(19) Europäisches Patentamt
European Patent Office
Office européen des brevets

(11) EP 1 202 744 B1

(12) # EUROPEAN PATENT SPECIFICATION

(45) Date of publication and mention of the grant of the patent:
**04.01.2006 Bulletin 2006/01**

(21) Application number: **00951266.6**

(22) Date of filing: **27.07.2000**

(51) Int Cl.:
*C12N 9/88* (2006.01) *C12N 5/10* (2006.01)

(86) International application number:
**PCT/DK2000/000425**

(87) International publication number:
**WO 2001/007065 (01.02.2001 Gazette 2001/05)**

(54) **PRODUCTION OF rhPBGD**
HERSTELLUNG VON RHPBGD
PRODUCTION DE RHPBGD

(84) Designated Contracting States:
**AT BE CH CY DE DK ES FI FR GB GR IE IT LI LU MC NL PT SE**
Designated Extension States:
**LT LV SI**

(30) Priority: **27.07.1999 WOPCT/DK99/01071**
**19.04.2000 WOPCT/DK00/00667**

(43) Date of publication of application:
**08.05.2002 Bulletin 2002/19**

(73) Proprietor: **Zymenex A/S**
**3400 Hilleroed (DK)**

(72) Inventors:
- **GELLERFORS, Pär**
  **S-181 63 Lidingö (SE)**
- **FOGH, Jens**
  **DK-3540 Lynge (DK)**

(74) Representative: **Plougmann & Vingtoft A/S**
**Sundkrogsgade 9,**
**P.O. Box 831**
**2100 Copenhagen O (DK)**

(56) References cited:
**WO-A-99/37325**

- **GROSS U ET AL: "Haem precursors and porphobilinogen deaminase in erythrocytes and lymphocytes of patients with acute intermittent porphyria" CELLULAR AND MOLECULAR BIOLOGY,US,TARRYTOWN, NY, vol. 43, no. 1, 1 February 1997 (1997-02-01), pages 29-35, XP002082339 ISSN: 0145-5680**
- **SASSA S: "Diagnosis and therapy of acute intermittent porphyria" BLOOD REVIEWS,GB,EDINBURGH, vol. 10, no. 1, 1 March 1996 (1996-03-01), pages 53-58, XP002082340**
- **GRANDCHAMP B: "Acute intermittent porphyria" SEMINARS IN LIVER DISEASE,DE,STUTTGART, vol. 18, no. 1, 1 January 1998 (1998-01-01), pages 17-24, XP002082341**
- **Molecular Cell Biology (Third edition - 1995) 299-300**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**SEQUENCE LISTING**

<110> HEMEBIOTECH A/S

<120> PRODUCTION OF rhPBGD AND NEW THERAPEUTIC METHODS FOR TREATING PATIENTS WITH ACUTE INTERMIT-TENT PORPHYRIA (AIP) AND OTHER PORPHYRIC DISEASES

<130> 23725PC1

<160> 22

<170> FastSEQ for Windows Version 3.0

<210> 1
<211> 5446
<212> DNA
<213> Homo sapiens

<400> 1

gaattctaac ataagttaag gaggaaaaaa aaatgagagt tattcgtgtc ggtacccgca 60
agagccagct tgctcgcata cagacggaca gtgtggtggc aacattgaaa gcctcgtacc 120
ctggcctgca gtttgaaatc attgctatgt ccaccacagg ggacaagatt cttgatactg 180
cactctctaa gattggagag aaaagcctgt ttaccaagga gcttgaacat gccctggaga 240
agaatgaagt ggacctggtt gttcactcct tgaaggacct gcccactgtg cttcctcctg 300
gcttcaccat cggagccatc tgcaagcggg aaaaccctca tgatgctgtt gtctttcacc 360
caaaatttgt tgggaagacc ctagaaaccc tgccagagaa gagtgtggtg ggaaccagct 420
ccctgcgaag agcagcccag ctgcagagaa agttcccgca tctggagttc aggagtattc 480
ggggaaacct caacacccgg cttcggaagc tggacgagca gcaggagttc agtgccatca 540
tcctggcaac agctggcctg cagcgcatgg gctggcacaa ccgggttggg cagatcctgc 600
accctgagga atgcatgtat gctgtgggcc agggggcctt gggcgtggaa gtgcgagcca 660
aggaccagga catcttggat ctggtgggtg tgctgcacga tcccgagact ctgcttcgct 720
gcatcgctga aagggccttc ctgaggcacc tggaaggagg ctgcagtgtg ccagtagccg 780
tgcatacagc tatgaaggat gggcaactgt acctgactgg aggagtctgg agtctagacg 840
gctcagatag catacaagag accatgcagg ctaccatcca tgtccctgcc cagcatgaag 900
atggccctga ggatgaccca cagttggtag gcatcactgc tcgtaacatt ccacgagggc 960
cccagttggc tgcccagaac ttgggcatca gcctggccaa cttgttgctg agcaaaggag 1020
ccaaaaacat cctggatgtt gcacggcaat tgaacgatgc ccattaataa gcttggctgt 1080
tttggcggat gagagaagat tttcagcctg atacagatta aatcagaacg cagaagcggt 1140
ctgataaaac agaatttgcc tggcggcagt agcgcggtgg tcccacctga ccccatgccg 1200
aactcagaag tgaaacgccg tagcgccgat ggtagtgtgg ggtctcccca tgcgagagta 1260
gggaactgcc aggcatcaaa taaaacgaaa ggctcagtcg aaagactggg cctttcgttt 1320
tatctgttgt ttgtcggtga acgctctcct gagtaggaca aatccgccgg gagcggattt 1380
gaacgttgcg aagcaacggc ccggagggtg gcgggcagga cgcccgccat aaactgccag 1440
gcatcaaatt aagcagaagg ccatcctgac ggatggcctt tttgcgtttc tacaaactct 1500
tttgtttatt tttctaaata cattcaaata tgtatccgct catgagacaa taaccctgat 1560
aaatgcttca ataatattga aaaaggaaga gtatgagtat tcaacatttc cgtgtcgccc 1620
ttattccctt ttttgcggca ttttgccttc ctgtttttgc tcacccagaa acgctggtga 1680
aagtaaaaga tgctgaagat cagttgggtg cacgagtggg ttacatcgaa ctggatctca 1740
acagcggtaa gatccttgag agttttcgcc ccgaagaacg ttttccaatg atgagcactt 1800
ttaaagttct gctatgtggc gcggtattat cccgtgttga cgccgggcaa gagcaactcg 1860
gtcgccgcat acactattct cagaatgact tggttgagta ctcaccagtc acagaaaagc 1920
atcttacgga tggcatgaca gtaagagaat tatgcagtgc tgccataacc atgagtgata 1980
acactgcggc caacttactt ctgacaacga tcggaggacc gaaggagcta accgcttttt 2040
tgcacaacat gggggatcat gtaactcgcc ttgatcgttg ggaaccggag ctgaatgaag 2100
ccataccaaa cgacgagcgt gacaccacga tgcctgtagc aatggcaaca acgttgcgca 2160
aactattaac tggcgaacta cttactctag cttcccggca acaattaata gactggatgg 2220

```
aggcggataa agttgcagga ccacttctgc gctcggccct tccggctggc tggtttattg 2280
ctgataaatc tggagccggt gagcgtgggt ctcgcggtat cattgcagca ctggggccag 2340
atggtaagcc ctcccgtatc gtagttatct acacgacggg gagtcaggca actatggatg 2400
aacgaaatag acagatcgct gagataggtg cctcactgat taagcattgg taactgtcag 2460
accaagttta ctcatatata ctttagattg atttaaaact tcatttttaa tttaaaagga 2520
tctaggtgaa gatccttttt gataatctca tgaccaaaat cccttaacgt gagttttcgt 2580
tccactgagc gtcagacccc gtagaaaaga tcaaaggatc ttcttgagat cctttttttc 2640
tgcgcgtaat ctgctgcttg caaacaaaaa aaccaccgct accagcggtg gtttgtttgc 2700
cggatcaaga gctaccaact ctttttccga aggtaactgg cttcagcaga gcgcagatac 2760
caaatactgt ccttctagtg tagccgtagt taggccacca cttcaagaac tctgtagcac 2820
cgcctacata cctcgctctg ctaatcctgt taccagtggc tgctgccagt ggcgataagt 2880
cgtgtcttac cgggttggac tcaagacgat agttaccgga taaggcgcag cggtcgggct 2940
gaacgggggg ttcgtgcaca cagcccagct tggagcgaac gacctacacc gaactgagat 3000
acctacagcg tgagctatga gaaagcgcca cgcttcccga agggagaaag gcggacaggt 3060
atccggtaag cggcagggtc ggaacaggag agcgcacgag ggagcttcca gggggaaacg 3120
cctggtatct ttatagtcct gtcgggtttc gccacctctg acttgagcgt cgatttttgt 3180
gatgctcgtc aggggggcgg agcctatgga aaaacgccag caacgcggcc tttttacggt 3240
tcctggcctt ttgctggcct tttgctcaca tgttctttcc tgcgttatcc cctgattctg 3300
tggataaccg tattaccgcc tttgagtgag ctgataccgc tcgccgcagc cgaacgaccg 3360
agcgcagcga gtcagtgagc gaggaagcgg aagagcgcct gatgcggtat tttctcctta 3420
cgcatctgtg cggtatttca caccgcatat ggtgcactct cagtacaatc tgctctgatg 3480
ccgcatagtt aagccagtat acactccgct atcgctacag atccggaaca taatggtgca 3540
gggcgctgac ttccgcgttt ccagacttta cgaaacacgg aaaccgaaga ccattcatgt 3600
tgttgctcag gtcgcagacg ttttgcagca gcagtcgctt cacgttcgct cgcgtatcgg 3660
tgattcattc tgctaaccag taaggcaacc ccgccagcct agccgggtcc tcaacgacag 3720
gagcacgatc atgcgcaccc gtggccagga cccaacgctg cccgagatgc gccgcgtgcg 3780
gctgctggag atggcggacg cgatggatat gttctgccaa gggttggttt gcgcattcac 3840
agttctccgc aagaattgat tggctccaat tcttggagtg gtgaatccgt tagcgaggtg 3900
ccgccggctt ccattcaggt cgaggtggcc cggctccatg caccgcgacg caacgcgggg 3960
aggcagacaa ggtatagggc ggcgcctaca atccatgcca acccgttcca tgtgctcgcc 4020
gaggcggcat aaatcgccgt gacgatcagc ggtccagtga tcgaagttag gctggtaaga 4080
gccgcgagcg atccttgaag ctgtccctga tggtcgtcat ctacctgcct ggacagcatg 4140
gcctgcaacg cgggcatccc gatgccgccg gaagcgagaa gaatcataat ggggaaggcc 4200
atccagcctc gcgtcgcgaa cgccagcaag acgtagccca gcgcgtcggc cgccatgccg 4260
gcgataatgg cctgcttctc gccgaaacgt ttggtggcgg gaccagtgac gaaggcttga 4320
gcgagggcgt gcaagattcc gaataccgca agcgacaggc cgatcatcgt cgcgctccag 4380
cgaaagcggt cctcgccgaa aatgacccag agcgctgccg gcacctgtcc tacgagttgc 4440
atgataaaga agacagtcat aagtgcggcg acgatagtca tgccccgcgc ccaccggaag 4500
gagctgactg ggttgaaggc tctcaagggc atcggtcgac gctctccctt atgcgactcc 4560
tgcattagga agcagcccag tagtaggttg aggccgttga gcaccgccgc cgcaaggaat 4620
ggtgcatgca aggagatggc gcccaacagt cccccggcca cggggcctgc caccataccc 4680
acgccgaaac aagcgctcat gagcccgaag tggcgagccc gatcttcccc atcggtgatg 4740
tcggcgatat aggcgccagc aaccgcacct gtggcgccgg tgatgccggc cacgatgcgt 4800
ccggcgtaga ggatccacag gacgggtgtg gtcgccatga tcgcgtagtc gatagtggct 4860
ccaagtagcg aagcgagcag gactgggcgg cggccaaagc ggtcggacag tgctccgaga 4920
acgggtgcgc atagaaattg catcaacgca tatagcgcta gcagcacgcc atagtgactg 4980
gcgatgctgt cggaatggac gatatcccgc aagaggcccg gcagtaccgg cataaccaag 5040
cctatgccta cagcatccag ggtgacggtg ccgaggatga cgatgagcgc attgttagat 5100
ttcatacacg gtgcctgact gcgttagcaa tttaactgtg ataaactacc gcattaaagc 5160
taatcgatga taagctgtca aacatgagtg atccgggctt atcgactgca cggtgcacca 5220
atgcttctgg cgtcaggcag ccatcggaag ctgtggtatg gctgtgcagg tcgtaaatca 5280
ctgcataatt cgtgtcgctc aaggcgcact cccgttctgg ataatgtttt ttgcgccgac 5340
atcataacgg ttctggcaaa tattctgaaa tgagctgttg acaattaatc atcggctcgt 5400
ataatgtgtg gaattgtgag cggataacaa tttcacacag gaaaca 5446
```

<210> 2
<211> 3225

<212> DNA
<213> Homo sapiens

<400> 2

```
aattcgtcaa gcagcagtat atgctgggtg gagccacaat cttcgccccc caggctgccg   60
ctttcattat gacggaagcg gttttcatca atcaggaaga agctgacttc cacacccagc  120
gaggcggccc agttttccag caggctacat ttacgttgta gcaattggcg ctcttcgcta  180
tcgagccagg attgatgaca gacccagata tccaggtcag aggaacaact ttgccctacg  240
gacgaggtgc tgcccatggt gtatacacca gtaattggaa gctcaccttt cggcggatcc  300
tgtactgaca ttccacgata cagttcaagc tcgttcaggt agtggcgttg agtttcatca  360
ggcgtgtaaa ggcaaatgcc tttgggaacg ttaccatcaa ggtagcccgg cattagcgga  420
tggtgatagt gcaacaatgt cggcagtaga ctgtagacct gttggaatgc aggccccata  480
gcagcaagcg cgcgatccac acgcaattga tttatggcat ccagtctctg tttcagagtc  540
tcaatataga ggtacaagac gtatcgcctg atttgctacc cgtcatgact gtgattccgc  600
caacatcaac ggtaacacgc ggcattcggg atatttcgta tgtcaaaggt aaccgttacc  660
acttttcgcg cctggttttt ttagtttcac gacgaaaaaa tggtctaaaa cgtgatcaat  720
ttaacacctt gctgattgac cgtaaagaaa gatgcgctac atacaagtgt agcaccgttt  780
attctctgta aattccttat tacaacggcg tgaaacgcct gtcaggatcc actgccagac  840
ctcattttac ggtttgcgca ggcgtctacg tttcaccaca acactgacat cactctggca  900
aggatgttag gatggaccac ggatgataat gacggtaaca agcatgttag acaatgtttt  960
aagaattgcc acacgccaaa gcccacttgc actctggcag gcacactatg tcaaagacaa 1020
gttgatggcg agccatccgg gcctggtcgt tgaactggta ccgatggtga cctcgagcgg 1080
cacgtaagag gttccaactt tcaccataat gaaataagat cactaccggg cgtatttttt 1140
gagttgtcga gattttcagg agctaaggaa gctaaaatgg agaaaaaaat cactggatat 1200
accaccgttg atatatccca atggcatcgt aaagaacatt ttgaggcatt tcagtcagtt 1260
gctcaatgta cctataacca gaccgttcag ctggatatta cggccttttt aaagaccgta 1320
aagaaaaata agcacaagtt ttatccggcc tttattcaca ttcttgcccg cctgatgaat 1380
gctcatccgg aattacgtat ggcaatgaaa gacggtgagc tggtgatatg ggatagtgtt 1440
cacccttgtt acaccgtttt ccatgagcaa actgaaacgt tttcatcgct ctggagtgaa 1500
taccacgacg atttccggca gtttctacac atatattcgc aagatgtggc gtgttacggt 1560
gaaaacctgg cctatttccc taaagggttt attgagaata tgtttttcgt ctcagccaat 1620
ccctgggtga gtttcaccag ttttgattta aacgtggcca atatggacaa cttcttcgcc 1680
cccgttttca ccatgggcaa atattatacg caaggcgaca aggtgctgat gccgctggcg 1740
attcaggttc atcatgccgt ttgtgatggc ttccatgtcg gcagaatgct taatgaatta 1800
caacagtact gcgatgagtg gcagggcggg gcgtaatttt cgagaccggc atgagtatcc 1860
ttgtcacccg cccgtctccc gctggagaag agttagtgag ccgtctgcgc acactggggc 1920
aggtggcctg gcattttccg ctgattgagt ttctccgggg tcaacaatta ccgcaacttg 1980
ctgatcaact ggcagcgctg gggagagcg atctgttgtt tgccctctcg caacacgcgg 2040
ttgcttttgc ccaatcacag ctgcatcagc aagatcgtaa atggccccga ctacctgatt 2100
atttcgccat tggacgcacc accgcactgg cactacatac cgtaagtgga cagaagattc 2160
tctacccgca ggatcgggaa atcagcgaag tcttgctaca attacctgaa ttacaaaata 2220
ttgcgggcaa acgtgcgctg atattacgtg gcaatggtgg tcgtgagcta attggggata 2280
ccctgacggc gcgcggtgct gaggtcactt tttgtgaatg ttatcaacga tgcgcaatcc 2340
attacgatgg tgcagaagaa gcgatgcgct ggcaagcccg cgaggtgacg atggtcgttg 2400
ttaccagcgg tgaaatgttg cagcaactct ggtcgctgat cccacaatgg tatcgtgagc 2460
actggttact acactgtcga ctattggtcg tcagtgagcg tttggcgaaa ctcgcccggg 2520
aactgggctg gcaagacatt aaggtcgccg ataacgctga caacgatgcg cttttacggg 2580
cattacaata actctcataa caggaagcca taatgacgga acaagaaaaa acctccgccg 2640
tggttgaaga gaccagggag gccgtggaca ccacgtcaca acctgtcgca acagaaaaaa 2700
agagtaagaa caataccgca ttgattctca gcgcggtggc tatcgctatt gctctggcgg 2760
cgggcatcgg tttgtatggc tggggtaaac aacaggccgt caatcagacc gccaccagcg 2820
atgccctggc taaccaactg acggcattgc aaaaagccca ggagagccaa aaagccgagc 2880
tggaaggcat tattaagcaa caagctgcac aacttaagca ggcgaatcgt cagcaagaaa 2940
cgctggcaaa acagttggat gaagtccaac aaaaggtcgc caccatttcc ggcagcgatg 3000
ctaaaacctg gctgctggct caggccgatt ttctggtgaa actcgccgga cggaagctgt 3060
ggagcgatca ggacgtcacg accgctgcag cgttgctgaa aagtgcagac gccagcctgg 3120
cggatatgaa tgacccgagt ctgattaccg ttcgtcgggc aattaccgat gatatcgcca 3180
gcctttctgc agtatcgcag gtggattatg acggcatcat cctta                 3225
```

<210> 3
<211> 1035
<212> DNA

<213> Homo sapiens

<400> 3

```
atgagagtga ttcgcgtggg tacccgcaag agccagcttg ctcgcataca gacggacagt      60
gtggtggcaa cattgaaagc ctcgtaccct ggcctgcagt ttgaaatcat tgctatgtcc     120
accacagggg acaagattct tgatactgca ctctctaaga ttggagagaa aagcctgttt     180
accaaggagc ttgaacatgc cctggagaag aatgaagtgg acctggttgt tcactccttg     240
aaggacctgc ccactgtgct tcctcctggc ttcaccatcg gagccatctg caagcgggaa     300
aaccctcatg atgctgttgt ctttcaccca aaatttgttg ggaagaccct agaaaccctg     360
ccagagaaga gtgtggtggg aaccagctcc ctgcgaagag cagcccagct gcagagaaag     420
ttcccgcatc tggagttcag gagtattcgg ggaaacctca acacccggct tcggaagctg     480
gacgagcagc aggagttcag tgccatcatc ctggcaacag ctggcctgca gcgcatgggc     540
tggcacaacc gggttgggca gatcctgcac cctgaggaat gcatgtatgc tgtgggccag     600
ggggccttgg gcgtggaagt gcgagccaag gaccaggaca tcttggatct ggtgggtgtg     660
ctgcacgatc ccgagactct gcttcgctgc atcgctgaaa gggccttcct gaggcacctg     720
gaaggaggct gcagtgtgcc agtagccgtg catacagcta tgaaggatgg gcaactgtac     780
ctgactggag gagtctggag tctagacggc tcagatagca tacaagagac catgcaggct     840
accatccatg tccctgccca gcatgaagat ggccctgagg atgacccaca gttggtaggc     900
atcactgctc gtaacattcc acgagggccc cagttggctg cccagaactt gggcatcagc     960
ctggccaact tgttgctgag caaaggagcc aaaaacatcc tggatgttgc acggcaattg    1020
aacgatgccc attaa                                                     1035
```

<210> 4
<211> 1113
<212> DNA
<213> Homo sapiens

<400> 4

```
cacacagcct actttccaag cggagccatg tctggtaacg gcaatgcggc tgcaacggcg      60
gaagaaaaca gcccaaagat gagagtgatt cgcgtgggta cccgcaagag ccagcttgct     120
cgcatacaga cggacagtgt ggtggcaaca ttgaaagcct cgtaccctgg cctgcagttt     180
gaaatcattg ctatgtccac cacaggggac aagattcttg atactgcact ctctaagatt     240
ggagagaaaa gcctgtttac caaggagctt gaacatgccc tggagaagaa tgaagtggac     300
ctggttgttc actccttgaa ggacctgccc actgtgcttc ctcctggctt caccatcgga     360
gccatctgca agcgggaaaa ccctcatgat gctgttgtct ttcacccaaa atttgttggg     420
aagaccctag aaaccctgcc agagaagagt gtggtgggaa ccagctccct gcgaagagca     480
gcccagctgc agagaaagtt cccgcatctg gagttcagga gtattcgggg aaacctcaac     540
acccggcttc ggaagctgga cgagcagcag gagttcagtg ccatcatcct ggcaacagct     600
ggcctgcagc gcatgggctg gcacaaccgg gttgggcaga tcctgcaccc tgaggaatgc     660
atgtatgctg tgggccaggg ggccttgggc gtggaagtgc gagccaagga ccaggacatc     720
ttggatctgg tgggtgtgct gcacgatccc gagactctgc ttcgctgcat cgctgaaagg     780
gccttcctga ggcacctgga aggaggctgc agtgtgccag tagccgtgca tacagctatg     840
aaggatgggc aactgtacct gactggagga gtctggagtc tagacggctc agatagcata     900
caagagacca tgcaggctac catccatgtc cctgcccagc atgaagatgg ccctgaggat     960
gacccacagt tggtaggcat cactgctcgt aacattccac gagggcccca gttggctgcc    1020
cagaacttgg gcatcagcct ggccaacttg ttgctgagca aaggagccaa aaacatcctg    1080
gatgttgcac ggcaattgaa cgatgcccat taa                                 1113
```

<210> 5
<211> 1035
<212> DNA
<213> Homo sapiens

<400> 5

```
atgagagtga ttcgcgtggg tacccgcaag agccagcttg ctcgcataca gacggacagt      60
gtggtggcaa cattgaaagc ctcgtaccct ggcctgcagt ttgaaatcat tgctatgtcc     120
accacagggg acaagattct tgatactgca ctctctaaga ttggagagaa aagcctgttt     180
accaaggagc ttgaacatgc cctggagaag aatgaagtgg acctggttgt tcactccttg     240
aaggacctgc ccactgtgct tcctcctggc ttcaccatcg gagccatctg caagcgggaa     300
aaccctcatg atgctgttgt ctttcaccca aaatttgttg ggaagaccct agaaaccctg     360
ccagagaaga gtgtggtggg aaccagctcc ctgcgaagag cagcccagct gcagagaaag     420
ttcccgcatc tggagttcag gagtattcgg ggaaacctca acacccggct tcggaagctg     480
gacgagcagc aggagttcag tgccatcatc ctggcaacag ctggcctgca gcgcatgggc     540
tggcacaacc gggtggggca gatcctgcac cctgaggaat gcatgtatgc tgtgggccag     600
ggggccttgg gcgtggaagt gcgagccaag gaccaggaca tcttggatct ggtgggtgtg     660
ctgcacgatc ccgagactct gcttcgctgc atcgctgaaa gggccttcct gaggcacctg     720
gaaggaggct gcagtgtgcc agtagccgtg catacagcta tgaaggatgg gcaactgtac     780
ctgactggag gagtctggag tctagacggc tcagatagca tacaagagac catgcaggct     840
accatccatg tccctgccca gcatgaagat ggccctgagg atgacccaca gttggtaggc     900
atcactgctc gtaacattcc acgagggccc cagttggctg cccagaactt gggcatcagc     960
ctggccaact tgttgctgag caaaggagcc aaaaacatcc tggatgttgc acggcaattg    1020
aacgatgccc attaa                                                     1035
```

<210> 6
<211> 1035
<212> DNA
<213> Homo sapiens

<400> 6

```
atgagagtga ttcgcgtggg tacccgcaag agccagcttg ctcgcataca gacggacagt      60
gtggtggcaa cattgaaagc ctcgtaccct ggcctgcagt ttgaaatcat tgctatgtcc     120
accacagggg acaagattct tgatactgca ctctctaaga ttggagagaa aagcctgttt     180
accaaggagc ttgaacatgc cctggagaag aatgaagtgg acctggttgt tcactccttg     240
aaggacctgc ccactgtgct tcctcctggc ttcaccatcg gagccatctg caagcgggaa     300
aaccctcatg atgctgttgt ctttcaccca aaatttgttg ggaagaccct agaaaccctg     360
ccagagaaga gtgtggtggg aaccagctcc ctgcgaagag cagcccagct gcagagaaag     420
ttcccgcatc tggagttcag gagtattcgg ggaaacctca acacccggct tcggaagctg     480
gacgagcagc aggagttcag tgccatcatc ctggcaacag ctggcctgca gcgcatgggc     540
tggcacaacc gggtggggca gatcctgcac cctgaggaat gcatgtatgc tgtgggccag     600
ggggccttgg gcgtggaagt gcgagccaag gaccaggaca tcttggatct ggtgggtgtg     660
ctgcacgatc ccgagactct gcttcgctgc atcgctgaaa gggccttcct gaggcacctg     720
gaaggaggct gcagtgtgcc agtagccgtg catacagcta tgaaggatgg gcaactgtac     780
ctgactggag gagtctggag tctagacggc tcagatagca tacaagagac catgcaggct     840
accatccatg tccctgccca gcatgaagat ggccctgagg atgacccaca gttggtaggc     900
atcactgctc gtaacattcc acgagggccc cagttggctg cccagaactt gggcatcagc     960
ctggccaact tgttgctgag caaaggagcc aaaaacatcc tggatgttgc acggcaattg    1020
aacgatgccc attaa                                                     1035
```

<210> 7
<211> 1034
<212> DNA
<213> Homo sapiens

<400> 7

```
atgagagtga ttcgcgtggg tacccgcaag agccagcttg ctcgcataca gacggacagt   60
gtggtggcaa cattgaaagc ctcgtaccct ggcctgcagt ttgaaatcat tgctatgtcc  120
accacagggg acaagattct tgatactgca ctctctaaga ttggagagaa aagcctgttt  180
accaaggagc ttgaacatgc cctggagaag aatgaagtgg acctggttgt tcactccttg  240
aaggacctgc ccactgtgct tcctcctggc ttcaccatcg gagccatctg caagcgggaa  300
aaccctcatg atgctgttgt cttcacccaa aatttgttgg gaagacccta gaaaccctgc  360
cagagaagag tgtggtggga accagctccc tgcgaagagc agcccagctg cagagaaagt  420
tcccgcatct ggagttcagg agtattcggg gaaacctcaa cacccggctt cggaagctgg  480
acgagcagca ggagttcagt gccatcatcc tggcaacagc tggcctgcag cgcatgggct  540
ggcacaaccg ggtggggcag atcctgcacc ctgaggaatg catgtatgct gtgggccagg  600
gggccttggg cgtggaagtg cgagccaagg accaggacat cttggatctg gtgggtgtgc  660
tgcacgatcc cgagactctg cttcgctgca tcgctgaaag ggccttcctg aggcacctgg  720
aaggaggctg cagtgtgcca gtagccgtgc atacagctat gaaggatggg caactgtacc  780
tgactggagg agtctggagt ctagacggct cagatagcat acaagagacc atgcaggcta  840
ccatccatgt ccctgcccag catgaagatg gccctgagga tgacccacag ttggtaggca  900
tcactgctcg taacattcca cgagggcccc agttggctgc ccagaacttg ggcatcagcc  960
tggccaactt gttgctgagc aaaggagcca aaaacatcct ggatgttgca cggcaattga 1020
acgatgccca ttaa                                                   1034
```

<210> 8
<211> 1035
<212> DNA
<213> Homo sapiens

<400> 8

```
atgagagtga ttcgcgtggg tacccgcaag agccagcttg ctcgcataca gacgggcagt   60
gtggtggcaa cattgaaagc ctcgtaccct ggcctgcagt ttgaaatcat tgctatgtcc  120
accacagggg acaagattct tgatactgca ctctctaaga ttggagagaa aagcctgttt  180
accaaggagc ttgaacatgc cctggagaag aatgaagtgg acctggttgt tcactccttg  240
aaggacctgc ccactgtgct tcctcctggc ttcaccatcg gagccatctg caagcgggaa  300
aaccctcatg atgctgttgt ctttcaccca aaatttgttg ggaagaccct agaaaccctg  360
ccagagaaga gtgtggtggg aaccagctcc ctgcgaagag cagcccagct gcagagaagg  420
ttcccgcatc tggagttcag gagtattcgg ggaaacctca acacccggct tcggaagctg  480
gacgagcagc aggagttcag tgtcatcatc ctggcaacag ctggcctgca gcgcatgggc  540
tggcacaacc gggttgggca gatcctgcac cctgaggaat gcatgtatgc tgtgggccag  600
ggggccttgg gcgtggaagt gcgagccaag gaccaggaca tcttggatct ggtgggtgtg  660
ctgcacgatc ccgagactct gcttcgctgc atcgctgaaa gggccttcct gaggcacctg  720
gaaggaggct gcagtgtgcc agtagccgtg catacagcta tgaaggatgg gcaactgtac  780
ctgactggag gagtctggag tctagacggc tcagatagca tacaagagac catgcaggct  840
accatccatg tccctgccca gcatgaagat ggccctgagg atgacccaca gttggtaggc  900
atcactgctc gtaacattcc acgagggccc cagttggctg cccagaactt gggcatcagc  960
ctggccaact tgttgctgag caagggagcc aaaaacatcc tggatgttgc acggcaattg 1020
aacgatgccc attaa                                                  1035
```

<210> 9
<211> 1035
<212> DNA
<213> Homo sapiens

<400> 9

```
atgagagtga ttcgcgtggg tacccgcaag agccagcttg ctcgcataca gacggacagt     60
gtggtggcaa cattgaaagc ctcgtaccct ggcctgcagt ttgaaatcat tgctatgtcc    120
accacagggg acaagattct tgatactgca ctctctaaga ttggagagaa aagcctgttt    180
accaaggagc ttgaacatgc cctggagaag aatgaagtgg acctggttgt tcactccttg    240
aaggacctgc ccactgtgct tcctcctggc ttcaccatcg gagccatctg caagcgggaa    300
aaccctcatg atgctgttgt ctttcaccca aaatttgttg ggaagaccct agaaaccctg    360
ccagagaaga gtgtggtggg aaccagctcc ctgcgaagag cagcccagct gcagagaaag    420
ttcccgcatc tggagttcag gagtattcgg ggaaacctca acacccggct tcggaagctg    480
gacgagcagc aggagttcag tgccatcatc ctggcaacag ctggcctgca gcgcatgggc    540
tggcacaacc gggtggggca gatcctgcac cctgaggaat gcatgtatgc tgtgggccag    600
ggggccttgg gcgtggaagt gcgagccaag gaccaggaca tcttggatct ggtgggtgtg    660
ctgcacgatc ccgagactct gcttcgctgc atcgctgaaa gggccttcct gaggcacctg    720
gaaggaggtt gcagtgtgcc agtagccgtg catacagcta tgaaggatgg gcaactgtac    780
ctgactggag gagtctggag tctagacggc tcagatagca tacaagagac catgcaggct    840
accatccatg tccctgccca gcatgaagat ggccctgagg atgacccaca gttggtaggc    900
atcactgctc gtaacattcc acgagggccc cagttggctg cccagaactt gggcatcagc    960
ctggccaact tgttgctgag caaaggagcc aaaaacatcc tggatgttgc acggcaattg   1020
aacgatgccc attaa                                                    1035
```

<210> 10

<211> 1034
<212> DNA
<213> Homo sapiens

<400> 10

```
atgagagtga ttcgcgtggg tacccgcaag agccagcttg ctcgcataca gacggacagt     60
gtggtggcaa cattgaaagc ctcgtaccct ggcctgcagt ttgaaatcat tgctatgtcc    120
accacagggg acaagattct tgatactgca ctctctaaga ttggagagaa aagcctgttt    180
accaaggagc ttgaacatgc cctggagaag aatgaagtgg acctggttgt tcactccttg    240
aaggacctgc ccactgtgct tcctcctggc ttcaccatcg gagccatctg caagcgggaa    300
aaccctcatg atgctgttgt ctttcaccca aaatttgttg ggaagaccct agaaaccctg    360
ccagagaaga gtgtggtggg aaccagctcc ctgcgaagag cagcccagct gcagagaaag    420
ttcccgcatc tggagttcag gagtattcgg ggaaacctca acacccggct tcggaagctg    480
gacgagcagc aggagttcag tgccatcatc ctggcaacag ctggcctgca gcgcatgggc    540
tggcacaacc gggtggggca gatcctgcac cctgaggaat gcatgtatgc tgtgggccag    600
ggggccttgg gcgtggaagt gcgagccaag gaccaggaca tcttggatct ggtgggtgtg    660
ctgcacgatc ccgagactct gcttcgctgc atcgctgaaa gggccttcct gaggcacctg    720
gaaggaggct gcagtgtgcc agtagccgtg catacagcta tgaaggatgg gcaactgtac    780
ctgactggag gagtctggag tctagacggc tcagatagca tacaagagac catgcaggct    840
accatccatg tccctgccca gcatgaagat ggccctgagg atgacccaca gttggtaggc    900
atcactgctc gtaacattcc acgagggccc cagttggctg cccagaactt gggcatcagc    960
ctggccaact tgttgctgag caaaggagcc aaaaacatcc tggatgttgc acggcaatta   1020
acgatgccca ttaa                                                     1034
```

<210> 11
<211> 1035
<212> DNA
<213> Homo sapiens

<400> 11

```
atgagagtga ttcgcgtggg tacccgcaag agccagcttg ctcgcataca gacggacagt       60
gtggtggcaa cattgaaagc ctcgtaccct ggcctgcagt ttgaaatcat tgctatgtcc      120
accacagggg acaagattct tgatactgca ctctctaaga ttggagagaa aagcctgttt      180
accaaggagc ttgaacatgc cctggagaag aatgaagtgg acctggttgt tcactccttg      240
aaggacctgc ccactgtgct tcctcctggc ttcaccatcg gagccatctg caagcgggaa      300
aaccctcatg atgctgttgt ctttcaccca aaatttgttg ggaagaccct agaaaccctg      360
ccagagaaga gtgtggtggg aaccagctcc ctgcgaagag cagcccagct gcagagaaag      420
ttcccgcatc tggagttcag gagtattcgg ggaaacctca acacccggct tcggaagctg      480
gacgagcagc aggagttcag tgccatcatc ctggcaacag ctggcctgca gcgcatgggc      540
tggcacaacc gggtggggca gatcctgcac cctgaggaat gcatgtatgc tgtgggccag      600
ggggccttgg gcgtggaagt gcgagccaag gaccaggaca tcttggatct ggtgggtgtg      660
ctgcacgatc ccgagactct gcttcgctgc atcgctgaaa gggccttcct gaggcacctg      720
gaaggaggct gcagtgtgcc agtagccgtg catacagcta tgaaggatgg gcaactgtac      780
ctgactggag gagtctggag tctagacggc tcagatagca tacaagagac catgcaggcc      840
accatccatg tccctaccca gcatgaagat ggccctgagg atgacccaca gttggtaggc      900
atcactgctc gtaacattcc acgagggccc cagttggctg cccagaactt gggcatcagc      960
ctggccaact tgttgctgag caaaggagcc aaaaacatcc tggatgttgc acggcaattg     1020
aacgatgccc attaa                                                      1035
```

<210> 12
<211> 3988
<212> DNA
<213> Homo sapiens

<400> 12

```
cacctgacgc gccctgtagc ggcgcattaa gcgcggcggg tgtggtggtt acgcgcagcg       60
tgaccgctac acttgccagc gccctagcgc ccgctccttt cgctttcttc ccttcctttc      120
tcgccacgtt cgccggcttt ccccgtcaag ctctaaatcg ggggctccct ttagggttcc      180
gatttagtgc tttacggcac ctcgacccca aaaaacttga ttagggtgat ggttcacgta      240
```

```
gtgggccatc gccctgatag acggtttttc gccctttgac gttggagtcc acgttcttta    300
atagtggact cttgttccaa actggaacaa cactcaaccc tatctcggtc tattcttttg    360
atttataagg gattttgccg atttcggcct attggttaaa aaatgagctg atttaacaaa    420
aatttaacgc gaattttaac aaaatattaa cgcttacaat ttccattcgc cattcaggct    480
gcgcaactgt tgggaagggc gatcggtgcg ggcctcttcg ctattacgcc agctggcgaa    540
agggggatgt gctgcaaggc gattaagttg ggtaacgcca gggttttccc agtcacgacg    600
ttgtaaaacg acggccagtg aattgtaata cgactcacta tagggcgaat tgggtaccgg    660
gccccccctc gaggtcgacg gtatcgataa gcttattaat gggcatcgtt caattgccgt    720
gcaacatcca ggatgttttt ggctcctttg ctcagcaaca agttggccag gctgatgccc    780
aagttctggg cagccaactg gggccctcgt ggaatgttac gagcagtgat gcctaccaac    840
tgtgggtcat cctcagggcc atcttcatgc tgggcaggga catggatggt agcctgcatg    900
gtctcttgta tgctatctga gccgtctaga ctccagactc ctccagtcag gtacagttgc    960
ccatccttca tagctgtatg cacggctact ggcacactgc agcctccttc caggtgcctc   1020
aggaaggccc tttcagcgat gcagcgaagc agagtctcgg gatcgtgcag cacacccacc   1080
agatccaaga tgtcctggtc cttggctcgc acttccacgc ccaaggcccc ctggcccaca   1140
gcatacatgc attcctcagg gtgcaggatc tgcccaaccc ggttgtgcca gcccatgcgc   1200
tgcaggccag ctgttgccag gatgatggca ctgaactcct gctgctcgtc cagcttccga   1260
agccgggtgt tgaggtttcc ccgaatactc ctgaactcca gatgcgggaa ctttctctgc   1320
agctgggctg ctcttcgcag ggagctggtt cccaccacac tcttctctgg cagggtttct   1380
agggtcttcc caacaaattt tgggtgaaag acaacagcat catgagggtt ttcccgcttg   1440
cagatggctc cgatggtgaa gccaggagga agcacagtgg gcaggtcctt caaggagtga   1500
acaaccaggt ccacttcatt cttctccagg gcatgttcaa gctccttggt aaacaggctt   1560
ttctctccaa tcttagagag tgcagtatca agaatcttgt cccctgtggt ggacatagca   1620
atgatttcaa actgcaggcc agggtacgag gctttcaatg ttgccaccac actgtccgtc   1680
tgtatgcgag caagctggct cttgcgggta cccacgcgaa tcactctcat gaattcctgc   1740
agcccggggg atccactagt tctagagcgg ccgccaccgc ggtggagctc cagcttttgt   1800
tccctttagt gagggttaat ttcgagcttg gcgtaatcat ggtcatagct gtttcctgtg   1860
tgaaattgtt atccgctcac aattccacac aacatacgag ccggaagcat aaagtgtaaa   1920
gcctggggtg cctaatgagt gagctaactc acattaattg cgttgcgctc actgcccgct   1980
ttccagtcgg gaaacctgtc gtgccagctg cattaatgaa tcggccaacg cgcggggaga   2040
ggcggtttgc gtattgggcg ctcttccgct tcctcgctca ctgactcgct gcgctcggtc   2100
gttcggctgc ggcgagcggt atcagctcac tcaaaggcgg taatacggtt atccacagaa   2160
tcaggggata acgcaggaaa gaacatgtga gcaaaaggcc agcaaaaggc caggaaccgt   2220
aaaaaggccg cgttgctggc gtttttccat aggctccgcc cccctgacga gcatcacaaa   2280
aatcgacgct caagtcagag gtggcgaaac ccgacaggac tataaagata ccaggcgttt   2340
ccccctggaa gctccctcgt gcgctctcct gttccgaccc tgccgcttac cggatacctg   2400
tccgcctttc tcccttcggg aagcgtggcg ctttctcata gctcacgctg taggtatctc   2460
agttcggtgt aggtcgttcg ctccaagctg ggctgtgtgc acgaaccccc cgttcagccc   2520
gaccgctgcg ccttatccgg taactatcgt cttgagtcca acccggtaag acacgactta   2580
tcgccactgg cagcagccac tggtaacagg attagcagag cgaggtatgt aggcggtgct   2640
acagagttct tgaagtggtg gcctaactac ggctacacta gaaggacagt atttggtatc   2700
tgcgctctgc tgaagccagt taccttcgga aaaagagttg gtagctcttg atccggcaaa   2760
caaaccaccg ctggtagcgg tggttttttt gtttgcaagc agcagattac gcgcagaaaa   2820
aaaggatctc aagaagatcc tttgatcttt tctacggggt ctgacgctca gtggaacgaa   2880
aactcacgtt aagggatttt ggtcatgaga ttatcaaaaa ggatcttcac ctagatcctt   2940
ttaaattaaa aatgaagttt taaatcaatc taaagtatat atgagtaaac ttggtctgac   3000
agttaccaat gcttaatcag tgaggcacct atctcagcga tctgtctatt tcgttcatcc   3060
atagttgcct gactccccgt cgtgtagata actacgatac gggagggctt accatctggc   3120
cccagtgctg caatgatacc gcgagaccca cgctcaccgg ctccagattt atcagcaata   3180
aaccagccag ccggaagggc cgagcgcaga agtggtcctg caactttatc cgcctccatc   3240
cagtctatta attgttgccg ggaagctaga gtaagtagtt cgccagttaa tagtttgcgc   3300
aacgttgttg ccattgctac aggcatcgtg gtgtcacgct cgtcgtttgg tatggcttca   3360
ttcagctccg gttcccaacg atcaaggcga gttacatgat cccccatgtt gtgcaaaaaa   3420
gcggttagct ccttcggtcc tccgatcgtt gtcagaagta agttggccgc agtgttatca   3480
ctcatggtta tggcagcact gcataattct cttactgtca tgccatccgt aagatgcttt   3540
tctgtgactg gtgagtactc aaccaagtca ttctgagaat agtgtatgcg gcgaccgagt   3600
tgctcttgcc cggcgtcaat acgggataat accgcgccac atagcagaac tttaaaagtg   3660
ctcatcattg gaaaacgttc ttcggggcga aaactctcaa ggatcttacc gctgttgaga   3720
tccagttcga tgtaacccac tcgtgcaccc aactgatctt cagcatcttt tactttcacc   3780
```

agcgtttctg ggtgagcaaa aacaggaagg caaaatgccg caaaaaaggg aataagggcg 3840
acacggaaat gttgaatact catactcttc ctttttcaat attattgaag catttatcag 3900
ggttattgtc tcatgagcgg atacatattt gaatgtattt agaaaaataa acaaataggg 3960
gttccgcgca catttccccg aaaagtgc 3988

<210> 13
<211> 1260
<212> DNA
<213> Homo sapiens

<400> 13

cacaggaaac agctatgacc atgattacgc caagctcgaa attaaccctc actaaaggga 60
acaaaagctg gagctccacc gcggtggcgg ccgctctaga actagtggat cccccgggct 120
gcaggaattc atgagagtga ttcgcgtggg tacccgcaag agccagcttg ctcgcataca 180
gacggacagt gtggtggcaa cattgaaagc ctcgtaccct ggcctgcagt ttgaaatcat 240
tgctatgtcc accacagggg acaagattct tgatactgca ctctctaaga ttggagagaa 300
aagcctgttt accaaggagc ttgaacatgc cctggagaag aatgaagtgg acctggttgt 360
tcactccttg aaggacctgc ccactgtgct tcctcctggc ttcaccatcg gagccatctg 420
caagcgggaa aaccctcatg atgctgttgt ctttcaccca aaatttgttg ggaagaccct 480
agaaaccctg ccagagaaga gtgtggtggg aaccagctcc ctgcgaagag cagcccagct 540
gcagagaaag ttcccgcatc tggagttcag gagtattcgg ggaaacctca acacccggct 600
tcggaagctg gacgagcagc aggagttcag tgccatcatc ctggcaacag ctggcctgca 660
gcgcatgggc tggcacaacc gggttgggca gatcctgcac cctgaggaat gcatgtatgc 720
tgtgggccag ggggccttgg gcgtggaagt gcgagccaag gaccaggaca tcttggatct 780
ggtgggtgtg ctgcacgatc ccgagactct gcttcgctgc atcgctgaaa gggccttcct 840
gaggcacctg gaaggaggct gcagtgtgcc agtagccgtg catacagcta tgaaggatgg 900
gcaactgtac ctgactggag gagtctggag tctagacggc tcagatagca tacaagagac 960
catgcaggct accatccatg tccctgccca gcatgaagat ggccctgagg atgacccaca 1020
gttggtaggc atcactgctc gtaacattcc acgagggccc cagttggctg cccagaactt 1080
gggcatcagc ctggccaact tgttgctgag caaaggagcc aaaaacatcc tggatgttgc 1140
acggcaattg aacgatgccc attaataagc ttatcgatac cgtcgacctc gagggggggc 1200
ccggtaccca attcgcccta tagtgagtcg tattacaatt cactggccgt cgttttacaa 1260

<210> 14
<211> 32
<212> DNA
<213> Homo sapiens

<400> 14

atccatgaat tccacgcaat gcagccccag tc 32

<210> 15
<211> 32
<212> DNA
<213> Homo sapiens

<400> 15

agtcgtaagc ttgcctggca ctgtcctcca tc 32

<210> 16
<211> 22
<212> DNA
<213> Homo sapiens

<400> 16

gtaatacgac tcactatagg gc 22

<210> 17
<211> 22
<212> DNA
<213> Homo sapiens

<400> 17

ctaaagggaa caaaagctgg ag 22

<210> 18
<211> 20
<212> DNA
<213> Homo sapiens

<400> 18

gcgcgtaata cgactcacta 20

<210> 19
<211> 20
<212> DNA
<213> Homo sapiens

<400>19

cctacgctgt gtcttgatct 20

<210> 20
<211> 20
<212> DNA
<213> Homo sapiens

<400> 20

ggcttcacca tgagcatgtc 20

<210> 21
<211> 993
<212> DNA
<213> Homo sapiens

<400> 21

```
atgcagcccc agtccgttct gcacagcggc tacttccacc cactacttcg ggcctggcag      60
acagccacca ccaccctcaa tgcctccaac ctcatctacc ccatctttgt cacggatgtt     120
cctgatgaca tacagcctat caccagcctc ccaggagtgg ccaggtatgg tgtgaagcgg     180
ctggaagaga tgctgaggcc cttggtggaa gagggcctac gctgtgtctt gatctttggc     240
gtccccagca gagttcccaa ggacgagcgg ggttccgcag ctgactccga ggagtcccca     300
gctattgagg caatccatct gttgaggaag accttcccca acctcctggt ggcctgtgat     360
gtctgcctgt gtccctacac ctcccatggt cactgcgggc tcctgagtga aaacggagca     420
ttccgggctg aggagagccg ccagcggctg gctgaggtgg cattggcgta tgccaaggca     480
ggatgtcagg tggtagcccc gtcggacatg atggatggac gcgtggaagc catcaaagag     540
gccctgatgg cacatggact tggcaacagg gtatcggtga tgagctacag tgccaaattt     600
gcttcctgtt tctatggccc tttccgggat gcagctaagt caagcccagc ttttggggac     660
cgccgctgct accagctgcc ccctggagca cgaggcctgg ctctccgagc tgtggaccgg     720
gatgtacggg aaggagctga catgctcatg gtgaagccgg gaatgcccta cctggacatc     780
gtgcgggagg taaaggacaa gcaccctgac ctccctctcg ccgtgtacca cgtctctgga     840
gagtttgcca tgctgtggca tggagcccag gccggggcat ttgatctcaa ggctgccgta     900
ctggaggcca tgactgcctt ccgcagagca ggtgctgaca tcatcatcac ctactacaca     960
ccgcagctgc tgcagtggct gaaggaggaa tga                                  993
```

<210> 22
<211> 330
<212> PRT
<213> Homo sapiens

<400> 22

```
Met Gln Pro Gln Ser Val Leu His Ser Gly Tyr Phe His Pro Leu Leu
 1              5                   10                  15
Arg Ala Trp Gln Thr Ala Thr Thr Thr Leu Asn Ala Ser Asn Leu Ile
            20                  25                  30
Tyr Pro Ile Phe Val Thr Asp Val Pro Asp Asp Ile Gln Pro Ile Thr
        35                  40                  45
Ser Leu Pro Gly Val Ala Arg Tyr Gly Val Lys Arg Leu Glu Glu Met
    50                  55                  60
Leu Arg Pro Leu Val Glu Glu Gly Leu Arg Cys Val Leu Ile Phe Gly
65                  70                  75                  80
Val Pro Ser Arg Val Pro Lys Asp Glu Arg Gly Ser Ala Ala Asp Ser
                85                  90                  95
Glu Glu Ser Pro Ala Ile Glu Ala Ile His Leu Leu Arg Lys Thr Phe
            100                 105                 110
Pro Asn Leu Leu Val Ala Cys Asp Val Cys Leu Cys Pro Tyr Thr Ser
        115                 120                 125
His Gly His Cys Gly Leu Leu Ser Glu Asn Gly Ala Phe Arg Ala Glu
    130                 135                 140
Glu Ser Arg Gln Arg Leu Ala Glu Val Ala Leu Ala Tyr Ala Lys Ala
145                 150                 155                 160
Gly Cys Gln Val Val Ala Pro Ser Asp Met Met Asp Gly Arg Val Glu
                165                 170                 175
Ala Ile Lys Glu Ala Leu Met Ala His Gly Leu Gly Asn Arg Val Ser
            180                 185                 190
Val Met Ser Tyr Ser Ala Lys Phe Ala Ser Cys Phe Tyr Gly Pro Phe
        195                 200                 205
Arg Asp Ala Ala Lys Ser Ser Pro Ala Phe Gly Asp Arg Arg Cys Tyr
    210                 215                 220
Gln Leu Pro Pro Gly Ala Arg Gly Leu Ala Leu Arg Ala Val Asp Arg
225                 230                 235                 240
Asp Val Arg Glu Gly Ala Asp Met Leu Met Val Lys Pro Gly Met Pro
                245                 250                 255
Tyr Leu Asp Ile Val Arg Glu Val Lys Asp Lys His Pro Asp Leu Pro
            260                 265                 270
Leu Ala Val Tyr His Val Ser Gly Glu Phe Ala Met Leu Trp His Gly
        275                 280                 285
Ala Gln Ala Gly Ala Phe Asp Leu Lys Ala Ala Val Leu Glu Ala Met
    290                 295                 300
Thr Ala Phe Arg Arg Ala Gly Ala Asp Ile Ile Ile Thr Tyr Tyr Thr
305                 310                 315                 320
Pro Gln Leu Leu Gln Trp Leu Lys Glu Glu
                325                 330
```

**Description**

FIELD OF THE INVENTION

The present invention relates to **a novel method for preparing rhPBGD in an *E. coli* host cell, which is hemC defective, as well as** -to an expression plasmid and a linear DNA fragment for use in the production of rhPBGD. **Finally, the invention also relates to an E. coli host strain, which is *hemC* defective**.

BACKGROUND OF THE INVENTION

Acute Intermittent Porphyria

Acute intermittent porphyria (AIP) is an autosomal dominant disorder in man caused by a defect (50% reduction of activity) of the third enzyme in the heme biosynthetic pathway porphobilinogen deaminase, (also known as porphobilinogen ammonia-lyase (polymerizing)), E.C. 4.3.1.8. (Waldenström 1937, J. Acta.Med. Scand. Suppl.82). In the following, this enzyme and the recombinant human form will be termed "PBGD" and "rhPBGD", respectively.

Important regulation of the heme biosynthetic pathway is delivered by the end product of the metabolic pathway, namely heme, which exerts a negative inhibition on the first rate-limiting enzymatic step (conducted by delta-aminolevulinic-synthetase) in the heme biosynthetic pathway (Strand et al. 1970, Proc. Natl. Acad. Sci. 67, 1315-1320). Deficiencies in the heme biosynthetic enzymes have been reported leading to a group of diseases collectively called porphyrias. A defect in the third enzymatic step leads to acute intermittent porphyria, AIP. The reduction in enzymatic PBGD activity makes this enzyme the rate limiting step in the heme biosynthetic pathway, with a concomitant increase in urinary and serum levels of delta-aminolevulinic acid (ALA) and porphobilonogen (PBG).

Clinical manifestation of AIP

The clinical manifestation of AIP involves abdominal pain and a variety of neuropsychiatric and circulatory dysfunctions. As a result of the enzymatic block, heme precursors such as PBG and ALA are excreted in excess amounts in the urine and stool. In acute attacks, high levels of PBG and ALA are also found in serum. These precursors are normally undetectable in serum in healthy individuals.The neuropsychiatric disturbances observed in these patients are thought to be due to interference of the precursors with the nervous system or due to the lack of heme. For instance, ALA bears a close resemblance to the inhibitory neurotransmitter 4-aminobutyric acid (GABA) and has been suggested to be a neurotoxin. (Jeans J. et al. 1996, American J. of Medical Genetics. 65, 269-273).

The AIP is a lifelong disease, which usually becomes manifest in puberty. Most precipitating factors exhibit an association with the first rate-limiting enzyme in the heme biosynthetic pathway through heme, the final product of the pathway. A lowering of the heme concentration will immediately increase the rate of ALA-synthetase. An overproduction of ALA then makes the partially deficient PBGD enzyme (50% activity) now rate-limiting with an accumulation of the heme precursors ALA and PBG. Drugs that induces cytochrome P450 such as barbiturates, estrogens, sulphonamides, progesterone, carbamyazepine, and phenytoin can all precipitate acute attacks. (Wetterberg L. 1976, In Doss M. Nowrocki P. eds. Porphyrias in Human Disease. Reports of the discussion. Matgurg an der Lahn, 191-202).

Existing treatment of AIP

The treatment of AIP as well as of other types of porphyrias such as variegata, hereditary coproporphyria, harderoporphyria, and aminolevulinic acid dehydratase deficiency, are basically the same. Existing therapies for AIP, are all aimed at reducing circulating PBG and ALA by inhibiting the first rate-limiting enzymatic step ALA-synthetase. This inhibition of ALA-synthetase is achieved by increasing circulating heme, since heme is a negative feed back regulator of ALA-synthetase. Hematin treatment, high caloric intake or inhibition of heme breakdown by Sn-mesoporphyrin administration are the existing therapies today. These therapies have shown limited efficacy.

Levels of ALA and PBG found in urine in patients with symptomatic AIP, are in the range of 1-203 mg/day and 4-782 mg/day, respectively. Normal excretion of ALA and PBG is very low (0-4 mg/day). Important is the observation that these patients also have elevated levels of ALA and PBG in serum. It was shown in a study that AIP patients had significantly elevated levels of ALA (96 μg %) and PBG (334 μg %) in serum in connection with acute attacks and that the severity of the attacks were correlated to high levels of ALA and PBG.

Hence, it is important to reduce the circulating levels of ALA and PBG in order to eliminate clinical symptoms and to normalize the heme pool.

Disclosure of the invention

By the term "catalyst" is herein meant either the relevant enzyme which is substituted as it is, or an enzymatically equivalent part or analogue thereof. One example of an enzymatically equivalent part of the enzyme could be a domain or subsequence of the enzyme which includes the necessary catalytic site to enable the domain or subsequence to exert substantially the same enzymatic activity as the full-length enzyme or alternatively a gene coding for the catalyst.
An example of an enzymatically equivalent analogue of the enzyme could be a fusion protein which includes the catalytic site of the enzyme in a functional form, but it can also be a homologous variant of the enzyme derived from another species. Also, completely synthetic molecules that mimic the specific enzymatic activity of the relevant enzyme would also constitute "enzymatic equivalent analogues".
The term "the heme biosynthetic pathway" refers to the well-known enzymatic steps (cf. e.g. Sassa S. 1996, Blood Review, 10, 53-58) which leads from glycin and succinyl-CoA to heme, and enzymes belonging to this synthetic pathway are delta-aminolevulininic acid synthetase, delta-aminolevulinic acid dehydratase, porphobilinogen deaminase, uroporphyrinogen III cosythetase, uroporphyrinogen decarboxylase, coproporphyrinogen oxidase, protoporphyrinogen oxidase and ferrochelatase. Hence, in line with the above, a catalyst used according to the invention is such an enzyme or an enzymatically equivalent part or analogue thereof.
**The present invention pertains to the production of recombinant** rhPBGD. Also described is the production of rhPBGD in high scale.
Preferred formulations of the catalyst are exemplified for, but not limited to, PBGD in the detailed description hereinafter.
A method for recombinant production comprises

a) introducing, into a suitable vector, a nucleic acid fragment which includes a nucleic acid sequence encoding the catalyst;
b) transforming a compatible host cell with the vector;
c) culturing the transformed host cell under conditions facilitating expression of the nucleic acid sequence; and
d) recovering the expression product from the culture

and optionally subjecting the expression product to post-translational processing, such as in vitro protein refolding, enzymatic removal of fusion partners, alkylation of amino acid residues, and deglycosylation, so as to obtain the catalyst.
For relatively small catalysts (e.g. those constituted mainly of the active site of the enzyme), the catalyst can alternatively be prepared by liquid-phase or solid-phase peptide synthesis.
A more detailed explanation of the recombinant production of the model enzyme PBGD is given in the detailed section hereinafter, but as mentioned herein the same considerations apply for all other peptide catalysts of the invention. One of the main advantages of producing the catalyst by recombinant or synthetic means is, that if produced in a non-human cell, the catalyst is free from any other biological material of human origin, thus reducing problems with known or unknown pathogens such as viruses etc.
Legends to figures:

Figure 1: Circular map of plasmid pPBGD1.1
Figure 2: Flow chart for construction of plasmid pExp0
Figure 3: Circular map of plasmid pExp0
Figure 4: Flow chart for construction of plasmid pExp1
Figure 5: Circular map of plasmid pExp1
Figure 6: Flow chart for construction of pExpl-M2
Figure 7: Circular map of plasmid pExp1-M2
Figure 8: Flow chart for construction of rhPBGD expression plasmid pExp1-M2-BB
Figure 9: Circular map of rhPBGD expression plasmid pExp1-M2-BB
Figure 10: PCR strategy for construction of the *EcoR1-Hind* III linear DNA-fragment
Figure 11: Structure of the *Eco*R I-*Hind* III linear DNA-fragment used for transformation
Figure 12: Respiration and growth data from fermentation PD14 with strain PBGD-2
Figure 13: rhPBGD expression in fermentation PD14 with strain PBGD-2
Figure 14: Chromatography on DEAE-Sepharose FF (DEAE1)
Figure 15: Chromatography on DEAE-Sepharose FF (DEAE2)
Figure 16: Chromatography on Butyl-Sepharose 4 FF
Figure 17: Circular map of rhPBGD-His expression plasmid pExp2
Figure 18: PBGD reaction mechanism
Figure 19: DEAE chromatography elution profile

Figure 20: SDS-PAGE gel of DEAE eluates
Figure 21: Cobalt chromatography elution profile
Figure 22: SDS-PAGE gel results of cobalt eluates
Figure 23 and
Figure 24: Ilustrate numbers in diagrams (Table19). The expression of PBGD in HeLa cells was increased up to 475 times from the basal activity and in NIH 3T3 cells up to 11 times.
Figure 25: Comparison of fermentations PD05 and PD06 with strain PBGD-2
Figure 26: Comparison of fermentations PD09, PD11 and PD12
Figure 27: Comparison of fermentations PD09, PD11 and PD12 with strain PBGD-1.
Figure 28: Comparison of fermentations PD14, PD16 and PD19 with strain PBGD-2.
Figure 29: Comparison of fermentations PD14, PD16 and PD19 with strain PBGD-2
Figure 30: Comparison of fermentations PD19, PD21 and PD22 with strain PBGD-2.
Figure 31: Comparison of fermentations PD19, PD21 and PD22 with strain PBGD-2.
Figure 32: Comparison of fermentations PD19, PD1501 and PD1502
Figure 33:: Comparison of fermentations PD19, PD1501 and PD1502 with strain PBGD-2.
Figure 34:
Figure 35: Stability studies: Single use aliquots of extract were routinely taken out of the freezer (-20°C) and the rhPBGD-activity was measured and plotted over time.
Figure 36.Description of oligos used for PCR amplification.
Figure 37. Enzyme concentration over 8 weeks at 40°C measured by HPLC. A decrease from 2 mg/ml to 0,5 mg/ml and 8 mg/ml to 2,5 was detected.
Figure 38. Enzyme specific activity measured during 8 weeks at 40°C. The activity was measured using the enzyme activity assay and the protein concentration was measured using HPLC.
Figure 39. The enzyme activity measured over 8 weeks at 40°C. A significant decrease over the first week was seen for the high concentration sample, 1b. After two weeks the decrease rate was the same for all samples.
Figure 40. rhPBGD concentration over 12 weeks at -20°C (freezer), 5°C (fridge), 25°C (RT) and freeze/thawed at each sampling. The measurement was performed using HPLC
Figure 41. rhPBGD activity over 12 weeks at -20°C (freezer), 5°C (fridge), 25°C (RT) and freeze/thawed at each sampling.
Figure 42. rhPBGD specific activity over 12 weeks at -20°C (freezer), 5°C (fridge), 25°C (RT) and freeze/thawed at each sampling. Measurements were performed using enzyme activity assay and HPLC.
Figure 43 The rhPBGD activity measured over 8 weeks. The stability study has been performed under nitrogen at -20°C ± 5°C, 5°C ± 3°C and at 25°C ± 2°C.
Figure 44. The rhPBGD activity measured over 8 weeks. The stability study has been performed under nitrogen at -20°C ± 5°C, 5°C ± 3°C and at 25°C ± 2°C.
Figure 45. The specific rhPBGD activity measured using the enzyme activity assay and BCA protein concentration assay. The stability study has been performed under nitrogen at -20°C ± 5°C, 5°C ± 3°C and at 25°C ± 2°C.

Sequence list:

Seq. ID NO 1: Sequence of the expression plasmid pExp1-M2-BB
Seq. ID NO 2: Sequence of the EcoR I - Hind III linear fragment used for transformation in the hemC disruption strategy
Seq. ID NO 3: Sequence of the erythropoietic form (PBGD 1.1)
Seq. ID NO 4: Sequence of the non-erythropoietic form (PBGD 1.1.1)
Seq. ID NO 5: Sequence of PDGB from Spleen (PBGD 1.3)
Seq. ID NO 6: Sequence of PDGB from bone marrow (PBGD 2.1)
Seq. ID NO 7: Sequence of PDGB from bone marrow (PBGD 2.2)
Seq. ID NO 8: Sequence of PDGB from lymph node (PBGD 3.1)
Seq. ID NO 9: Sequence of PDGB from lymph node (PBGD 3.3)
Seq. ID NO 10: Sequence PDGB from total brain (PBGD 5.3)
Seq. ID NO 11: Sequence of PDGB from total brain (PBGD 6.1)
Seq. ID NO 12: Sequence of PDGB, Fig 1
Seq. ID NO 13: Sequence of (PBGD)
Seq. ID NO 14: Primer named ICO 549
Seq. ID NO 15: Primer named ICO 550
Seq. ID NO 16: Primer named ICO 383

Seq. ID NO 17: Primer named ICO 384
Seq. ID NO 18: Primer named ICO 618
Seq. ID NO 19: Primer named ICO 616
Seq. ID NO 20: Primer named ICO 617

DETAILED DISCLOSURE OF THE INVENTION

Herein described is a method wherein **rhPBGD** has been prepared by a method comprising

a) introducing, into a suitable vector, a nucleic acid fragment which includes a nucleic acid sequence encoding the catalyst;
b) transforming a compatible host cell with the vector;
c) culturing the transformed host cell under conditions facilitating expression of the nucleic acid sequence; and
d) recovering the expression product from the culture

and optionally subjecting the expression product to post-translational processing, such as in vitro protein refolding, enzymatic removal of fusion partners, alkylation of amino acid residues, and deglycosylation, so as to obtain the catalyst.

An example **of a rhPBGD** is a recombinant human PBGD based on any of Seq. ID NO 3 and Seq. ID NO 4.

The human PBGD cDNA sequence is selected from Seq. ID NO 3 and Seq. ID NO 4.

The following description of preferred embodiments of the invention will focus on recombinant production of PBGD and formulations thereof **but other aspects of the invention are mentioned in the following sections of the description as well. The invention is directed to an EcoRI - *Hin*DIII linear DNA fragment as shown in SEQ ID NO.: 2, an expression plasmid as shown in SEQ ID NO.: 1, an *E. coli* host strain, which is hemC defective, a production strain, which is made heme-by transforming with the *Eco*RI - *HinDIII* linear DNA fragment**.

Production of recombinant human PBGD (rhPBGD)

As mentioned above, it is preferred to administer recombinant human versions of the various enzymes of the heme biosynthetic pathway. In the following will be described recombinant production of one of these enzymes, namely PBGD.

The gene for the erythropoietic PBGD, which is located in the human genome in the chromosomal region 11q 24, is composed of 15 exons spanning 10 kb of DNA and is shown in Grandchamp B. et al. 1996, J. of Gastroenerology and Hepatology 11, 1046-1052.

The gene coding the erythropoietic PBGD enzyme (344 amino acids) (Raich N. et al 1986, Nucleic. Acid. Res, 14, 5955-5968), will be cloned from a human erythropoietic tissue by use of a nested PCR (polymerase chain reaction) strategy.

The PBGD coding region will be inserted in a plasmid and transformed into a suitable host cell (a bacterium such as E. *coli* and B. *subtilis,* or a fungus such as *Saccharomyces).* The expression of the PBGD gene will be regulated by a promoter which is compatible with the selected host cell.

For bacterial production: An endogenous ATG sequence is located at the $NH_2$-terminal end of the PBGD structural gene for initiation of translation and cytoplasmic expression. Alternatively insert in front of the PBGD coding region a bacterial signal sequence for example an *E. coli* periplasmic enzyme signal peptide or a signal peptide from a secreted enterotoxin or endotoxin in *E. coli,* to obtain secretion in *E. coli.*

A plasmid used for production of rhPBGD in *E. coli* was constructed in the following way:

Construction of a plasmid harboring the coding region of human wild type PBGD (pBPGD1.1)

Introduction:

The erythropoietic expressed form of porphobilinogen deaminase (PBGD) (Raich N. et al. Nucleic Acids Research 1986 14(15): 5955-67) was cloned and sequence determined. Two forms of PBGD are known. The erythropoietic form is expressed specifically in erythroid progenitors and the constitutive form is expressed in all cells (Grandchamp B. et al. 1987, Eur J Biochem. 162(1):105-10). The two are expressed from the same gene and are identical except for the addition of 17 amino acids at the amino terminus of the constitutive form through alternative exon usage. It was decided to clone and express the erythropoietic form. There are three sequences for PBGD in the Genebank, the two isoforms mentioned above and the genomic sequence (Yoo HW. et al. 1993, Genomics. 15(1):21-9). These all have nucleotide differences translating to amino acid changes. Before choosing a specific sequence to be expressed for a human therapeutic it was therefore necessary to determine what is the wild type allele. To accomplish this, PBGD cDNA

clones were isolated and sequenced from a number of sources to define the most common amino acid usage. Oligonucleotide primers were designed to amplify the coding region from cDNAs by Polymerase Chain Reaction (PCR) (Saiki R.K. et al. 1985, Science 230(4732): 1350-4). These were used to isolate cDNAs from 5 sources of mRNA which were then cloned into a plasmid vector. Eight of these clones were sequenced and along with the published sequences define a wild type allele, which should be the most common amino acid sequence in the population. This wild type allele will be used for protein expression.

Strategy:

A nested PCR strategy was devised to clone PBGD. The first primer set, (see Table 1) Ico379 and Ico382, are 20mers that bind to sequence outside of the coding region. Ico379 is specific for the 5' untranslated region of the mRNA (cDNA) of the erythropoietic form of PBGD. The binding site is in an exon region not expressed in the constitutive form of the enzyme. Ico382 binds to the 3' untranslated region of both forms of PBGD. Internal to these are a second set of oligonucleotide primers to be used for the second round of PCR, Ico375 and Ico376, designed to distal ends of the PBGD coding region. Ico375 has 22 bases of sequence homologous to the 5' end of the coding region of the erythropoietic form of PBGD with the ATG start codon followed by an *EcoR* I endonuclease cleavage site for cloning of the PCR product and 4 bases of sequence to ensure efficient restriction. Ico376 has 33 bases homologous to the 3' end of the PBGD coding region with 3 bases changed internally to introduce a *Mun I*/*Mfe* I endonuclease cleavage site through silent mutations and ending with the TAA stop codon. This restriction site will be used to easily introduce sequence encoding a His-Tag to the DNA with oligonucleotide adapters or to enable other 3' modifications. Following the stop codon is a second stop codon to ensure good termination of translation and a *Hind* III endonuclease cleavage site for cloning the PCR product followed by 4 bases to ensure efficient restriction. The EcoR I and *Hind* III endonuclease cleavage sites introduced onto the ends of the PBGD PCR product ligate into the respective unique restriction sites in the high copy number pBluescriptII SK-(Stratagene) vector for sequencing and will then be used to move the PBGD DNA into an E. *coli* expression vector for production of recombinant human porphobilinogen deaminase, rhPBGD.

PCR:

Six cDNAs were used as a PCR source; spleen, bone marrow, lymph node, lung, whole brain and adipose tissue each from a different pool of human donors (produced by Donald Rao using BRL Superscript II with 500 ng Clontech poly-A RNA in 20 μl reaction volumes per manufacturers instructions except adipose which was made from 5 μg of Clontech total RNA from a single donor). List of equipment and supplies used (see lists below). One μl of each cDNA (approximately 25ng) was amplified with Advantage cDNA polymerase mix (Clontech) with 0.2mM dNTP (PE/ABI) and 0.3 μM each of Ico379 and Ico382 in 50 μl reaction volumes. Two cycle PCR was used, with an initial heat denaturation step at 94°C for 1' 40" then 28 cycles of 96°C for 16" and 68°C for 2'. A final extension of 6' at 74°C ensured that extension products were filled out. One fifth of the reaction was run out on a 1.2% agarose gel with 2 μl of 6X ficol loading dye in 0.5X TBE buffer (Maniatis T., E.F. Fritsch, J. Sambrook. Molecular Cloning (A laboratory Manual) Cold Spring Harbor Laboratory. 1982). The predicted band of 1.1 kb. was observed by ethidium bromide staining with all sources but lung tissue cDNA. These bands were excised and DNA was isolated with Microcon-30 with micropure inserts (Amicon/Millipore) per manufacturers instructions and buffer exchanged with $dH_2O$. One tenth of the recovered DNA was amplified with Advantage cDNA polymerase mix (Clontech) with 0.2mM dNTP (PE/ABI) and 0.3μM each of the internal nested oligonucleotides (Ico375 and Ico376) at 0.3 μM in 50μl reactions. Two cycle PCR was used again with an initial heat denaturation step at 94°C for 1' 40" then 2 cycles of 96°C for 16" and 68°C for 2' then 13 cycles of 96°C for 16" and 72°C for 2' with a final extension of 6' at 74°C. Ten μl of the 50 μl reactions were run on a 1.2% agarose gel with 2 μl 6X loading dye. The resulting bands were of the expected size, 1.05 kb. The remainder of the PCR reactions were passed through Chromaspin-400 columns (Clontech) per manufacturers instructions to remove reaction components and to exchange buffer with TE (10mM Tris-HCl pH8.0/ 1mM EDTA). The DNA containing eluates were washed with $dH_2O$ and concentrated with Microcon-100 spin-filters (Amicon/Millipore) as described by the manufacturer's instructions.

Cloning:

The purified PBGD DNA was digested for 6 hours with 40 Units each of EcoR I and *Hind* III in EcoR I "U" buffer (New England Biolabs (NEB)) in 50 μl reactions at 37°C. Enzymes were heat killed for 20 minutes at 68°C and reactions were spun in Microcon 100 spin-filters to remove small DNA end pieces, washed with $dH_2O$ and concentrated. One fifth of the resulting DNA was ligated with approximately 50 ng *E*coR I and *Hind* III digested and twice gel purified pBluescriptII SK- (Stratagene) and 200 units T4 DNA ligase (NEB cohesive end units) for 15 hours at 16°C. The ligase was heat killed at 75°C for 10 minutes. The reactions were then buffer exchanged with $dH_2O$ and concentrated in Microcon-100 spin filters and volumes taken up to 5 μl with $dH_2O$. One μl each was electroporated into 25 μl DH10B Electromax cells

(Gibco/BRL) at 2.5Kv/2000hms/25μF in 0.1cm cuvets with a BioRad electroporator. One ml of SOC medium (Gibco/BRL) was added and the cells were outgrown at 37°C for one hour at 250 rpm. Cells were plated out on LB plates (Maniatis T., E.F. Fritsch, J. Sambrook. Molecular Cloning (A laboratory Manual) Cold Spring Harbor Laboratory. 1982) with 150 μg/ml ampicillin. The efficiency of all five were approximately twice background (vector ligated without insert). Colony PCR was used to analyze 18 transformants of each electroporation for the presence of PBGD. An internal PBGD specific primer (IC0381) was used with a pBluescript specific primer (IC0385) to both confirm identity and proper orientation in the vector. The 25 μl reactions were set up on ice to inactivate proteases with primer concentrations of 0.4 μM, 0.125U Taq polymerase (Fisher), and 0.2mM dNTP(PE/ABI.) Two cycle PCR was used, with an initial heat denaturation step at 94°C for 1' 40" a further denaturing step at 96°C for 20 seconds, then 30 cycles of 96°C for 16" and 68°C for 1' with a final extension of 4' at 74°C. Five μl of 6X loading dye was added and 12.5 μl each were run out on a 1.2% agarose gel. Results are as follows: 12/18 positive colonies for spleen; 10/18 for bone marrow; 8/18 for lymph node; 9/18 for brain and 10/18 for adipose tissue. Two positive colonies each for the first 3 and 1 each for the latter two were grown up in 25 ml. liquid LB culture with 150 μg/ml ampicillin over night at 37°C with 250 rpm. Plasmid DNA was purified from the cultures with Qiagen's Tip-100 DNA purification kit per manufacturer's instructions. UV absorbance at 260nm was used to determine the plasmid yields which varied from between 131 and 169 μg of highly purified DNA.

Sequencing:

Sequencing reactions of double stranded plasmid DNA with Big Dye terminator cycle sequencing were performed in a 9700 thermocycler (Perkin Elmer/Applied Biosystems) Two vector primers (ICO383 and ICO384) and two PBGD specific internal primers (ICO380 and ICO381) were used for all 8 plasmids. In addition a fifth vector primer (ICO385) was used for the brain and adipose derived clones. Reaction conditions were per manufacturers protocol as follows: 500 ng plasmid DNA and 4 pmol oligonucleotide primer with 8 μl ready mix in 20 μl volumes with 30 cycles of 96°C for 12" and 60°C for 4'. Extension products were purified by isopropanol precipitation. To each reaction 20 μl of $dH_2O$ and 60 μl isopropanol were added. These were mixed by inversion and allowed to sit at room temperature for 15 minutes then spun for 40' at 3250 rpm in a Beckman GS-6KR centrifuge with the GH3 rotor and Microplate + carriers. Reactions were inverted then spun at 1680 rpm for 1' to remove liquid from the pelleted DNA. DNA sequence analysis was performed at the University of Washington Biochemistry Department sequencing Laboratory with an Applied Biosystems 377 sequencer.

Analysis:

The inserts of all 8 clones were confirmed to be PBGD by complete double strand sequence analysis (see sequences 1 - 8). Each has some change(s) from the published sequences. Some changes are unique and some are shared with other clones (see Table 2 and Table 3). For differences found only in one clone, it is difficult to distinguish between PCR or cloning artifacts and actual allelic variations without additional sampling. However, when the same base difference is found in more than one sequence it is unlikely to be from cloning errors. From the alignment of all 11 PBGD sequences a set of common bases emerged, the consensus or wild type allele sequence. Five of the eight clones (1.1, 1.3, 2.1, 3.3, and 5.3.) have the wild type amino acid sequence. Within this set with wild type amino acid sequence, there is only one difference at the nucleic acid level. At position 555, 4 of the 5 sequences have a dGTP while 1 along with the published erythropoietic and genomic PBGD have a dTTP. These appear to be two common alleles, which result in no amino acid difference. There are 2 base changes between clone number 1.1 and the published erythropoietic PBGD. An adenine to guanine change at base 513 (Leu 171) is a silent mutation, which is also present in 9 out of the 11 sequences, compared. The second difference is a cytosine to adenine substitution,at base 995 (Thr 332.) This is not a silent change, with a threonine to asparagine non-conservative mutation. It appears however that the difference is an error in the published erythropoietic PBGD sequence since all 10 other sequences have an adenine at this position. In addition to these natural variations, there are three additional silent mutations introduced during the cloning at positions 1017, 1018 and 1020 to create a *Mun* I site for future manipulations. The PBGD gene was ligated into pBluescript SK plasmid generating the pSK-PBGD 3988 bp plasmid, which was sequenced.

Conclusion:

For any recombinant therapeutic protein it is important that the wild type allele be used to reduce the potential for immunogenicity. We feel confident through our survey of the literature and analysis of PBGD sequence from different individuals that clone number 1.1 represents the most prevalent "wild type" allele in the population with respect to amino acid sequence. Clone number 1.1 contains the consensus wild type amino acid sequence and differs from the published erythropoietic PBGD sequence by only one amino acid. Because this difference is found in all the other PBGD clones besides the erythropoietic PBGD sequence, it, rather than the published erythropoietic sequence, is deemed to be the

prevalent wild type sequence. For this reason PBGD encoded by clone number 1.1 was chosen for production of recombinant human porphobilinogen deaminase (rhPBGD). In the following, the plasmid encoding the human wild type PBGD in clone number 1.1 will be termed "pPBGD1.1".

Equipment and supplies lists are shown in appendix 1 and 2, respectively.

Appendix 1 Equipment list

| Item | Manufacturer | Serial Number |
|---|---|---|
| Pipetman P-1000 | Gilson | N55287E |
| Pipetman P-200 | Gilson | N52324E |
| Pipetman P-20 | Gilson | N53465M |
| Pipetman P-10 | Gilson | P626586 |
| 5415C centrifuge | Eppendorf | 5415B68381 |
| GS-6KR centrifuge | Beckman | NGD97J18 |
| Avanti J-25 I centrifuge | Beckman | JJY97J14 |
| DU 640B Spectrophotometer | Beckman | 4323015 |
| Genie II vortex | VWR | 2-241186 |
| GeneAmp PCR system 2400 | Perkin Elmer (PE) I Applied *Biosystems* (ABI) | 803N6021903 |
| GeneAmp PCR system 2400 | PE / ABI | 803S7100104 |
| GeneAmp PCR system 9700 | PE / ABI | 805S7121566 |
| 1545 incubator | VWR | 0902597 |
| heat block 1 | VWR | 0795 |
| heat block 1 | VWR | 0511 |
| Gene Pulser II Apparatus | BioRad | 340BR2745 |
| Pulse Controller Plus | BioRad | 339BR1377 |
| Power Pac 1000 | BioRad | 286BR00770 |
| Sub Cell | BioRad | 16S/8860 |
| Wide-Mini Sub Cell | BioRad | 02S/7951 |
| Foto/Prep transilluminator | Fotodyne | PTG1-0997-2831 |
| Elutrap Electro-separator | Schleicher + Schuell | Order No. 57880 |
| Innova 4000 incubator | New Brunswick Scientific | 890165366 |
| Power Mac G3 computer | Macintosh | XA8061A3BBW |
| Trinitron Multiscan 200GS monitor | Sony | 8057052 |
| DNA analysis Software: Geneworks | Intelligenetics | Version 2.5.1 |

Appendix 2 Supplies List

| Item | Supplier | Cat No. | Lot No. |
|---|---|---|---|
| Human Spleen Poly A+ RNA | Clontech | 6542-1 | 7120266 |
| Human Bone Marrow Poly A+ RNA | Clontech | 6573-1 | 56714 |

Table continued

| Item | Supplier | Cat No. | Lot No. |
|---|---|---|---|
| Human Lung Poly A+ RNA | Clontech | 6524-1 | 7050104 |
| Human Lymph Node Poly A+ RNA | Clontech | 6594-1 | 6120292 |
| Human Brain Poly A+ RNA | Clontech | 6516-1 | 63101 |
| Human Adipose Total RNA | Clontech | D6005-01 | 7907005 |
| Superscript II reverse transcriptase | Gibco/BRL | 18064-014 | JM6418 |
| 100 mM dNTP set | Pharmacia | 27-2035-01 | 6072035011 |
| pbluescriptII SK- phagemid | Stratagene | 212206 | 0270702 |
| Advantage cDNA polymerase mix | Clontech | 8417-1 | 8060354 |
| GeneAmp dNTP | PE/ABI | N-808-0007 | H0172.4,H0553 |
| Xba-I endonuclease | New England Biolabs (NEB) | 145S | 30 |
| Pvu-II endonuclease | NEB | 151L | 14 |
| EcoR-I endonuclease | NEB | 101L | 25 |
| Hind-III endonuclease | NEB | 104S | 49 |
| Tris six-Pack "C" | Sigma | T-PAC-C | 77H9049 |
| 0.5 M EDTA pH 8.0 | Sigma | E-7889 | 16H8924 |
| Chromaspin TE 400 | Clontech | K1323-1 | 7090795 |
| Chromaspin 400 DepC $dH_2O$ | Clontech | K1333-1 | 7040086 |
| Quiaquick gel extraction kit | Qiagen | 28704 | BY97017/0397/119 |
| Microcon-30 | Amicon | 42410 | L8JM4330B |
| Microcon-100 | Amicon | 42413 | L8DM3296A |
| Micropure 0.22$\mu$m | Amicon | 42544 | CCB017 |
| Seakem GTG agarose | FMC | 50074 | 709397 |
| 100 bp DNA Ladder | NEB | 323-1 | 3 |
| 123 bp DNA Ladder | Gibco/BRL | 15613-029 | JK9706 |
| T4 DNA Ligase | NEB | 202S | 64 |
| Ampicillin | Sigma | A-9518 | 76H0434 |
| LB media | Gibco/BRL | 12795-084 | 12E1072B |
| Bacto Agar | Difco | 0140-07-4 | 106728JA |
| DH10B electromax | Gibco/BRL | 18290-015 | KHN430 |
| SOC media | Gibco/BRL | 15544-042 | 1010351 |
| Taq polymerase | Fisher | FB-6000-15 | H0436 |
| TaqStart antibody | Clontech | 5400-1 | 6070479 |
| Qiafilter Midi DNA isolation kit | Qiagen | 12243 | PO No. 514 |
| Isopropanol | Sigma | I-9516 | 47H3724 |
| Big Dye terminator cycle sequencing kit | PE/ABI | 4303152 | 9803008 |

Table 1 Oligonucleotide primers used for PCR amplification and sequencing of PBGD:

Ico375-pbgds (32 mer) coding region 5' end w/ EcoRI site sense
5' CGT GGA ATT CAT GAG AGT GAT TCG CGT GGG TA 3'

Ico376-pbgda (47 mer) coding region 3'end w/ HindIII site antisense
5' GGA GAA GCT TAT TAA TGG GCA TCG TTC AAT TGC CGT GCA ACA TCC AG 3'

Ico379-esnonc (20 mer) erythropoietic form non-coding sense
5' TCG CCT CCC TCT AGT CTC TG 3'

Ico380-sinter (21 mer) internal coding sense
5' CAG CAG GAG TTC AGT GCC ATC 3'

Ico381-ainter (21 mer) internal coding antisense
5' GAT GGC ACT GAA CTC CTG CTG 3'

Ico382-anonc (20 mer) non-coding antisense
5' CAG CAA CCC AGG CAT CTG TG 3'

Ico383-pSKT7 (22 mer) pBluescript T7 promoter

5' GTA ATA CGA CTC ACT ATA GGG C 3'

Ico384- pSKpjrev (22 mer) pBluescript reverse1
5' CTA AAG GGA ACA AAA GCT GGA G 3'

Ico385- pSKrev (21 mer) pBluescript reverse2
5' CAG CTA TGA CCA TGA TTA CGC 3'

Table 2 Variation of PBGD clones from published erythroid sequence:

| PBGD clone | Differences from Erythroid mRNA silent | non-silent | total diffs | Genebank No. | Reference/Source |
|---|---|---|---|---|---|
| Erythroid | 0 | 0 | 0 | X04217 | Raich.N. et. al. 1986, Nucleic Acids Res. 14 (15), 5955-5968 |
| Constitutive | 1 | 2 | 3 | X04808 | Grandchamp.B. et. al. 1987, Eur. J. Biochem. 162 (1), 105-110 |
| Genomic | 1 | 2 | 3 | M95623 | Yoo,H.W. et. al. 1993, Genomics 15 (1), 21-29 |
| 1.1 | 1 | 1 | 2 | - | Spleen (Clontech mRNA Lot No. 7120266) |
| 1.3 | 2 | 1 | 3 | - | Spleen (Clontech mRNA) |
| 2.1 | 2 | 1 | 3 | - | Bone Marrow (Clontech mRNA) |
| 2.2 | 2 | 2 | 4 | - | Bone Marrow (Clontech mRNA) |
| 3.1 | 2 | 4 | 6 | - | Lymph Node (Clontech mRNA) |
| 3.3 | 3 | 1 | 4 | - | Lymph Node (Clontech mRNA) |
| 5.3 | 2 | 1 | 3 | - | Total Brain (Clontech mRNA) |
| 6.1 | 3 | 2 | 5 | - | Adipose Tissue (Clontech mRNA) |

Table 2:

Summary of the number of differences in amino acid sequence of our sequenced PBGD clones and clones from Genebank entries for the constitutive and genomic PBGD with published Erythropoietic PBGD sequence. Shown in different columns are the total number of silent mutations with a DNA base change not causing a corresponding amino acid change, the number of non-silent mutations with a DNA change causing an amino acid difference and the sum of the two types of mutations. Not included in this table are the three silent mutations introduced into the clones to create an internal *Mun* I endonuclease cleavage site. Note that clone number 1.1 which will be used for production of recombinant human porphobilinogen deaminase (rhPBGD) has only one of each type of difference with the least number of total differences.

Expression plasmids

Construction of the basic expression plasmid pExp0

The basic expression plasmid pExp0 was constructed by excising the PBGD coding sequence (cDNA) from plasmid pPBGD1.1 (see Figure 1) with EcoR I and *Hind* III and inserting it into the vector pKK223-3 (Pharmacia, Catalogue # 27-4935) cut with the same enzymes, thus operatively linking it to the IPTG-inducible tac promoter (Amann E. et al. 1983, Gene 25(2-3):167-178). Figure 1 shows the construction details. Plasmid pExp0 was constructed for a preliminary assessment of the expression levels and does not directly lead to the construction of the final expression plasmid.

Construction of the final expression plasmid

The final expression plasmid pExp1-M2-BB (Figure 9) was constructed in a multi-step process. The individual steps used and all the intermediate plasmids are outlined below.

Construction of plasmid pExp1

Plasmid pExp1 was first constructed with modifications to the 5' untranslated region and the initial part of the coding sequence both aimed at improving translation efficiencies (Gold L. and Stormo G.D. 1990, Methods Enzymol 185:89-93). The changes are indicated below, and include, insertion of a second ribosome binding site, an AT-rich sequence preceding the ATG and three silent base substitutions shown in boldface.

```
AATTCTAACA TAAGTTAAGG AGGAAAAAAA A ATG AGA GTT ATT CGT GTC GGT AC
                                   Met-Arg-Val-Ile-Arg-Val-Gly
```

A naturally occurring Kpn I site six amino acid residues into the coding sequence of the human cDNA for PBGD (pPBGD1.1) was exploited for this purpose. Oligonucleotides ICO386 and ICO387 were designed to provide upon annealing to each other a 5' *EcoR* I adhesive end and a 3' *Kpn* I sticky end and the elements described above including the codons for the first six amino acid residues as shown. Oligonucleotides ICO386 and ICO387 were annealed and ligated with the Kpn I-*Hind* III, PBGD fragment from pPBGD1.1 into *EcoR* I -*Hind* III linearised pBluescript II SK- (Stratagene, Catalogue # 212206) to yield plasmid pPBGD1.1Tra. In the second step, the *EcoR* I-*Hind* III fragment from pPBGD1.1Tra was ligated into pKK223-3 cut with the same enzymes resulting in plasmid pExp1 (Figures 4 and 5).

Construction of plasmid pExp1-M2

The tetracycline resistance gene was next restored using the following strategy. Plasmid pExp1 was cut with *Sal* I and *Bam*H I and the 5349 base-pair fragment containing part of the tetracycline coding sequence and the bulk of the plasmid was isolated. Into this was ligated the *Sal* I-*Hind* III fragment from pBR322 (New England BioLabs, Catalogue # 303-3S) containing rest of the coding sequence and an adapter formed by annealing oligonucleotides IC0424 and IC0425 to each other. The adapter contains part of the tetracycline promoter and provides *Hind* III and BamH I overhangs for ligation but destroys the *Hind* III and *BamH* I restriction sites. The resulting plasmid was called pExp1-M2 (Figures 6 and 7).

Construction of plasmid pExp1-M2-BB

In the final step the rop gene contained between BsaA I and BsaBI was deleted to increase copy number (Makrides S.C. 1996, Microbiol.Rev. 60(3):512-538). For this the plasmid pExp1-M2 was cycled through the *dam* minus strain, JM110 (F' *[traD36 proA$^+$ proB$^+$ lacI$^q$ $\Delta$ (lacZ)M15] dam dcm supE44 hsdR17 thi leu thr rpsL lacY galK galT ara tonA tsx $\Delta$ (lac-proAB) lambda$^-$)* as restriction with *BsaB* I is blocked by overlapping *dam* methylation. It was then cut with *BsaA* I and *BsaB* I to excise the rop gene and the 5446 base-pairs long linear fragment was circularised by blunt-end ligation to yield the production plasmid pExp1-M2-BB (Figures 8 and 9).

Construction of the hemC-deletion host and the final expression strain

The parent strain JM105 (F' *[traD36 proA+ proB+ lacI$^q$ $\Delta$(lacZ)M15] $\Delta$(pro-lac) hsdR4 sbcB15 rpsL thi endA1 lambda$^-$),* a derivative of E. *coli* K12 was obtained from Parmacia, Catalogue # 27-1550-01. The *hemC* gene coding for the endogenous *E. coli* Porphobilinogen Deaminase was partially deleted. This was necessary to ensure that the purified product (rhPBGD) was free from contaminating E. *coli* PBGD as the E. *coli* and human enzymes are very similar in properties (Jordan P.M. 1994, Wiley, Chichester (Ciba Found Symp 180), p70-96) and may co-purify. The *hem*C-deletion host was derived from JM105 according to Scheme A. First a hemin-permeable variant was obtained by a three-step process. This was essential as a *hemC*-deletion mutant would require hemin for good growth and *E. coli* K12 strains are not freely permeable to hemin.

Scheme A. Scheme for obtaining *hemC*-deletion strain:

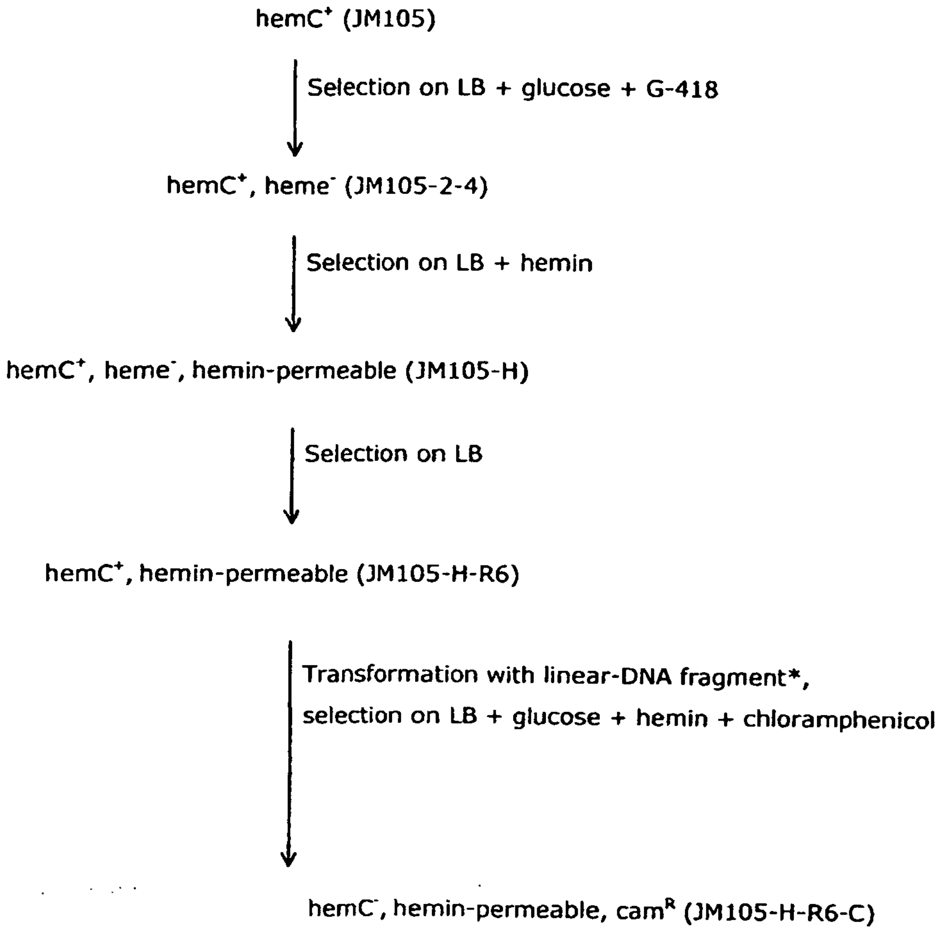


All mutant isolation was spontaneous. Approximately $5\times10^{-8}$-$5\times10^{-9}$ cells were plated on selective media. The media compositions are included as Appendix 1.

**[0049]** * For details on the linear DNA-fragment see Figure 11.

In the first step, a heme-minus mutant was isolated carrying a defect in any of the biosynthetic steps leading to the formation of heme. Heme strains fall into the general class of respiration deficient mutants that are defective in active transport and consequently resistant to low levels of antibiotics of the aminoglycoside family such as gentamicin (Lewis L.A. et al. 1991, Microbiol.Immunol. 35(4):289-301). Several spontaneous mutants were isolated as a dwarf colonies on LB+glucose +G-418 (gentamicin)-containing plates (Lewis L.A. et al. 1991, Microbiol.Immunol. 35(4):289-301). These were screened for their ability to respond weakly to hemin, indicating that they were heme (as opposed to other respiration deficient mutants which would not respond to hemin at all). One such heme-strain (JM105-2-4, see Scheme A) which could also revert back spontaneously to robust growth on LB (as this is essential for the third step, see below) was selected. This strain was next plated on LB+hemin to obtain a better grower in the presence of hemin and was called JM105-H. It showed improved growth only in the presence of hemin, which meant that it still was heme but had become hemin-permeable. To restore the functionality of the heme biosynthetic pathway in JM105-H, spontaneous revertants were isolated on LB plates and only those retained which resembled the starting strain JM105 in growth, both untransformed and after transformation with the expression plasmid. One such strain used in this study was called JM105-H-R6 and should have retained the heme-permeable trait of its parent strain.

Strain JM105-H-R6 was transformed with the *EcoR I-Hind* III fragment (see Scheme A), to obtain the *hemC*-deletion host called JM105-H-R6-C by homologous gene replacement. This strain has the genotye, *F'[traD36 proA+ proB+ $lacI^q$ Δ(lacZ)M15] Δ(pro-lac) hsdR4 sbcB15 rpsL thi end A1 lambda*$^-$ hemC:CAT hemin-permeable. It was transformed with the expression plasmid pExp1-M2-BB to yield the final production strain PBGD-2 (PBGD-2 was deposited under the Budapest Treaty on 9 July 1999 with DSMZ (Deutsche Sammlung von Mikroorganismen und Zellkulturen, GmbH, Mascheroder Weg 1b, D-38124 Braunschweig, Germany) under the accession No. DSM 12915).

In order to obtain the *EcoR* I-*Hind* III fragment, a multiple PCR strategy was used. Olignudeotide pairs IC0437, ICO438 and IC0505, ICO440 were used to amplify separately, portions of *E. coli* JM105 genomic DNA segments flanking

the *hemC* gene (see Figure 10). These amplified gene products were digested with pairs of enzymes EcoR I, *Xho* I and *Xho* I, *Hind* III respectively, and in essence, assembled together between the EcoR I and *Hind* III sites of pUC19 to give plasmid phemCd. Next the fragment containing the chloramphenicol-resistance gene was PCR amplified from plasmid pBC SK+ (Stratagene, Catalogue # 212215) using oligonucleotides ICO510 and ICO511. This product was cut with *Xho* I and inserted into plasmid phemCd at the *Xho* I site. In essence, the plasmid having the Cam gene in the orientation shown was called phemCdCm and formed the source of the *EcoR* I-*Hind* III linear DNA-fragment depicted in Figure 11.

In Figure 11 the structure of the linear DNA-fragment used for the transformation is shown. The genetic organization of the *E. coli* polypeptides depicted by gray arrows (cyaA, hemC-5', hemC-3', hemX and hemD) is derived from the GenBank report Accession number AE000456. The black arrow represents the 767 base-pairs long PCR fragment carrying the cholamphenicol-resistance gene (Cam), encoding chloamphenicol acetyltransferase (CAT), replacing 806 base-pairs of the *hemC* coding sequence. HemC-5' and hemC-3' correspond to 149 and 16 base-pairs respectively, of the coding sequence of the disrupted *hemC* gene. EcoR I, *Xho* I and *Hind* III are engineered restriction sites. The sequence of this 3225 base-pairs long fragment is shown in Seq. ID NO 2. The two *Xho* I sites are at positions 1072 and 1839 in the sequence, respectively.

Table 34 Oligonucleotide primers used in the construction of the production strain PBGD-2

ICO386 (54 mer) Construction of plasmid pExp1
5' AAT TCT AAC ATA AGT TAA GGA GGA AAA AAA AAT GAG AGT TAT TCG TGT CGG TAC 3'

ICO387 (46 mer) Construction of plasmid pExp1
5' CGA CAC GAA TAA CTC TCA TTT TTT TTT CCT CCT TAA CTT ATG TTA G 3'

ICO424 (32 mer) Construction of plasmid pExp1-M2
5' GAT CAC TCA TGT TTG ACA GCT TAT CAT CGA TT 3'

ICO425 (31 mer) Construction of plasmid pExp1-M2
5' AGC TAA TCG ATG ATA AGC GTC AAA CAT GAG T 3'

ICO437 (32 mer) Amplification of product P1
5' AGT CAG AAT TCA GAC GCA CGG CGG TAC GAT AA 3'

ICO438 (32 mer) Amplification of product P1
5' ATT CAC TCG AGG TCA CCA TCG GTA CCA GTT CA 3'

ICO440 (32 mer) Amplification of product P2
5' AGA TCA AGC TTC GGC CAG ACG CAG GTT ATC TA 3'

ICO505 (34 mer) Amplification of product P2
5' ATA CAC TCG AGA CCG GCA TGA GTA TCC TTG TCA C 3'

ICO510 (30 mer) Amplification of Cam gene
5' ACT GAC CTC GAG CGG CAC GTA AGA GGT TCC 3'

ICO511 (29 mer) Amplification of Cam gene
5' ACT GAA CTC GAG AAT TAC GCC CCG CCC TG 3'

Accordingly, the cDNA used for expressing rhPBGD was derived from plasmid pPBGD1.1. The starting host strain was derived from JM105 and is called JM105-H-R6-C. The genotype and the details on its construction are described above. A large part of the coding region of the *hemC* gene was replaced by the Cam gene, encoding chloramphenicol acetyltransferase. This gene replacement was confirmed by PCR amplification of the segment of the E. *coli* genome followed by restriction analysis of the amplified product. As a result of the gene replacement, the strain is resistant to chloramphenicol and grows extremely poorly on LB medium. Growth improves when LB medium is supplemented with hemin.

Expression construct

The expression plasmid in the final production strain is pExp1-M2-BB. Its construction is described above. A detailed map of the plasmid showing the open reading frames and functionally relevant regions is shown in Figure 9. The complete DNA sequence is included in Seq. ID NO 1. All synthetic adapters and linkers used during the construction

have been sequenced along with all junctions created during ligation which directly impinge upon the expression of the cloned gene.

Production strain

The final production strain is called PBGD-2. It was obtained by introducing the expression plasmid pExp1-M2-BB into the host strain JM105-H-R6-C, essentially, by rendering the cells competent with 100mM $CaCl_2$ (Morrison D.A. 1979, Methods.Enzymol. 68:326-331) and selecting for transformants on LB + ampicillin media at 30°C. The plasmid is a derivative of pBR322 without the *rop* gene and should be present extrachromosomally in moderate copy number at 30°C with a slightly higher copy number at elevated temperatures 37°C and greater (Makrides S.C. 1996, Microbiol.Rev. 60(3):512-538). It has both the ampicillin and tetracycline resistance genes as selectable markers. It also expresses rhPBGD which can complement the *hemC* defect of the host strain. As a result, the production strain should grow normally on LB/M9 media, be resistant to the antibiotics ampicillin and tetracycline and also be resistant to the antibiotic chloramphenicol (because of the presence of the Cam gene in the genome). It was confirmed to have all these characteristics.

Expression

The expression of rhPBGD is driven by the tac promoter which is regulated by the copy of the *lacI$^q$* gene present in the host. Due to the modifications made to the system as described in the study plan, the uninduced level of expression is 1.8 units/mg (see Appendix 3 for assay details), which amounts to approximately 10% of the total soluble protein. The culture is grown throughout at 30°C and no induction step is used to increase expression.

Evaluation and conclusions

The expression system developed for the production of rhPBGD in *E. coli* is a stable system, producing good amounts of rhPBGD in a constitutive manner when the cells are grown at 30°C. The host strain employed is partially deleted for the gene producing the endogenous *E. coli* porhobilinogen deaminase. After transformation with the expression plasmid, the resulting production strain PBGD-2 grows as well as the strain PBGD-1 (which is JM105 carrying the same expression plasmid) and makes the same amount of rhPBGD.

Alternative expression construct:

Expression plasmid pExp1-M2-Puc-BB and expression of rhPBGD in *E. coli*

The plasmid pExp1-M2 was digested with Pvu I and Afl III and the larger of the two fragments corresponding to a size of 4745 base-pairs was isolated. This was ligated to the 1257 base-pairs long Pvu I-AflIII fragment derived from pUC19 containing the origin of replication and part of the ampicillin resistance gene to obtain plasmid pExp1-M2-Puc. This was passaged through JM110 and cut with BsaA1 and BsaB1 to excise the rom gene contained between the two sites and blunt-ended together to yield the final expression plasmid pExp1-M2-Puc-BB. The pExp1-M2-Puc-BB plasmid has been fully sequenced and differs from pExp1-M2-BB only in that C in position 2769 is T in pExp1-M2-Puc-BB.

Expression of rhPBGD in *E. coli*

The *E. coli* K12 host strain JM105 genotype endA thi rpsL sbcB15 hsdR4 Δ(lac-proAB) [F'traD36 proAB lacI$^q$ Δ(lacZ)M15] containing the expression plasmid pExp1-M2-Puc-BB was grown in LB broth containing 100 μg/ml ampicillin at to mid-log phase at 30°C from a 1 to 40 dilution of an overnight inoculum. The culture was then split into three and growth was continued for another 4 hours at 30°C, 37°C and 42°C respectively. Cells were spun down from 1 ml samples and frozen at -20°C. The thawed cell pellets were resuspended in 200-300 μl of B-PER reagent PIERCE Cat. # 78243, incubated at room temperature for 10 minutes, spun at 16,000 for 10 minutes and PBGD activity was determined in the supernatants. Total protein was estimated by the Bradford method using the BioRad reagent Cat # 500-0006 and bovine serum albumin as stsndard. The specific activities in the crude lysates obtained at the three growth temperatures are tabulated below. The results clearly show an increase of PBGD units/mg with increasing temperature in the range from 30°C to 40°C.

| Temperature | PBGD Units/mg |
|---|---|
| 30°C | 363 |

Table continued

| Temperature | PBGD Units/mg |
|---|---|
| 37°C | 573 |
| 42°C | 1080 |

A bacterial promoter for example the tryptophane (trp) promoter or the lac promoter or alternatively an alkaline phosphatase promoter, should be inserted before the PBGD coding region for efficient transcription in prokaryotes for example E. coli.

The E. coli cell containing plasmid (pExp1 or pExp1-M2 Puc-BB), may be fermented to stationary phase between 24-48 hours, in a medium containing casein hydrolysate, or yeast extract, glucose, vitamins and salts. pH oxygen may be monitored by electrodes during fermentation. Temperature will be kept at 37 +/- 2 C during the fermentation.

Fermentation and Purification

rhPBGD may be recovered from E. coli after fermentation by an extraction procedure involving for example ribipress, sonication, osmotic shock or total solubilization by detergent for example Tween 80, Triton X-100 or Brij. rhPBGD will be recovered from fermentation medium after production in yeast or from a total cellular extract using detergents such as Triton X-100, Tween 80 or Brij. rhPBGD will be recovered from mammalian culture medium or from a total cellular extract by ion-exchange chromatography or affinity chromatography.

rhPBGD may be purified from E. coli extract or from yeast medium or total cellular extract or from mammalian culture medium or total mammalian cellular extract by binding to an ion-exchange column for example DEAE-Sepharose or MonoQ-Sepharose and eluted with for example NaCl and Sodium phosphate buffer pH 7-8 or the corresponding potassium salts.

Alternatively, rhPBGD may be recovered from extracts by binding to an affinity chromatography column for example an anti-PBGD affinity column. rhPBGD will be eluted by lowering the pH to 4-2, or a thiol specific affinity column. rhPBGD has been "tagged" with thiols residues when a thiol affinity column step is used. Thiols will be removed by a specific enzymatic cleavage step to generate authentic rhPBGD.

The ion-exchange or affinity purified rhPBGD will be further purified by hydrophobic interaction chromatography on for example, TSK Phenyl 5 PW column or Octyl-Sepharose or Phenyl-Sepharose columns.

Binding of rhPBGD may be done at high ionic strength for example in 10-50 mM Tris-HCl pH 7-8, 1M NaCl or 10-15 mM Sodium phosphate pH 7-8, 0.5 M $MgSO_4$ and eluted by lowering the ionic strength for example with 10-50 mM Tris-HCl pH 7-8 or 10-50 mM Sodium phosphate pH 7-8.

Three hydrophobic interaction steps will be applied consecutively.

rhPBGD is further purified with preparative RP-HPLC for example C12 or C18 matrixes. The rhPBGD is be eluted from the column by a gradient of 10-50 mM Sodium phosphate and 1-10% acetonitrile buffer.

Formulation of rhPBGD is done by passing the enzyme over a G-100 Sephadex column and eluting it in an isotonic solution for example 0.9%NaCl and 10-50mM Sodium phosphate pH7.0 +/- 0.5 or Sodium phosphate, glycin, mannitol or the corresponding potassium salts.

For the preparation of a medicament, the formulation solution of rhPBGD may be sterile filtered and filled aseptically in glass vials and lyophilised.

Alternatively, the sterile filtered rhPBGD solution is formulated in for example, lipid vesicles constituting phosphatidylcholine or phosphatidylethanolamine or combinations of these or incorporated into erythrocyte ghosts.

Reconstitution of lyophilised rhPBGD may be done in water for injection.

Alternatively, rhPBGD is formulated in a sustained release formulation involving a biodegradable microspheres, for example in polylactic acid, polyglycolic acid or mixtures of these.

Alternatively, rhPBGD is lyophilized in a two-compartment cartridge, where rhPBGD will be in the front compartment and water for reconstitution in the rear compartment. This two compartment cartridge may be combined with an injection device to administer either rhPBGD by a needle or needle less (by high pressure) device.

Alternatively, rhPBGD may be formulated in a physiological buffer containing an enhancer for nasal administration.

Alternatively, rhPBGD is formulated in an oral formulation containing for example, lipid vesicles (phospatidylcholine, phosphatidylethanolamine, sphingomyeline) or dextrane microspheres.

Although recombinant production of PBGD is preferred for the treatment of AIP, it can alternatively be produced from human red blood cells.

A general production and manufacturing of recombinant PBGD may be done by the following steps.

Recombinant PBGD production process; an outline

A: Fermentation

1. Master cell bank
2. Working cell bank
3. Production of seed culture
4. Fermentation in large fermenter (250 L >)

B. Purification

1. Cell concentration by filtration/centrifugation
2. Cell disruption
3. Ultrafiltration
4. Chromatography ion exchange, DEAE-Sepharose, MonoQ-Sepharose
5. Hydrophobic interaction chromatography (Octyl/phenyl-Sepharose, TSK Phenyl, SPW, Phenyl -Sepharose
6. Chromatography ion exchange (MonoQ)
7. Formulation by Gel filtration Sephadex G-100

C. Manufacturing

1. Sterile filtration
2. Aseptic filling
3. Lyophilization

EXAMPLE 1

Fermentation of recombinant human Porphobilinogen Deaminase (rhPBGD)

Strain PBGD-1 is an E. *coli* K12 host strain JM105 genotype endA thi rpsL sbcB15 hsdR4 △(lac-proAB) [F'traD36 proAB lacI$^q$ △(lacZ)M15] containing the expression plasmid pExp1-M2-BB. Strain PBGD-2 has the same expression plasmid pExp1-M2-BB but the host cell is deleted for the hemC gene to facilitate rhPBGD purification. Since the strain PBGD-2 was not ready at the start of the study, the decision was made to start the study with strain PBGD-1. Both the strains are resistant against both tetracycline and ampicillin, but due to regulatory advantages it was decided to use oxytetracycline as selection pressure. To focus the first part of the study on the expression level of rhPBGD, and not the strain stability, it was decided to start the development with selection pressure in the fermenter. When the expression level was satisfactory strain stability without selection pressure in the fermenter should be investigated. Preliminary tests performed showed that the expression level of rhPBGD was 1,5 times higher at 37°C compared to the expression at 30°C. At 42°C it was as much as 3 times higher. Based on this knowledge one would suggest a temperature induction to either 37°C or 42°C during the fermentation to boost the rhPBGD production. However, at higher fermentation temperatures the strain stability might be a problem. The time frame was too narrow to study the rhPBGD expression at all three temperatures, so the decision was to start the study without temperature induction and to keep the temperature at 30°C during the whole process.

Short description of the work

During the first two months the strain PBGD-1 was cultivated on agar plates and in shake flasks to obtain information about the strain characteristics. In parallel to this purchase of study dedicated chemicals, build up of the documentation system and technology transfer of the analytical methods took place. When the PBGD-1 intermediary cell bank was prepared the actual fermentation work started. First two "simple" 1-L batch fermentations of strain PBGD-1 were used to test the newly designed substrate and to calculate the maximum growth rate for the strain. After that three 10 L fed batch fermentations of strain PBGD-1was performed.

As soon as the strain PBGD-2 was avaible and an intermediary cell bank was prepared, this strain was implemented in the fermentation procedure developed for strain PBGD-1. At present two 10-L fermentations of strain PBGD-2 have been performed.

The general outline of the fermentation is starting with inoculum preparation on M9-tc agar plates and shake flasks. The

cells are incubated at 30°C for 24 h on M9-tc agar plates and are then transferred to M9-tc shake flasks. The shake flasks are incubated for 12-14 h at 30°C. The broth from 1-2 shake flasks are used to inoculate the 10 L fermenter containing a minimal medium supplemented with yeast extract, trace elements, thiamine and oxytetracycline as selection pressure. The fermentation starts with a 14-h batch phase where the cells grow at maximum growth rate. The glucose feed is started after 14 h and the feed rate profile is varied between 25-75 ml/h of a 600 $gl^{-1}$ glucose solution.

Broth taken from shake flasks and fermentations have been used to develop the down stream processing and to test and adjust the analytical methods provided. The general outline in the down stream processing is concentration of the fermentation broth on a 0,22 μm cross flow membrane followed by diafiltration (washing) with a buffer to exchange 90-95 % of the substrate with buffer. The diafiltered cell concentrate is homogenised in a homogeniser, where the pressure has been varied between 600 - 1000 bars. The cell debris is then removed from the homogenate either by filtration on the same membrane as mentioned above or by centrifugation. Finally the extract is sterile filtered into sterile containers.

Results

Fermentation

The maximum growth rate for strain PBGD-1 was determined in shake flask experiments and in 1-L batch fermentations. The results are summarized in Table 4 below. The reason for the lower values in the shake flask with fermenter medium is probably acetic acid production and hence lower pH since the pH is not controlled. No experiments have been performed to calculate the maximum growth rate of strain PBGD-2, but from the fact that the batch phase has the same duration as for PBGD-1 we can draw the conclusion that the maximum growth rate is approximately the same.

Table 4 Maximum growth rates

| Conditions | Maximum growth rate ($\mu_{max}$) [$h^{-1}$] |
|---|---|
| M9-tc Shake flask | 0,3 |
| Shake flask with fermenter substrate | 0,3 |
| 1 L Fermenter with pH controlled at 7,0 | 0,4 |

The developed substrate for the fermentation is given in Table 5 on the next page. When implementing strain PBGD-2 it seems like this strain has different requirements on either the amount of yeast extract or the thiamine concentration in the substrate. When using the substrate developed for strain PBGD-1 the growth stops or lags during the fermentation (PD14). When adding extra yeast extract and thiamine the growth starts again. This pattern is repeated at least two times during the fermentation.

Table 5 Fermenter substrate

| Component | Mw [g/mol] | Concentration | Unit |
|---|---|---|---|
| $(NH_4)_2SO_4$, | 114,12 | 2,70 | [g/l] |
| $KH_2PO_4$ | 136,08 | 3,25 | [g/l] |
| $K_2HPO_4$*$3H_2O$ | 228,23 | 2,80 | [g/l] |
| $C_6H_5Na_3O_7$*$2H_2O$ | 258,07 | 0,60 | [g/l] |
| Yeast extract | | 5,00 - 20,0 | [g/l] |
| $C_6H_{12}O_6$*$H_2O$ | 198,17 | 10,00 | [g/l] |
| $MgSO_4$*$7H_2O$ | 246,50 | 1,07 | [g/l] |
| Thiamine chloride $C_{12}H_{18}Cl_2N_4OS$*$xH_2O$ | | 1,00 -10,0 | [mg/l] |
| $H_3BO_3$ | 61,83 | 2,1 | [mg/l] |
| $CuSO_4$*$5H_2O$ | 249,70 | 10,5 | [mg/l] |
| $FeCl_3.6H_2O$ | 270,30 | 35,5 | [mg/l] |

Table continued

| Component | Mw [g/mol] | Concentration | Unit |
|---|---|---|---|
| $MnSO_4*H_2O$ | 169,02 | 6,6 | [mg/l] |
| $ZnSO_4*7H_20$ | 287,50 | 5,3 | [mg/l] |
| $CoCl_2*6H_2O$ | 237,93 | 9,3 | [mg/l] |
| $CaCl_2*2H_2O$ | 147,02 | 14,0 | [mg/l] |
| $Na_2MoO_4*2H_2O$ | 241,95 | 9,3 | [mg/l] |
| HCl | 34,46 | 6,9 | [ml/l] |
| Oxytetracycline $C_{22}H_{24}N_2O_9*HCl$ | 496,90 | 6,0 | [mg/l] |

The strains seem to utilise different components in the yeast extract in a sequential order. The metabolism and respiration is different for different compounds. This gives rise to an irregular fermentation pattern with large changes in the respiration of the population during the fermentation, e.g. the $CO_2$ and the $O_2$ outlet gas analysis and the dissolved oxygen tension (DOT) signal (see Figure 12).

As the fermentation proceeds, the fermentation broth is gradually coloured bright pink. When centrifuging broth for dry weight analysis it is observed that it is the actual cells and not the supernatant that is pink. The colonies on the M9-tc agar plates used to inoculate the shake flasks are not coloured pink, they are rather yellow or white like "normal" *E.coli* cells.

The colonies on the agar plates used for the colony forming units (CFU) analysis from the fermentation are also pink. However, on the CFU plates from PD14, the first fermentation with the new strain PBGD-2, a small portion of yellow or white colonies was observed. This observation was made already from the plates spread with broth from the inoculum shake flask. The percentage of yellow-white cells was varying in the range 2-8 % during the fermentation. Both the white and red colonies were resistant against the antibiotic oxytetracycline. When observing the white and red colonies in the microscope they both appeared as *E.coli* rod like cells. It was hard to see any clear difference, but possibly the white cells were a little bit shorter than the red ones. To investigate this further shake flask cultivation were started with one red and one yellow colony. The CFU analysis showed that there were only red colonies from the shake flask inoculated with the red colony, but that the white colony gave rise to approximately 70% white and 30% red cells. The rhPBGD activity and protein concentration were measured in the broth from these shake flasks. The results are shown in Table 6 below. The difference in the protein concentration and the rhPBGD activity is in accordance with the difference in the $OD_{620}$ reached in the shake flask, probably due to different size of the inoculum colony.

Table 6 rhPBGD activity and total protein from single colony shake flasks

| Start colony | Protein [mg/ml broth] | PBGD activity [U/L broth] | Specific activity [U/mg protein] |
|---|---|---|---|
| White | 0,01 | 9 | 0,8 |
| Red | 0,04 | 27 | 0,7 |

In the Table 7 below a summary of the final values of the fermentations are given. The lower $OD_{620}$ and Dw (dry weight) values in fermentation PD12 is a result of the lower amount of glucose that totally was fed into the fermenter in this fermentation (600 ml compared to approximately 850 ml in PD11 and PD14). It is also interesting to notice the very high expression and specific activity of rhPBGD in fermentation PD14 compared to the earlier fermentations.

Table 7 Summary of final fermentation results

| Batch | Strain | Time [h] | $OD_{620}$ | Dw [g/l] | PBGD activity [U/ml broth] | Specific PBGD activity [U/mg protein] |
|---|---|---|---|---|---|---|
| PD11 | PBGD-1 | 27 | 82 | 29 | 7,7 | 2,6 |
| PD12 | PBGD-1 | 31 | 59 | 19 | 15,3 | 1,8 |
| PD14 | PBGD-2 | 30 | 87 | 32 | 39 | 3,1 |

Until now we have achieved the best fermentation results in fermentation PD14.
In the Figures 12 and 13 the fermentation results from this fermentation with the new strain PBGD-2 are shown. After

a 14-h batch phase the glucose feed is started according to a schedule with three step changes in the feed rate. However after 16 h the glucose begins to accumulate in the fermenter due to that something else is limiting the growth more. The glucose feed is then stopped and restarted when the glucose concentration becomes limiting again.

The respiration pattern (i.e. $CO_2$, $O_2$ and DOT signals) indicated that something in the substrate was depleted after 14,5 and 22,3 h and 26,3 h (see Figure 12). When extra yeast extract and thiamine was added to the fermenter growth respiration increased dramatically for a while. There was a steady increase in the $OD_{620}$ and Dw during the whole fermentation and the final values are rather high. The increase in produced amount of rhPBGD correlates very well with the increase in biomass. This is something that has been observed also in the other fermentations. However, in fermentation PD14 there also seems to be a steady increase in the specific activity of the produced rhPBGD. Something that has been much less pronounced in the other fermentations.

Down stream processing

The different broths have been concentrated 1,9 - 6,9 times. The different values reflect problems with clogging of the membrane. This problem can probably be avoided by not concentrating the broth too much. Instead a somewhat longer diafiltration has to be done. The homogenisation has given a good yield of released enzyme compared to sonication. Removal of cell debris is In the laboratory scale rather easily done by centrifugation. For the production scale it would be preferred to use membrane filtration and because of that filtration has been tested. However, so far the transmission of enzyme through the membrane has been low resulting in low yields. This yield may be improved by better controlled filtration parameters or extended diafiltration. Otherwise a separator could be used in the production.

In Table 8 some data from the down stream processing are shown.

Table 8 Summary of down stream results

| | | Sterile filtered extract | | | Yield from broth, % | |
|---|---|---|---|---|---|---|
| Broth | Debris removal by | protein mg/ml | activity U/ml | Spec. activity U/mg protein | protein | U |
| PD11 | Filtration | 2,7 | 5,1 | 1,9 | 30-60 1) | 35-45 1) |
| PD12 | Centrifugation | 31 | 84 | 2,7 | 79 | 120 2) |
| PD14 | Centrifugation | 32 | 92 | 2,9 | 85 | 67 |
| PD14 | Filtration | 3,8 | 11 | 2,8 | 15 | 18 |
| 1) Uncertainties in analysis, because the methods were not fully evaluated at this time. | | | | | | |
| 2) Uncertainty in volume because of a tube leakage. | | | | | | |

Conclusions

Strain PBGD-2 has a maximum growth rate of approximately 0,4 $h^{-1}$ in the fermenter substrate. This is similar to the maximum growth rate of strain PBGD-1, however the substrate requirement seems to be different for strain PBGD-2. An increase of the initial yeast extract and thiamine concentration in the substrate to 20 $gl^{-1}$ and 10 $mgl^{-1}$ respectively supports growth to a biomass similar to those achieved with the old strain PBGD-1.

The general fermentation process outline is a 14 h batch phase followed by a 16 h feed phase were the glucose feed rate is increased in three steps.

The production of rhPBGD correlates very well with the biomass production and the specific activity of the rhPBGD also seems to increase during the fermentation.

The best result so far with strain PBGD-2 is a rhPBGD concentration of 39 U/ml and a specific activity of 3,1 U/mg protein after 30-hour fermentation. The final dry weight and $OD_{620}$was 32 $gl^{-1}$ and 87 respectively. The plasmid stability is good during the fermentation when oxytetracycline is present as selection pressure.

EXAMPLE 2

Development of a purification process for recombinant human Porphobilinogen Deaminase (rhPBGD)

Introduction

For the capture step a weak anion exchange (DEAE-Sepharose FF) matrix has been tested primarily, because this has been the most common initial step for purification of PBGD. The disadvantage with anion exchange is that

endotoxins and DNA adsorb on this type of gel. There is a risk that these impurities are coeluted with rhPBGD. To use a cation exchange in this project is not possible, because pI for rhPBGD is too low. For that reason a hydrophobic gel has also been tested as a capture step.

Material and methods

Cell extract

Cell extract (PD12, see Example 1) was supplied frozen (8x50 ml) from Biogaia. After the initial thawing a precipitation was found in the sample. The extract was centrifuged and the next day a new precipitation was found. This means that the extract has to be centrifuged in connection to a chromatography experiment. The protein content in the extract (BCA) was estimated to 29 mg/ml and the enzyme activity was found to be 63 U/ml. The pH and conductivity were estimated to 7.0 and 6 mS/cm, respectively.

Ion-Exchange

A DEAE- Sepharose FF hitrap (1 ml) column was used. The gel was equilibrated with Tris-HCl 25 mM, pH 8.5. The pH of the extract was adjusted to pH 8.5 with NaOH 5 M and the sample volume applied on the gel was 1.4 or 2.0 ml. After the sample has been applied, the gel was washed with 15 column volumes with equilibration buffer. For the desorption of the gel the following KCl concentrations have been tested: 40, 120, 150 and 300 mM. Finally, after every experiment the gel was cleaned with NaOH 1 M.

Hydrophobic interaction chromatography

A Butyl-Sepharose 4 FF hitrap (1 ml) column was used. The gel was equilibrated with potassium phosphate 1.0-1.3 M pH 7.5. To the extract, potassium phosphate (2.5 M) was added to an end concentration of 1.0-1.3 M and the sample volume applied on the gel was 2.0 ml. After the sample has been applied, the gel was washed with 15 column volumes with equilibration buffer. For the desorption of the gel 500 mM, 20 mM potassium phosphate and water were tested. Finally, after every experiment the gel was cleaned with NaOH 1 M.

Results

Ion-exchange

In Figures 14 and 15 chromatograms from two DEAE runs are shown. In Table 9 the results from these runs are shown. The difference between these experiments are that peak b in the first run was desorbed with 120 mM KCl and 150 mM in the second. Further, in the first run less sample was applied and the gel was also desorbed with 300 mM KCl. The recovery was in the best experiment found to be 75 % and the yield 47 %. To get this recovery and yield 300 mM KCl has to be used. The purity of rhPBGD in peak b (DEAE2) was estimated to 31 % (RPC).

Hydrophobic interaction chromatography

In Figure 16 a chromatogram from a Butyl run is shown. In Table 10 the result from the run is shown. In this experiment 1.3 M potassium phosphate was used and the desorption was done with water. Conductivity in peak b was found to be 60 mS/cm. The recovery was calculated to 78 % and the yield 75 %. In an investigation it was found that a precipitation was formed in the extract at a potassium phosphate concentration of 1.5 M. The purity of rhPBGD in peak b was estimated to 40 % (RPC).

Comments and conclusions

From the results of the experiments it can be seen that the mass balance in all experiments are not in balance. This seems to be valid for all analyses. The main reasons for this are probably insecurity of the analyses and that all proteins are not eluted from the gel. The first reason is confirmed by the enzyme activity that seems to be too high in the extract when high concentration of potassium phosphate is added. The second reason is confirmed by the elution peak with NaOH in ion-exchange experiments. This peak is not analyzed. For the hydrophobic matrix a cleaning with organic solution can be necessary. The conclusion of the results so far is that the Butyl-Sepharose 4 FF seems to be the best alternative for the capture step. The main reason for that is the higher yield of rhPBGD. Another advantage to use Butyl-Sepharose 4 FF is the small peak after cleaning with NaOH 1M compared with the large peak in DEAE-Sepharose FF runs. This probably means that few impurities stick on Butyl matrix. On the other hand there is a risk that a precipitation is formed when adding potassium phosphate. A desalting before the next chromatography step can be necessary, caused by the high ion strength in the product peak.

Table 9 Ion-exchange

| Exp. | Applied | | | Peak a | | | Peak b | | | Peak c | | |
|---|---|---|---|---|---|---|---|---|---|---|---|---|
| | BCA | A280 | Act. | BCA | A280 | Act. | BCA | A280 | Act. | BCA | A280 | Act. |
| | mg | mg | U | mg | mg | U | mg | mg | U | mg | mg | U |
| DEAE1 | 37 | 166 | 76 | 10 | 122 | 21 | 5 | 6 | 29 | 9 | 11 | 7 |
| DEAE2 | 42 | 229 | 129 | 17 | 143 | 56 | 10 | 14 | 25 | - | - | - |

Table 10 Hydrophobic interaction chromatography

| Exp. | Applied | | | Peak a | | | Peak b | | | Peak c | | |
|---|---|---|---|---|---|---|---|---|---|---|---|---|
| | BCA | A280 | Act. | BCA | A280 | Act. | BCA | A280 | Act. | BCA | A280 | Act. |
| | mg | mg | U | mg | mg | U | mg | mg | U | mg | mg | U |
| Butyl | 31 | 137 | 93 | 7 | 84 | 3 | 11 | 23 | 70 | - | - | - |

EXAMPLE 3

Development of a method for the purification of recombinant human Porphobilinogen deaminase with a "His-Tag" (rhPBGD-His)

Nature and purpose of the study

Many groups have reported in the literature on the purification of porphobilinogen deaminase from various sources including *E. coli* and Human erythrocytes (Anderson P. M. and R. J. Desnick, 1979, The Journal of Biological Chemistry 255(5): 1993-99, Awan S.J. et al. 1997, Biochemistry 36(30): 9273-82, Grandchamp B. et al. 1987, Eur.J.Biochem. 162(1): 105-10, Jordan P.M. 1994, Wiley, Chichester (Ciba Found Symp 180), p70-96, Jordan P.M. et al. 1988, Biochhem.J. 254:427-435, Lambert R. et al. Wiley, Chichester (Ciba Found Symp 180), p97-110, Louie G.V. et al. 1996, Proteins 25(1): 48-78, Maniatis T., E.F. Fritsch, J. Sambrook. Molecular Cloning (A laboratory Manual) Cold Spring Harbor Laboratory. 1982, Miyagi K. et al. 1979, Proc.Natl.Acad.Sci. 76(12):6172-76, Racich N. 1986, Nucleic Acids Research 14(15): 5955-67, Shoolingin-Jordan P.M. et al. 1997, Methods in Enzymology, 281:317-327). Most use a combination of ion exchange, hydrophobic interaction and size exclusion chromatography to obtain fairly pure protein

preparations. With the engineering of 5 additional Histidine residues on the C-terminus of recombinant human porphobilinogen deaminase, rhPBGD we have a convenient "Tag" to help with purification. Histidine has an affinity to electropositive transition metals such as nickel, copper, zinc and cobalt. When a series of 6 or more electron-rich histidine residues are expressed on the end of a protein they can function as an anchor, firmly attaching the protein to a solid support coated with metal ions. Very thorough washing can be done without dislodging the bound moiety. Elution can be accomplished in one of two ways, either by decreasing the pH to protonate the imidizole nitrogen (pKa of 5.97) of histidine, or by including imidizole, a molecule identical to the histidine side chain, in the elution buffer which competitively dislodges the tagged protein off the support. The purpose of this study is to obtain pure rhPBGD-His for antibody production and for use as a standard in assays and protein purification.

Study objectives

The objective of this study is to obtain 10 mg of highly pure active rhPBGD-His.

Study Plan

Plan outline

Optimize induction time for the expression system and lysis
Purify 10 mg of rhPBGD-His for antibody production and standard
2 liter scale induction and lysis of strain
DEAE ion exchange chromatography
Immobilized metal affinity chromatography
Characterization of rhPBGD-His
SDS-PAGE
Amino acid analysis
Specific activity
HPLC
Mass spectrometry
Amino terminal sequencing

Plan body

Expression of rhPBGD-His is regulated by the bacterial Taq promoter, a derivative of the lac promoter which is inducible with IPTG (See Figure 17 for plasmid map). Different proteins are produced at different rates in *E. coli* upon induction. This necessitates the optimization of the time required for optimum rhPBGD-His yield upon induction. To accomplish this a culture in mid-log phase will be induced with an excess of IPTG and expression followed at timepoints with activity and protein concentration measurements. After induction the cells must be lysed to release rhPBGD-His. Of the options available, sonication is the best for this scale of purification. It is compatible with any buffer system and should not be damaging to the protein. To follow efficiency of lysis, absorbance at 600nm will be measured after each cycle. For use as a standard and for antibody production at least 10 mg of rhPBGD-His will be purified.
For protein purification a 2 liter flask culture of the strain producing rhPBGD-His is sufficient. The culture will be inoculated with a fresh over-night culture of cells and grown to mid log phase then induced with IPTG.
Plans are to utilize a two step purification process. After lysis the debris will be removed by centrifugation and supernatant loaded onto a DEAE ion exchange column. This will remove the vast majority of contaminants from the lysate and leave a limited number of protein contaminants in the elution fractions containing rhPBGD-His. Protein will be loaded in a high pH and low ionic strength buffer to ensure binding of the weakly charged rhPBGD-His. Extensive washing will be used to remove material that is not firmly bound to the column. A very shallow step gradient of KCl will be used to elute rhPBGD-His. This should separate the different forms of rhPBGD-His with differing charge properties from each other. Separation of different charged forms of PBGD, by ion exchange chromatography, has beenreported by others (Anderson P. M. and R. J. Desnick, 1979, The Journal of Biological Chemistry 255(5): 1993-99, Jordan P.M. et al. 1988, Blochhem.J. 254:427-435, Miyagi K. et al. 1979, Proc.Natl.Acad.Sci. USA 76(12):6172-76).
The second chromatographic step planned is a column containing Talon fast flow immobilized cobalt metal affinity resin (Clontech). This makes use of the 6-residue histidine tract at the amino terminus of the recombinant protein. Initially, a metal chelating resin (Pharmacia) charged with nickel (Sigma) was tried for purification of rhPBGD-His but it was found to bind other proteins in the lysate as well which coeluted with rhPBGD-His. Although this cobalt resin has less binding affinity to His tagged proteins than nickel it retains the high binding capacity and is more discriminating which proteins

to bind. This leads to elution from the metal with a lower concentration of imidizole or with higher pH and achieves a higher level of purity. The cobalt is also bound more tightly to the matrix by a tetradentate metal chelator, effectively eliminating the leaching of metal ions from the solid support during purification. The loss of reactive metal ions during elution is common problem with nickel based affinity columns (personal communications) which can lead to unwanted precipitation of purified proteins.
rhPBGD-His will be characterized by the following methods:
The first measure of protein purity will be by SDS-PAGE (polyacrylamide gel electrophoresis). This method will also give an indication of the molecular weight of the protein being produced.
To determine the specific activity of rhPBGD-His in the preparation it is first necessary to accurately determine protein concentration in solution. Amino acid analysis will be used as an accurate method. The method also provides the amino acid composition of the protein. The concentration can be used to establish an extinction coefficient of rhPBGD-His. Activity of the enzyme is an important measure of correct structure of the enzyme. The proper structure, equivalent to that produced in humans, is essential for rhPBGD-His to be used as a therapeutic. Any deviation from the natural structure can cause activation of the patient's immune system. Historically, activity of porphobilinogen deaminase has been measured in one of two ways, either by the metabolism of porphobilinogen substrate or by the formation of preuroporphyrinogen product. In the reaction catalyzed by PBGD, porphobilinogen monomers are covalently attached one at a time starting from the free alpha position of the dipyrromethane cofactor. After four molecules are added the linear tetramer of PBG, preuroporphyrinogen, is spontaneously released by hydrolysis from the cofactor, regenerating the active holoenzyme with covalently attached cofactor for further reactions (See Figure 18). After release the tetrapyrrole is circularized by the next enzyme of the heme pathway, uroporphyrinogen III synthase, forming uroporphyrinogen III, the central ring of heme and vitamin B12 in animals and chlorophyll in plants. The linear preuroporphyrinogen molecule can instead be oxidized to uroporphyrin with benzoquinone creating a molecule, which absorbs light at 405 nm. This is the basis for the activity assay used to measure PBGD activity
The rhPBGD-His preparation will be further characterized by mass spectrometry, which will give an accurate measure of rhPBGD-His molecular weight and potentially identify molecular heterogeneity in the preparation. rhPBGD-His can exist with 1,2,3 and 4 substrate molecules bound to it. Each substrate molecule added to the holoenzyme will add roughly 209 daltons to the mass, which is detectable through mass spectrometry. Characterization by reversed phase HPLC will provide purity data.
Amino terminal sequencing of rhPBGD-His will be used to ensure the correct amino terminus.

Materials and Methods

Induction and Lysis:

From a freshly streaked colony, a culture of pExp-2 in JM105 was grown for 13.5 hours in 100 ml LB (10g/l) bacto-tryptone, 5g/l bacto-yeast extract, 10 g/l NaCl pH 7.0) + 100 μg/ml ampicillin in a 500 ml baffled flask at 37°C at 350 rpm. The optical density measured at 600nm reached 1.6. This culture was used to inoculate 2 liters of terrific broth (12g/l bacto-tryptone, 24g/l bacto-yeast extract, 4ml/l glycerol, 2.31g/l KH2PO4, 12.54g/l $K_2HPO_4$ (Maniatis T., E.F. Fritsch, J. Sambrook. Molecular Cloning (A laboratory Manual) Cold Spring Harbor Laboratory. 1982) with 100 μg/ml ampicillin and split into four 2 liter baffled flasks with 400 ml each and two 1 liter baffled flasks with 200 ml culture each. These were grown at 37°C with 350 rpm in a New Brunswick Scientific Innova 4000 incubator. When reaching an optical density of 0.7 at 600 nm the Taq promoter was induced with 4 mM IPTG, causing rhPBGD-His protein to be made. Growth was followed by hourly readings of absorbance at 600 nm. After 9 hours the cultures were stopped by chilling to 0°C after an absorbance of 1.93 was reached. The culture was centrifuged, 4 X 250ml at a time, for 10 min at 4,000xg in a Beckman Avanti J25I centrifuge with a JLA-16.250 rotor. Supernatant was decanted and the remainder of the culture was added to the cell pellets and spun for an additional 10 min. The pellets were resuspended in 2 pools of 250 ml 50 mM Tris/HCl pH 8.5 (prechilled) each and stored for 8 hours on ice. Cells were centrifuged for 10 min at 4,000xg, liquid decanted and resulting pellets weighed in the bottles to determine the wet weights. Cells were then resuspended in 400 ml ice cold 50 mM Tris/HCl pH 8.5 and lysed by sonication with a Branson Sonifier 450 with 1/2 inch diameter stepped, tapped horn. Each round was for 30 seconds at maximal power with constant duty cycle in a Pyrex 150 ml glass beaker on ice. Lysate was mixed between cycles by either drawing into a 50ml pipet a few times or by pouring between beakers on ice. Progress of lysis during sonication was ascertained by reading absorbance of the lysate at 600 nm.After six rounds of sonication for each of the 100 ml aliquots of cells, debris was removed by centrifuging at 16,000xg for 30 minutes at 4°C. Lysate was then pooled and vacuum filtered through a 0.22 μm Durapore membrane (Millipore) to remove any remaining particulate matter.

DEAE Sepharose Chromatography:

The first chromatographic step in purifying rhPBGD-His was by ion exchange chromatography on a DEAE Sepharose fast flow column (Pharmacia). A 2.5 X 50 cm Spectrum LC column with degassed resin was washed extensively with degassed 25 mM Tris/HCl pH 8.5 buffer. Filtered lysate (380 ml) was applied to the column at 5 ml/min.

The column was then washed with 720 ml 25 mM Tris/HCl pH 8.5. Elution of bound rhPBGD-His was with a shallow step gradient of KCl from 50 to 120 mM in 10 mM increments in 25 mM Tris/HCl pH 8.5 and degassed. Volumes for each step varied from between 105 and 470 ml depending on the elution profile (see Table 11).

Table 11

| | | | | | | | | |
|---|---|---|---|---|---|---|---|---|
| 0 mM KCl | 470 | 60 | 70 | 80 | 90 | 100 | 110 | 120 |
| 720 ML | 50 | 120 | 175 | 270 | 105 | 130 | 180 | 300 |

Fractions were collected about every 50 ml. Absorbance at 280 nm was followed closely during elution The next step was only applied after the absorbance had declined following a peak. BioRad's protein assay II in microtiter format was used per manufacturer's protocol to assay the amount of protein in each fraction. Coomassie stained 10% acrylamide Bis/Tris gels (Novex) were then prepared, with 5 μg protein in each lane, to characterize the purity of each peak.

Cobalt Affinity Chromatography:

The resin slurry was degassed prior to pouring into a 2.5 × 30 cm Spectrum LC column. It was then washed extensively with degassed 25 mM Tris/HCl pH 8.5/ 150 mM NaCl at a flow rate of 5 ml/min. The sodium chloride was included to decrease protein to protein ionic interactions and to reduce ion exchange effects with the column matrix itself. A relatively high pH of 8.5 was used to keep rhPBGD-His well above the pI, and therefore negatively charged, to maintain high solubility during the purification. Two consecutive rhPBGD-His affinity purifications were then run on the column. The first sample loaded was a sterile filtered pool of the entire first peak of eluate of activity from the DEAE sepharose column including fractions 9 through 12. The column was then washed with 2 liters of 25mM Tris pH8.5/150mM NaCl at 3 ml/min. To elute bound contaminants the column was then washed with 100 ml of 25mM Tris pH8.5/150mM NaCl/5mM imidizole at 5 ml/min followed by 100 ml of 10mM imidizole buffer solution. Elution of his tagged protein was with 25mM Tris pH 8.5/150mM NaCl/50mM imidizole at 5 ml/min. A final elution with 100 mM imidizole was included to be certain all rhPBGD-His was eluted. To prepare the column for the second loading it was merely washed with ~100 ml of 25mM Tris pH8.5/150mM NaCl. It was hoped that rhPBGD-His would displace the imidizole bound to the column (which turned out to be the case). The second loading of the column was with a sterile filtered pool with ~900 ml of all remaining peaks of activity from the DEAE Sepharose column at a flow rate of 5 ml/min. The column was then washed with 2 liters of 25mM Tris pH8.5/150mM NaCl at 5 ml/min, followed by imidizole containing buffers as with the first run above.

Polyacrylamide Gel Electrophoresis: (SDS-PAGE)

Gel electrophoresis was with the Novex system with Nupage 10% Bis/Tris gels run at 125V for 2 hours with or without reducing agent. Staining was with 50% methanol / 10% acetic acid / 0.25% Coomassie brilliant blue R-250 for 2 to 4 hours. Destaining was in 30% methanol / 10% acetic acid in a Bio-Rad gel destainer.

Amino Acid Analysis:

Amino acid analysis was performed by AAA Laboratory (6206 $89^{th}$ Avenue Southeast, Mercer Island, Washington 98040). rhPBGD-His was hydrolyzed for 20 hours with 6N-HCl /0.05% mercaptoethanol / 0.02% phenol at 115°C. Serine was increased by 10% and Threonine increased by 5% to compensate for destruction of the individual acids during hydrolysis. A Beckman 7300 Amino Acid Analyzer was used coupled with System Gold software. Analysis was performed by post-column derivitization with ninhydrin using the ion-exchange chromatographic methods developed by Moore and Stein.

PBGD Activity Assay:

We performed assays in 96 well microtiter format with validation in cuvets. Procedures were derived from published procedures (Awan S.J. et al. 1997, Biochemistry 36(30): 9273-82, Shoolingin-Iordan P.M. et al. 1997, Methods

in Enzymology, 281:317-327). From 0.125 to 8 μg of purified rhPBGD-His protein per well have been used to determine enzymatic activity. Assay buffer is 50 mM Tris/HCl pH 8.2 with 1.0 mg/ml BSA (Sigma fraction 5) and 10 mM DTT. A Perkin Elmer 9700 PCR machine was used for thermal regulation, allowing for tight control of the temperature and reaction time. Assays have been started in two ways. One method was to start the reactions at 37°C with prewarmed substrate in a PCR block. Strategic placement of pauses in a thermocycle program was used with beeping at defined intervals for both addition of the substrate and for stopping the reaction. An example cycle program is shown in Table 12 with reaction times varying from 10, 20, 40 and 60 minutes.

The reaction block is a 96 well block with tubes arranged in an 8x12 matrix.
It is kept throughout at 37C. The reaction is initiated by adding PBG to eight tubes in the first row using an eight-channel pipettor. The addition is staggered so that each row receives PBG every 30 seconds. A ten second pause and beep interval is setup every 20 seconds to signal each addition at the end of the period. In this fashion all the 96 reactions are started which takes a total of six minutes. At the end of a further four-minute incubation, the first three rows are stopped in a staggered manner giving a total of a ten-minute incubation period. This procedure is repeated for the next three rows after an additional ten minutes amounting to a total of twenty-minute reaction time. This scheme is illustrated in Table 12. The p@37 represents the 10-second beep period which is configured in the thermocyclor as a pause plus beep interval.

Table 12

| start add PBG | | | stop 10 min | | | stop 20 min | | | stop 40 min | | | stop 60 min | |
|---|---|---|---|---|---|---|---|---|---|---|---|---|---|
| \|-----12X------\| | | | \|------3X------\| | | | \|------3X------\| | | | \|------3X------\| | | | \|-----3X------\| | |
| 37 20 sec | p@37 10 sec | 37 4 min | 37 20 sec | p@37 10 sec | 37 10 min | 37 20 sec | p@37 10 sec | 37 20 min | 37 20 sec | p@37 10 sec | 37 20 min | 37 20 sec | p@37 10 sec |

Reactions were stopped by acidification with HCl / p-benzoquinone solution. The final concentration of HCl used was 1 molar. Benzoquinone, which oxidizes the uroporphyrinogen to uroporphyrin, was used at a final concentration of 0.002% w/v (from 0.2% stock solution in methanol). At defined intervals the 150 μl samples were removed from the reaction tubes and added to 850 μl HCl / p-benzoquinone solution in wells of a 96 well X 2 ml plate on ice. The second method of initiating the assay was to set up the reactions complete with substrate on ice then to transfer to the PCR block for incubation at 37°C. Following the reaction the block was brought to 4°C to stop the reaction after which samples were removed and added to HCl / p-benzoquinone solution. For both methods the incubation was allowed to proceed for 20 minutes on ice and in the dark after the last addition of reaction solutions. Then the plate was centrifuged for 10 min at 3750 rpm in a swing out rotor in a GS-6KR centrifuge to pellet precipitated protein (mostly BSA). 250 μl was removed to a Corning 96 well assay plate. Absorbance was measured at 405 nm with a 605 nm reference wavelength in a BioTek FL-600 plate reader. Selected samples (normally the standard curve) were diluted 10X with 1M HCl and read in a quartz cuvet in a Beckman DU640B spectrophotometer at 405.5 nm. A 605 nm reference wavelength was used to subtract out background absorbance. These measurements in cuvets produced a conversion factor from 1 cm pathlength reads to the plate data. Analysis was performed using the KC4 software included with the plate reader and with excel spreadsheets. An extinction coefficient of 548 $M^{-1}cm^{-1}$ was used to quantitate the oxidized reaction product (Shoolingin-Jordan P.M. et al. 1997, Methods in Enzymology, 281:317-327).

HPLC:

HPLC analysis was performed at the University of Washington Mass Spectrometry Analysis Facility for HPLC. Samples were prepared free of salts for mass spectrometry analysis by HPLC on a C4 column and eluted with an increasing gradient of acetonitrile. The instrument used was an Applied Biosystems (ABI) 140A Solvent Delivery System with an ABI 785A Programmable Absorbance Detector.

Mass Spectrometry:

Mass spectrometric analysis was performed at the University of Washington Mass Spectrometry Analysis Facility. One tenth of the HPLC run within the main elution peak was diverted prior to the absorbance detector to a Perkin Elmer SCIEX AP13 Biomolecular Mass Analyzer for electro-spray mass spectrometry. Analysis was by HyperMass method on an average of 16 peaks (for Cobalt run #1 eluate).

Amino Terminal Sequencing:

Amino terminal sequence analysis was perfomed at the University of Washington Mass Spectrometry Analysis Facility. An ABI 477A Protein Sequencer was used with an ABI 120A PTH Analyzer.

Results

Purification:

Induction and Lysis:

Growth of the 2-liter culture of bacteria slowed down after the first hour but growth still continued to 9 hrs (see Table 13).

Table 13

| start | 1 hr | 2 hr | 3 hr | 4 hr | 5 hr | 6 hr | 7 hr | 8 hr | 9 hr |
|---|---|---|---|---|---|---|---|---|---|
| 0.699 | 1.300 | 1.521 | 1.607 | 1.660 | 1.732 | 1.797 | 1.841 | 1.890 | 1.927 |

After about 3 hrs of induction cells tended to clump together with most turbidity settling out of the broth by gravity in about an hour. Final density of cells stayed low for growth in a rich media such as terrific broth but final weight of pellets was adequate. The total wet weight was 35.3 g, corresponding to 17.7g/liter culture. Interestingly the cells were orange/pink probably due to various intermediates in the heme biosynthetic pathway. It is clear from the low growth rate and final densities achieved that cultures were limited by the amount of oxygen available.

Lysis by sonication was essentially complete after 5 cycles as seen by following absorbance readings (Table 14).

Table 14

| # rounds | 0 | 1 | 2 | 3 | 4 | 5 | 6 | 7 |
|---|---|---|---|---|---|---|---|---|
| OD600 | 30.33 | 20.15 | 12.90 | 9.43 | 6.14 | 3.86 | 3.31 | 2.99 |
| % down | - | 34 | 36 | 27 | 35 | 37 | 14 | 9.7 |

It appears from the % decrease of optical density that for each of the first 5 rounds of sonication, about the same percentage of cells were lysed. After this the percentage of newly lysed cells dropped rapidly. For each of the first 4 rounds viscosity of the lysate was relatively high due to the presence of unfragmented genomic DNA but this decreased significantly after further rounds from shearing of the DNA into smaller fragments.

DEAE Sepharose:

Elution of proteins from the DEAE ion exchange column occurred in 4 distinct peaks as seen in the elution profile in Figure 19 and by protein assay in Table 15. SDS-PAGE analysis of the eluted fractions shows that these peaks contain four separate peaks of rhPBGD-His eluted with the step gradient of KCl (Figure 20). The first and major peak was eluted in fractions 9 through 13 with 50 to 70 mM KCl. As seen by gel analysis (see Figure 20) purity was fairly good for a first step of the purification, especially in fractions 10 through 13. The second peak eluted in fractions 15 through 18 with 80 mM KCl. The major contaminant in this peak, in about equal molar proportions to desired product, was a protein running at about 5 kDa smaller than rhPBGD-His. The third peak in fractions 20 through 23 eluted with 90 to 100 mM KCl and had less visible contaminants than the second peak. The fourth and final peak eluted in fractions 26 through 29+ with 110 to 120 mM KCl. The fractions were split into 2 pools for further purification. The first pool, comprising the major peak of rhPBGD-His elution contained fractions 9 through 12. The second pool contained fractions

13 through 29 along with the next 50 ml of 120 mM KCl elution buffer. These two pools eluted by ion exchange contained 877 mg protein out of 3253 mg loaded, corresponding to a 3.7 fold decrease in total protein (See Table 15).

Cobalt Affinity:

From the first cobalt run the majority of rhPBGD-His eluted in a sharp peak with a volume of 30 ml upon addition of 50 mM imidizole (see Table 16 for protein assay and Figure 22 for SDS-PAGE results). A final elution with 100 mM imidizole released no detectable protein absorbing at 280nm. In the second cobalt run (Figure 21) surprisingly, the first imidizole wash of 5 mM eluted a small uncolored peak of absorbance with a volume of about 50 ml. The second wash with 10 mM imidizole then eluted a larger and broader orange/pink colored peak of about 150 ml. Further elution with 50 mM imidizole yielded a large sharp uncolored peak of 23 ml.

Characterization:

Amino Acid Analysis:

Amino acid analysis of 3 of the fractions (Cobalt run #1 50 mM imidizole eluate (in duplicate,) Cobalt run #2 10 and 50 mM imidizole eluates) yielded conclusive data that rhPBGD-His was being purified. Results from the analysis allowed for a very accurate measure of protein concentration calculated from the concentration of individual amino acids (see Table 17).

Specific Activity:

Specific activity of the first 50 mM imidizole eluate of rhPBGD-His from the cobalt column turns out to be high at approximately 24 U/mg (Units are in μmol PBG consumed per mg protein in one hour). Activity of rhPBGD-His was found to be strongly dependent on pH with a sharp rise from 7.0 to 8.0 where it approached a plateau. The optimum was around pH 8.2. The optimum PBG substrate concentration was found to be around 1 mM. rhPBGD-His had activity with all concentrations of PBG, however with amounts less than 1 mM the reaction was limited by available substrate, decreasing both the Vmax and the linearity over time as substrate was depleted. It was not found to be necessary to decolorize remaining benzoquinone with sodium metabisulfite as used in a published assay (Shoolingin-Jordan P.M. et al. 1997, Methods in Enzymology, 281:317-327). Strangely enough if acidification and oxidation were done in a smaller total volume (240 μl vs 1 ml) as done by this research group then a highly colored product develops during the incubation on ice. This product must be decolorized with a saturated solution of sodium metabisulfite to obtain accurate reaction absorbances. There was no significant difference in enzymatic activity found as measured by these two variations of the method.

Generally assays have been set up with both time and enzyme concentration as variables. This allows for a more detailed analysis of the results with a built in validation. If activity is fairly linear from different timepoints at any enzyme concentration then it can be inferred that substrate is not limiting and that reaction measurements are valid over that range. If a measurement is taken at only one timepoint then there is no indication of whether the enzyme is still functioning at V-max.

Reaction volumes in 96 well format have been limited by the size of PCR tubes to 150 μl. Volumes from between 50 and 150 μl have been tried with a noticeable increase in linearity over time and with increasing enzyme amounts seen with the larger volumes. Additional increases in volume would make even more substrate available and dilute the protein further, thereby increasing the linearity over time and enzyme concentrations. However the increase in cost of the assay from PBG substrate would be substantial.

For routine analysis of similar protein preparations at similar concentrations it should be possible to standardize the assay and use far fewer data points and still obtain an accurate measure of PBGD activity. Optimally a standard curve of rhPBGD-His of known activity would be included to validate the results and to simplify analysis. Basically multiple variables including time would be internally controlled. With a four-parameter logistic curve of the standards one could use any time point and a wide range of sample concentrations to obtain accurate activity measurements. Single use aliquots of highly pure rhPBGD-His could be stored frozen for use as standards.

Mass Spectrometry:

Mass spectrometry of the 50 mM imidizole elution peaks from the two cobalt runs yielded molecular weights of:

1$^{st}$ cobalt run eluate: 38,816.8, standard deviation = 3.68
2$^{nd}$ cobalt run eluate: 38,814.6, standard deviation = 4.70

1st cobalt eluate dialyzed for antibodies: 38,817.1, STD deviation = 3.33

These weights correspond to the holoenzyme without any additional substrate molecules attached.

Evaluation and conclusions

We found that with a simple two step purification process involving ion exchange and cobalt affinity chromatography we could achieve a yield of 173 mg/l rhPBGD-His with a purity of greater than 98% starting from a bacterial crude lysate. Each one of the enzyme intermediate complexes is stable and can be independently isolated (Anderson P. M. and R. J. Desnick, 1979, The Journal of Biological Chemistry 255(5): 1993-99, Jordan P.M. et al. 1988, Biochhem.J. 254:427-435, Miyagi K. et al. 1979, Proc.Natl.Acad.Sci.USA 76(12):6172-76). This may be a major contributing factor to the differential binding of different enzyme fractions to the DEAE ion exchange matrix. Due to the negative charges contributed by acetate and propionate side groups on the growing chain of porphobilinogen molecules it could be theorized that binding affinity to the ion exchange resin would be in the order; E<ES<ES2<ES3. That would imply that the first peak could be the holoenzyme followed by the others in the same order of the reaction progression. The cobalt column also eluted rhPBGD-His in different fractions during the second run. It is strange that the elution profile from the second run was different from the first. It would be expected that all closely related proteins with a his-tag would bind to cobalt with the same affinity. This implies that either the His-tag is partially digested away or partially obscured due to protein conformational changes or charge interactions. The difference in elution characteristics may also be due to differences between the various enzyme-substrate intermediate complexes as hinted by the color difference in the 10 mM elution peak. From a report in the literature by Jordan P.M. 1994, Wiley, Chichester (Ciba Found Symp 180), p70-96, the ES2 intermediate complex has a pink colored chromophore. The 10 mM imidizole fraction from the second cobalt column run has a pink color while the other fractions do not. This implies a separation of different enzyme substrate intermediates in different fractions. If the colored protein peak is predominantly composed of the ES2 intermediate then it could be extrapolated that the peak at 5 mM would be ES3. Whatever would be decreasing the binding of ES2 to cobalt whether conformational or charge related as compared to ES would likely be enhanced with the ES3 intermediate. The peak released with 50 mM imidizole and with stronger binding to DEAE could then be the ES form. Holoenzyme by itself may be the remaining form, purified during the first cobalt run, binding less tightly to DEAE due to a higher pI and elute from nickel with 50 mM imidizole. When the mass of the two 50 mM cobalt eluates were compared however there was no significant difference detected. Both corresponded to the weight expected for holoenzyme alone. Unfortunately no reliable mass measurement of the 10 mM eluate was obtained due possibly to precipitation problems with a lower rhPBGD-His protein concentration. If a difference of elution characteristics between the different enzyme substrate intermediates is occurring then a likely explanation would be due to the large conformational changes that take place during the course of the reactions From E to ES4 (Jordan P.M. 1994, Wiley, Chichester (Ciba Found Symp 180), p70-96, Louie G.V. et al. 1996, Proteins 25(1): 48-78). The C-terminal His-tag on the third domain of the protein could become partially hidden and rendered sterically less accessible when the reaction proceeds past the ES1 form. A direct interaction between the his-tag and the growing substrate chain would be less likely. At a pH of 8.5 histidines should be in an electron rich unprotonated state and the substrate complex should also be in an electron rich state even though acidic side chains are neutralized by basic amino acids in the catalytic cleft (Jordan P.M. 1994, Wiley, Chichester (Ciba Found Symp 180), p70-96, Louie G.V. et al. 1996, Proteins 25(1): 48-78). Conformational changes in rhPBGD-His occurring during the reaction could conceivably make accessible other charge groups for interaction with the his-tag either on the surface or perhaps the same ones meant for dampening charges from the growing substrate polymer in the cleft.

Equipment and supplies lists are shown in appendix 4 and 5, respectively.

Appendix 4 Equipment list:

| Item | Manufacturer | Serial Number |
|---|---|---|
| Pipetman P-1000 | Gilson | N55287E |
| Pipetman P-200 | Gilson | N52324E |
| Pipetman P-20 | Gilson | N53465M |
| Pipetman P-10 | Gilson | P626586 |
| 5415C centrifuge | Eppendorf | 5415B68381 |

Table continued

| Item | Manufacturer | Serial Number |
|---|---|---|
| GS-6KR centrifuge | Beckman | NGD97J18 |
| Avanti J-25 I centrifuge | Beckman | JJY97J14 |
| DU 640B Spectrophotometer | Beckman | 4323015 |
| Genie II vortex | VWR | 2-241186 |
| GeneAmp PCR system 2400 | Perkin Elmer (PE) / Applied Biosystems (ABI) | 803N6021903 |
| GeneAmp PCR system 2400 | PE / ABI | 803S7100104 |
| GeneAmp PCR system 9700 | PE / ABI | 80557121566 |
| 1545 incubator | VWR | 0902597 |
| BioTek FL-600 plate reader | BioTek | |
| ProTeam LC System 210 | ISCO | |
| Nupage Electrophoresis System | NOVEX | |
| Gel Destainer | BioRad | |
| Power Pac 200 | BioRad | |
| Power Pac 1000 | BioRad | |
| Innova 4000 incubator | New Brunswick Scientific | 890165366 |
| Innova 4000 incubator | New Brunswick Scientific | |
| Power Mac G3 computer | Macintosh | XA8061A3BBW |
| Trinitron Multiscan 200GS monitor | Sony | 8057052 |
| DNA analysis Software: Geneworks | Intelligenetics | Version 2.5.1 |
| Sonifier 450 | Branson | |
| 1/2" diameter stepped disruptor horn | Branson | |
| 2.5 × 50 cm LC column | Spectrum | |
| 1.5 × 30 cm LC column | Spectrum | |

Appendix 5 Supplies List

| Item | Supplier | Cat # | Lot # |
|---|---|---|---|
| Ampicillin | Sigma | A-9518 | 76H0434 |
| Bacto Agar | Difco | 0140-07-4 | 106728JA |
| Tris six-pack "C" | Sigma | T-PAC-C | 77H9049 |
| Trizma Base | Sigma | T-8524 | 28H5436 |
| HCl | Sigma | H-1758 | 37H3495 |
| PBG (5mg) | Sigma | P-1134 | 77H0930 |
| PBG (1mg) | Sigma | P-1134 | 36H1297 |
| BSA (fraction 5) | Sigma | A-6003 | 87H7603 |
| DTT | Sigma | D-9779 | 105H7711 |
| Methanol | Fisher | A452SK-4 | 982215 |

Table continued

| Item | Supplier | Cat # | Lot # |
|---|---|---|---|
| P-Benzoquinone | Acros | 10563-0050 | A011202801 |
| DEAE Sepharose Fast Flow | Pharmacia | 17-0709-01 | 256288 |
| Chelating Sepharose Fast Flow | Pharmacia | 17-0575-01 | 253865 |
| Nickel Sulfate | Sigma | N73-100 | 985482 |
| Talon Superflow Metal Affinity Resin | Clontech | 8908-2 | 8110601 |
| Centricon Plus-80 (Biomax 8) | Millipore | UFC5 BFC 02 | Not available |
| Nupage 10% Bis/Tris gels | Novex | NP0302 | Various |
| Protein Assay Kit II | BioRad | 500-0002 | 59163A,62171A |
| Centricon-10 | Millipore | 4321 | L8PM2042 |
| KCl | Sigma | P-9333 | 68H01001 |
| NaCl | Sigma | S-3014 | 97H1151 |
| Imidizole | Fisher | 03196-500 | 985421 |
| Coming microtiter plate | Fisher | 07-200-89 | Not available |
| Costar 96 well × 2ml plate | Fisher | 097-61-117 | Not available |
| MicroAmp Reaction Tubes | Perkin Elmer | N801-0838 | S18N8-41 |
| MicroAmp Full Plate Cover | Perkin Elmer | N801-0550 | 090397 |
| Spectra/Por 2.1 Biotech DispoDialyzers MWCO: 15k | Spectrum | 135030 | 11987 |

Table 156 Protein assay results on DEAE fractions (with BioRad's Protein assay II kit):

| sample | vol(ml) | mM KCl | protein mg/ml | rhPBGD-His mg/ml | mg prot | gel # | well # | pool | protein mg pool |
|---|---|---|---|---|---|---|---|---|---|
| DEAE Load | 380 | 0 | 8,56 | | 3253 | 1 | 2 | | |
| DEAE FT | 380 | 0 | 1,33 | | 505 | 1 | 3 | | |
| DEAE #1 | 16 | 0 | 0,26 | 0,00 | 4 | 1 | 4 | | |
| 2 | 60 | 0 | 0,23 | 0,00 | 14 | 1 | 5 | | |
| 3 | 100 | 0 | 0,044 | 0,00 | 4 | 1 | 6 | | |
| 4 | 215 | 0 | 0,002 | 0,00 | 0 | 1 | 7 | | |
| 5 | 320 | 0 | 0,005 | 0,00 | 2 | 1 | 8 | | |
| 6 | 75 | 50 | 0,11 | 0,00 | 8 | 1 | 9 | | |
| 7 | 100 | 50 | 0,19 | 0,00 | 19 | 1 | 10 | | |
| 8 | 100 | 50 | 0,14 | 0,00 | 14 | 1 | 11 | | |
| 9 | 94 | 50 | 0,61 | 0,31 | 57 | 1 | 12 | 1 | 334 |
| 10 | 100 | 50 | 1,44 | 1,15 | 144 | 2 | 1 | 1 | \| |
| 11 | 38 | 60 | 1,05 | 0,84 | 40 | 2 | 2 | 1 | \| |
| 12 | 135 | 60-70 | 0,69 | 0,48 | 93 | 2 | 3 | 1 | V |
| 13 | 100 | 70 | 0,4 | 0,10 | 40 | 2 | 4 | 2 | 543 |
| 14 | 50 | 80 | 0,43 | 0,09 | 22 | 2 | 5 | 2 | \| |
| 15 | 50 | 80 | 0,96 | 0,34 | 48 | 2 | 6 | 2 | \| |
| 16 | 50 | 80 | 0,98 | 0,49 | 49 | 2 | 7 | 2 | \| |
| 17 | 50 | 80 | 0,57 | 0,29 | 29 | 2 | 8 | 2 | \| |
| 18 | 50 | 80-90 | 0,48 | 0,14 | 24 | 2 | 9 | 2 | \| |
| 19 | 50 | 90 | 0,42 | 0,13 | 21 | 2 | 10 | 2 | \| |
| 20 | 50 | 90 | 0,61 | 0,24 | 31 | 3 | 1 | 2 | \| |
| 21 | 50 | 90-100 | 0,93 | 0,70 | 47 | 3 | 2 | 2 | \| |
| 22 | 50 | 100 | 1,08 | 0,86 | 54 | 3 | 3 | 2 | \| |
| 23 | 50 | 100 | 0,57 | 0,34 | 29 | 3 | 4 | 2 | \| |
| 24 | 50 | 110 | 0,41 | 0,08 | 21 | 3 | 5 | 2 | \| |
| 25 | 50 | 110 | 0,61 | 0,09 | 31 | 3 | 6 | 2 | \| |
| 26 | 50 | 110 | 0,69 | 0,17 | 35 | 3 | 7 | 2 | \| |
| 27 | 50 | 110-120 | 0,73 | 0,44 | 37 | 3 | 8 | 2 | \| |
| 28 | 28 | 120 | 1,07 | 0,86 | 30 | 3 | 9 | 2 | \| |
| 29 | 50 | 120 | 1,08 | 0,76 | 54 | 3 | 10 | 2 | V |

Table 16 Second cobalt run fractions with other samples in Figure 20 gel

| Sample description | Imidizole mM | volume ml | Conc. mg/ml | Total mg | Gel lane | Ico-pure mg |
|---|---|---|---|---|---|---|
| Co-1 load | 0 | 367 | 0,78 | 286,3 | 2 | |
| Co-1 FT | 0 | 367 | 0,22 | 80,7 | 3 | |
| Co-1 Eluate | 50 | 30 | 6,46 | 193,8 | 4 | 193,8 |
| MI #1 Nickel | 500 ? | | 9,65 | | 5 | |
| PBGD-1 Lys | 0 | | 1,73 | | 6 | |
| Ab prep #1 | 0 | 3,3 | 4,00 | 13,2 | 7 | |
| Cobalt-2 FT | 0 | 900 | 0,23 | 207,0 | 8 | |
| FT tail | 0 | 30 | 0,02 | 0,6 | - | |
| Cobalt-2 w1 | 5 | 100 | 0,01 | 1,0 | - | |
| " w2 | 5 | 50 | 0,14 | 7,0 | - | |
| " w3 | 5 | 52 | 0,02 | 1,0 | - | |
| " w4 | 5 | 100 | 0,04 | 4,0 | - | |
| " w5 | 10 | 50 | 0,07 | 3,5 | - | |
| " w6 | 10 | 50 | 0,81 | 40,5 | 9 | |
| " w7 | 10 | 50 | 0,81 | 40,5 | 10 | 101,5 |
| " w8 | 10 | 50 | 0,41 | 20,5 | 11 | |
| " w9 | 10 | 130 | 0,20 | 26,0 | - | |
| " E1 | 50 | 22,5 | 2,29 | 51,5 | 12 | 51,5 |
| " E2 | 50 | 30 | 0,11 | 3,3 | - | |

Total mg highly pure rhPBGD-His (by amino acid analysis) = 346,8

Table 17 rhPBGD-His amino acid analysis:

Cobalt eluates

| aa | #aa | % whole | #1 umol/ml ——— aa | #1 [PBGD] | #2 aa | #2 [PBGD] | #3 aa | #3 [PBGD] | #4 ——> aa | #4 [PBGD] |
|---|---|---|---|---|---|---|---|---|---|---|
| Ala | 29 | 8,31 | 4,8220 | 0,166 | 0,5920 | 0,020 | 1,6950 | 0,058 | 4,5406 | 0,157 |
| Arg | 21 | 6,02 | 3,7279 | 0,178 | 0,4550 | 0,022 | 1,3050 | 0,062 | 3,5129 | 0,167 |
| Asn | 10 | 2,87 | | | | | | | | |
| Asp | 19 | 5,44 | 4,7157 | 0,163 | 0,5755 | 0,020 | 1,6510 | 0,056 | 4,4442 | 0,153 |
| Cys | 4 | 1,15 | | | | | | | | |
| Gln | 19 | 5,44 | | | | | | | | |
| Glu | 21 | 6,02 | 6,7648 | 0,169 | 0,8152 | 0,020 | 2,3592 | 0,059 | 6,3524 | 0,159 |
| Gly | 27 | 7,74 | 4,3933 | 0,163 | 0,6120 | 0,023 | 1,5595 | 0,058 | 4,1137 | 0,152 |
| His | 18 | 5,16 | 2,5664 | **0,143** | 0,2539 | **0,014** | 0,7947 | **0,044** | 2,4518 | **0,136** |
| Ile | 20 | 5,73 | 3,0511 | **0,153** | 0,3661 | 0,018 | 1,0561 | 0,053 | 2,8381 | 0,142 |
| Leu | 43 | 12,32 | 7,0235 | 0,163 | 0,8565 | 0,020 | 2,4506 | 0,057 | 6,6020 | 0,154 |
| Lys | 18 | 5,16 | 2,9241 | 0,162 | 0,3538 | 0,020 | 1,0427 | 0,058 | 2,7135 | 0,151 |
| Met | 6 | 1,72 | 0,8691 | **0,145** | 0,0996 | **0,017** | 0,3072 | **0,051** | 0,8252 | **0,138** |
| Phe | 9 | 2,58 | 1,4713 | 0,163 | 0,1825 | 0,020 | 0,5216 | 0,058 | 1,4045 | 0,156 |
| Pro | 16 | 4,58 | 2,7268 | 0,170 | 0,3708 | 0,023 | 1,1194 | 0,070 | 2,8441 | 0,178 |
| Ser | 18 | 5,16 | 2,8356 | 0,158 | 0,3570 | 0,020 | 1,0121 | 0,056 | 2,8680 | 0,159 |
| Thr | 20 | 5,73 | 3,4426 | 0,172 | 0,4272 | 0,021 | 1,2154 | 0,061 | 3,2746 | 0,164 |
| Trp | 2 | 0,57 | | | | | | | | |
| Tyr | 3 | 0,86 | 0,5150 | 0,172 | 0,0626 | 0,021 | 0,1734 | 0,058 | 0,4823 | 0,161 |
| Val | 26 | 7,45 | 4,0526 | **0,156** | 0,4920 | 0,019 | 1,4292 | 0,055 | 3,7740 | 0,145 |

| | #1 | #2 | #3 | #4 |
|---|---|---|---|---|
| Avg umol/mol rhPBGD-His: (w/o bold values) | 0,167 | 0,021 | 0,059 | 0,159 |
| Avg mg/ml rhPBGD-His: (MW= 38759,4 ) | 6,46 | 0,81 | 2,29 | 6,17 |
| Volume (ml): | 30 | 100 | 22,5 | 5 |
| amount in fraction (mg): | 193,7 | 80,8 | 51,6 | 30,8 |

| sample | run # | imidazole | Notes |
|---|---|---|---|
| #1 | 1 | 50 mM | Major peak of cobalt eluate (from 50->60mM KCl elution from DEAE) |
| #2 | 2 | 10 mM | 70->120mM KCl elution from DEAE ; cobalt fractions W-6,7 |
| #3 | 2 | 50 mM | " fraction E-1 |
| #4 | 1 | - | #1 dialyzed -> PBS+ 5mM Tris/Cl pH 8.0 (used for 2nd round Antibodies) |

Analysis done at: AAA Laboratory
By: Lowell Ericsson, Nancy Ericsson
Address: 6206 89th Ave SE, Mercer Island, Washington 98040-4599

Example 3

**P-9808: Summary and method of a high scale fermentation process using *E.coli* and a down stream process for production of extract containing active recombinant human Porphobilinogen Deaminase (rhPBGD)**

The most important facts about the process is summarised in table 20 below.

Table 18

| Step | Methods for Fermentation process |
|---|---|
| 1. Strain propagation on agar plates. | 4 pcs. of M9H-Tc (6 $mgl^{-1}$) agar plates are inoculated with cells from a Working Cell Bank (WCB) cryo vial stored at < -70 °C.<br>The agar plates are incubated upside down for 24 ± 4 h at 30 ± 1 °C |
| 2. Shake flask cultivation | Two 1 L baffled shake flasks containing 250 ml M9H-Tc (6 $mgl^{-1}$) substrate are incubated with growth from 1 ½ M9H-Tc (6 $mgl^{-1}$) agar plate each. The shake flasks are incubated in a rotary shaker at 135 ± 15 rpm for 12-14 h at 30 ± 1 °C.<br>After incubation the broth from both shake flasks is pooled together in a container suitable for transfer to the 14 L inoculum fermenter. |
| 3. Inoculum fermentation | The 14 L fermenter containing 8 ± 0,4 L MM5Y-Tc (6 $mgl^{-1}$) substrate is inoculated with 500 ml broth both shake flasks.<br>The inoculum fermentation is performed as a batch fermentation with the conditions described below.<br>When the $OD_{620}$ is in the interval 7-10 the broth is transferred to a container suitable for transfer ot the broth to |
| | the 1500 L fermenter.<br>Fermenter: 14 L Chemap fermenter with standard type configuration<br>Temperature: controlled at 30 ± 1 °C during fermentation.<br>pH : controlled at 7,0 ± 0,2 by titration with 12,5% (w/v) $NH_3$ and 2 M $H_2SO_4$<br>$pO_2$: controlled > 20 % by manual increases in stirrer speed or aeration.<br>Initial stirrer speed: 800 + 100 rpm<br>Initial aeration: 4,5 $Nlmin^{-1}$ ( 0,5 WM)<br>Fermentation time: Approximately 9 h ($OD_{620}$ is in the interval 7-10) |

Table continued

| Step | Methods for Fermentation process |
|---|---|
| 4. Production fermentation | The 1500 L fermenter containing 850 ± 100 L MM20Y-Tc substrate is inoculated with the broth from the 14 L inoculum fermenter. Fermentation conditions are given below.<br>Fermenter: 1500 L Chemap fermenter with standard type configuration<br>Temperature: Controlled at 30 ± 1 °C, when fermentation is stopped the broth is cooled down to 20-25 °C.<br>pH : Controlled at 7,0 ± 0,2 by titration with 25 % (w/v) $NH_3$ and 2 M $H_2SO_4$<br>$pO_2$: Controlled > 20 % by manual increases in stirrer speed or aeration.<br>Initial stirrer speed: 200 ± 50 rpm, max 400 rpm.<br>Initial aeration: 425 ± 100 $Nlmin^{-1}$ ( 0,5 VVM); max 775 $Nlmin^{-1}$<br>$CO_2$ in oulet gas: Controlled < 7 % by manual increases in aeration.<br>Glucose feed (600 $gl^{-1}$): Initiated when glucose concentration < 0,5 $gl^{-1}$<br>0-7 h after feed start 3,0 $lmin^{-1}$<br>7-14 h after feed start 6,0 $lmin^{-1}$<br>14 h - End fermentation 9,0 $lmin^{-1}$ |
| | 2 M $MgSO_4 \times 7 H_2O$ feed: Constant feed rate of 490 $mlh^{-1}$<br>initiaded at the same time as the glucose feed<br>Fermentation time : Fermentation is stopped when $OD_{620}$ = 100 ± 20<br>(≈28-30 h.) |
| **Step** | **Methods for Down stream process** |
| 5.Cell concentration using membrane filtration | 300 liter broth is concentrated 2 times with a cross-flow membrane at a temperature of 15-25°C. After concentration cells are washed with a volume of 2 times the cell concentrate volume. The buffer used is 50 mM sodium-phosphate buffer + 1,34 mM EDTA, pH 7,4.<br>Filter equipment: Biomax 1000 K, Millipor, Pellicon, 4 × 2 $m^2$ During filtration temperature, pressure and permeate-flux are controlled.<br>End volume: 150 ± 10 liter.<br>Permeate-flux: 15 ± 2 $lm^{-2}h^{-1}$. |
| 6. Homogenisation | Cell concentrate is homogenized 800 bar, 3 passages with a flow-rate of 100 ± 10 $lh^{-1}$. Homogenate temperature is set at 15-25°C between passages. After the third passage homogenate is diluted 2,5 times with 50 mM sodium-phosphate buffer+1,34 mM EDTA, pH 7,4. During homogenization temperature, pressure and permeate-flux are controlled.<br>Homogenizer: Rannie Type LAB 10-51VH, Max pressure 1500 bar. Supplier APV |

Table continued

| Step | Methods for Down stream process |
|---|---|
| 7. Cell debris removal by membrane filtration | Diluted homogenate is concentrated 2,5 times with a cross-flow membrane at a controlled temperature between 15 and 25°C. The concentrate is then dia filtered with the same membrane to a theoretical yield of 85 ±5 % with 50 mM sodium-phosphate buffer + 1,34 mM EDTA, pH 7,4. Permeate (Extract) is gently stirred in a tank with nitrogen flushed over the surface. During filtration temperature, pressure and permeate-flux are controlled.<br>Filter equipment: Biomax 1000 K, Millipor, Pellicon, 4 × 2 $m^2$<br>End volume permeate: 450 ± 100 liter.<br>Permeate-flux: 12 ± 2 $lm^{-2}h^{-1}$. |
| 8. Final filtration | Permeate from Cell debris removal is filtered through a 0,22 |
| | μm filter into 20 liter containers. Each container is filled to a weight of 10 kg. The filter is integrity tested according to specified instructions. Filled containers are transported to a freeze-house according to instructions. Maximum time from packaging to delivery at the freeze-house is 6 hours. Extract is kept at the freeze-house until it is released for delivery to BioInvent where the final Down-stream process take place.<br>End volume filtered extract: 350 ± 100 liter.<br>Filter: Durapore CVGL71TP3<br>Containers: 20 liter Flex-Boy<br>Temperature freeze-house: < 18°C |

METHOD OF PURIFICATION OF rhPBGD

INTRODUCTION

The scope of this document is to give an overview of the current method of purification of crude rhPBGD.

Cell extract is supplied by BioGaia AB frozen in 20 liter Flexboy bags, filled to 10 liters. After thawing, purification is achieved i three steps commencing with a HIC step on Streamline Phenyl FF Sepharose, followed by a IEC step on DEAE FF Sepharose, ending with an affinity step on Cibacrone Blue FF Sepharose.
The main fraction of the affinity purification step is diafiltrated with the formulation buffer before freezing.

STEP 1: HIC, STREAMLINE PHENYL FF SEPHAROSE

A Pharmacia BPG-200 column is packed with gel to a volume of 10,8 liters (h=310 mm, diam. 200 mm). Flow is 0,5 L/min.

Crude extract (e.g. 50-100 liters) is thawed overnight in room temperature, then diluted immediately before loading onto column, with approximately 20 - 45 liters of 2,5 M $K_2HPO_4$(aq), pH 7,5.
Column is equilibrated with 0,75 M $K_2HPO_4$(aq), pH 7,5. Following sample loading and washing with 0,75 M $K_2HPO_4$(aq), pH 7,5, elution is performed using water.

The main fraction (23-28 liters, pH 7,2-7,8, cond. 15-20 mS/cm) is stored in a coldroom overnight.

Chromatography sequence

| No. | Action | Buffer | Col. vol. |
|---|---|---|---|
| 1. | Equilibration | 0,75 M $K_2HPO_4$(aq), pH 7,5 | 1 |
| 2. | Sample load | Sample | Ca. 12 |
| 3. | Wash | 0,75 M $K_2HPO_4$(aq), pH 7,5 | 3 |

Table continued

| No. | Action | Buffer | Col. vol. |
|---|---|---|---|
| 4. | Elution | Water | 5 |

STEP 2: IEC, DEAE FF SEPHAROSE

A Pharmacia BPG-300 column is packed with gel to a volume of 12,5 liters (h=180 mm, diam. 300 mm). Flow is 0,5 L/min.

Main fraction from step 1 is diluted with water to a conductivity of 4-5 mS/cm (4-6 volumes water) and split into two equal halves. Step 2 is run twice following each other as follows: The column is equilibrated with 10 mM $K_2HPO_4$(aq), pH 7,5. The sample is loaded onto the column. Washing is performed first with 10 mM $K_2HPO_4$(aq), pH 7,5 (about 2 col. vol.), followed by 10 mM $K_2HPO_4$(aq), 6 M urea, pH 7,5 (about 2 col. vol. for the purpose of removing ECP). Elution is performed with 10 mM $K_2HPO_4$(aq), 100 mM KCl(aq), pH 7,5. The column is washed with 10 mM $K_2HPO_4$(aq), 1,5 M KCl(aq), pH 7,5.
The main fraction (20-25 liters, pH 7,2-8,0, cond. 10-15 mS/cm) from each of the step 2 is pooled and stored in a coldroom overnight.

Chromatography sequence

| No. | Action | Buffer | Col. vol. |
|---|---|---|---|
| 1. | Equilibration | 10 mM $K_2HPO_4$(aq), pH 7,5 | 1 |
| 2. | Sample load | Sample | Ca. 5 |
| 3. | Wash | 10 mM $K_2HPO_4$(aq), pH 7,5 | 2 |
| 4. | Wash | 10 mM $K_2HPO_4$(aq), 6 M urea, pH 7,5 | 2 |
| 5. | Wash | 10 mM $K_2HPO_4$(aq), pH 7,5 | 2 |
| 6. | Elution | 10 mM $K_2HPO_4$(aq), 100 mM KCl(aq), pH 7,5 | 3 |
| 7. | Wash | 10 mM $K_2HPO_4$(aq), 1,5 M KCl(aq), pH 7,5 | 2 |
| 8. | Wash | 10 mM $K_2HPO_4$(aq), pH 7,5 | 2 |

STEP 3: AFFINITY CHROMATOGRAPHY, CIBACRONE BLUE FF SEPHAROSE

A Pharmacia BPG-300 column is packed with gel to a volume of 12,5 liters (h=180 mm, diam. 300 mm). Flow is 467 mL/min.

The pooled fractions from the two step 2 runs are diluted with one volume of 10 mM $K_2HPO_4$(aq), pH 7,5. The column is equilibrated with 10 mM $K_2HPO_4$(aq), pH 7,5.
After loading of sample and washing with 10 mM $K_2HPO_4$(aq), pH 7,5, the column is washed with 10 mM $K_2HPO_4$(aq), 300 mM KCl(aq), pH 7,5 followed by elution of product with 10 mM $K_2HPO_4$(aq), 450 mM KCl(aq), pH 7,5. The column is then washed with 10 mM $K_2HPO_4$(aq), pH 7,5.

The main fraction (10-15 liters, pH 7,0-8,0, cond.ca. 35 mS/cm) is stored in a coldroom overnight.

A fermentation process using *E. Coli* and a down stream process for production of aseptic extract containing active recombinant human Porphobilinogen Deaminase (rhPBGD) on a commercial scale. The subsequent purification process for the production of an active bulk product .

STUDY OBJECTIVES

The study objectives are stated in the Study Protocol chapter 3.1 and are shown in the text below.

- Development of a cGMP *E. Coli* fermentation process up to 600 L scale and cell extraction process.
- Development and qualification of in-process analytical tests, rhPBGD tests and specifications. Verification of in process analytical tests - The goal was a fermentation yield of at least 100 $mgl^{-1}$ rhPBGD and with 50% overall yield of the final purification process.
- Delivery of a frozen, aseptic *E.coli* extract containing active rhPBGD_from BioGaia to BioInvent in appropriate, approved containers including small scale sample for verification and acceptance criteria.

- The aseptic rhPBGD extract must meet the criteria of the Specification for aseptic extract for purification of recombinant human Porphobilinogen Deaminase (rhPBGD) as bolow.

**Specification for aseptic extract for purification of recombinant human Porphobilinogen Deaminase (rhPBGD).**

The analytical tests are performed on filtered extract.
Description: The extract is buffered with 50 mM sodium phosphate, 1.3 mM EDTA, pH 7,4 and filtered through a 0,22 μm filter.

| TEST | METHOD NO. | LIMIT |
|---|---|---|
| Content: | | |
| RhPBGD activity (Units/ml) | E 001 | > 20 |
| RhPBGD specific activity (Units/mg) | E 001/P 001 | > 4.0 |
| Other Tests: | | |
| Protein concentration (mg/ml) | P 001 | 4.5 - 10.0 |
| SDS-PAGE | S 001 | equal to |
| Bacterial counts ( Cfu/ml) | Ph.Eur | reference < 10 |
| PH | Ph.Eur. | 7.0 - 8.0 |

Materials and Methods

Materials

Media / substrates used in this study are given below.
In the tables 21-26 below the media compositions for the final developed process are given. From start to the experiment PD14 the thiamine concentration in substrate MM5Y Tc was only 1 mg/l, but was thereafter increased to 10 mg/l starting with experiment PD16.

**1. M9H-Tc (6 $mgl^{-1}$), M9H-Amp (100 $mgl^{-1}$) and M9H-Chl (25 $mgl^{-1}$) agar plates.**

The M9H-Tc (6 $mgl^{-1}$) agar plates are used in inoculum procedure while the M9H-Amp (100 $mgl^{-1}$) and M9H-Chl (25 $mgl^{-1}$) were used for controls of the strain identity.

Table 19. Composition of the M9H agar plates

| **Component** | **Chemical formula** | **Conc.** | **Unit** |
|---|---|---|---|
| di-Sodium hydrogen phosphate | $Na_2HPO_4$ | 6,00 | $gl^{-1}$ |
| Potassium dihydrogen phosphate | $KH_2PO_4$ | 3,00 | $gl^{-1}$ |
| Sodium chloride | NaCl | 0,50 | $gl^{-1}$ |
| Ammonium chloride | $NH_4Cl$ | 1,00 | $gl^{-1}$ |
| Glucose monohydrate | $C_6H_{12}O_6 \times 1H_2O$ | 2,00 | $gl^{-1}$ |
| Thiamine chloride hydrochloride | $C_{12}H_{18}Cl_2N_4OS\ xXH_2O$ | 1,00 | $mgl^{-1}$ |
| Magnesium sulphate heptahydrate | $MgSO_4 \times 7\ H_2O$ | 25,0 | $mgl^{-1}$ |
| Bacto Agar | ------------------- | 15,0 | $gl^{-1}$ |
| **One of the antibiotics below are added** | | | |
| Chloramphenicol | $C_{11}H_{12}Cl_2N_2O_5$ | 25,0 | $mgl^{-1}$ |
| Ampicillin sodium salt | $C_{16}H_{19}N_3O_4SNa$ | 100 | $mgl^{-1}$ |
| Oxytetracycline hydrochloride | $C_{22}H_{24}N_2O_9 \times HCl$ | 6,00 | $mgl^{-1}$ |

**M9H-Tc (6 mgl$^{-1}$) shake flask**

The M9H-Tc (6 mgl$^{-1}$) shake flasks were used in the initial batch experiments and in the inoculum preparation for all experiments.

Table 20. Composition of the M9H-Tc shake flasks.

| Component | Chemical formula | Conc. | Unit |
|---|---|---|---|
| di-Sodium hydrogen phosphate | $Na_2HPO_4$ | 6,00 | gl$^{-1}$ |
| Potassium dihydrogen phosphate | $KH_2PO_4$ | 3,00 | gl$^{-1}$ |
| Sodium chloride | NaCl | 0,50 | gl$^{-1}$ |
| Ammonium chloride | $NH_4Cl$ | 1,00 | gl$^{-1}$ |
| Glucose monohydrate | $C_6H_{12}O_6 \times 1H_2O$ | 10,0 | gl$^{-1}$ |
| Thiamine chloride hydrochloride | $C_{12}H_{18}Cl_2N_4OS \times XH_2O$ | 1,00 | mgl$^{-1}$ |
| Magnesium sulphate heptahydrate | $MgSO_4 \times 7\ H_2O$ | 0,25 | gl$^{-1}$ |
| Oxytetracycline hydrochloride | $C_{22}H_{24}N_2O_9 \times HCl$ | 6,00 | mgl$^{-1}$ |

**MM5Y-Tc (6 mgl$^{-1}$) shake flask and fermenter medium**

The MM5Y-Tc (6 mgl$^{-1}$) medium was used for all fermentations with strain PBGD-1 and in the inoculum fermentations in the scale up of the process when the strain PBGD-2 was used. Shake flask cultivations of both strain PBGD-1 and PBGD-2 was performed with this substrate.

Table 21. MM5Y-Tc (6 mgl$^{-1}$) shake flask and fermenter medium

| Component | Chemical formula | Conc.* | Unit |
|---|---|---|---|
| Ammonium sulfate | $(NH_4)_2SO_4$ | 2,67 | gl$^{-1}$ |
| Potassium dihydrogen phosphate | $KH_2PO_4$ | 3,26 | gl$^{-1}$ |
| di-Potassium hydrogensulfate trihydrate | $K_2HPO_4 \times 3\ H_2O$ | 2,84 | gl$^{-1}$ |
| tri-Sodium citrate dihydrate | $C_6H_5Na_3O_7 \times 2\ H_2O$ | 0,60 | gl$^{-1}$ |
| Yeast extract | ------------------ | 5,00 | gl$^{-1}$ |
| Glucose monohydrate | $C_6H_{12}O_6 \times 1H_2O$ | 11,0 | gl$^{-1}$ |
| Magnesium sulfate heptahydrate | $MgSO_4 \times 7\ H_2O$ | 0,25 | gl$^{-1}$ |
| Thiaminechloride hydrochloride | $C_{12}H_{18}Cl_2N_4OS \times XH_2O$ | 10,0 | mgl$^{-1}$ |
| Boric acid | $H_3BO_3$ | 2,50 | mgl$^{-1}$ |
| Cupper(II)sulfate pentahydrate | $CuSO_4 \times 5H_2O$ | 10,1 | mgl$^{-1}$ |
| Iron(III)chloride hexahydrate | $FeCl_3 \times 6H_2O$ | 34,1 | mgl$^{-1}$ |
| Manganese(II)sulfate monohydrate | $MnSO_4 \times 1H_2O$ | 6,40 | mgl$^{-1}$ |
| Zinc sulfate heptahydrate | $ZnSO_4 \times 7H_2O$ | 5,00 | mgl$^{-1}$ |
| Cobalt(II)chloride hexahydrate | $CoCl_2 \times 6H_2O$ | 8,90 | mgl$^{-1}$ |
| Calcium chloride dihydrate | $CaCl_2 \times 2H_2O$ | 13,9 | mgl$^{-1}$ |
| Sodium molybdate dihydrate | $Na_2MoO_4 \times 2H_2O$ | 8,90 | mgl$^{-1}$ |
| Hydrochloric acid, fuming, 37% | HCl | 66,0 | □ll$^{-1}$ |
| Oxytetracycline hydrochloride | $C_{22}H_{24}N_2O_9 \times HCl$ | 6,00 | mgl$^{-1}$ |
| *Concentration after inoculum. | | | |

**MM20Y Fermenter medium**

The MM20Y Fermenter medium was used in all main fermentations with strain PBGD-1, except for batch PD 14.

Table 22. MM20Y Fermenter medium

| **Component** | **Chemical formula** | **Conc.*** | **Unit** |
|---|---|---|---|
| Ammonium sulfate | $(NH_4)_2SO_4$ | 2,67 | gl$^{-1}$ |
| Potassium dihydrogen phosphate | $KH_2PO_4$ | 3,26 | gl$^{-1}$ |
| di-Potassium hydrogensulfate trihydrate | $K_2HPO_4 \times 3\ H_2O$ | 2,84 | gl$^{-1}$ |
| tri-Sodium citrate dihydrate | $C_6H_5Na_3O_7 \times 2\ H_2O$ | 0,60 | gl$^{-1}$ |
| Yeast extract | ------------------------------- | 20,0 | gl$^{-1}$ |
| Glucose monohydrate | $C_6H_{12}O_6 \times 1H_2O$ | 11,0 | gl$^{-1}$ |
| Magnesium sulfate heptahydrate | $MgSO_4 \times 7\ H_2O$ | 0,25 | gl$^{-1}$ |
| Thiaminechloride hydrochloride | $C_{12}H_{18}Cl_2N_4OS \times XH_2O$ | 10,0 | mgl$^{-1}$ |
| Boric acid | $H_3BO_3$ | 2,50 | mgl$^{-1}$ |
| Cupper(II)sulfate pentahydrate | $CuSO_4 \times 5H_2O$ | 10,1 | mgl$^{-1}$ |
| Iron(III)chloride hexahydrate | $FeCl_3 \times 6H_2O$ | 34,1 | mgl$^{-1}$ |
| Manganese(II)sulfate monohydrate | $MnSO_4 \times 1H_2O$ | 6,40 | mgl$^{-1}$ |
| Zinc sulfate heptahydrate | $ZnSO_4 \times 7H_2O$ | 5,00 | mgl$^{-1}$ |
| Cobalt(II)chloride hexahydrate | $CoCl_2 \times 6H_2O$ | 8,90 | mgl$^{-1}$ |
| Calcium chloride dihydrate | $CaCl_2 \times 2H_2O$ | 13,9 | mgl$^{-1}$ |
| Sodium molybdate dihydrate | $Na_2MoO_4 \times 2H_2O$ | 8,90 | mgl$^{-1}$ |
| Hydrochloric acid, fuming, 37% | HCl | 66,0 | □ll$^{-1}$ |
| Oxytetracycline hydrochloride | $C_{22}H_{24}N_2O_9 \times HCl$ | 6,00 | mgl$^{-1}$ |
| *concentration after inoculum | | | |

**LB / TSA agar plates**

LB or TSA agar plates were for determination of colony forming units (CFU) and as a reference in the plasmid stability controls.

Table 23: Composition of LB agar plates

| Component | Concentration | Unit |
|---|---|---|
| Tryptone | 10,0 | gl$^{-1}$ |
| Yeast extract | 5,00 | gl$^{-1}$ |
| NaCl | 10,0 | gl$^{-1}$ |
| Bacto Agar | 15,0 | gl$^{-1}$ |

Table 24: Composition of TSA agar plates

| Component | Concentration | Unit |
|---|---|---|
| Tryptone | 15,0 | gl$^{-1}$ |
| Soytone | 5,00 | gl$^{-1}$ |
| NaCl | 5,00 | gl$^{-1}$ |

Table continued

| Component | Concentration | Unit |
|---|---|---|
| BactoAgar | 15,0 | g/l |

**Methods**

Methods used in II. Development of lab scale fermentation process

Each cultivation/fermentation started with material from a cryo vial of the intermediary cell bank (ICB) for either the PBGD-1 (PDWS1:1-80) [(7)] or the PBGD-2 strain (PDWS2:1-80) [(7)] stored at < -70°C in an ultra freezer. Material from these cryo vials was transferred to M9H-Tc (6 $mgl^{-1}$) agar plates (attachment 2, table 1) with a sterile platinum loop. These plates were then incubated upside down for 23-33 hours at 30 °C.

1-L baffled shake flasks containing 250 ml M9H-Tc (6 $mgl^{-1}$) substrate or MM5Y-Tc (6 $mgl^{1}$) substrate (attachment 2, tables 2 and 3) were inoculated with colonies from 1- 1 ½ agar plate each, depending on the experimental design. The transfer was made either directly to the shake flask with a sterile platinum loop or via a test tube containing 9,9 ml sterile 0,9 % (w/v) NaCl solution. In the latter case the solution was vortexed before inoculum to dissolve the cell "pellet" properly.

Depending on the experimental design the M9H-Tc (6 $mgl^{-1}$) shake flasks were incubated for 9-14 hours in an air incubator at 135 rpm and 30 °C. The $OD_{620}$ at the end of the incubation in these experiments varied between 0,8-1,8. When the experiments only involved shake flask cultivation in either M9H-Tc (6 $mgl^{-1}$) or MM5Y-Tc (6 $mgl^{-1}$) substrate the incubation time varied between 12-55 h and the final $OD_{620}$ varied between 0,3-4,0. Depending on experimental design and the volume needed for the subsequent down stream processing development three different fermenters (2 L, 14 L and 20 L) were used. The fermenters were incubated with 250-500 ml broth from the M9H-Tc (6 $mgl^{-1}$) shake flasks and this in combination with different substrates and volumes in the fermenter made the $OD_{620}$ after the inoculum vary in the range 0,05-2,0. Since the initial glucose concentration always was 10 $gl^{-1}$ this made the length of the batch phase vary between 8,5-15,2 h. When the initial glucose was consumed (end of batch phase) different glucose feed profiles were investigated to control the fermentation. The general fermentation conditions during the development of the lab scale fermentation are given in table 1 below.

**Table 25. General fermentation conditions during the development of a lab scale fermentation process**

| Parameter | Comments |
|---|---|
| Temperature | Controlled at 30 °C |
| pH | Controlled at 7,0 by titration with 12,5 % $NH_3$ and 2M $H_2SO_4$ |
| $pO_2$ (dissolved oxygen) | Manually controlled > 20 % by changes in aeration and stirrer speed |
| $CO_2$ | Manually controlled < 7 % by changes in aeration |
| Aeration | 0,5-1,2 VVM |
| Stirrer speed | 500-1200 rpm |
| $MgSO_4 \times 7H_2O$ feed | Initiated when glucose feed started, constant feed rate 0,3 $gl^{-1}$ $h^{-1}$ based on initial volume |

Methods used for III. Scale up of fermentation process.

Simulated large scale fermentations.

In the simulated large scale fermentations an extra 9 L inoculum fermentation was performed before the main fermentation to introduce an extra 5-6 generations in the total process. The inoculum fermentation and the main fermentation were performed in the same fermenter. The procedures for the inoculum preparation on M9H-Tc (6 $mgl^{-1}$) agar plates and 1 L shake flasks containing 250 ml M9H-Tc (6 $mgl^{-1}$) substrate were the same as described above in chapter 6.2.1. The inoculum fermenter containing 9 L MM5Y-Tc (6 $mgl^{-1}$) substrate (attachment 2, table 3) was inoculated with 500 ml broth from two shake flasks and the initial $OD_{620}$ varied between 0,1-0,2. When the $OD_{620}$ reached 7- 8

(9,5-13,0 h) in the 9 L inoculum fermentation 130-150 ml broth was withdrawn and cooled down to 0-8 °C. The volume of the withdrawn broth was calculated to give an initial $OD_{620}$ of 0,1-0,2 in the main fermenter. The remaining broth in the fermenter was removed and the fermenter was rinsed once with 7 L sterile water before 7 L sterile MM20Y substrate (attachment 2, table 4) was transferred to the fermenter. The fermenter was allowed to reach the right fermentation conditions before the fermenter was inoculated with the cold stored broth. This procedure took approximately 1,5 h.After the inoculum the fermentations were performed according to the recently developed lab scale process.

850 L Scale up fermentations

Four M9H-Tc (6 $mgl^{-1}$) agar plates were inoculated with material from one cryo vial of strain PBGD-2 and incubated at 30 °C for 23,2-27,2 h. Two 1 L shake flasks, each containing 250 ml M9H-Tc (6 $mgl^{-1}$) substrate, were inoculated with growth from 1 ½ agar plate each. The shake flasks were incubated in an air incubator at 30 °C and 135 rpm for 12-13,2 h. Broth from the two shake flasks was pooled together in a sterile bottle with connections suitable for sterile transfer to the fermenter. The 14 L fermenter containing 9 L MM5Y-Tc (6 $mgl^{-1}$) substrate was then inoculated with the 500 ml broth with an $OD_{620}$ of 1,7. The conditions for the 14 L inoculum fermenter are summarised in table 28 below.

Table 26. Inoculum fermentation conditions during 850 L Scale up fermentations

| Parameter | Value |
|---|---|
| Temperature | 30 °C |
| pH | 7,0 |
| Aeration | 0,5 WM (4,5 $Nlmin^{-1}$) |
| Initial stirrer speed | 800 rpm |
| pO2 (dissolved oxygen tension) | > 20 % by manual control of stirrer speed |
| Fermentation time | 8,5-9,2 h ( $OD_{620}$ 7-10 ) |

After 8,5-9,2 h when the $OD_{620}$ in the 14 L fermenter had reached 7,4 -8,6 the broth was transferred to a sterile plastic container suitable for transfer to the 1500 L fermenter containing 850 L MM20Y substrate. The initial $OD_{620}$ in the 1500 L fermenter was 0,17-0,18. Based on the lab scale development and the simulated large scale fermentations the following fermentation strategy was postulated for the 850 L fermentations. When the initial 10 $gl^{-1}$ glucose was consumed (glucose concentration < 0,5 $gl^{-1}$) a stepwise increasing glucose feed (600 $gl^{-1}$) profile (3,0 $lh^{-1}$ 0-7 h after feed start, 6,0 $lh^{-1}$ 7-14 h after feed start and finally an increase to 9,0 $lh^{-1}$ for the remaining fermentation) was started. A constant feed (490 $mlh^{-1}$) of a 2M $MgSO_4 \times 7H_2O$ feed was started at the same time as the glucose feed. The fermentation conditions in the 1500 L fermenter are summarised in Table 27.below.

Table 27. Conditions in 1500 L fermenter during 850 L Scale up fermentations

| Parameter | Comments |
|---|---|
| Temperature | Controlled at 30 °C |
| $p_H$ | Controlled at 7,0 by titration with 25 % $NH_3$ and 2M $H_2SO_4$ |
| Pressure | 0,2 bar overpressure |
| $pO_2$ (dissolved oxygen) | Manually controlled > 20 % by changes in aeration and stirrer speed |
| $CO_2$ | Manually controlled < 7 % by changes in aeration |
| Aeration | 0,5-0,8 VVM (425-775 $Nlmin^{-1}$) |
| Stirrer speed | 200-400 rpm |
| Glucose feed (600 $gl^{-1}$) | 3,0 $lh^{-1}$ 0-7 h after feed start, 6,0 $lh^{-1}$ 7-14 h after feed start and finally an increase to 9,0 $lh^{-1}$ for the remaining |
| | fermentation |

Table continued

| Parameter | Comments |
|---|---|
| $MgSO_4 \times 7\ H_2O$ feed | Initiated when glucose feed starts, constant feed rate 0,3 $gl^{-1}\ h^{-1}$ based on initial volume |
| Fermentation time | 28-30 h |

7.2.3 Methods used in IV. Development and Scale-up of downstream process

Downstream process

During the lab scale development of a down stream process 1-15 L broth was processed (PD07-PD22). A twenty fold scale up to a process taking care of 300 L broth (PD1501 and PD1502) was done to produce material for toxicological and clinical studies.

**Cell concentration**

In all experiments the broth was concentrated 1,5-6,9 times using a cross flow membrane followed by dia filtration (washing) using a buffer to exchange 90-95% of the substrate. The permeate-flux (i.e. flow-rate through the membrane) was controlled between 7-15 $lm^2h^{-1}$ using a permeate pump. The filters used in the experiments were a 0,2 μm Sartocon filter (Sartorius) and a 1000 K Biomax filter, v-screen (Millipore), (table 30).

Table 28. Filters tested for cell concentration

| **Filter used** | **Batch** |
|---|---|
| 0,2 μm Sartocon | PD09, PD11, PD12, PD14, PD16, PD19 and PD21 |
| 1000 K Biomax, v-screen | PD22, PD1501 and PD1502 |

After the concentration the cell concentrate was dia filtered with buffer using the same filter and parameters as used for the concentration. The different buffers tested are given in table 29 below. The membrane filtrations were made at ambient temperature in the laboratory scale and controlled at 15-25 °C in during scale up.

**Table 29. Different buffers tested for cell-concentration**

| Buffer | Batch |
|---|---|
| 20 mM Tris, 0,67 mM EDTA, pH 8,2 | PD09, PD11 and PD12 |
| 50 mM Tris,1,34 mM EDTA, pH 8,2 | PD14 |
| 50 mM Tris, 1,34 mM EDTA, pH 7,4 | PD16 |
| 50 mM sodium-phosphate, 1,34 mM EDTA, pH 7,4 | PD21, PD22, PD1501 and PD1502 |

Homogenisation

The concentrated and washed cells were homogenised with a laboratory homogeniser (Niro Soavi Panda (10 $lh^{-1}$)) or a production scale homogeniser (Rannie Type LAB 10-51 VH (100 $lh^{-1}$), APV). The conditions tested were 600-1000 bar, 1-3 passages at ambient temperature. Between passages the temperature was set at 15-25°C in the production scale. Table **30** below summarises the conditions used for each experiment. The homogenate was analysed for rhPBGD-activity and protein concentration (Procedures E001 and P001).

**Table 30. Conditions tested for homogenisation**

| **Pressure** | **No of passages** | **Batch** |
|---|---|---|
| 600 bar | 1 | PD09 |
| 600 bar | 2 | PD09 |

**Table continued**

| Pressure | No of passages | Batch |
|---|---|---|
| 600 bar | 3 | PD21 |
| 800 bar | 1 | PD09, PD11, PD12, PD14, PD16, PD19 |
| 800 bar | 3 | PD19, PD21, PD22, PD1501, PD1502 |
| 1000 bar | 3 | PD19 |

Cell debris removal by membrane filtration

Before cell debris removal by membrane filtration the homogenate was diluted 2-3 times with the same buffer used for the cell concentration step.
Filters tested for cell debris removal were 0,2 μm Sartoconfilter, 1000 K Biomax filter (v-screen) and 500 K Biomax filter (v-Screen). The homogenate was concentrated 1,5-2,5 times and dia filtrated in order to get 80-99 % theoretical yield of rhPBGD, with buffers specified in table 33 below. The theoretical yield is a calculated (calculations not shown) yield based on the assumptions that there are no interactions what so ever between the filter surface and the rhPBGD (i.e. 100 % transmission). Since there in practice are many interactions (e.g. fouling, electrostatic forces) the real yield is off course lower. The theoretical yield is mainly used as a to tool to be able to compare results from different filtration runs.

**Table 31. Buffers tested for cell debris removal.**

| Buffer | Batch |
|---|---|
| 20 mM Tris, 0,67 mM EDTA, pH 8,2 | PD11 and PD12 |
| 50 mM Tris,1,34 mM EDTA, pH 8,2 | PD14 |
| 50 mM Tris, 1,34 mM EDTA, pH 7,4 | PD16 |
| 50 mM Na-phosphate, 1,34 mM EDTA, pH 7,4 | PD21, PD22, PD1501 and PD1502 |

The permeate-flux during membrane filtration was controlled between 7-15 $lm^{-2}$ $h^{-1}$ using a permeate pump. To prevent oxidation of the extract in the stirred holding tank a stream of nitrogen was flushed over the permeate surface (only PD1502). The Membrane filtrations were made at ambient temperature in the laboratory scale and controlled between 15-25 °C in production scale. The permeate was assayed for rhPBGD activity, protein concentration and SDS-Page (Procedure: E001, P001 and S001).

Cell debris removal by centrifugation

During the development of a method for cell debris removal centrifugation was initially used in the laboratory scale in order to produce material for further down stream experiments. The homogenised material was centrifuged at 10 000 × g for 20 min in a Sorvall centrifuge (Beckman). The supernatant was collected, saved and assayed for rhPBGD-activity, protein concentration and SDS-Page (Procedures: E001, P001 and S001).

Final filtration

Permeate from membrane filtrated homogenate and supernatant from centrifuged homogenate were respectively filtered through a 0,22 μm retentive filter (Sartobran or Durapore) into autoclaved containers. The filters were integrity tested and the extract assayed for CFU, rhPBGD-activity, protein concentration and SDS-Page (procedures: K-M50, E001, P001 and S001).

Stability studies, rhPBGD-activity

Membrane filtered extract from different batches PD22 and PD1501 were kept at -20°C for several months. Single use aliquots were routinely taken out of the freezer and the rhPBGD-activity was measured and plotted over time (procedure: E001).

Analytical methods

*Preparation of undisrupted cells for analysis, Procedure K-M45*

Cells for analysis were prepared and lysed according to procedure K-M45. The procedure is mainly based on a report made by Pieter Jan Oort, Icogen[4].
The sample (10 ml) was centrifuged for 10 min at 7700 × g. The supernatant was poured of and the pellet re suspended in 5 ml 25 mM sodium-phosphate, 0,9 % NaCl, pH 7,4. The new cell suspension was centrifuged for another 10 min at 7700 × g and the pellet was re suspended in 50 mM sodium-phosphate, 1,34 mM EDTA, pH 7,4. This cell suspension was then sonicated for 3 x 45 s in a Soniprep 150 Sonicator followed by centrifugation for 10 min at 7700 x g. The supernatant was saved and the volume measured. The pellet was re suspended once more in 50 mM sodium-phosphate, 1,34 mM EDTA, pH 7,4, re sonicated for 3 x 45 s and centrifuged at - 7700 x g for 10 min. The volume of the supernatant was measured and pooled with the first supernatant. Finally, the total protein concentration and the rhPBGD activity were analysed according to the procedures P 001 and E 001, respectively. From these analyses the specific rhPBGD activity was calculated.

*Porphobilinogen Deaminase, rhPBGD Assay, Procedure E001*

rhPBGD-activity was measured according to procedure E001. The procedure is based on the methods published by Jordan et al (1988, 1997) [(1) and (6)] .

*Protein determination, Procedure P001*

Protein concentrations were determined by procedure P001using the BCA protein assay kit (Pierce). Bovine serum albumin was used as a reference standard (Pierce Instructions, 1997) [(5)].

***SDS-polyacrylamide gel electrophoresis, Procedure S001***

rhPBGD was analysed by SDS-PAGE on Novex NuPAGE gels according to procedure S001. Electrophoresis System with NuPAGE 4-12 % gels was used for analysis (Novex, 1997, Laemmli 1970) [(2)].

A.I Process start

The final strain was not fully developed by Icogen Inc. at the initiation of the study (1/2/99). Hence the development was started with the intermediary strain designated PBGD-1, an *E. coli* K12 host strain JM105 with genotype endA thi rpsL sbcB15 hsdR4 Δ(lac-proAB) [F'traD36 proAB lacI$^q$ Δ(lacZ)M15 ] containing the expression plasmid pExp1-M2-BB [(8)]. When the final strain PBGD-2 was delivered, the process developed so far for the intermediary strain PBGD-1 was implemented on this strain. Both the PBGD-1 and 2 strains contain the expression plasmid pExp1-M2-BB encoding rhPBGD and the only difference between the strains is that the hem C gene (encoding endogenous PBGD) has been deleted in the PBGD-2 strain to facilitate the purification of rhPBGD.
Strain genotype and information about agar plate and shake flask cultivation from Icogen formed the basis for the first experiments at BioGaia Fermentation. Articles and discussions with HemeBiotech formed the basis for the analysis methods set up.

A II. Development of lab scale fermentation

The overall strategy for the development of the fermentation process was outlined as follows. The use of a minimal medium in the inoculum steps should facilitate the stability of the host, and a minimal medium supplemented with yeast extract and peptone should facilitate growth and production in the main fermentation. To reach high cell densities a concentrated glucose feed was used to control the growth rate in the feed phase. In the expression plasmid pExpl-M2-BB the rop gene has been deleted (8) which means that the expression of rhPBGD can be temperature regulated. Initially it was decided to start with a fermentation temperature of 30°C, which means that no temperature induction was used. If the productivity at this temperature was unsatisfactory the temperature could be increased to 37 °C or 42 °C to increase the productivity. Oxytetracycline was chosen as selection pressure, but if possible with regard to plasmid stability, the main fermentation should run without any selection pressure at all.

A.1 Initial batch experiments

The study was initiated 1/2/99 and the intermediary strain PBGD-1 was delivered 4/2/99. An initial M9H-Tc (6 $mgl^{-1}$)( attachment 2, table 2) shake flask cultivation (PD03) was performed to study the growth in the M9H-Tc (6 $mgl^{-1}$) inoculum medium recommended by Icogen Inc. A fermenter medium designated MM5Y-Tc (6 $mgl^{-1}$) was designed based on BioGaia Fermentations know how from other recombinant *E.coli* fermentations. This medium was first tested in a shake flask cultivation (PDO4) before two 1 L batch fermentations (PD05 and PD06) were performed with two variants of the medium. PD06 was performed in MM5Y-Tc (6 $mgl^{-1}$) substrate complemented with 2 $gl^{-1}$ tryptone (table 8) to investigate if tryptone could facilitate growth .
In a batch cultivation exponential growth continues for a relatively few generations until nutrients are depleted or toxic products accumulates. Due to this growth begins to slow and thereafter the micro organisms enter the stationary phase, where a steady state cell number is reached.

In all these batch experiments the initial glucose concentration was 10 $gl^{-1}$. The parameters defined below were analysed or calculated, and the results are summarised in table 34 below.

$$\text{Maximum growth rate } (\mu_{max}) \quad dX / dt = \mu \times X,$$

where X = Dry cell weight

Optical density ($OD_{620}$) The optical density of a cell suspension was measured by light transmission through it. The absorbance was measured at 620 nm with water as reference

Dry cell weight (Dw) Dry cell weight is determined from a known volume of cell suspension that is washed free of extraneous materials, dried in an oven, and then weighed

Colony forming units (CFU) The CFU technique involves growth of micro-organisms from a suspension on LB or TSA agar media (attachment 2 table 5 and 6). When a single micro organism divides on an agar medium, it forms a colony of cells, which can be seen by the naked eye

Glucose concentration Enzymatic analysis with a YSI 2000 instrument

Table 32. Summary of initial batch experiments with strain PBGD-1

| | PD03 | PD04 | PD05 | PD06 |
|---|---|---|---|---|
| Cultivation type | 250 ml shake flask | 250 ml shake flask | L fermentation | 1L fermentation |
| Substrate | M9H-Tc | MM5Y-Tc | MM5Y-Tc | MM5Y-Tc + 2 $gl^{-1}$ peptone |
| $\mu_{max}$ | 0,3 $h^{-1}$ | 0,3 $h^{-1}$ | 0,4 $h^{-1}$ | 0,4 $h^{-1}$ |
| Stationary $OD_{620}$ | 0,8 | 3,3 | 11 | 8 |
| Stationary Dw | 0,17 $gl^{-1}$ | 1,5 $gl^{-1}$ | 3,9 $gl^{-1}$ | 2,9 $gl^{-1}$ |
| Stationary CFU | $1*10^9$ $ml^{-1}$ | $1*10^9$ $ml^{-1}$ | $4*10^9$ $ml^{-1}$ | $3*10^9$ $ml^{-1}$ |
| Residual glucose | Not analysed | 6 $gl^{-1}$ | 0 $gl^{-1}$ | 0 $gl^{-1}$ |

In the shake flask cultivations growth stops before glucose is consumed and the growth rates and stationary values for $OD_{620}$, Dw and CFU are lower than in the controlled fermentations. The reason is the decreasing pH in the broth due to acetate formation during the shake flask cultivation (pH was not controlled). The shake flask cultivation in "rich" fermenter medium MM5Y-Tc allowed growth to higher stationary values compared to shake flask cultivation in M9H-Tc medium. In the fermentations growth stops because the glucose is depleted, and not due to decreasing pH, since pH was controlled at 7,0. A comparison of fermentations PD05 and PD06 indicates that the addition of 2 $gl^1$peptone to the MM5Y-Tc medium did not have any positive effect on the growth rate (attachment 3, fig 1.). Hence it was decided to use the MM5Y-Tc medium without any peptone.
The poor correlation between $OD_{620}$, Dw and CFU in the shake flask experiments can be explained by a combination of the following facts. The Dry weight determinations are difficult to perform at such low cell densities and the viability (CFU) of the cells can vary much since the cultivation conditions are not well controlled. Viability is a parameter that is influenced by many parameters in a complex way, and hence it is very difficult to get a good correlation between $OD_{620}$, Dw and CFU, especially in uncontrolled shake flasks with low cell densities.

Final statement: The strain PBGD-1 has the same growth rate (0,3 $h^{-1}$) in shake flasks with either M9H-Tc (6 $mgl^{-1}$) or MM5Y-Tc (6 $mgl^{-1}$) substrate. The growth rate in MM5Y-Tc (6 $mgl^{-1}$) substrate increases to 0,4 $h^{-1}$ when the

strain grows under controlled conditions in a fermenter. The MM5Y-Tc (6 $mgl^{-1}$) substrate supplements growth up to at least an $OD_{620}$ and a Dw of approximately 10 and 4 $gl^{-1}$ respectively.

A.2 Fed batch fermentations

A.2.1 Fed batch fermentations with strain PBGD-1

After the establishment of knowledge about some general strain characteristics during the initial batch experiments, the next step was to proceed to fed batch fermentations to reach higher cell densities and product concentrations. In a fed batch fermentation, a concentrated feed of the limiting substrate is fed into the fermenter at a rate ensuring that the respiration and heat evolution does not exceed the capacity of the fermenter the process is designed for. Hence, until any other substrate component is depleted or toxic by product accumulation limits growth, growth can proceed and the stationary phase is avoided. Eventually the increasing starvation of the limiting substrate (due to the increasing biomass) means the fermentation anyway will reach a stationary phase. However this occurs at much higher cell densities than in a batch phase. In this study the stationary phase was never reached in the fed batch fermentations and we are hence from now referring to final values instead.

The strategy was to start a concentrated glucose feed (600. $gl^{-1}$) when the initial 10 $gl^{-1}$ glucose was reduced below 0,5 $gl^{-1}$. Three feed batch fermentations were performed to establish a glucose feed profile matching the oxygen transfer in BioGaia Fermentations 1500 L production fermenter and not giving glucose accumulation in the broth. Glucose accumulation was defined as a glucose concentration > 0,1 $gl^{-1}$_glucose since this was the lower detection limit for real time glucose measurements in broth. Glucose accumulation occurs when the growth rate varies due to that various compounds in the yeast extract are utilised by the cells. A temporary drop in the growth rate leads to an accumulation of glucose if the glucose feed rate is based on a previous higher growth rate. When glucose accumulates in the broth, acetate production is likely to occur due to overflow metabolism. This means lower yields, but more importantly this can cause growth inhibition as a result of the acetate accumulation. Hence the feed profile has to be adjusted according to the actual growth profile of the strain. The three feed batch fermentations performed with strain PBGD-1 are summarised in table 35 below and graphs with growth and production of rhPBGD versus fermentation time are given in fig 26. and fig 27.

Table 33. Summary of feed batch experiments with strain PBGD-1

| | PD09 | PD11 | PD12 |
|---|---|---|---|
| type | 7 L fermentation | 7 L fermentation | 7 L fermentation |
| Substrate | MM5Y-Tc | MM5Y-Tc | MM5Y-Tc |
| Batch phase | 0-15,2 h | 0-13,9 h | 0-13,1 h |
| Feed phase | 15,2-20,7 h | 13,9-27,5 h | 13,1- 31,0 h |
| Achieved glucose feed profile | 70 $mlh^{-1}$ 15,2-20,7 h | 78 $mlh^{-1}$ 13,9-15,1 h<br>0 $mlh^{-1}$ 15,1-16,1 h<br>43 $mlh^{-1}$ 16,1-17,3 h<br>53 $mlh^{-1}$ 17,3-18,7 h<br>72 $mlh^{-1}$ 18,7-27,5 h | 43 $mlh^{-1}$ 13,1-17,5 h<br>90 $mlh^{-1}$ 17,5-18,2 h<br>60 $mlh^{-1}$ 18,2-19,2 h<br>0 $mlh^{-1}$ 19,2-22,3 h<br>75 $mlh^{-1}$ 22,3-23,3 h<br>28 $mlh^{-1}$ 22,3-31,0 h |
| Total glucose added | 313 g | 586 g | 430 g |
| Glucose accumulation | No measurements made during feed phase. | Peak value 3,4 $gl^{-1}$ between 13,9-16,0 h and | Peak values 1,0 $gl^{-1}$ 1,9 $gl^{-1}$ between 13,1- 22,0 h and 22,3-25,0 respectively |
| Final Dw | 14,0 $gl^{-1}$ | 28,8 $gl^{-1}$ | 19,2 $gl^{-1}$ |
| Final $OD_{620}$ | 37 | 82 | 59 |
| Final CFU | Not analysed | 3,5*$10^9$ $ml^{-1}$ | 6*$10^9$ $ml^{-1}$ |
| Final rhPBGD | Not analysed | 7,7 $Uml^{-1}$ | 15,3 $Uml^{-1}$ |
| Final specific rhPBGD | Not analysed | 2,6 $Umg^{-1}$ | 1,8 $Umg^{-1}$ |

Table continued

| | PD09 | PD11 | PD12 |
|---|---|---|---|
| Final plasmid stability | Not analysed | LB-Tc: 100% of growth on LB | LB-Tc: 100% of growth on LB |

The objective with fermentation PD09 was to use a constant glucose feed (600 $gl^{-1}$) rate of 75 $mlh^{-1}$ to reach a high cell density and a good production of rhPBGD. Unfortunately, practical limitations made it impossible to make any measurements during the batch phase. However, respiration (data not shown) during the fed batch phase seemed to be low in comparison to the glucose feed rate, and hence there was a possibility that glucose accumulation could have occurred.

The strategy was to use the same constant glucose feed rate (75 $mlh^{-1}$) in fermentation PD11 and monitor the glucose concentration every hour to ensure that no glucose accumulation occurred. Glucose analyses revealed glucose accumulation (3,4 $gl^{-1}$) directly after feed start (13,9 h) and hence the glucose feed was stopped until the cells consumed the excess glucose. When the glucose concentration decreased below 0,1 $gl^{-1}$ (16,1 h) the glucose feed was started again, however at a lower feed rate (43 $mlh^{-1}$). The glucose feed rate was then stepwise increased without any glucose accumulation until a final feed rate of 72 $mlh^{-1}$ was reached.

Based on the experience from PD11 it was decided to start the glucose feed (600 $gl^{-1}$) with a lower constant feed rate (40 $mlh^{-1}$) to avoid glucose accumulation directly after feed start. After 4 h the glucose feed rate should be increased to 75 $mlh^{-1}$. Despite the lower initial glucose feed rate a slow glucose accumulation occurred at the start of the glucose feed (13,1 h). Due to an operator mistake the feed rate was not decreased and was even increased according to the plan after 17,5 h. As soon as the operator realised the mistake the glucose feed rate was decreased (18,2 h) and stopped after 19,2 h. After 23,3 h the glucose feed was started at 75 ml/h but had to be decreased to 28 $mlh^{-1}$ h later to avoid glucose accumulation. The glucose feed was then kept at 28 $mlh^{-1}$ for the remaining fermentation.
From Table 33 it can be seen that the different glucose feed profiles determines the outcome of the fermentations. As expected the final $OD_{620}$ and Dw values correlates well to the total amount of glucose added to the fermenter. Again it is seen that the number of viable cells (CFU) is influenced by fermentation conditions in a complex way. The number of viable cells is lower in PD11 than in PD12 even though both $OD_{620}$ and Dw values are approximately 50 % higher in PD11. Also the difference between the rhPBGD expression in PD11 and PD12 is hard to explain, but also this indicates that the production of rhPBGD not only is linked to the cell density but also depends on the physiological status of the cells.

**Final statement:** The fermenter medium MM5Y-Tc (6 $mgl^{-1}$) supplemented growth up to $OD_{620}$ and Dw values of approximately 80 and 30 $gl^{-1}$ respectively. The expression levels of the rhPBGD had already at this point in the fermentation development reached acceptable levels. After the initial batch phase it was necessary to find an initial feed rate of the glucose feed (600 $gl^{-1}$) profile lower than 40 $mlh^{-1}$ and not giving glucose accumulation.

A.2.2 Fed batch fermentations with strain PBGD-2

The final strain PBGD-2 had the same expression plasmid pExp1-M2-BB encoding rhPBGD as strain PBGD-1, but the host cell was deleted for the hemC gene to facilitate rhPBGD purification._Since report existed of any differences in the properties of strain PBGD-2 in comparison to strain PBGD-1, the strategy was to implement this strain in the process developed for strain PBGD-1 so far. One difference was that a new inoculum procedure was introduced to shorten the initial batch phase. The main fermenter was inoculated with 500 ml broth from two 250 ml shake flasks cultivated 12 h instead of 250 ml from one incubated for 10 h. Three fed batch fermentations were performed to adjust the fermentation substrate and to adjust the glucose feed profile to the new strain. The results from these fermentations are summarised in table 36 and graphs with growth and production of rhPBGD versus fermentation time are given fig 28. and fig 29.

Table 34. Summary of feed batch experiments with strain PBGD-2

| | PD14 | PD16 | PD19 |
|---|---|---|---|
| Cultivation type | 7 L fermentation | 7 L fermentation | 7 L fermentation |
| Substrate | MM5Y-Tc + extra yeast extract and thiamine | MM20Y | MM20Y |
| Initial glucose conc. | 10 $gl^{-1}$ | 10 $gl^{-1}$ | 10 $gl^{-1}$ |
| Batch phase | 0-14,3 h | 0-10,8 h | 0-10,3 h |

Table continued

| | PD14 | PD16 | PD19 |
|---|---|---|---|
| Feed phase | 14,3-30,0 h | 10,8-30,0 h | 10,3- 30,0 h |
| Achieved glucose feed profile | 10 $mlh^{-1}$ 14,3-15,3 h<br>20 $mlh^{-1}$ 15,3-15,5 h<br>40 $mlh^{-1}$ 15,5-22,3 h<br>75 $mlh^{-1}$ 22,3-30,0 h | 25 $mlh^{-1}$ 10,8-15,0 h<br>50 $mlh^{-1}$ 15,0-16,8 h<br>37 $mlh^{-1}$ 16,8-17,3 h<br>0 $mlh^{-1}$ 17,3-18,3 h<br>50 $mlh^{-1}$ 18,3-18,7 h<br>34 $mlh^{-1}$ 18,7-19,6 h<br>37 $mlh^{-1}$ 19,6-20,0 h<br>40 $mlh^{-1}$ 20,0-22,6 h<br>50 $mlh^{-1}$ 22,6-23,0 h<br>55 $mlh^{-1}$ 23,0-24,3 h<br>60 $mlh^{-1}$ 24,3-25,3 h<br>75 $mlh^{-1}$ 25,3-30,0 h | 23 $mlh^{-1}$ 10,3-17,3 h<br>49 $mlh^{-1}$ 17,3-24,3 h<br>73 $mlh^{-1}$ 24,3-30,0 h |
| Total glucose added | 577 g | 610 g | 652 g |
| Glucose acc. | No accumulation | Peak values 1,8 $gl^{-1}$ and 0,3 $gl^{-1}$ between 15,0-18,0 h and 18,7-19,6 h respectively | No accumulation |
| Final Dw | 32,0 $gl^{-1}$ | Measurement error | Measurement error |
| Final $OD_{620}$ | 87 | 93 | 96 |
| Final CFU | 9*$10^9$ $ml^{-1}$ | 30*$10^9$ $ml^{-1}$ | 37*$10^9$ $ml^{-1}$ |
| Final rhPBGD | 39 $Uml^{-1}$ | 42 $Uml^{-1}$ | 66 $Uml^{-1}$ |
| Final spec. rhPBGD | 3,1 $Umg^{-1}$ | 1,9 $Umg^{-1}$ | 4,8 $Umg^{-1}$ |
| Final plasmid stability | Not analysed | 102 % of growth on LB | 100 % of growth on LB |

Based on the results from PD12, it was decided to implement a step wise increasing glucose feed profile (25 $mlh^{-1}$ 0-4 h after feed start, 50 $mlh^{-1}$ 4-8 h after feed start and finally 75 $mlh^{-1}$ for the remaining fermentation) to avoid glucose accumulation in fermentation PD14. However, during the end of the batch phase a dramatic decrease in the growth rate (observed by $OD_{620}$, $pO_2$ (dissolved oxygen tension), $CO_2$ and $O_2$ trends) was observed (data not shown). Since glucose analysis showed that there was no glucose limitation (glucose conc. was 5,7-0,7 $gl^{-1}$) the hypothesis was that the initial yeast extract or thiamine was depleted. After 14,5 h an addition of 35 g yeast extract and 21 mg thiamine was made and growth was re-established. Hence this test verified the postulated hypothesis. After 16 h another decrease in growth was observed and a second addition of 35 g yeast extract and 21 mg thiamine was made (20,3 h). Growth was again re established and continued until 24 h, when again a week tendency of decreasing growth was observed. Since interpretation of the growth patterns was difficult at this stage, only 4 g of yeast extract was added to study the response of the addition. Since there was no clear response, it was not thought that any more additions were necessary during the rest of the fermentation. The depletion of yeast extract and/or thiamine made it necessary to alter the intended glucose feed profile to avoid glucose accumulation.

Based on the results from fermentation PD14 a new fermentation substrate MM20Y-Tc (attachment 2, table 4) was designed to avoid depletion of yeast extract or thiamine in fermentation PD16. In the new substrate the concentrations of yeast extract and thiamine was increased from 5 $gl^{-1}$ and 1 $mgl^{-1}$ to 20 $gl^{-1}$ and 10 $mgl^{-1}$ respectively.

Plasmid stability and rhPBGD production without oxytetracycline as selection pressure in the main fermenter was also tested in PD16. The concentration of oxytetracycline (6 $mgl^{-1}$) in the inoculum shake flasks was however kept the same as in the earlier experiments. The change to the new MM20Y substrate resulted in a smooth growth pattern during the whole fermentation. The intention was to keep the same glucose feed profile as originally planned for fermentation PD14. Hence the glucose feed was started at 25 $mlh^{-1}$ after 10,8 h. After 15 h when the glucose feed rate was increased to 50

$mlh^{-1}$ glucose started to accumulate in the broth. The glucose feed rate was decreased after 16 h (0,7 g/l) and stopped after 17 h since the glucose concentration in the broth still was increasing (1,8 $gl^{1}$). After 18 h when the accumulated glucose was consumed (< 0,1 $gl^{-1}$) the glucose feed was started at 50 $mlh^{-1}$ and again glucose accumulation was observed (0,3 $gl^{-1}$). The feed rate was now decreased to and kept at 34 $mlh^{-1}$ until the excess glucose was consumed and a stable situation without glucose accumulation was established. Finally the glucose feed rate was increased step wise to a final value of 75 $mlh^{-1}$ according to table 36 above.

To avoid the glucose accumulation observed in PD16 the glucose feed profile was changed for PD19 to the following; 25 $mlh^{-1}$ 0-7 h after feed start, 50 $mlh^{-1}$ 7-14 h after feed start and 75 $mlh^{-1}$ during the rest of the fermentation. Since there was no sign of plasmid loss or decrease in rhPBGD production in PD16 it was decided to exclude oxytetracycline in the main fermenter also in experiment PD19.

In PD19 the new glucose feed profile did not give raise to any glucose accumulation, and hence no changes in the planned glucose profile was necessary. As in PD16 no plasmid loss was observed and the rhPBGD production reached even higher values than in PD16. Also in these fermentations the final $OD_{620}$ values correlates well with the total amount of glucose added to the fermentation. A measurement error makes it impossible to compare the results of the dry weight determinations. The number of viable cells (CFU) was greatly improved when changing to the new substrate composition MM20Y, and shows a fairly good reproducibility between PD16 and PD19. There is no clear trend for the rhPBGD production, but it seems to be a tendency that the rhPBGD production is positively influenced by both the increases in cell density and viability in fermentations PD16 and PD19.

**Final statement:** The fermenter medium MM5Y-Tc (6 $mgl^{-1}$) was not able to supplement strain PBGD-2 up to the $OD_{620}$ and Dw values reached with strain PBGD-1. The new designed fermenter medium MM20Y with increased concentrations of yeast extract and thiamine was able to supplement growth up to $OD_{620}$ and Dw of approximately 90 and 30 $gl^{-1}$ respectively. The expression of rhPBGD was in these fermentations at very good levels. After the initial batch phase the final glucose feed (600 $gl^{-1}$) profile for a 7 L fermentation was designed like follows: 25 $mlh^{-1}$ 0-7 h after feed start, 50 $mlh^{-1}$ 7-14 h after feed start and 75 $mlh^{-1}$ 14-21 h after feed start. There were no negative effects on plasmid stability and rhPBGD expression when excluding oxytetracycline as selection pressure in the main fermenter.

A.3 III. Scale up of fermentation

The scale up part of the development was divided into two parts. First simulated large scale fermentations were performed in laboratory fermenters to study the effect of the increasing number of generations on plasmid stability and product quality. These tests were followed by the actual 850 L fermentations to investigate the effects from increasing the fermentation scale on plasmid stability and product quality.

BioGaia Fermentations 1500 L production fermenters are normally inoculated with broth from one 14 L fermenter. To mimic the fermentations PD14-PD19 the $OD_{620}$ after inoculation should be approximately 0,1. When using a working volume of 9 L in the he 14 L fermenter the final $OD_{620}$ necessary to achieve the same inoculum conditions in 850 L can be calculated as follows

$$OD_{620} \times 9\ L = 0{,}1 \times 850\ L \rightarrow OD_{620} = 9{,}4$$

Inoculating the 14 L fermenter containing 9 L substrate with 500 ml broth (two 1 L shake flasks) with an $OD_{620}$ of approximately 1,0 gives an $OD_{620}$ initial of approximately 0,1-0,2. With a growth rate of 0,4 $h^{-1}$ it will take 9-11 h to reach an $OD_{620}$ of about 9. Since a growth rate of 0,4 $h^{-1}$ is equivalent to a generation time of 1,7 h (ln2/0,4) this corresponds to 5-6 extra generations compared to the earlier lab scale fermentations.

An estimation of the number of generations in different steps of the entire process is given in table 11 below. The extra inoculum fermentation increases the total number of generations in the process with 15-25 %, an increase that could have effects on plasmid stability and product quality.

Table 35. Estimated number of generations in different process steps

| Process step | Estimated number of generations |
|---|---|
| M9H-Tc agar plates | 8 - 12 |
| 0,25 L M9H-Tc Shake flasks | 4 - 5 |
| 9 L Inoculum fermentation | 5 - 6 |
| 850 L Main fermentation | 9 - 11 |

Table continued

| Process step | Estimated number of generations |
|---|---|
| Σ Total process | 26 - 34 |

A.3.1 Results from simulated large scale fermentations

It was decided to use a rich substrate MM5Y-Tc (6 $mgl^{-1}$) in the inoculum fermenter for two reasons. The first reason was that an increase in the growth rate from 0,3 $h^{-1}$ to 0,4 $h^{-1}$ would decrease the process time with approximately 5 h. The second reason was the hesitation whether or not the M9H-Tc (6 $mgl^{-1}$) substrate could support growth up to $OD_{620}$ values in the range 7-10. Table 36 below compares the results from the simulated large scale fermentations with the developed lab scale process and the growth and rhPBGD production versus tim-e are shown in, fig 30 and fig 31.

Table 36. Results from simulated large scale fermentations in comparison to the developed lab scale process.

| | PD19 | PD21 | PD22 |
|---|---|---|---|
| Cultivation type | 7 L fermentation | Simulated large scale: 9 L inoculum + 7 main fermentation | Simulated large scale: 9 L inoculum + 13 L main fermentation |
| Substrate | MM20Y | MM20Y | MM20Y |
| Initial glucose conc. | 10 $gl^{-1}$ | 10 $gl^{-1}$ | 10 $gl^{-1}$ |
| Batch phase | 0-10,3 h | 0-9,25 h | 0-8,50 h |
| Feed phase | 10,3- 30,0 h | 9,25-30,0 h | 8,50-30,0 h |
| Achieved glucose feed profile | 23 $mlh^{-1}$ 10,3-17,3 h<br>49 $mlh^{-1}$ 17,3-24,3 h<br>73 $mlh^{-1}$ 24,3-30,0 h | 20 $mlh^{-1}$ 9,25-11,0 h<br>24 $mlh^{-1}$ 11,0-13,3 h<br>25 $mlh^{-1}$ 13,3-16,3 h<br>41 $mlh^{-1}$ 16,3-20,3 h<br>43 $mlh^{-1}$ 20,3-23,3 h<br>65 $mlh^{-1}$ 23,3-25,0 h<br>70 $mlh^{-1}$ 25,0-30,0 h | 43 $mlh^{-1}$ 8,5-10,0 h<br>40 $mlh^{-1}$ 10,0-13,0 h<br>50 $mlh^{-1}$ 13,0-14,0 h<br>45 $mlh^{-1}$ 14,0-15,3 h<br>60 $mlh^{-1}$ 15,3-16,0 h<br>80 $mlh^{-1}$ 16,0-17,0 h<br>85 $mlh^{-1}$ 17,0-22,3 h<br>150 $mlh^{-1}$ 22,3-23,3 h<br>121$mlh^{-1}$ 23,3-24,0 h<br>135 $mlh^{-1}$ 24,0-25,0 h<br>130 $mlh^{-1}$ 25,0-26,0 h<br>135 $mlh^{-1}$ 26,0-27,0 h<br>150 $mlh^{-1}$ 27,0-28,0 h<br>130 $mlh^{-1}$ 28,0-29,0 h<br>150 $mlh^{-1}$ 29,0-30,0 h |
| Total glucose added | 652 g | 622 g | 1303 g (702 g )* |
| Glucose acc. | No accumulation | No accumulation | No accumulation |
| Final Dw | Measurement error | 36,9 $gl^{-1}$ | 38,8 $gl^{-1}$ |
| Final $OD_{620}$ | 96 | 96 | 95 |
| Final CFU | 37*$10^9$ $ml^{-1}$ | 21* $10^9$ $ml^{-1}$ | 46*$10^9$ $ml^{-1}$ |

Table 36. Results from simulated large scale fermentations in comparison to the developed lab scale process (continued).

| | | | |
|---|---|---|---|
| Final rhPBGD | 66 $Uml^{-1}$ | 66 $Uml^{-1}$ | 69 $Uml^{-1}$ |
| Final spec. rhPBGD | 4,8 $Umg^{-1}$ | 4,2 U $mg^{-1}$ | 3,8 U $mg^{-1}$ |
| Final plasmid stability | 100 % of growth on LB | 100 % of growth on LB | 96 % of growth on LB |
| *recalculated to a 7 L fermentation | | | |

There was still a rather good correlation between the amount of glucose added to the fermentation (when adjusted for the differences in volume) and the final Dw and $OD_{620}$ values. When comparing the results from fermentations PD21 and PD22 with PD19 there is a very good reproducibility in all measured parameters. Since PD19 when the final glucose feed profile was fixed the reproducibility in CFU values has improved greatly. This is due to that we now have more controlled and similar conditions during the fermentations. The levels of rhPBGD expression and the plasmid stability are very good. Based on the SDS Page assay it was not possible to detect any differences in the produced rhPBGD from PD21 and PD22 when compared to PD19.

Final statement: The 5-6 extra generations introduced in the inoculum procedure do not have any influence on the growth pattern, productivity or quality of the produced rhPBGD. The fermentation process seems to be very reproducible.

A.3.2 850 L Scale Up fermentations

Two scale up fermentations were performed according to the outline of the simulated large scale fermentations. The only differences were that all 9 L broth from the inoculum fermenter were used for inoculation and that the broth was used directly for inoculation instead of being stored at 0-8 °C for approximately 1,5 h as in the simulated large scale fermentations. Results from the scale up fermentations are summarised in table 39 and the growth and rhPBGD production versus time are shown in fig32 and fig 32.

Table 37. Results from 850 L Scale up fermentations in comparison to the developed lab scale fermentation

| | PD19 | PD1501 | PD1502 |
|---|---|---|---|
| Cultivation type | 7 L fermentation | Scale Up fermentation 9 L inoculum + 850 L main fermentation | Scale Up fermentation 9 L inoculum + 850 L main fermentation |
| Substrate | MM20Y | MM20Y | MM20Y |
| Initial glucose conc. | 10 $gl^{-1}$ | 10 $gl^{-1}$ | 10 $gl^{-1}$ |
| Batch phase | 0-10,3 h | 0-9,0 h | 0-8,5 h |
| Feed phase | 10,3- 30,0 h | 9,0-30,0 h | 8,5-28,0 h |
| Glucose feed profile | 23 $mlh^{-1}$ 10,3-17,3 h<br>49 $mlh^{-1}$ 17,3-24,3 h<br>73 $mlh^{-1}$ 24,3-30,0 h | Averaged values from process data<br>3,3 $lh^{-1}$ 9,00 - 16,5 h<br>5,5 $lh^{-1}$ 16,5 - 23,5 h<br>10,5 $lh^{-1}$ 23,5 - 30,0 h | Averaged values from process data<br>3,3 $lh^{-1}$ 9,00 - 15,5 h<br>5,7 $lh^{-1}$ 15,5 - 22,5 h<br>9,8 $lh^{-1}$ 22,5 - 28,0 h |
| Tot. glucose added | 652 g | 89,5 kg ( 737 g )* | 79,9 kg (658 g )* |
| Glucose acc. | No accumulation | No accumulation | No accumulation |
| Final Dw | Measurement error | 40,7 $gl^{-1}$ | 37,7 $gl^{-1}$ |
| Final $OD_{620}$ | 96 | 111 | 106 |
| Final CFU | $37*10^9$ $ml^{-1}$ | $89*10^9$ $ml^{-1}$ | $140*10^9$ $ml^{-1}$ |
| Final rhPBGD | 66 $Uml^{-1}$ | 62 $Uml^{-1}$ | 59 $Uml^{-1}$ |

Table continued

| | PD19 | PD1501 | PD1502 |
|---|---|---|---|
| Final spec. rhPBGD | 4,8 $Umg^{-1}$ | 3,8 U $mg^{-1}$ | 4,1 U $mg^{-1}$ |
| Final plasmid stability | 100 % of growth on LB | 100 % of growth on LB | 99 % of growth on LB |
| *Recalculated to 7 L | | | |

To get conditions similar to batch PD1501 in the down stream processing it was decided to stop the fermentation at an $OD_{620}$close to the final value in fermentation PD1501. Hence PD1502 was stopped after 28 h instead of 30 h. One common explanation to this kind of differences in growth pattern are quality differences between different batches of yeast extract, even from the same manufacturer.

When comparing the fermentations PD1501 and PD1502 with PD19 there is a very good reproducibility in all measured parameters, except for the CFU values. It seems like the amount of viable cells increases dramatically when increasing the fermentation volume (i.e. changing from homogeneous well mixed conditions in a lab scale fermenter to a inhomogeneous production fermenter where the cells encounter constantly fluctuating conditions). This is a phenomenon that has been reported for other fermentation processes as well, but on the other hand there are also examples of the opposite. The viability of a population of cells are influenced by a many parameters in a complex way and is impossible to explain the variations based on these very limited experiments performed during this study. No changes in the pattern on the SDS Page gel indicated any changes in the quality of the produced rhPBGD. A comparison of the SDS-Page gels from PD22 (13 L), PD1501(850 L) and PF1502 (850 L) are shown in fig 34. Also the plasmid stability and the rhPBGD productivity was unaffected by the increase in scale.

Final statement: The scale up from a 7-13 L lab fermentation to a 850 L production fermentation do not have any influence on the growth pattern, productivity or quality of the produced rhPBGD. The developed process is very reproducible even when comparing lab scale and large scale fermentations.

A.4 Results from IV. Development and Scale-up of down stream process

A.4.1 Cell concentration

In a series of experiments the broth was concentrated 1,9-6,9 times. The lower concentration factors reflected problems with clogging of the membrane surface. The optimal concentration factor was about 2. Such a low concentration factor demands a large buffer volume for dia filtration. The dia filtration volume has to be at least two times the concentration volume to exchange about 90 % of the substrate in the broth. No differences were seen in dia filtration results using different buffers (table 31).

**Final statement**: As a 1000 K filter was chosen for the cell debris removal (see 7.4.3) the same filter was used for cell concentration for practical reasons when scaling up to production scale. The broth was then concentrated around 2 times with a dia filtration volume of two times the concentration volume with a buffer consisting of 50 mM sodium-phosphate, 1,34 mM EDTA, pH 7,4. Permeate-flux was set at 15 $lm^{-2}$ $h^{-1}$ and the temperature controlled between 15 and 25°C (PD1501-PD1502).

A.4.2 Homogenisation

There were no significant differences in protein concentration and rhPBGD-activity using different pressures and number of passages. However, after three passages at 800 bar, disruption of cells was considered to be optimum based on viscosity and results from cell debris removal. After one passage the homogenate was highly viscous due to the presence of unfragmented genomic DNA. The viscosity decreases significantly after further passages from shearing of the DNA into smaller parts. This is important to prevent clogging on the membrane surface. An Increasing number of passages gave less viscous homogenate (table 40).

**Final statement**: Parameters chosen for scaling up to production scale were 800 bar, 3 passages since disruption of cells was then maximised. Homogenate between passages was kept in a tank with a refrigerated jacket. To facilitate the cell debris removal, it was decided to dilute the homogenate 2,5 times after the third passage. A buffer consisting of 50 mM sodium-phosphate, 1,34 mM EDTA, pH 7,4 was used for the dilution. Between passages temperature at the homogenate in the tank was controlled between 15 and 25°C (PD1501-PD1502).

**Table 38. Homogenisation of E-coli cells containing rhPBGD-activity, Batch PD19**

| Pressure Bar | Number of passages | Total protein mg/ml | Activity U/ml | Comments |
|---|---|---|---|---|
| 800 | 1 | 14,2 | 67 | Viscous |
| 600 | 3 | 14,6 | 72 | Not viscous |
| 800 | 3 | 15,5 | 79 | Not viscous |
| 1000 | 3 | 14,8 | 76 | Not viscous |

A.4.3 Cell debris removal

When centrifuged extracts exceeded 10 mg protein $ml^{-1}$ problems with precipitation and a significant decrease in pH occurred. Membrane filtered extracts did not give the same problems with precipitation as centrifuged extracts. When testing different buffers with different pH during cell debris removal it was obvious that the ionic strength of the buffer had to be at least 50 mM, with a pH around 7,4 to avoid later decrease in pH. It was also concluded that rhPBGD was more stable in buffers with a pH around 7,4 than pH 8,2. The final choice of buffer was 50 mM sodium-phosphate, 1,34 mM EDTA, pH 7,4.

The produced extract was dark pink in colour. When standing at room temperature with vigorous stirring extract became brownish-red in a couple of hours. This was probably due to an oxidation process taking part in the extract.

The transmission of proteins through the membrane was low using a 0,2 μm filter. The rhPBGD activity yield was only around 20 %. The yield was however affected positively by changing the homogenisation parameters from one passage to three passages (table 41).

Table 39 . Summary of rhPBGD yield after cell debris removal using different pressures during homogenisation. Filter used was 0,2 μm, Batch PD19.

| Pressure Bar | Number of passages | Yield Total protein mg/ml | Yield Activity U/ml | Specific activity U/mg protein |
|---|---|---|---|---|
| 800 | 1 | 1,2 | 4 | 3,3 |
| 600 | 3 | 1,6 | 7 | 4,3 |
| 800 | 3 | 3,5 | 16 | 4,6 |
| 1000 | 3 | 1,7 | 10 | 5,9 |

Exchanging the micro filter (0,2 μm) for ultra filters (500 K and 1000 K) resulted in less fouling at the filter surface and protein concentration increased to acceptable yields (Table 156). The smaller membrane area and flatter surface minimised product hold up and adsorption, which in this case increases yields. Furthermore, the transmission of total protein seems to be lower than the transmission of rhPBGD when using an ultra filter (1000 K), resulting in a higher specific activity (table 16). In all experiments the homogenate was dia filtered to the same theoretical yield (90%) to enable comparison of the results.

Final statement: For scaling up to production scale a 1000 K filter was chosen for cell debris removal since this filter gave a good yield of rhPBGD with a high specific activity. Diluted homogenate was decided to be concentrated approximately 2,5 times and then dia filtered with 50 mM sodium-phosphate, 1,34 mM EDTA, pH 7,4 to get a theoretical yield of rhPBGD of about 95 %. Nitrogen was flushed over the permeate surface in order to prevent oxidation (PD1502). Permeate-flux was set at 15 l $m^{-2}h^{-1}$ and the temperature was set at 15- 25°C (PD1501-PD1502).

Table 40. Summary of rhPBGD yield from Batch PD21 using different filters during cell debris removal. Homogenisation parameters: 800 bar, 3 passages. The yield of rhPBGD from membrane filtration was compared to centrifuged material.

| Cell debris removal by | Yield Total protein | | Yield Activity | | Specific activity **U/mg protein** |
|---|---|---|---|---|---|
| | mg/ml | %* | U/ml | %* | |
| 0,2 μm filter | 2,2 | 30 | 7 | 21 | 3,3 |
| 500 K filter | 4,8 | 65 | 22 | 69 | 4,6 |
| 1000 K filter | 3,1 | 42 | 21 | 66 | 6,8 |

Table continued

| Cell debris removal by | Yield Total protein mg/ml | %* | Yield Activity U/ml | %* | Specific activity **U/mg protein** |
|---|---|---|---|---|---|
| centrifuged | -- | 80 | -- | 90 | 5,1 |
| * Yield is given in % compared to homogenate. | | | | | |

A.4.4 Final filtration

Membrane filtered extracts contained less particles and was thereby easier to filter than centrifuged extract, where problems with clogging on the filter surface occurred. The clogging made it difficult to perform integrity tests. A white slippery precipitate was always seen in extract before the final filtration. When analysing the dissolved precipitate spectrofotometrically at $OD_{260}$ /$OD_{280}$ resulting in a ratio near 2, it was concluded that it contained nucleic acid.

A.4.5 Scale up of down stream process (PD22, PD1501 and PD1502)

The entire final process was at first tested at a 15 L scale (batch PD22) ending with a rhPBGD yield of 75 %. When scaling up to 300 L(PD1501) problems with precipitation in the broth occurred and the rhPBGD yield decreased to 46 %. When processing batch PD1502 no precipitation was seen and the rhPBGD yield increased to 77 %. The results are summarised in tables 43 and 44.

The low yields of rhPBGD from batch PD1501 was probably due to several factors:

Using the same filter unit at cell concentration and cell debris removal commonly saves both money and time but when a white precipitate occurred in the broth it resulted in problems cleaning the filter between cell concentration and cell debris removal. The composition of the white insoluble precipitate was analysed and the results are shown in table 19. To avoid the precipitate formation in PD1502 the substrate preparation was carefully monitored. No new component was added until the former component was completely dissolved. No precipitate was formed in batch PD1502.

The filter area was small in comparison to the processed volume, which increased the chances for clogging on the filter surface. Dia-filtration was only performed to achieve a theoretical yield, rhPBGD, of 90 %.

In batch PD1502 the concentration of rhPBGD in the extract was low compared to batch PD22 but the yield was slightly higher. The lower concentration was due to an operator mistake using an increased dia filtration volume at cell debris removal during the process of PD1502. If a smaller dia filtration volume during cell debris removal in batch PD1502 had been used it would have resulted in a higher concentration of rhPBGD but the yield had then decreased. The concentration of rhPBGD was also higher in starting material (homogenate) from batch PD22 and PD1501 than from PD1502. Furthermore a the specific activity in the extracts from batch PD22 and PD1501 are higher than in the extract from PD1502.

To compare rhPBGD-protein amount with rhPBGD-activity, samples from different stages in the process were analysed with SDS-PAGE according to procedure S001. In all batches tested (PD22, PD1501 and PD1502) a major band was seen in a position corresponding to a molecular weight around 40kD and with almost the same mobility as the rhPBGD-His standard. We therefor conclude that this band corresponds to rhPBGD and is responsible for the rhPBGD-activity measured in the samples 34.

Table 41. Parameters of the final extraction process for the last laboratory batch PD22 and the two production batches PD1501 and PD1502

| Batch | PD22 | PD1501 | PD1502 |
|---|---|---|---|
| Volume broth processed (l) | 15 | 300 | 300 |
| Filter used for Cell concentration | 1000K, 0,5 $m^2$ | 1000K, 4 $m^2$ | 1000K, 8 $m^2$ |
| Permeate Flow rate (Flux) Cell concentration | 11,0 $lm^{-2}h^{-3}$ | 14,5 $lm^{-2}h^{-1}$ | 13,9 $lm^{-2}h^{-1}$ |
| Homogenisation parameters | 800 bar, 3 passages | 800 bar, 3 passages | 800 bar, 3 passages |
| Filter used for Cell debris removal | 1000K, 0,5 $m^2$ | 1000K, 4 $m^2$ | 1000K, 8 $m^2$ |
| Permeate Flow rate (Flux) Cell debris removal | 11,6 $lm^{-2}h^{-1}$ | 11,5 $lm^{-2}h^{-1}$ | 12,0 $lm^{-2}h^{-2}$ |
| CF (concentration factor cell debris removal) | 2,0 | 2,2 | 2,5 |

Table continued

| Batch | PD22 | PD1501 | PD1502 |
|---|---|---|---|
| Dia-filtration cell debris removal % theoretical yield | 93 | 90 | 98 |
| Volume extract after cell debris removal | 25 liter | 450 liter | 670 liter |
| Dilution factor extract volume /broth | 1,7 | 1,5 | 2,2 |
| volume | | | |
| Filter used for sterile-filtration | Sartobran P, 0,2 $m^2$ | Durapore CVGL71 | Durapore CVGL71 |

Table 42. Results from the extraction of the last laboratory and the two production batches using the final process.

| **Batch** | **PD22** | **PD1501** | **PD1502** |
|---|---|---|---|
| Homogenate protein (mg/ml) | 17,4 | 18,6 | 15,0 |
| Homogenate activity (U/ml; kU) | 82; 1280 | 71; 25420 | 51; 17950 |
| Specific activity, homogenate (U/mg) | 4,7 | 3,8 | 3,4 |
| Extract protein (mg/ml; g) | 7,5; 261 | 5,3; 2370 | 5,0;3300 |
| Extract (U/ml; kU) | 38; 950 | 25;11620 | 21; 13860 |
| Yield protein from homogenate (%) | 72 | 36 | 62 |
| Yield activity from homogenate (%) | 74 | 46 | 77 |
| Specific activity, extract (U/mg) | 5,1 | 4,7 | 4,2 |

Table 43 Composition of the white insoluble precipitate from broth (Batch PD1501).

| **Composition** | **mg/kg dry weight** |
|---|---|
| Sodium (Na) | 510 |
| Potassium (K) | 5 800 |
| Calcium (Ca) | 900 |
| Magnesium (Mg) | 190 000 |
| Phosphate (P) | 230 000 |
| | |

Final Statement: The scale up of the extraction process from a 15 L laboratory scale to a 300 L production scale, with a longer product hold up, seems not to have any important influence on the quality of the extract produced. However, it should be mentioned that in stability studies, extract from the production scale batch PD1501 was more stabile over time in the freezer than the laboratory batch PD22 (See 7.A.4.6).

A.4.6 Stability studies, rhPBGD-activity

When the final extract was stored in freezer (-20°C) extract from the final laboratory batch (PD22) gave a decrease in activity of approximately 10 percent monthly while extract from a production batch (PD1501) resulted in a 3 percent decrease in activity over the same time (Fig 35).

Evaluation and conclusions

We have developed a fermentation and down stream process for production of sterile filtered permeate containing recombinant human Porphobilinogen Deaminase (rhPBGD) on a commercial scale. The process is briefly outline

as follows.

The fermentation process is started with cells from cryo vials stored in an ultra freezer at a temperature < -70 °C. In all inoculum steps (agar plates, shake flasks and inoculum fermenter) 6 $mgl^{-1}$ oxytetracycline is used as selection pressure to ensure a good plasmid stability. In all process steps the temperature is 30 °C. The minimal medium agar plates are inoculated with cells from the cryo vial and are incubated for 24 ± 4 h. Two 1 L shake flasks are inoculated with growth from 1 ½ agar plate each and are incubated for 13 ± 1 h before the broth is pooled and used to inoculate an14 L inoculum fermenter. The inoculum fermenter containing 9 L minimal medium supplemented with 5 $gl^{-1}$ yeast extract. When $OD_{620}$ is 7-10 (after approximately 9 h) the broth is transferred to the 1500 L production fermenter containing 850 L minimal medium supplemented with 20 $gl^{-1}$ yeast extract and 10 $mgl^{-1}$ thiamine. No selection pressure is used in the production fermenter. After an initial batch phase (approximately 8 h) growth is controlled by a stepwise increasing glucose feed (600 $gl^{-1}$) profile. Totally about 120 L glucose (600 $gl^{-1}$) solution is fed into the fermenter and together with the $NH_3$ (25% w/w) and $MgSO_4$ $7H_2O$ also fed into the fermenter this gives a final fermentation volume of about 1000 L. The fermentation is stopped when $OD_{620}$ is 100 ± 20 (approximately 30 h) and the broth is cooled down to 20-25 °C before downstream processing starts. At the end of fermentation the dry cell weight is about 40 $gl^{-1}$ and the rhPBGD activity is about 60 $Uml^{-1}$.

The process described for rhPBGD extraction from the PBGD-2 strain of *E.coli* cells is based largely on the use of membrane filtration with a 1000 K ultra filter both for cell concentration and cell debris removal. During cell concentration and cell debris removal the temperature is controlled between 15 and 25 °C. Disruption of cells is accomplished by homogenisation at 800 bar for 3 passages at ambient temperature. Between passages homogenate is kept in a tempered (15-25°C) tank. Finally extract is filtered through a 0,22 μm retentive filter into autoclaved containers. By means of the process developed, rhPBGD can be extracted from 300 L broth in less than 30 hours with an overall yield of about 75 % with a concentration in the extract of approximately 25-30 U/ ml.

The 850 L fermentation process developed gives a very good and reproducible expression of rhPBGD without temperature induction. Plasmid stability is good even without selection pressure in the 1500 L production fermenter. No negative Scale up effects was encountered during Scale up. The 300 L down stream process is reproducible and has a good overall yield. The down stream process is foreseen to be easy to further scale up to give a process taking care of all 1000 L broth produced in the fermentation. Hence the developed process are therefore at this stage found suitable for large scale production of rhBPGD.

List of figures

Fig 25. Comparison of fermentations PD05 and PD06 with strain PBGD-2.
Fig 26. Comparison of fermentations PD09, PD11 and PD12
Fig 27: Comparison of fermentations PD09, PD11 and PD12 with strain PBGD-1.
Fig 28. Comparison of fermentations PD14, PD16 and PD19 with strain PBGD-2.
Fig 29. Comparison of fermentations PD14, PD16 and PD19 with strain PBGD-2.
Fig 30. Comparison of fermentations PD19, PD21 and PD22 with strain PBGD-2.
Fig 31. Comparison of fermentations PD19, PD21 and PD22 with strain PBGD-2.
Fig 32. Comparison of fermentations PD19, PD1501 and PD1502.
Fig 33. Comparison of fermentations PD19, PD1501 and PD1502 with strain PBGD-2.
Fig34. Summary of Fermentation and down stream process visualised by SDS-Page. Comparison between different samples
Fig 35. Stability studies: Single use aliquots of extract were routinely taken out of the freezer (-20°C) and the rhPBGD-activity was measured and plotted over time.

**Example 4**

Stability data for rhPBGD

Stability study 1 - Selection of formulation buffer

To find the best suitable formulation conditions for rhPBGD-His, the enzyme was formulated in a phosphate buffer containing mannitol and glycine as protein stabilizers. Different pH, ion strength and enzyme concentration were investigated, Table 44. The study was performed at 40°C and 75% relative humidity during 8 weeks.

Table 44. Formulations

| Sample no. | pH | Ion strength (mM) | rhPBGD-His conc. (mg/ml) |
|---|---|---|---|
| 1a, 2c, 3a | 7,5 | 10 | 2 |
| 1b | 7,5 | 10 | 8 |
| 2a | 6,5 | 10 | 2 |
| 2b | 7,0 | 10 | 2 |
| 2d | 8,0 | 10 | 2 |
| 2e | 8,5 | 10 | 2 |
| 3b | 7,5 | 50 | 2 |
| 3c | 7,5 | 100 | 2 |

The samples were sterile filtered and aliquots of 170 μl were dispensed into 300 μl glass vials with a Teflon/silicon crimp cap. Samples were collected for analysis on week 0, 1, 2, 4 and 8 and were analysed for enzyme activity (enzyme activity assay), protein concentration (HPLC) and degradation/aggregation (HPLC, SDS-PAGE and IEF).

The enzyme precipitated in all samples except for sample no. 2e (pH 8,5). Sample no. 1b (8 mg/ml) showed a higher precipitation rate than samples with lower enzyme concentration. This sample turned yellow after one week, all other samples turned yellow after 2 weeks. The HPLC chromatograms showed that a prepeak to the rhPBGD-His peak was formed during storage at 40ºC, and that this prepeak increase with time. The peak for pure rhPBGD-His decreased with time. Figure 1, shows the amount enzyme calculated from the area under the peak in HPLC chromatograms.

The enzyme activity, Figure 37, decreased from 16 Units/ml (U/ml) to 2 U/ml in 8 weeks, for sample no. 1b the decrease in enzyme activity was more pronounced the first week, 40 U/ml to 10 U/ml, corresponding to the precipitation seen in Figure 44. The specific enzyme activity is shown in Figure 3. It seemed like high protein concentration was detrimental for the enzyme activity (sample no. 1b).

SDS-PAGE gels showed bands from aggregates as well as from scissoring after one week for all samples.

On the isoelectric focusing, IEF, gels four bands corresponding to the four catalytic forms of the enzyme (E, E1, E2 and E3) were seen on day 0. During storage at 40°C it seemed like the first band (E) was weakened and the second band (E1), which is more acidic was getting stronger. The number of more acidic bands increased over time. This was probably due to deamidation.

Conclusion

rhPBGD-His was not stable at 40°C. However, the only formulation buffer in which no visible precipitation of the enzyme was detected was no. 2e (pH 8,5). High concentration of the enzyme seemed to be critical for both precipitation and enzyme activity. All samples turned yellow as well as precipitated, except for no. 2e that just turned yellow. The decreased enzyme activity can for example be a result of enzyme aggregation, scissoring and/or deamidation.

Stability study 2 - Storage temperature

This study was performed on rhPBGD without the five histidines. Stability study 1 showed that rhPBGD-His has its best stability in pH 8,5. Since rhPBGD has its isoelectric point about 0,5 pH units lower than the histidine tagged enzyme a formulation buffer with pH 8,0 was chosen in this study. The formulation buffer is described in Table 45.

Table 45. Formulation buffer for rhPBGD, 3,67mM phosphate buffer with mannitol and glycine, pH 8 and ion strength 10mM.

| Excipient | | Concentration (mM) |
|---|---|---|
| | $Na_2HO_4•2H_2O$ | 3,16 |
| | $NaH_2PO_4•H_2O$ | 0,51 |
| | Glycine | 27 |
| | Mannitol | 222 |

The samples were prepared as described in stability study 1. The study was performed over 12 weeks using two concentrations of rhPBGD, 10 mg/ml and 2 mg/ml. The examined temperatures were -20°C ± 5°C, 5°C ± 3°C and 25°C ± 2°C. The material was also tested for stability when freeze/thawed. The assays were the same as in stability study 1.

The enzyme concentration was relatively stable in this study compared to the stability study 1, Figure 47. Precipitation was only seen at 25°C and only for the high concentrated formulation. The precipitation appeared after one week, at the same time as a yellow colour. After eight weeks the high concentrated formulation stored at 5°C had turned yellow. For the 2 mg/ml formulation the yellow colour appeared only at 25°C, and then not until after eight weeks of storage. (The fluctuations seen in Figure 47 was probably due to variability in the dilution of the samples and integration of HPLC chromatograms.)

Regarding the enzyme activity there was no significant difference between the two concentrations or the temperatures -20°C and 5°C, Figure 41 and 42. The samples stored at 25°C showed a lower final enzyme activity.

The material used in this study showed at time 0 two bands at higher molecular weight than pure rhPBGD and one with lower molecular weight, on SDS-PAGE gels. This pattern did not change for any of the samples during the 12 weeks.

On the IEF gels there were two extra bands for samples stored at 5°C and 25°C, at lower pH than the original bands. These bands were more pronounced in the 10 mg/ml samples. For the samples stored at 5°C these bands appeared after 4 weeks and for the samples stored at 25°C after 1 week. This might be due to deamidations.

Conclusion

The rhPBGD formulation was best preserved at -20°C over 12 weeks. Samples stored at 5°C showed changes after 4 weeks on IEF gels, not recorded for -20 samples. Except for this 5°C seemed acceptable for storage of the rhPBGD formulation over 12 weeks.

Stability study 3 - Simulation of toxicology selection study

This 12 weeks study was performed using the same formulation buffer as in stability study 2, Table 45. The purpose of this study was to certify that the rhPBGD should be stable during the animal selection study. The formulation for the 58 days long selection study will be kept frozen (-20°C) until the day before use. At this point it will be thawed at 5°C over night. During use it will probably be stored at room temperature (25°C). In this study a formulation with a concentration of 10 mg/ml of rhPBGD will be used.

The samples were sterile filtered and aliquots of 400 µl sample were dispensed into 3 ml glass vials with rubber stopper. The vials were flushed with nitrogen and subsequently caped with a plastic seal. A formulation with 10 mg/ml of rhPBGD was used, and its stability at -20°C ± 5°C for 12 weeks, at 5°C ± 3°C for 4 weeks and at 25°C ± 2°C for 2 weeks was examined. The protein concentration in the samples was in this study measured using BCA, Figure 43.

The variability in protein concentration seen in the HPLC measurements was reduced using this method. However, the variability in the data seen in Figure 43 was probably due to variability in dilution of the samples.

This high concentrated formulation was from origin yellowish in colour. After 8 weeks, the formulation had become yellow. No precipitation was seen after 8 weeks (the study is not finished).

The enzyme activity was stable during the investigated time period at each temperature, Figure 44. This was true even for the specific enzyme activity, Figure 45. There were some fluctuations, however these were probably assay related.

The material used in this study showed form origin two bands at higher molecular weight than pure rhPBGD and one with lower molecular weight, on SDS-PAGE gels. One weak extra band at even lower molecular weight appeared after 2 weeks at 25°C and after 4 weeks at 5°C. This band had not appeared after 8 weeks at -200C.

The samples showed at time 0 two extra bands on the IEF gels. These were more acidic than the E3 band, however very weak,. For samples stored at 5°C and at 25°C the intensity of these bands increased over time.

Conclusion

The rhPBGD will stay stable during the selection study if treated as described. Thus, rhPBGD is stable at -20°C for 8 weeks, and except for deamidaion at 5°C for 4 weeks and at 25°C for 2 weeks. Note, that this study was performed using nitrogen in the test vials.

<u>References:</u>

Anderson P. M. and R. J. Desnick. 1979, Purification and Properties of Uroporphyrinogen I Synthase from

Human Erythrocytes: Identification of Stable Enzyme-Substrate Intermediates The Journal of Biological Chemistry 255(5) :1993-99

Andersson, Christer, Thesis, 1997, ISBN 91/7191/280/0, pp. 22-23

Amann, E., Brosius, J., and Ptashne, M. 1983, Vectors bearing a hybrid trp-lac promoter useful for regulated expression of cloned genes in Escherichia coli. *Gene* 25(2-3):167-178

Awan S.J. et al. 1997, Reconstitution of the Holoenzyme Form of *E. coli* Porphobilinogen Deaminase from Apoenzyme with Porphobilinogen and Preuroporphyrinogen: A Study Using Circular Dichroism Spectroscopy Biochemistry 36 (30) :9273-82

Gold, L. and Stormo, G.D. 1990, High-level translation initiation. *Methods Enzymol.* 185 : 89-93: 89-93

Grandchamp B. et al. 1987, Tissue-specific expression of porphobilinogen deaminase. Two isoenzymes from a single gene. Eur J Biochem. Jan;162(1):105-10

Grandschamp B. et al. 1996, J. of Gastroenerology and Hepatology 11, 1046-1052

Herrick A.L. et al. 1989, Lancet 1, 1295-1297

Jeans J. et al. 1996, American J. of Medical Genetics, 65, 269-273

Jordan P.M., S.D. Thomas and M.J. Warren. 1988, Purification, crystallization and

properties of porphobilinogen deaminase from a recombinant strain of *Escherichia coli* K12. Biochem. J. 254: 427-435

Jordan, P.M. The biosynthesis of uroporphyrinogen III: mechanism of action of porphobilinogen deaminase. In: The biosynthesis of the tetrapyrrole pigments 1994 Wiley, Chister (Ciba Found Symp 180), p70-96

Lambert R. et al. 1994, Structural studies on porphobilinogen deaminase. In: The biosynthesis of the tetrapyrrole pigments. Wiley, Chichester (Ciba Found Symp 180), p97-110

Lewis, L.A., Li, K.B., Gousse, A., Pereira, F., Pacheco, N., Pierre, S., Kodaman, P., and Lawson, S. 1991, Genetic and molecular analysis of spontaneous respiratory deficient (res- ) mutants of Escherichia coli K-12. *Microbiol.Immunol.* 35(4):289-301

Lithner F. et al. 1984, Acta.Med.Scand. 215, 271-274

Louie, G.V. et al. 1996, The three-dimensional structure of Escherichia coli porphobilinogen deaminase at 1.76-A resolution Proteins 25(1):48-78

Makrides, S.C. 1996, Strategies for achieving high-level expression of genes in Escherichia coli. *Microbiol.Rev.* 60(3):512-538

Maniatis T., E.F. Fritsch, J. Sambrook. Molecular Cloning (A laboratory Manual) Cold Spring Harbor Laboratory. 1982

Miller et al. 1989, BioTechniques 7, 980-990

Miyagi K. et al. 1979, Uroporphyrinogen I synthase from human erythrocytes: Separation, purification, and properties of isoenzymes. Proc. Natl. Acad. Sci. 76:12 6172-76

Morrison D.A. 1979, Transformation and preservation of competent bacterial cells by freezing. *Methods Enzymol.* 68, 326-331.

Mustajoki et al. 1989, Sem. Hematol. 26, 1-9

Raich N. et al. 1986, Molecular cloning and complete primary sequence of erythrocyte porphobilinogen deaminase. Nucleic Acids Research 14(15): 5955-67

Raich N. et al. 1986, Nucleic. Acid. Res, 14, 5955-5968

Saiki R.K. et al. 1985, Enzymatic amplification of beta-globin genomic sequences and restriction site analysis for diagnosis of sickle cell anemia. Science, 20;230(4732):1350-4.

Sassa S. 1996, Blood Review, 10, 53-58

Shoolingin-Jordan P.M., M.J. Warren and S.J. Awan, 1997, Dipyrromethane Cofactor Assembly of Porphobilinogen Deaminase: Formation of Apoenzyme and Preparation of Holoenzyme. Methods in Enzymology 281: 317-327

Stephens P.E. et. al. Biochem J. 248, 1-11, 1987

Strand et al. 1970, Proc. Natl. Acad. Sci. 67, 1315-1320

Tishler P.V. et al. 1985, Am.J.Psychiatry 142,1430-1436

Waldenström J. 1937, Acta.Med. Scand. Suppl.82

Welland F.H. et al. 1964, Metabolism, 13, 232

Wetterberg L. 1967, Svenska bokförlaget Nordstedt, Stockholm

Wetterberg L. 1976, In Doss M. Nowrocki P. eds. Porphyrias in Human Disease. Reports of the discussion. Matgurg an der Lahn, 191-202

Yeung L. et al. 1983, Q J. Med 52, 92-98

Yoo H.W. et al. 1993, Hydroxymethylbilane synthase: complete genomic sequence and amplifiable polymorphisms in the human gene. Genomics Jan; 15(1):21-9

References for the method relating to example 7 (A)

(1) Jordan P.M. et al (1988) Purification, crystallisation and properties of porphobilinogen deaminase from a recombinant strain of Escherichia coli K12.Biochem. J 254, 427-435
(2) Laemmli U.K. (1970) Cleavage of structural proteins during the assembly of the head of bacteriophage T4. Nature 227, 680-685
(3) Novex (1997) NuPAGE Electrophoresis System - Instruction Booklet.
(4) Oort P.J. Purification of Recombinant HIS-Tagged Human Erythropoietic Porphobilinogen Deaminase (rPBGD-HIS) Report number 3
(5) Pierce Instructions (1997) BCA Protein assay Kit, version 4/1997
(6) Shoolingin-Jordan P.M. et al. (1977) Dipyrromethane cofactor assembly of porphobilinogen deaminase: Formation of apoenzyme and preparation of holoenzyme. Methods in Enzymology 281, 317-327
(7) Technical Report Master and Working Cell Bank Development, HemeBiotech Internal no: 01000043
(8) Development of expression system for recombinant human porphobilinogen deaminase, HemeBiotech Internal no: 0199003

## Claims

1. An *EcoR I - Hind* III linear DNA fragment as shown In Seq. ID NO:2.

2. An expression plasmid pExp1-M2-BB as shown In Seq. ID NO: 1.

3. An E. coli host strain which is *hemC* defective and is obtainable by transformation with a DNA fragment according to claim 1

4. A production strain, which is a strain according to claim 3 transformed with an expression plasmid encoding rhPBGD.

5. A production strain which is PBGD-2 (DSM 12915).

**6.** A method for the preparation of rhPBGD by a method comprising

a) introducing, into a suitable vector, a nucleic add fragment which includes a nucleic add sequence encoding PBGD;
b) transforming a compatible host cell, according to claim 3 with the vector;
c) culturing the transformed host cell under conditions facilitating expression of the nucleic acid sequence;
d) recovering the expression product from the culture.

**7.** A method for the preparation of rhPBGD according to claim 6 the method comprising culturing a production strain according to claim 4 under conditions facilitating expression of rhPBGD and recovering the rhPBGD from the culture.

**8.** A method according to claims 6 or 7, further comprising a purification step

**9.** A method according to claim 8 wherein the purification is performed with a His-Tag (rhPBGD-His).

**10.** A method for purification of rhPBGD-His as defined in claim 9 from a bacterial crude lysate, said method comprising DEAE ion exchange chromatography and cobalt affinity chromatography.

**Patentansprüche**

**1.** Lineares EcoR I-*Hind* III-DNA-Fragment, wie es in SEQ ID NO:2 gezeigt ist.

**2.** Expressionsplasmid pExp1-M2-BB, wie es in SEQ ID NO:1 gezeigt ist.

**3.** E.coli-Wirtsstamm, der hemC-defekt ist und durch Transformation mit einem DNA-Fragment gemäß Anspruch 1 erhältlich ist.

**4.** Produktionsstamm, der ein Stamm gemäß Anspruch 3 ist, der mit einem Expressionsplasmid transformiert ist, das für rhPBGD codiert.

**5.** Produktionsstamm, der PBGD-2 ist (DSM 12915).

**6.** Verfahren zur Herstellung von rhPBGD durch ein Verfahren, umfassend

a) Einführen eines Nucleinsäurefragments, das eine Nucleinsäuresequenz umfasst, die für PBGD codiert, in einen geeigneten Vektor;
b) Transformieren einer kompatiblen Wirtszelle nach Anspruch 3 mit dem Vektor;
c) Kultivieren der transformierten Wirtszelle unter Bedingungen, die eine Expression der Nucleinsäuresequenz erleichtern;
d) Gewinnen des Expressionsprodukts aus der Kultur.

**7.** Verfahren für die Herstellung von rhPBGD nach Anspruch 6, wobei das Verfahren umfasst: Kultivieren eines Produktionsstamms nach Anspruch 4 unter Bedingungen, die eine Expression von rhPBGD erleichtern, und Gewinnung des rhPBGD aus der Kultur.

**8.** Verfahren nach Anspruch 6 oder 7, das außerdem einen Reinigungsschritt umfasst.

**9.** Verfahren nach Anspruch 8, wobei die Reinigung mit einem His-Tag (rhPBGD-His) durchgeführt wird.

**10.** Verfahren zur Reinigung von rhPBGD-His, wie in Anspruch 9 definiert, aus einem bakteriellen Rohlysat, wobei das Verfahren DEAE-Ionenaustausch-Chromatographie und Kobaltaffinitätschromatographie umfasst.

**Revendications**

**1.** Fragment d'ADN linéaire EcoR I-*Hind* III. tel que représenté dans Seq. ID N° : 2.

**2.** Plasmide d'expression pExp1-M2-88 tel que représenté dans Seq. ID N° : 1.

**3.** Souche hôte de E. *coli* qui est déficiente en *hemC* et qui peut être obtenue par transformation avec un fragment d'ADN selon la revendication 1.

**4.** Souche de production, qui est une souche selon la revendication 3 transformée avec un plasmide d'expression codant pour rhPBGD.

**5.** Souche de production qui est PBGD-2 (DSM 12915).

**6.** Méthode de préparation de rhPBGD par une méthode comprenant :

a) l'introduction, dans un vecteur adapté, d'un fragment d'acide nucléique qui comprend une séquence d'acide nucléique codant pour PBGD ;
b) la transformation d'une cellule hôte compatible, selon la revendication 3, avec le vecteur ;
c) la culture de la cellule hôte transformée dans des conditions facilitant l'expression de la séquence d'acide nucléique ;
d) la récupération du produit d'expression de la culture.

**7.** Méthode de préparation de rhPBGD selon la revendication 6, la méthode comprenant la culture d'une souche de production selon la revendication 4 dans des conditions facilitant l'expression de rhPBGD et la récupération de rhPBGD de la culture.

**8.** Méthode selon les revendications 6 ou 7, comprenant en outre une étape de purification.

**9.** Méthode selon la revendication 8, dans laquelle la purification est réalisée avec une étiquette His (rhPBGD-His).

**10.** Méthode de purification de rhPBGD-His tel que défini dans la revendication 9 d'un lysat bactérien brut, ladite méthode comprenant une chromatographie par échange d'ions sur DEAE et une chromatographie d'affinité sur cobalt.

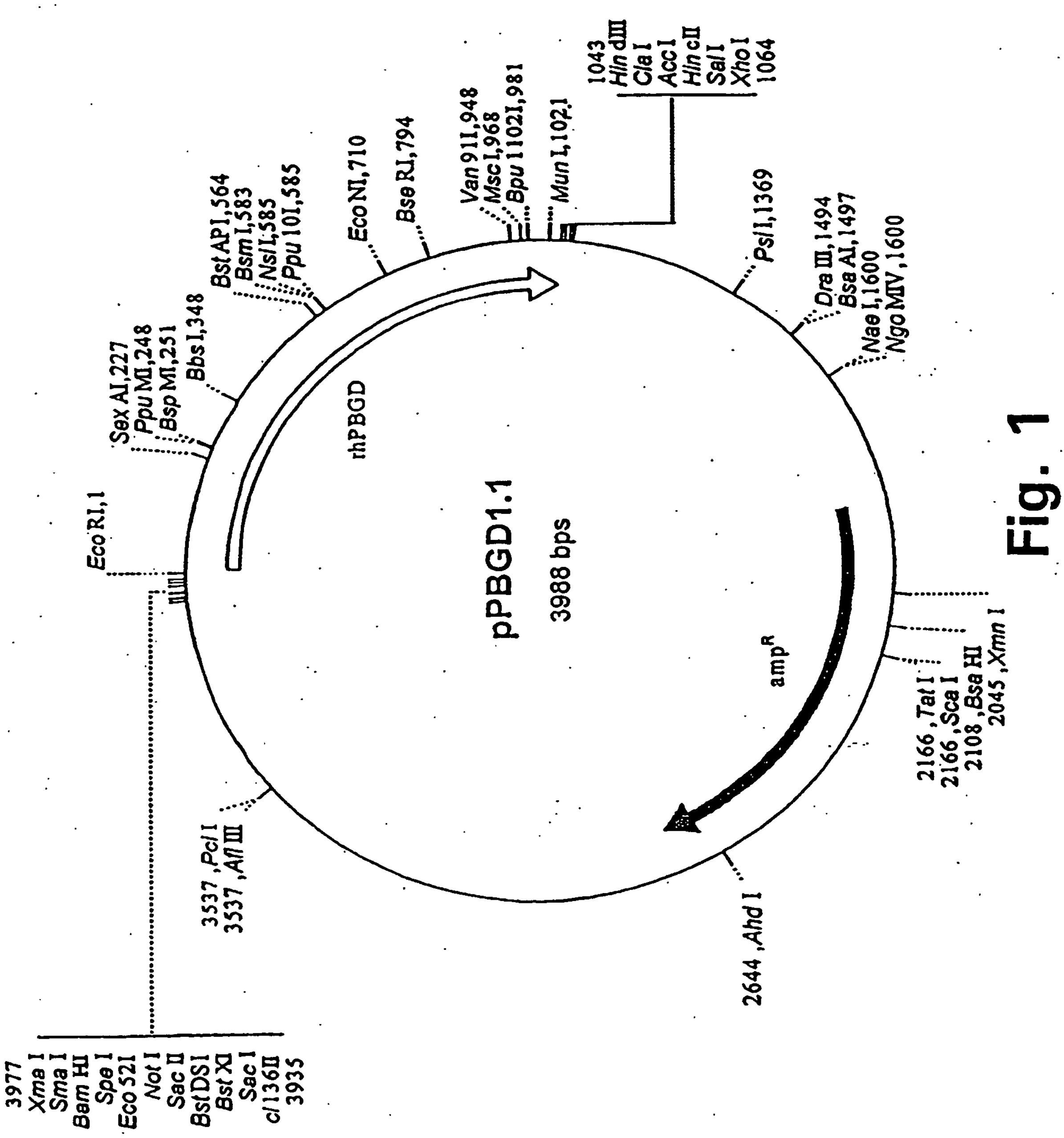
pPBGD1.1
3988 bps
rhPBGD
amp^R
EcoRI,1
SexAI,227
PpuMI,248
BspMI,251
BbsI,348
BstAPI,564
BsmI,583
NsiI,585
Ppu10I,585
EcoNI,710
BseRI,794
Van91I,948
MscI,968
Bpu1102I,981
MunI,1021
1043
HindIII
ClaI
AccI
HincII
SalI
XhoI
1064
PstI,1369
DraIII,1494
BsaAI,1497
NaeI,1600
NgoMIV,1600
2166 ,TatI
2166 ,ScaI
2108 ,BsaHI
2045 ,XmnI
2644 ,AhdI
3537 ,PclI
3537 ,AflIII
3977
XmaI
SmaI
BamHI
SpeI
Eco52I
NotI
SacII
BstDSI
BstXI
SacI
cl136II
3935

Fig. 1

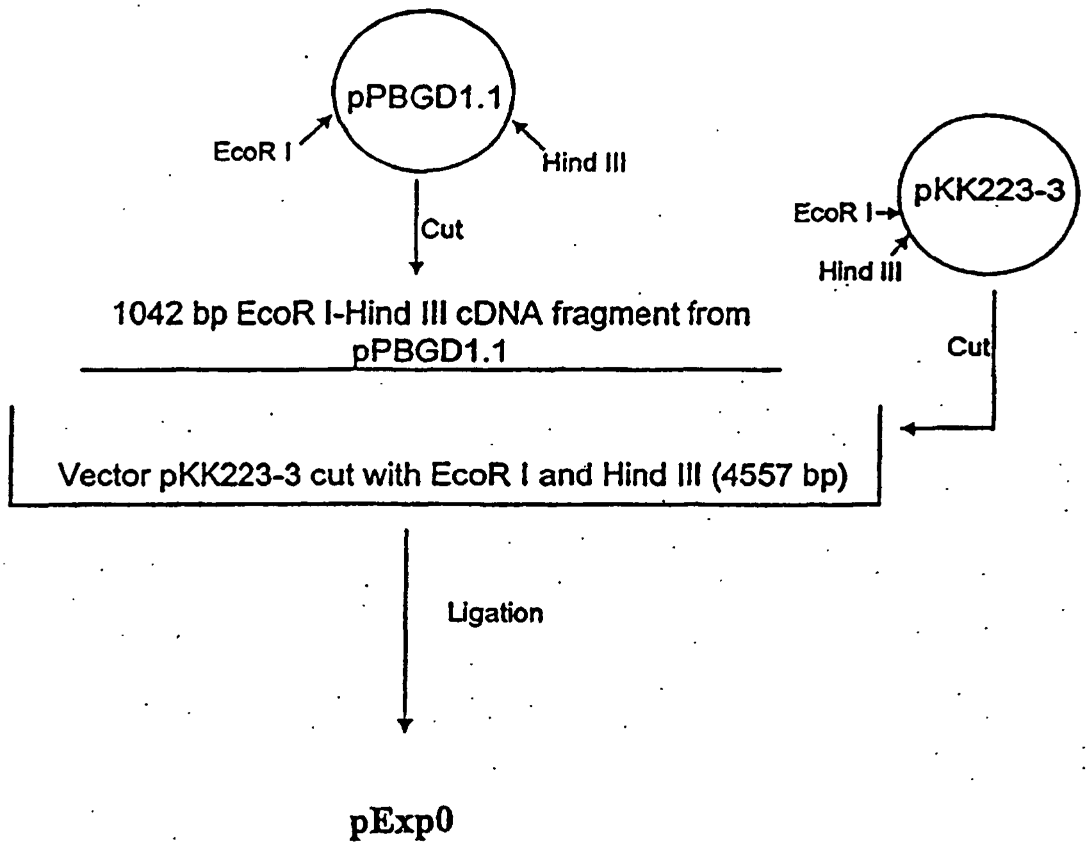
pPBGD1.1
EcoR I
Hind III
Cut
pKK223-3
EcoR I
Hind III
1042 bp EcoR I-Hind III cDNA fragment from pPBGD1.1
Cut
Vector pKK223-3 cut with EcoR I and Hind III (4557 bp)
Ligation
pExp0

Fig. 2

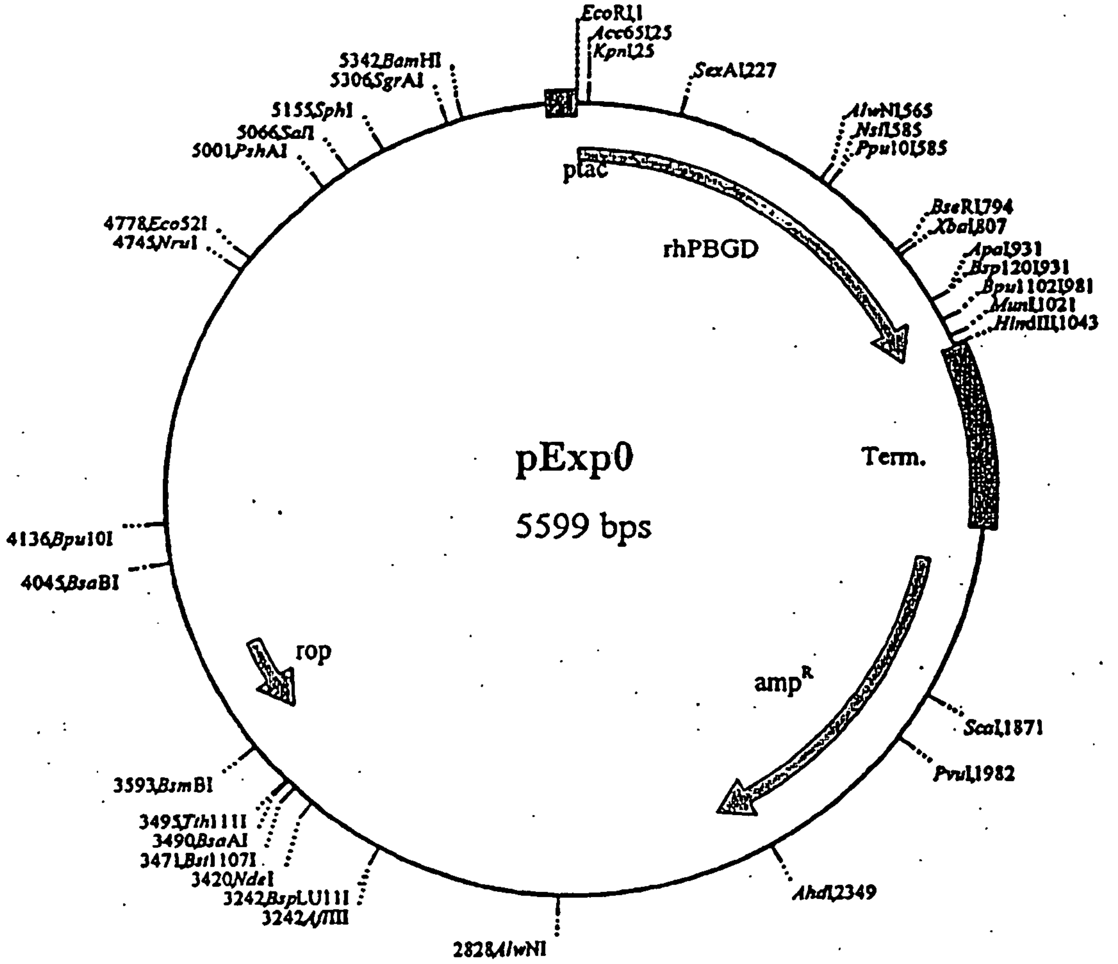
pExp0
5599 bps
ptac
rhPBGD
Term.
amp^R
rop
EcoRI,1
Acc65I,25
KpnI,25
SexAI,227
AlwNI,565
NsiI,585
Ppu10I,585
BseRI,794
XbaI,807
ApaI,931
Bsp120I,931
Bpu1102I,981
MunI,1021
HindIII,1043
ScaI,1871
PvuI,1982
AhdI,2349
2828,AlwNI
3242,AflIII
3242,BspLU11I
3420,NdeI
3471,Bst1107I
3490,BsaAI
3495,Tth111I
3593,BsmBI
4045,BsaBI
4136,Bpu10I
4745,NruI
4778,Eco52I
5001,PshAI
5066,SalI
5155,SphI
5306,SgrAI
5342,BamHI

# Fig. 3

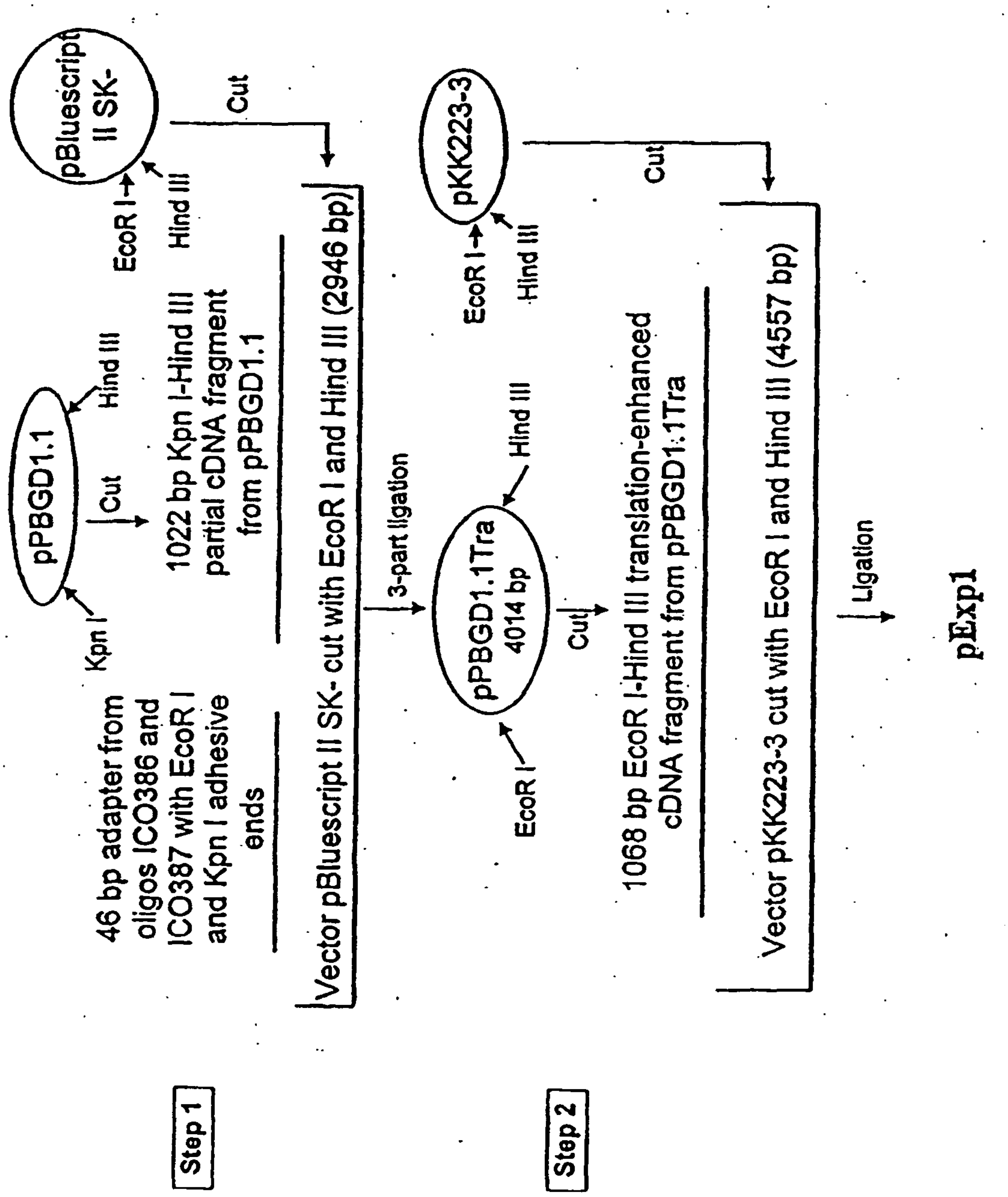
Step 1
46 bp adapter from oligos ICO386 and ICO387 with EcoR I and Kpn I adhesive ends
Kpn I
pPBGD1.1
Hind III
Cut
1022 bp Kpn I-Hind III partial cDNA fragment from pPBGD1.1
pBluescript II SK-
EcoR I
Hind III
Cut
Vector pBluescript II SK- cut with EcoR I and Hind III (2946 bp)
3-part ligation
Step 2
EcoR I
pPBGD1.1Tra 4014 bp
Hind III
Cut
1068 bp EcoR I-Hind III translation-enhanced cDNA fragment from pPBGD1.1Tra
pKK223-3
EcoR I
Hind III
Cut
Vector pKK223-3 cut with EcoR I and Hind III (4557 bp)
Ligation
pExp1

Fig. 4

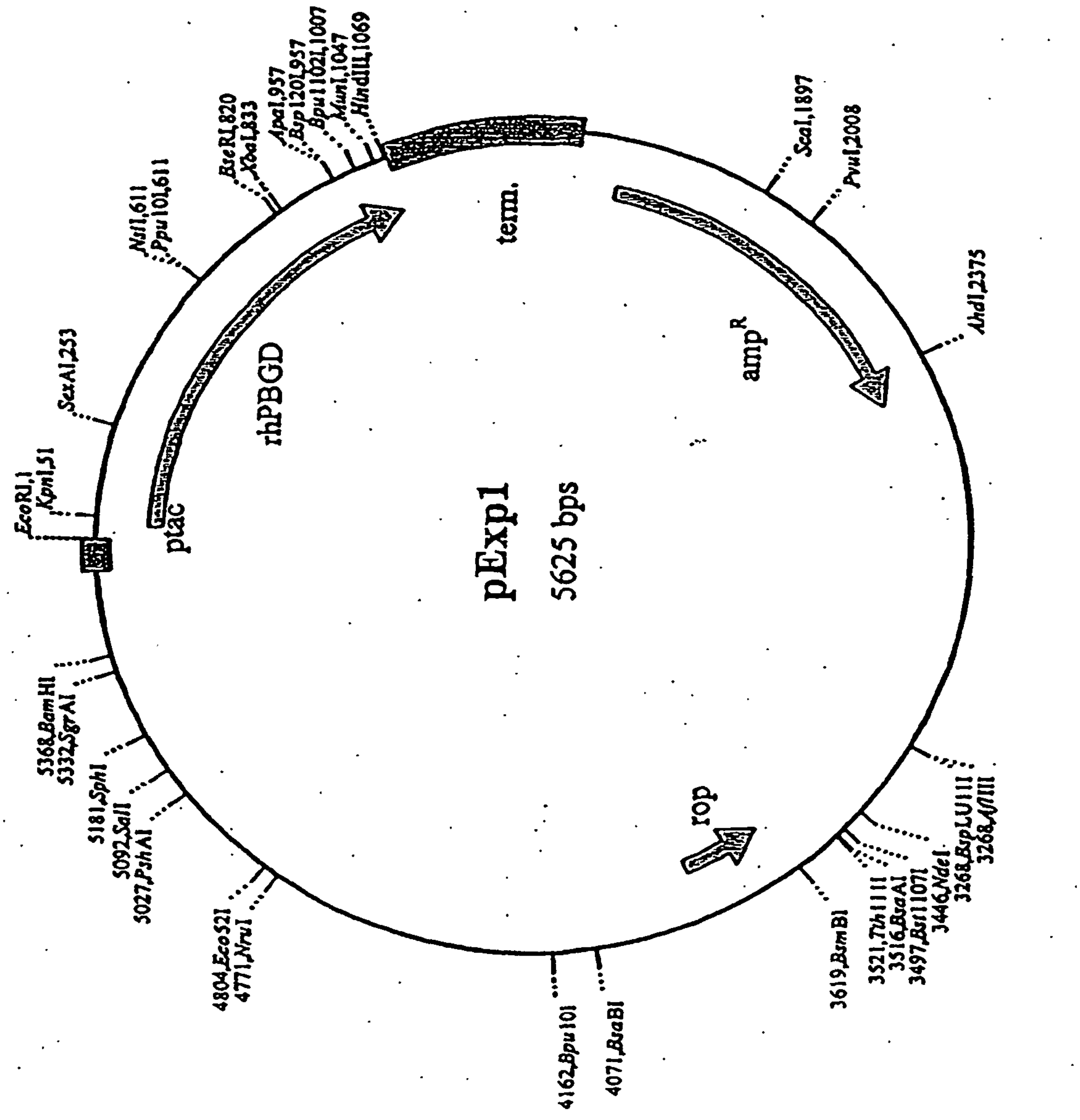
pExp1
5625 bps
ptac
rhPBGD
term.
amp^R
rop
EcoRI,1
KpnI,51
SexAI,253
NsiI,611
Ppu10I,611
BseRI,820
XbaI,833
ApaI,957
Bsp120I,957
Bpu1102I,1007
MunI,1047
HindIII,1069
ScaI,1897
PvuI,2008
XhoI,2375
3268,AflIII
3268,BspLU11I
3446,NdeI
3497,Bst1107I
3516,BsaAI
3521,Tth111I
3619,BsmBI
4071,BsaBI
4162,Bpu10I
4771,NruI
4804,Eco52I
5027,PshAI
5092,SalI
5181,SphI
5332,SgrAI
5368,BamHI

Fig. 5

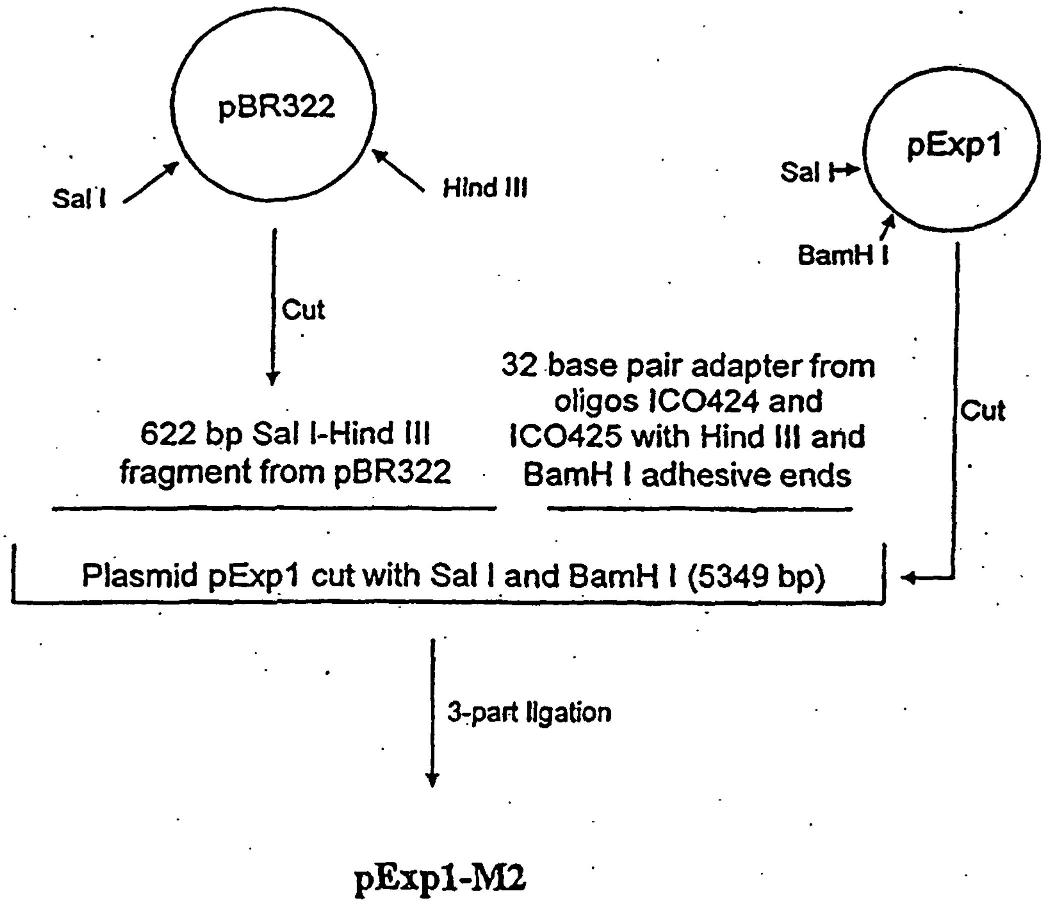
pBR322
Sal I
Hind III
pExp1
Sal I
BamH I
Cut
Cut
622 bp Sal I-Hind III fragment from pBR322
32 base pair adapter from oligos ICO424 and ICO425 with Hind III and BamH I adhesive ends
Plasmid pExp1 cut with Sal I and BamH I (5349 bp)
3-part ligation
pExp1-M2

Fig. 6

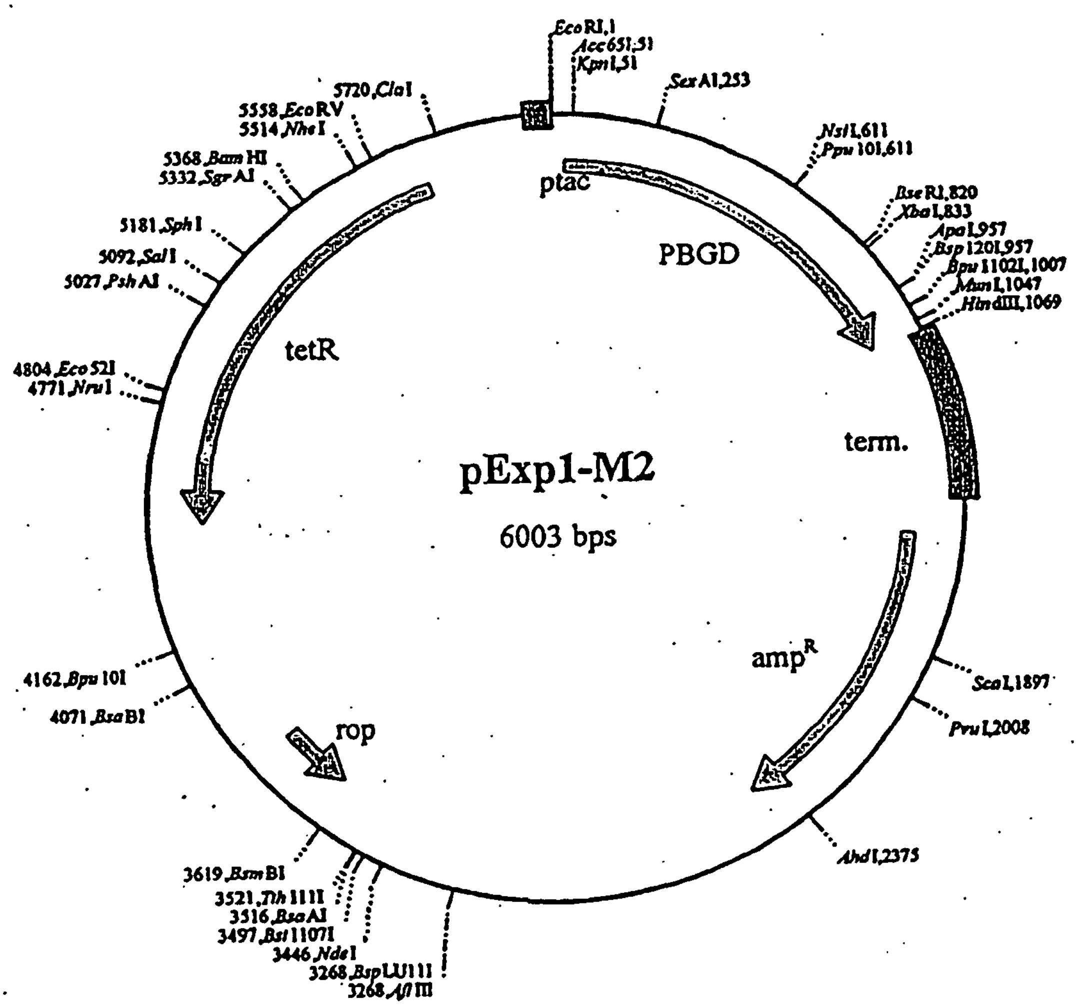
EcoRI,1
Acc65I,51
KpnI,51
SexAI,253
NsiI,611
Ppu10I,611
BseRI,820
XbaI,833
ApaI,957
Bsp120I,957
Bpu1102I,1007
MunI,1047
HindIII,1069
ScaI,1897
PvuI,2008
AhdI,2375
3619,BsmBI
3521,Tth111I
3516,BsaAI
3497,Bst1107I
3446,NdeI
3268,BspLU11I
3268,AflIII
4162,Bpu10I
4071,BsaBI
4804,Eco52I
4771,NruI
5720,ClaI
5558,EcoRV
5514,NheI
5368,BamHI
5332,SgrAI
5181,SphI
5092,SalI
5027,PshAI
ptac
PBGD
term.
amp^R
rop
tetR
pExp1-M2
6003 bps

Fig. 7

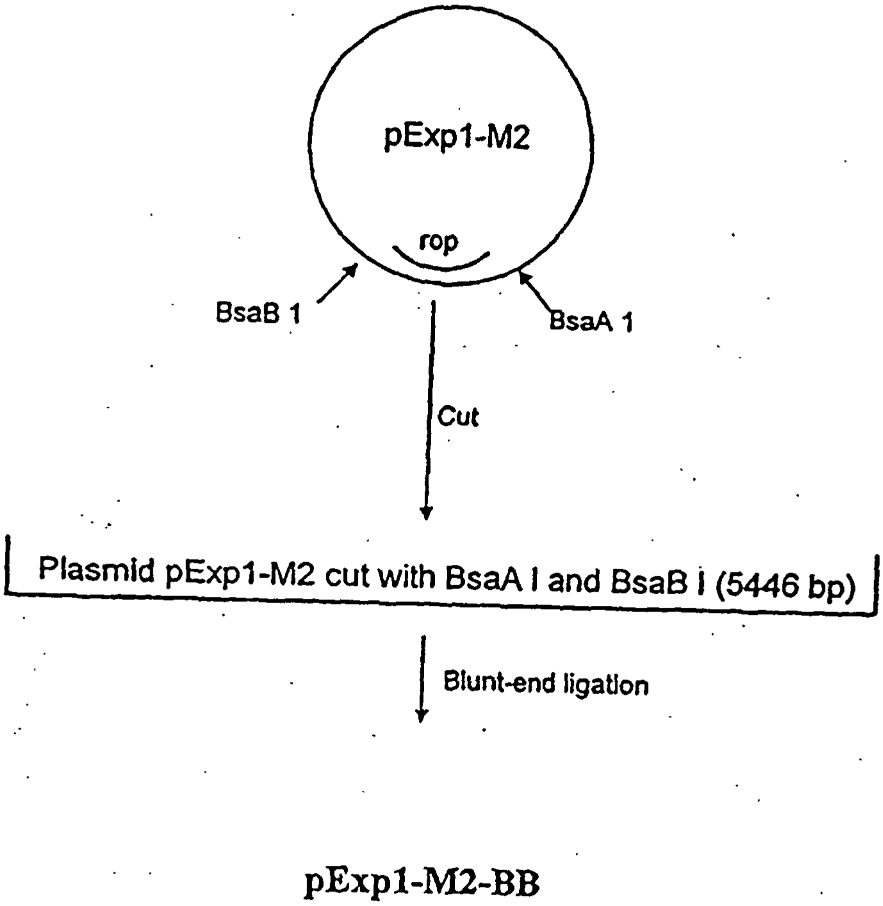
pExp1-M2
rop
BsaB 1
BsaA 1
Cut
Plasmid pExp1-M2 cut with BsaA I and BsaB I (5446 bp)
Blunt-end ligation
pExp1-M2-BB

Fig. 8

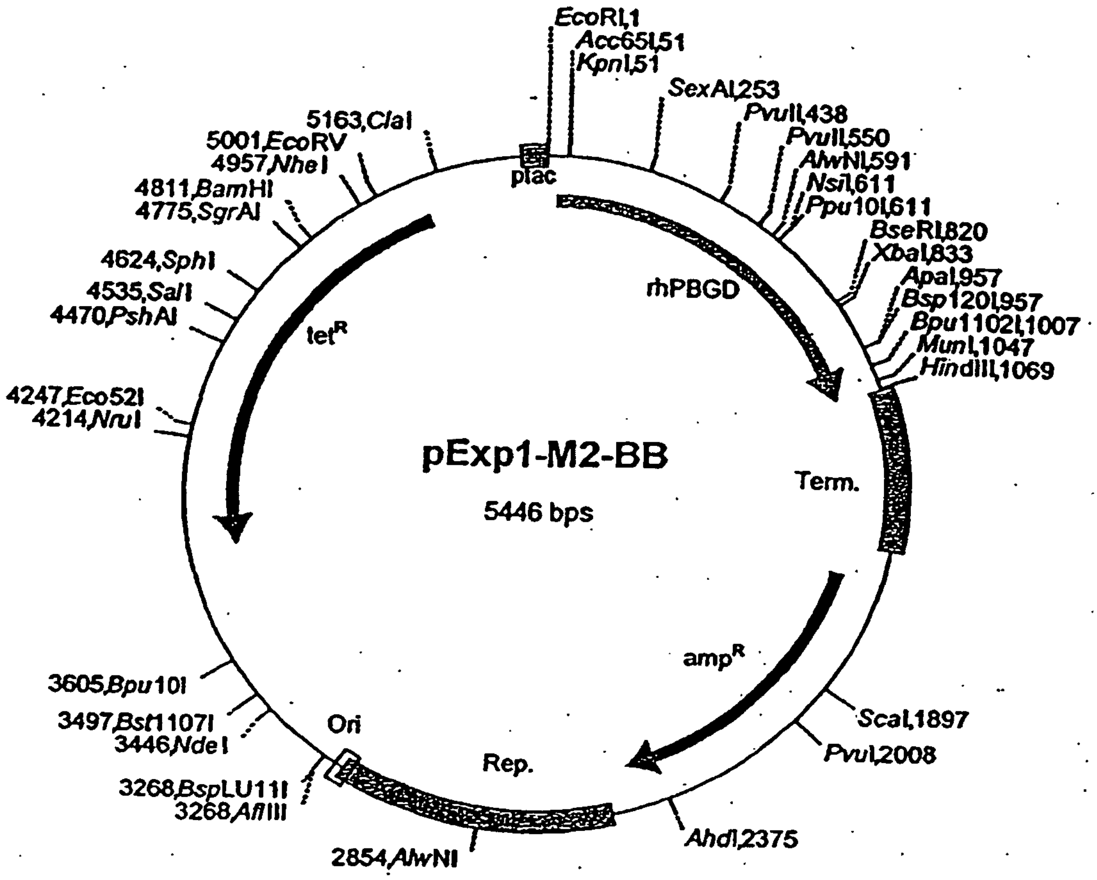


PLASMID FEATURES:

| Start | End | Name |
|---|---|---|
| 33 | 1067 | rhPBGD (open reading frame) |
| 1593 | 2453 | amp$^R$ (open reading frame) |
| 5105 | 3915 | tet$^R$ (open reading frame) |
| 5376 | 1 | ptac (promoter) |
| 1075 | 1501 | Term. (terminator region) |
| 3214 | 2519 | Rep. (replication region) |
| 3214 | | Ori (origin of replication) |

Fig. 9

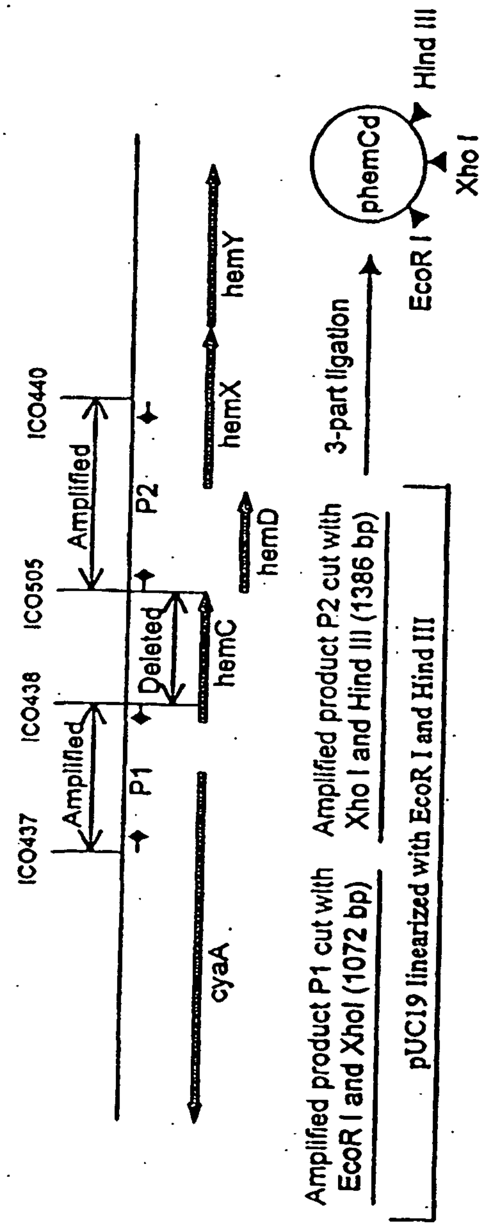
ICO437
ICO438
ICO505
ICO440
Amplified
Deleted
Amplified
P1
P2
cyaA
hemC
hemD
hemX
hemY
Amplified product P1 cut with EcoR I and XhoI (1072 bp)
Amplified product P2 cut with Xho I and Hind III (1386 bp)
pUC19 linearized with EcoR I and Hind III
3-part ligation
phemCd
EcoR I
Xho I
Hind III

Fig. 10

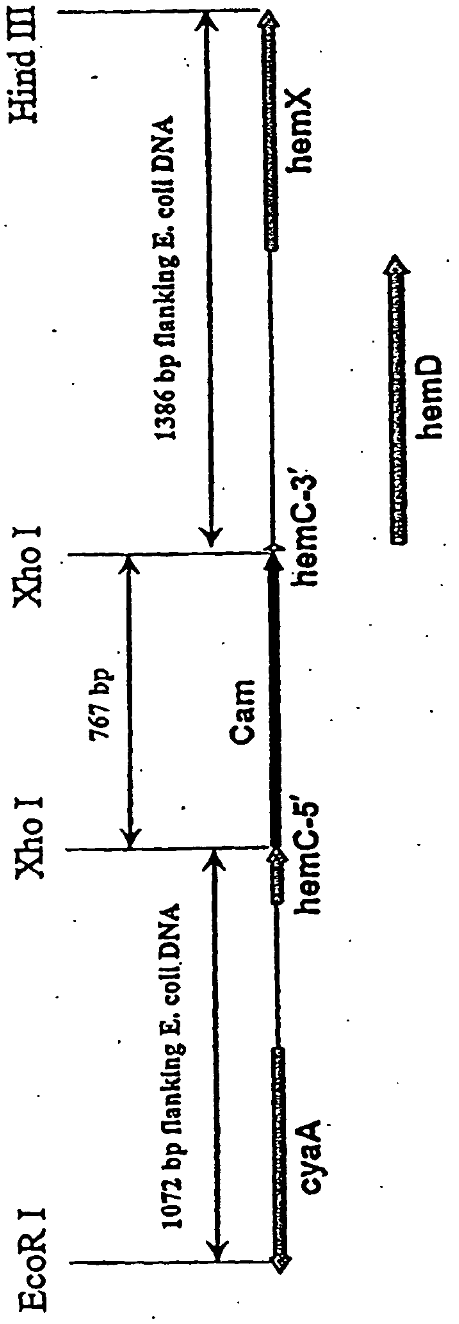
Hind III
Xho I
Xho I
EcoR I
1386 bp flanking E. coli DNA
767 bp
1072 bp flanking E. coli DNA
hemX
hemC-3'
Cam
hemC-5'
cyaA
hemD

Fig. 11

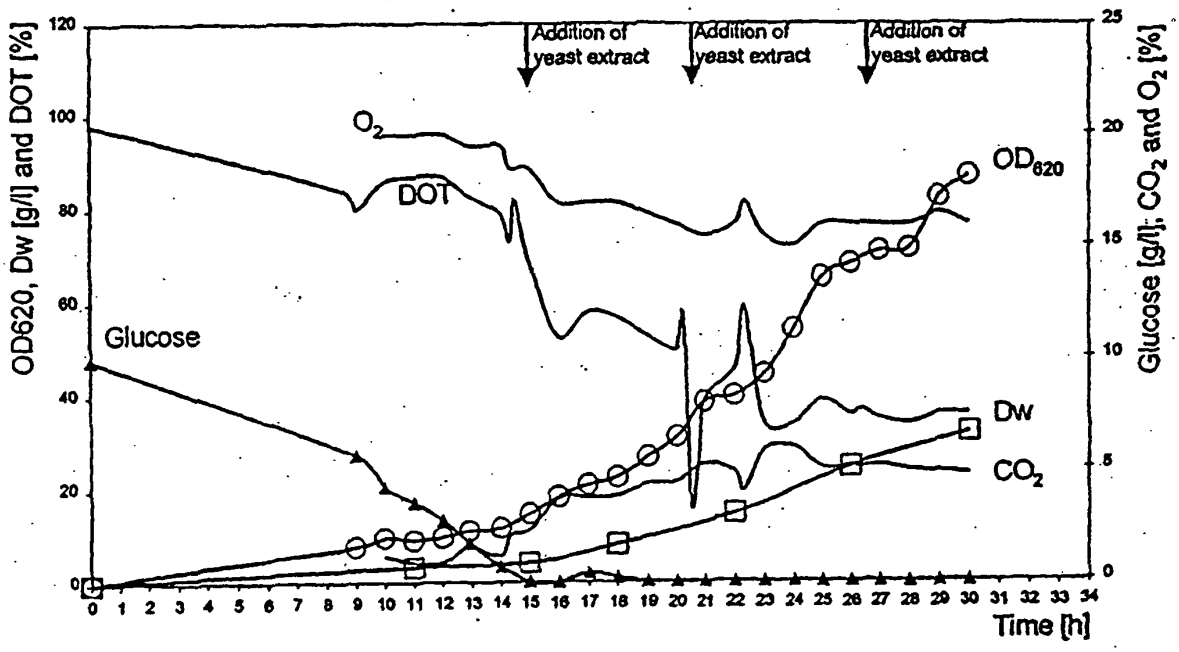
Addition of yeast extract
Addition of yeast extract
Addition of yeast extract
O2
DOT
Glucose
OD620
Dw
CO2
OD620, Dw [g/l] and DOT [%]
Glucose [g/l]; CO2 and O2 [%]
Time [h]
0
20
40
60
80
100
120
0
5
10
15
20
25
0 1 2 3 4 5 6 7 8 9 10 11 12 13 14 15 16 17 18 19 20 21 22 23 24 25 26 27 28 29 30 31 32 33 34

Fig. 12

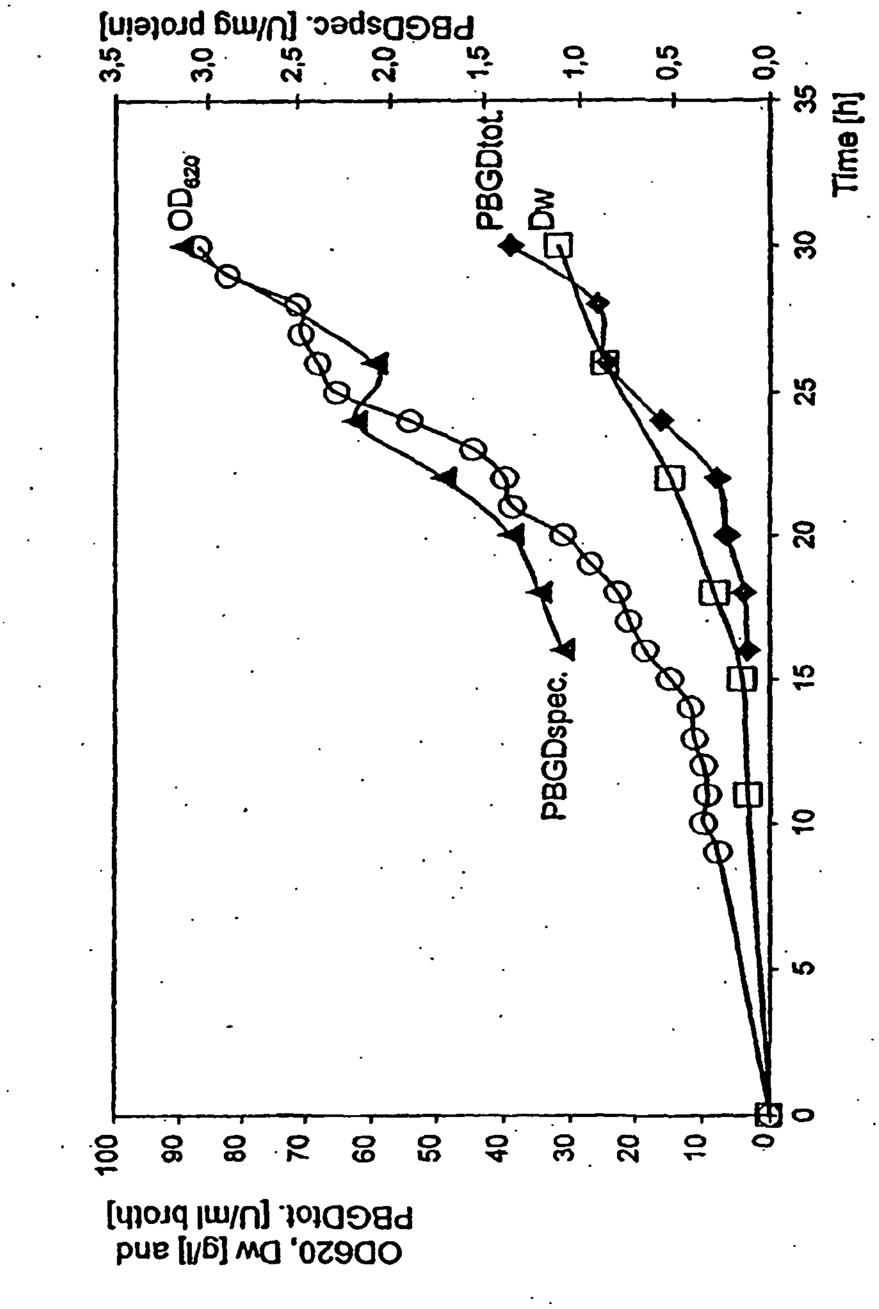

PBGDspec. [U/mg protein]
3,5
3,0
2,5
2,0
1,5
1,0
0,5
0,0
OD620, Dw [g/l] and PBGDtot. [U/ml broth]
100
90
80
70
60
50
40
30
20
10
0
0
5
10
15
20
25
30
35
Time [h]
$OD_{620}$
PBGDtot.
Dw
PBGDspec.


Fig. 13

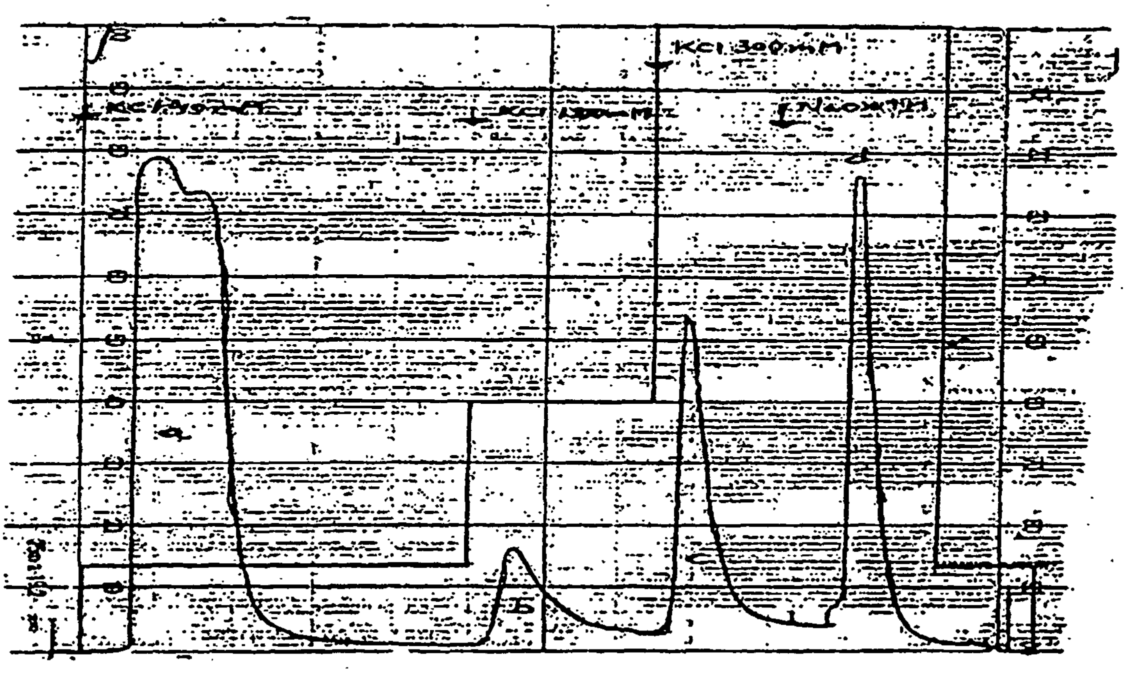

Chromatography on DEAE-Sepharose FF (DEAE1). Peak a flow through the gel 40 mM KCl, Peak b 120 mM KCl Peak c 300 mM KCl Peak d NaOH 1 M.

Fig. 14

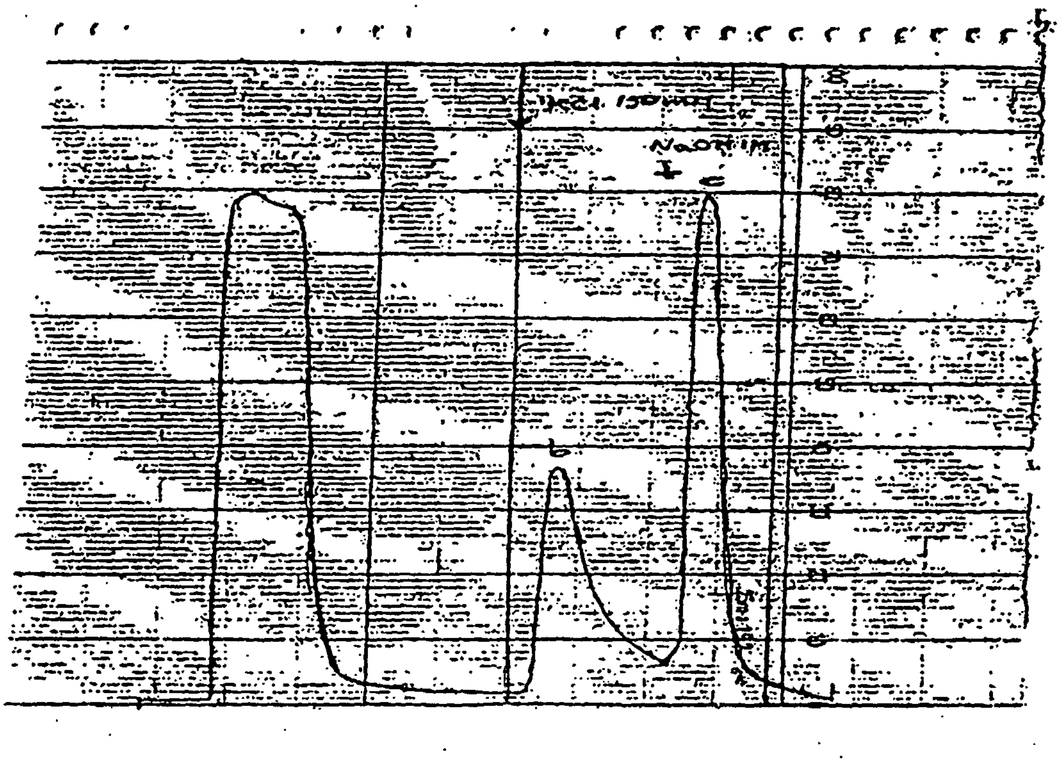

Chromatography on DEAE-Sepharose FF (DEAE2). Peak a flow through the gel 40 mM KCl, Peak b 150 mM KCl Peak c NaOH 1 M.

# Fig. 15

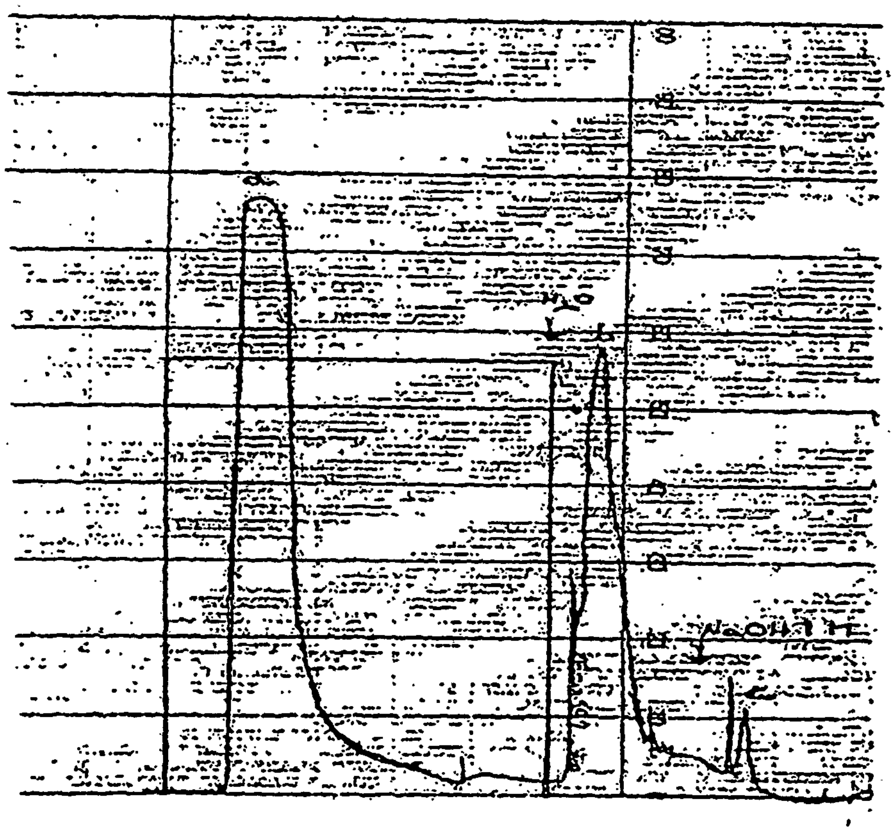


Chromatography on Butyl-Sepharose 4 FF. Peak a flow through the gel, Peak b water Peak c NaOH 1 M.

Fig. 16

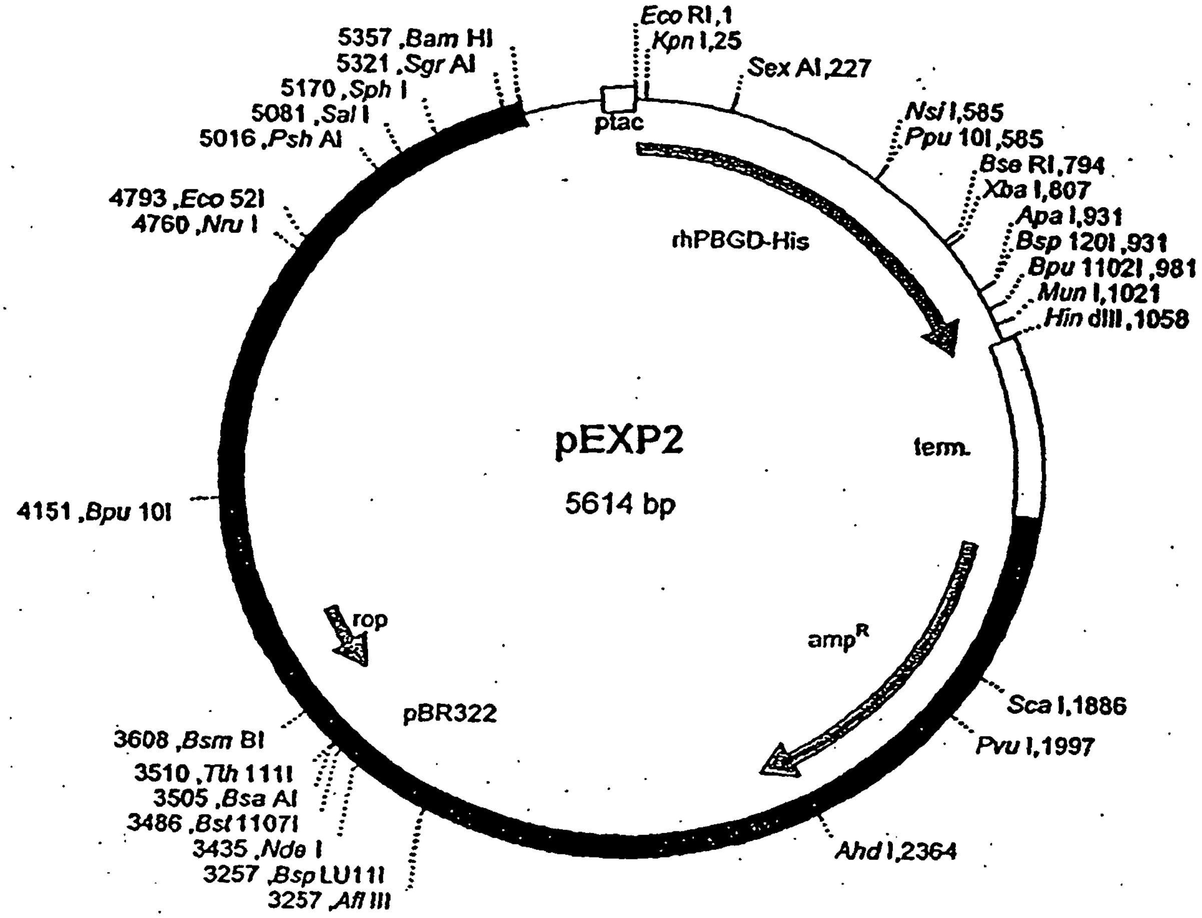


PLASMID FEATURES:

| Start | End | Name |
|---|---|---|
| 7 | 1056 | rhPBGD-His |
| 1064 | 1490 | terminator region |
| 1582 | 2442 | $amp^R$ |
| 3820 | 3629 | rop |
| 5362 | 1490 | pBR322 |
| 5544 | 1 | ptac (promoter) |

Fig. 17

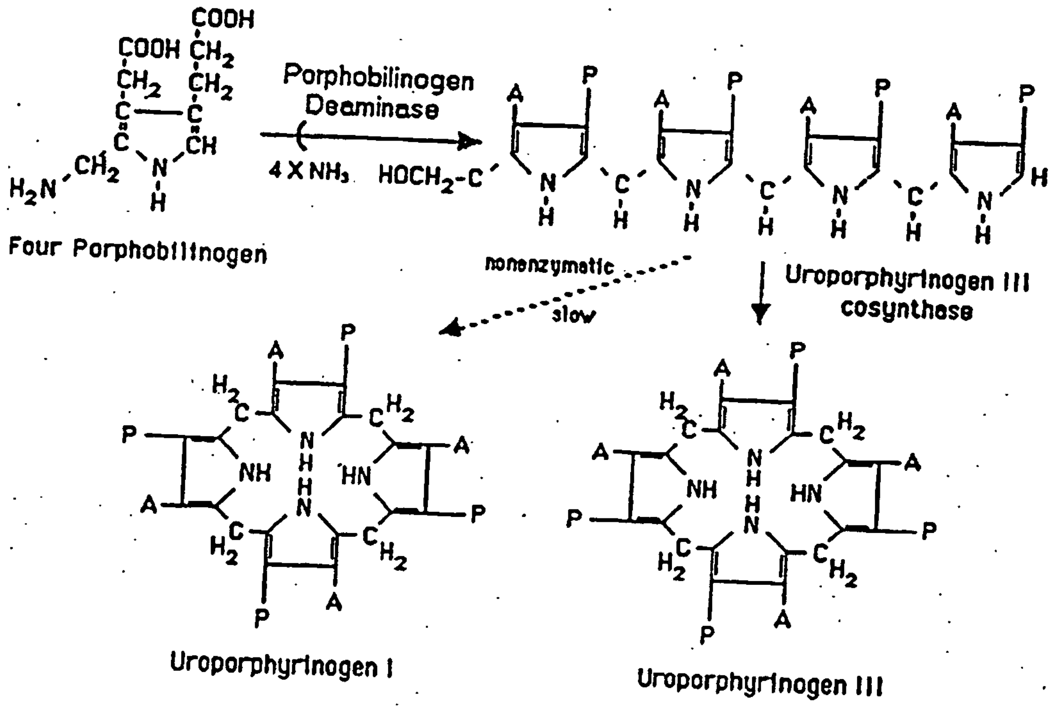
Porphobilinogen
Deaminase
4 X NH₃
nonenzymatic
slow
Uroporphyrinogen III
cosynthase

# Fig. 18

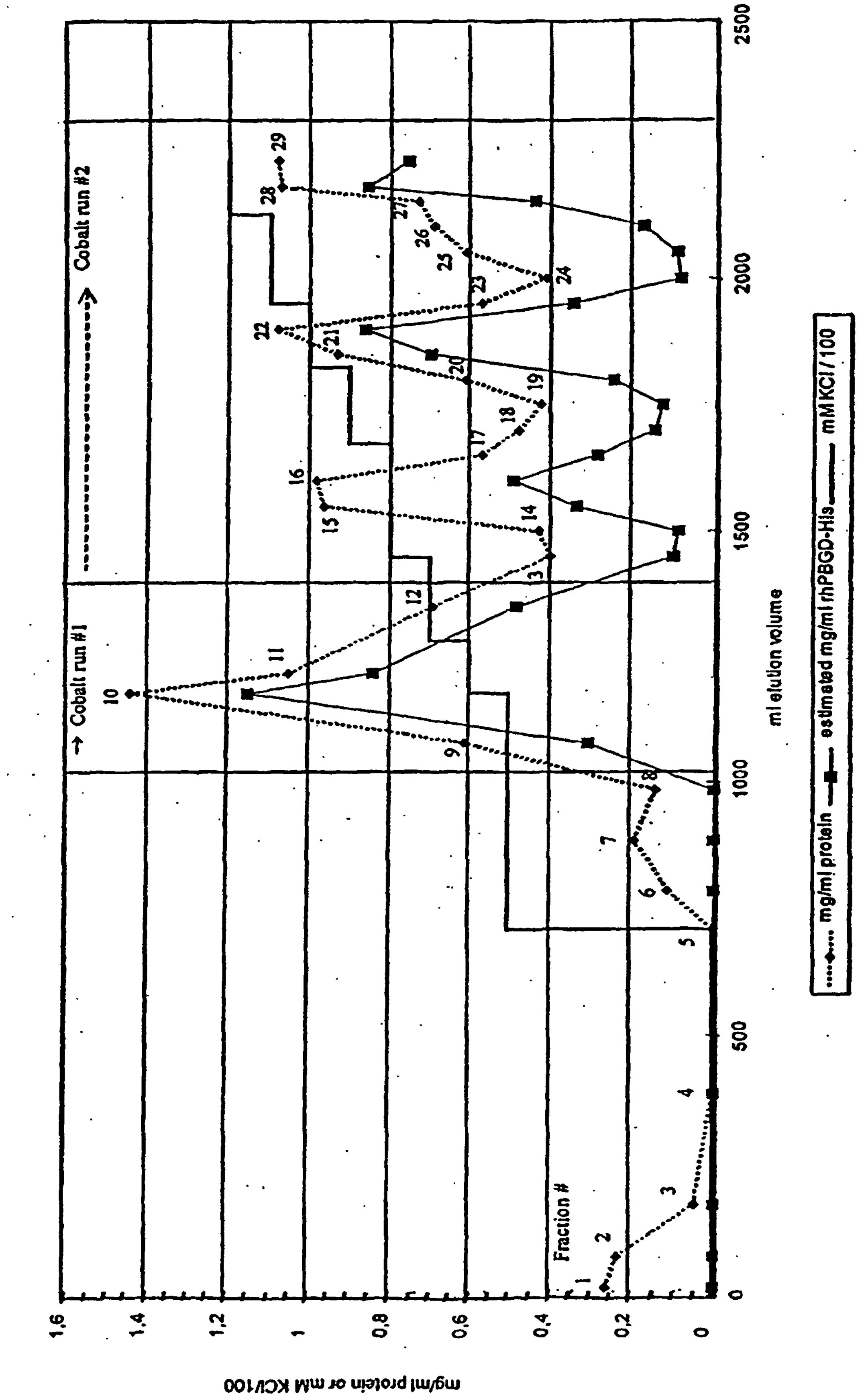
mg/ml protein or mM KCl/100
ml elution volume
Cobalt run #1
Cobalt run #2
Fraction #
mg/ml protein
estimated mg/ml rhPBGD-His
mMKCl/100

Fig. 19

DEAE Sepharose Column Fractions:

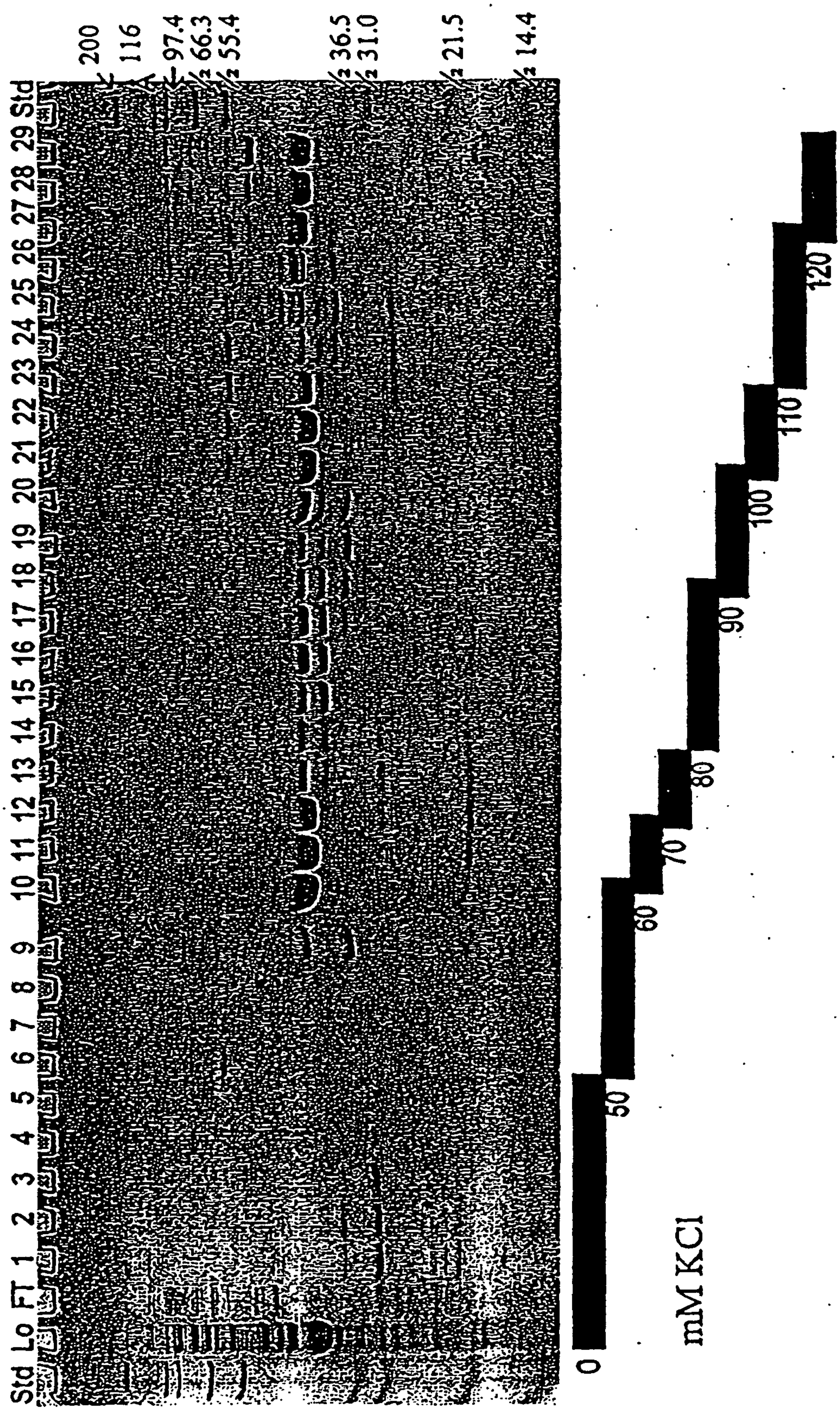

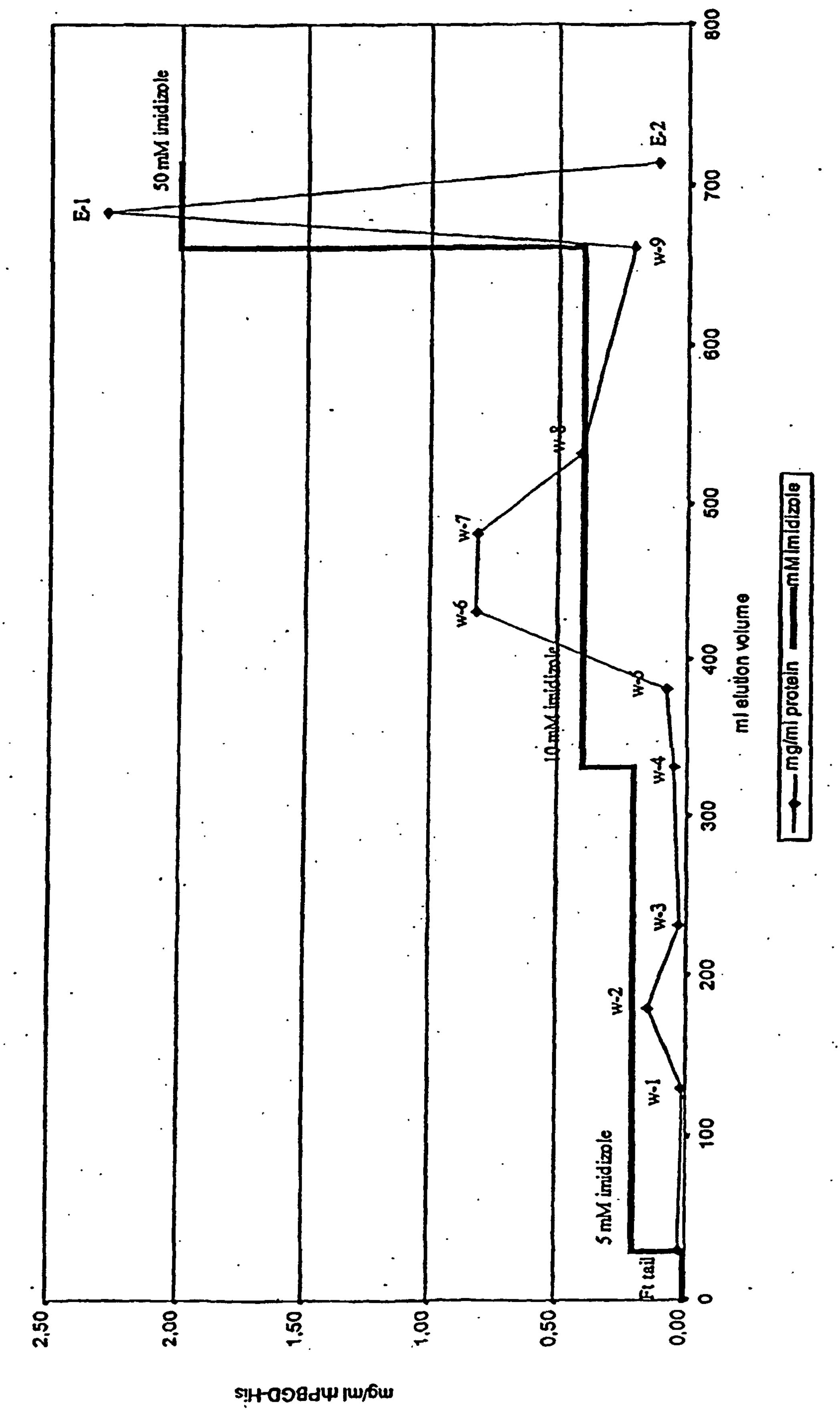
mg/ml rhPBGD-His
2,50
2,00
1,50
1,00
0,50
0,00
0
100
200
300
400
500
600
700
800
ml elution volume
Ft tail
5 mM imidizole
10 mM imidizole
50 mM imidizole
w-1
w-2
w-3
w-4
w-6
w-7
w-8
w-9
E-1
E-2
mg/ml protein
mM imidizole

Fig. 21

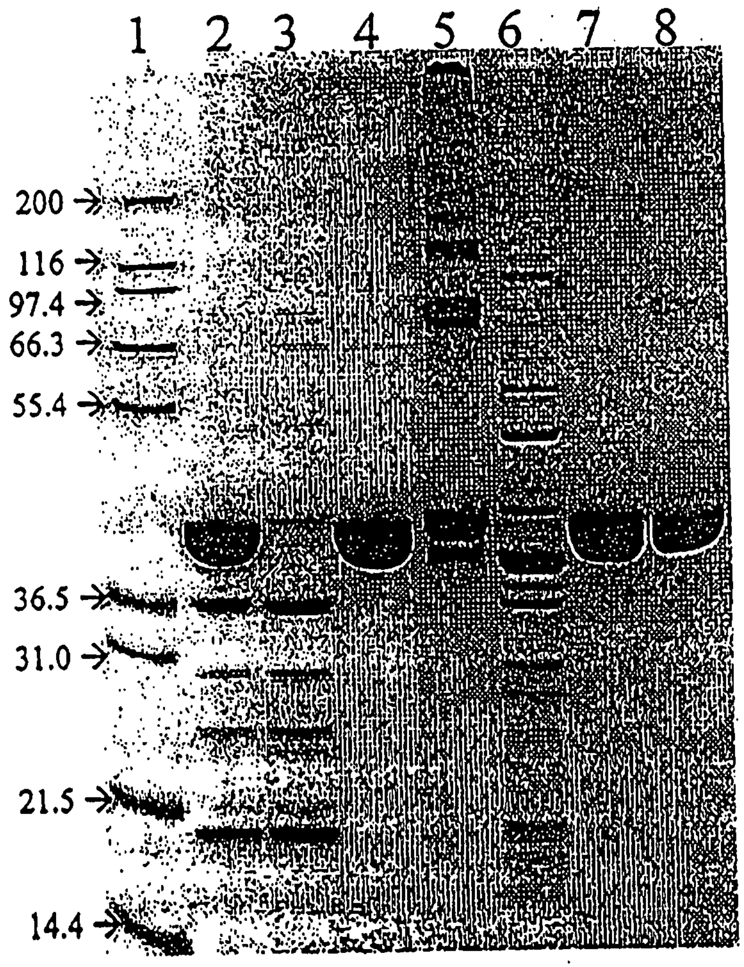


| | |
|---|---|
| 1 | Mark 12 molecular weight standards (Novex) |
| 2 | Cobalt load #1 (DEAE fractions 9 -> 12) |
| 3 | Flowthrough from cobalt load #1 |
| 4 | 50 mM imidizole eluate from cobalt run #1 |
| 5 | Concatamerized nickel purified rhPBGD-His |
| 6 | Flowthrough from cobalt load #2 |
| 7 | 10 mM imidizole eluate from cobalt run #2 |
| 8 | 50 mM imidizole eluate from cobalt run #2 |

Fig. 22

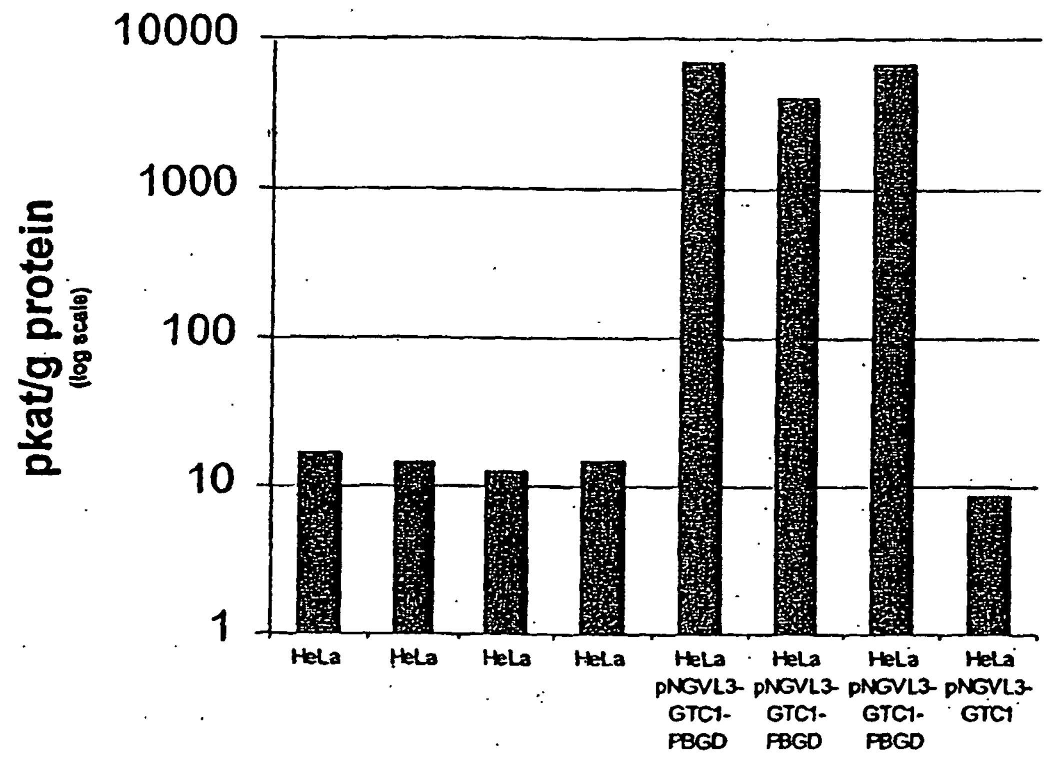


*Fig 23: PBGD activity related to protein concentration in HeLa cells.*

**Fig. 23**

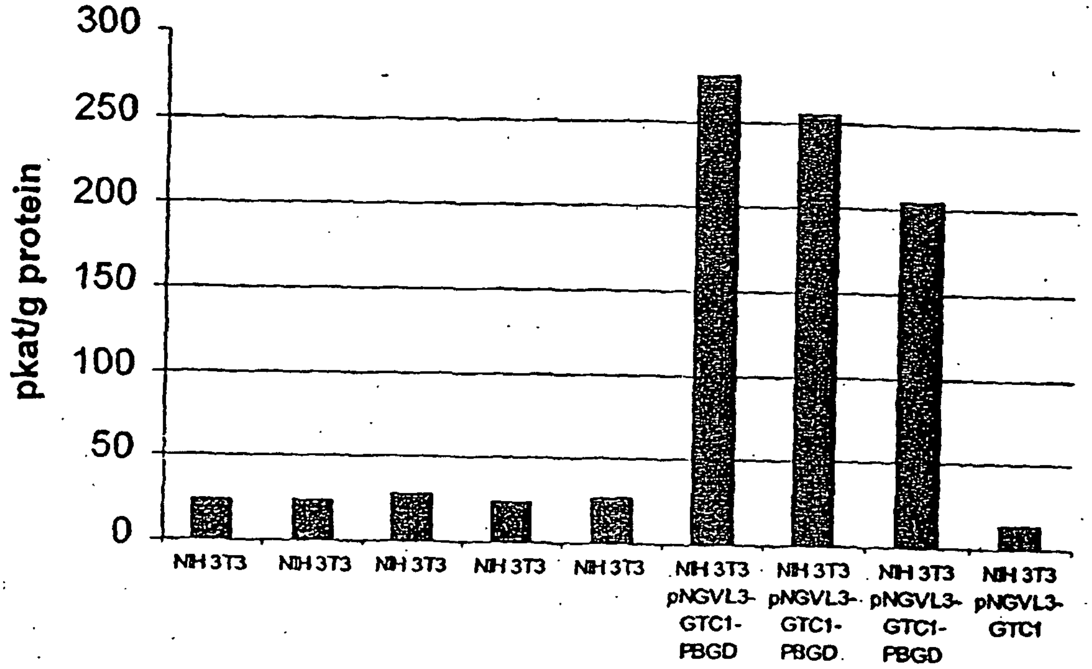


*Figure 24: PBGD activity related to protein concentration in NIH 3T3 cells.*

**Fig. 24**

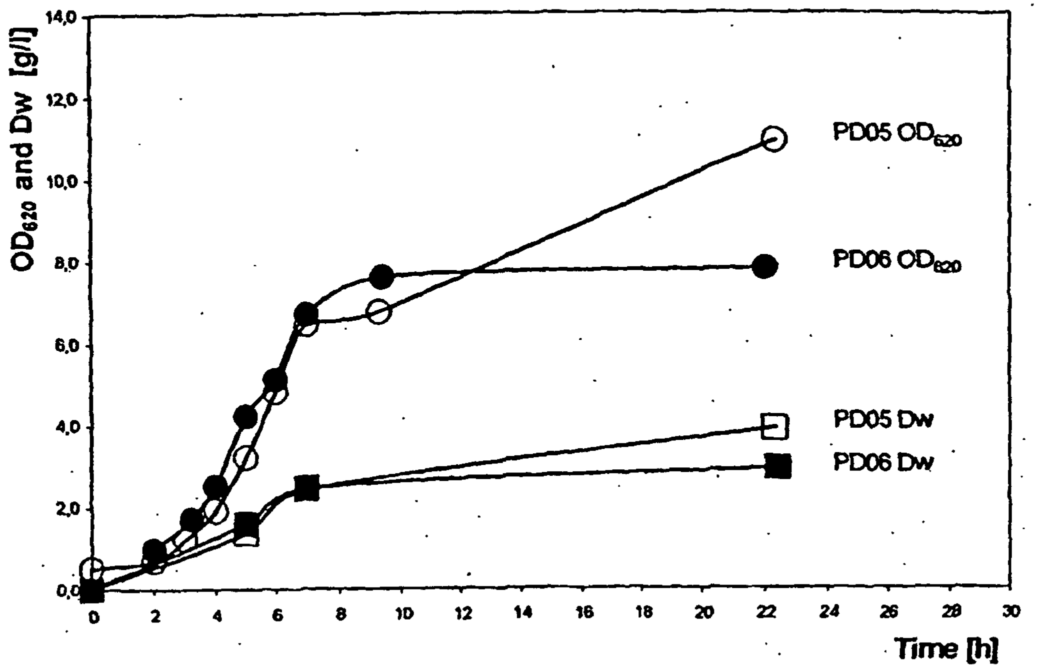


Fig 25. Comparison of fermentations PD05 and PD06 with strain PBGD-2.

Fig. 25

Fig 26. Comparison of fermentations PD09, PD11 and PD12

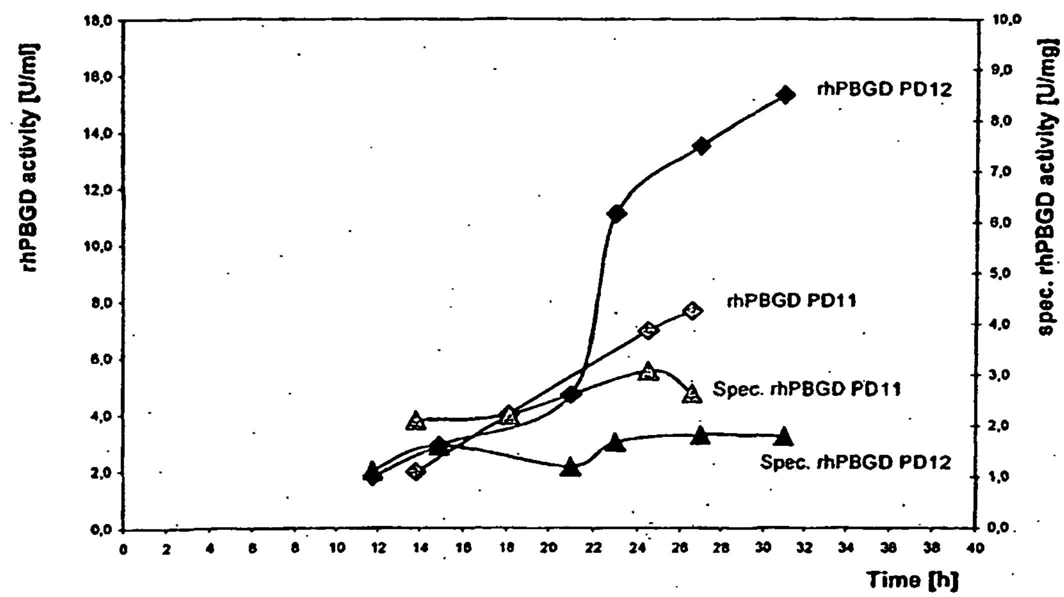


Fig. 26

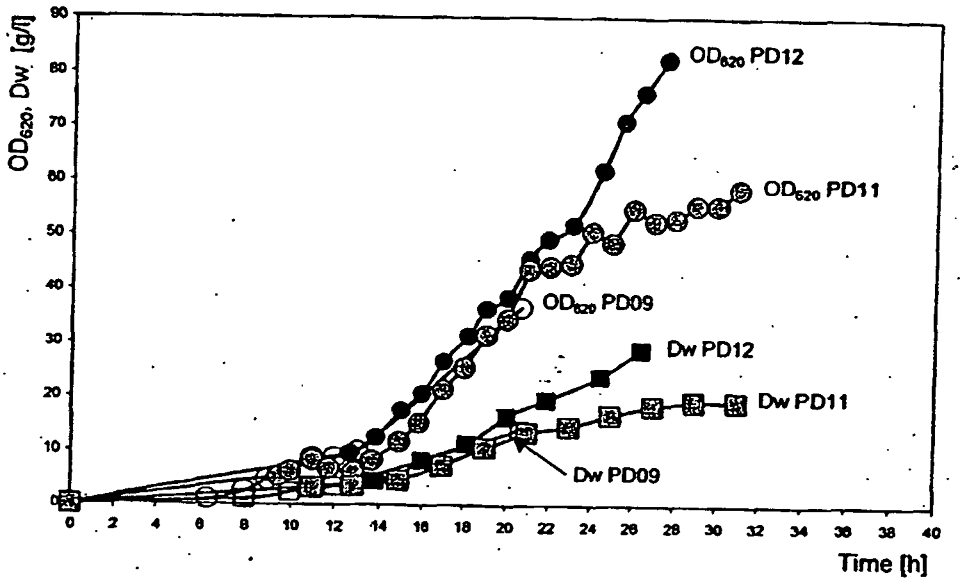


Fig 27: Comparison of fermentations PD09, PD11 and PD12 with strain PBGD-1.

# Fig. 27

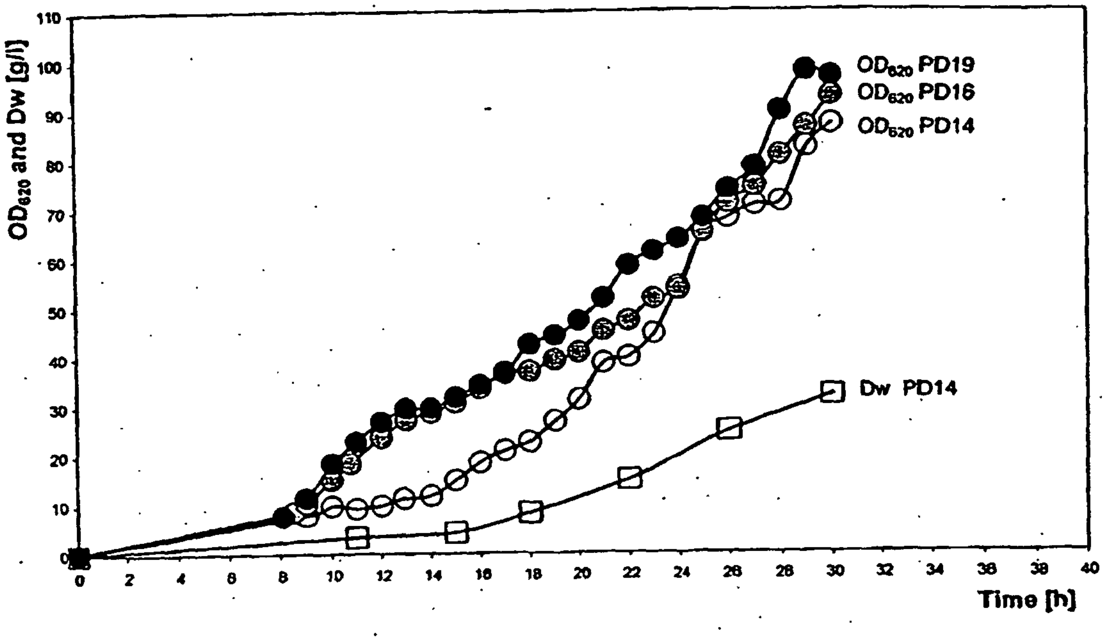


Fig 28.Comparison of fermentations PD14, PD16 and PD19 with strain PBGD-2.

Fig. 28

Fig 29.Comparison of fermentations PD14, PD16 and PD19 with strain PBGD-2.

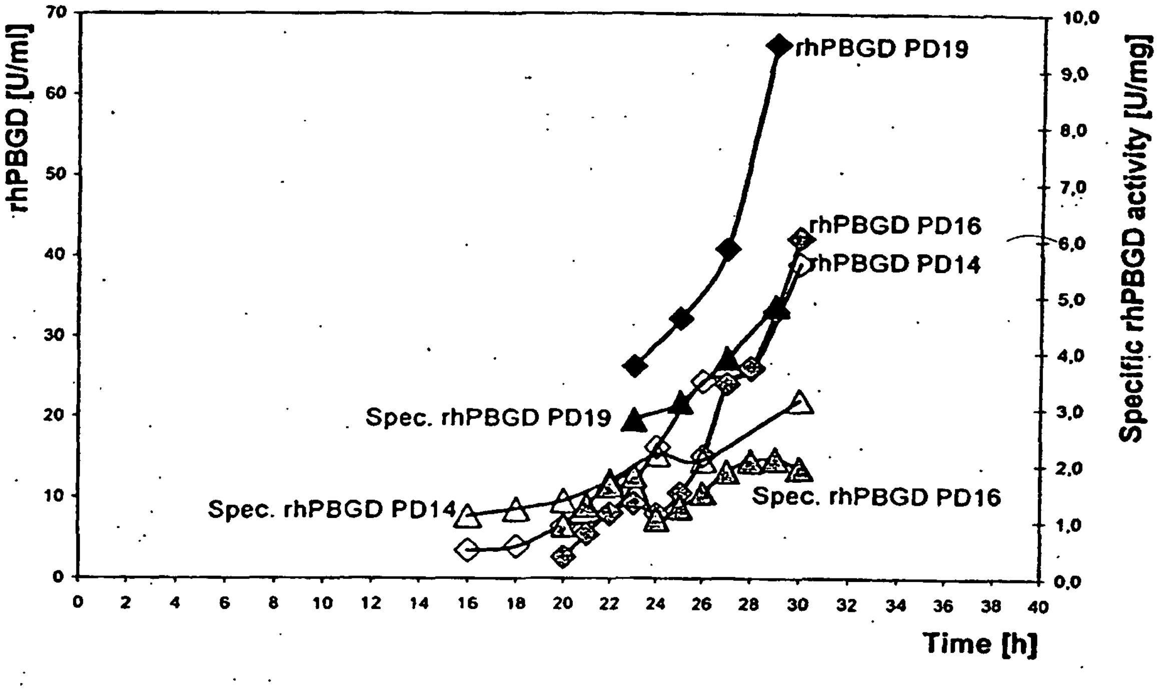


Fig. 29

Fig 30.Comparison of fermentations PD19, PD21 and PD22 with strain PBGD-2.

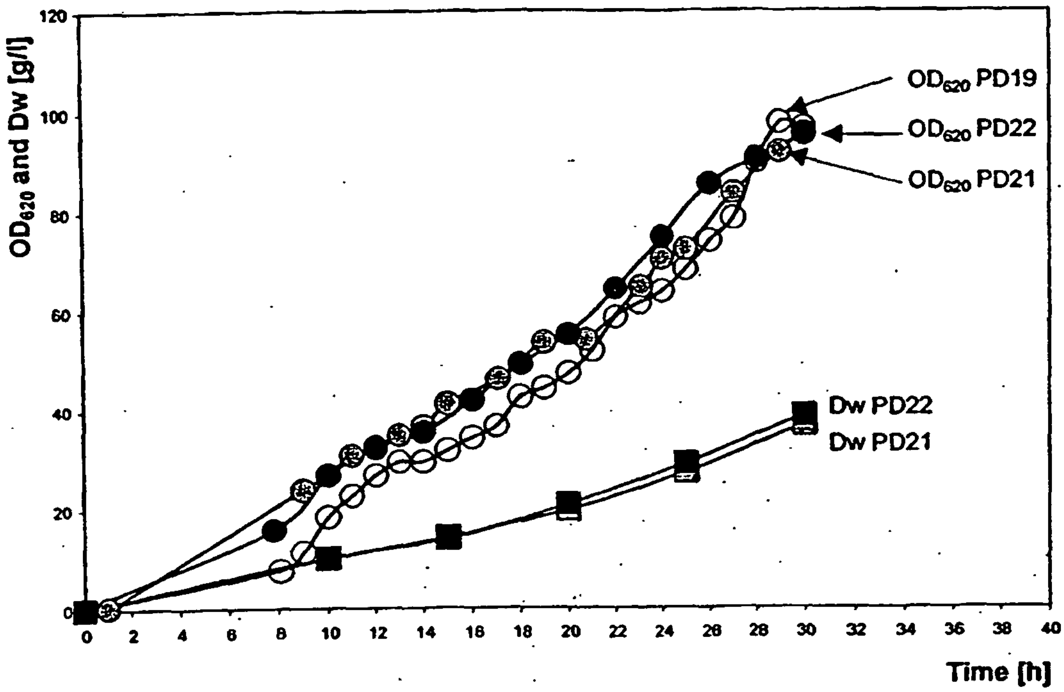


Fig. 30

Fig 31. Comparison of fermentations PD19, PD21 and PD22 with strain PBGD-2.

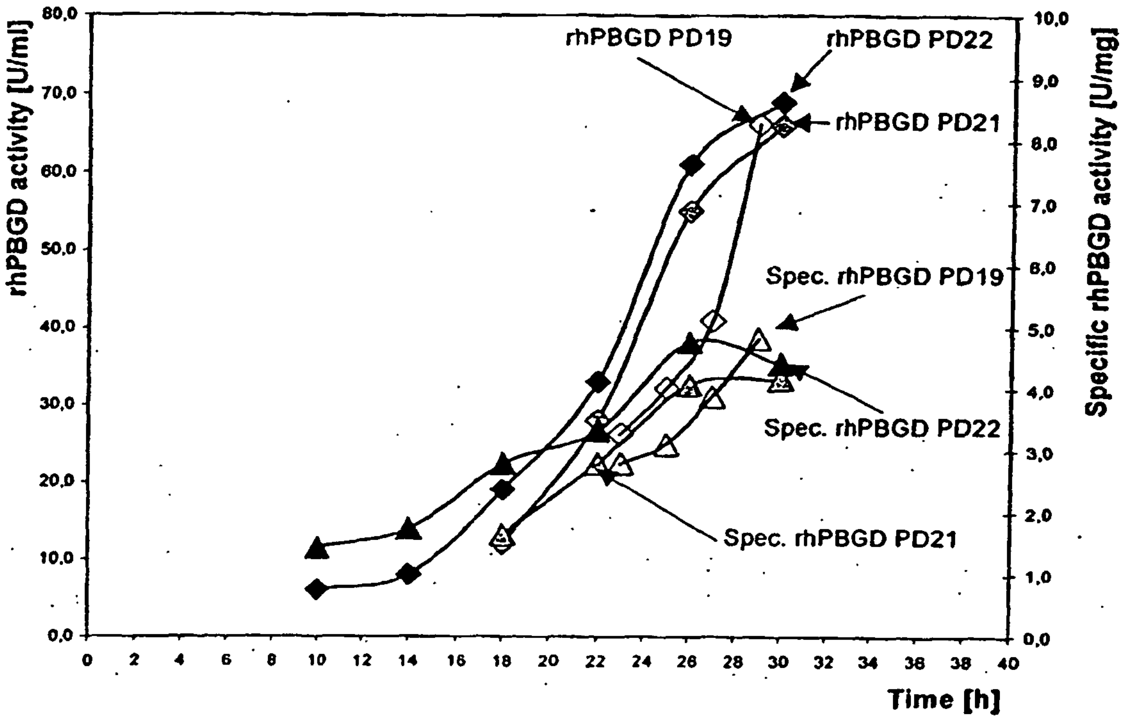


# Fig. 31

Fig 32. Comparison of fermentations PD19, PD1501 and PD1502.

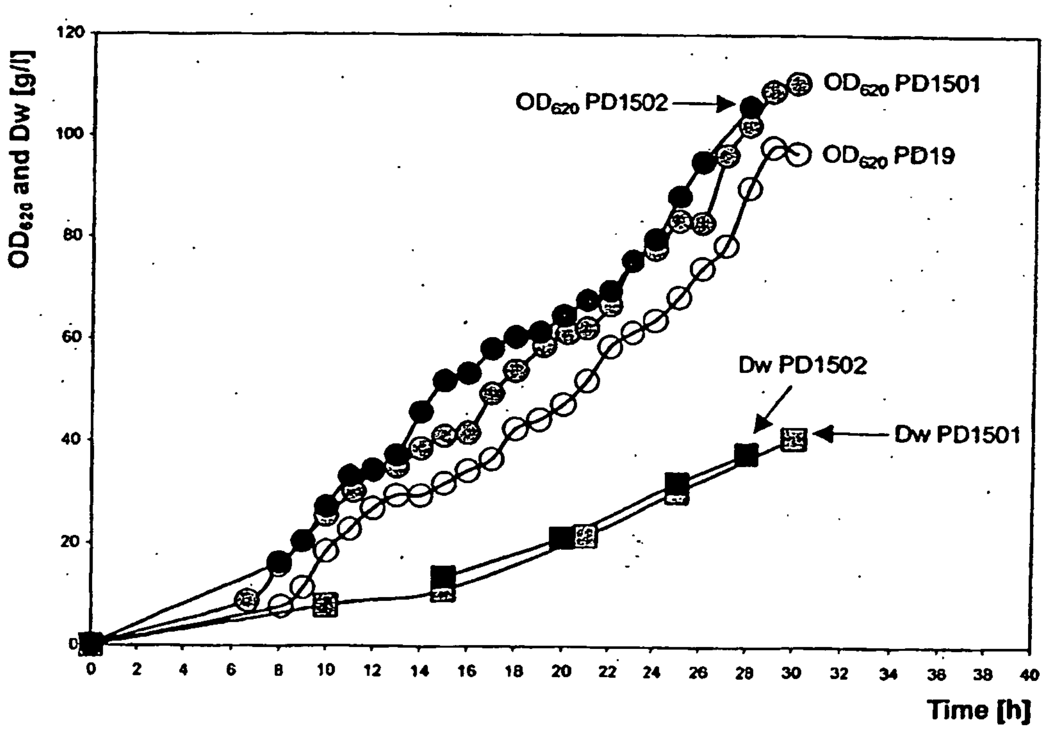


Fig. 32

Fig 33. Comparison of fermentations PD19, PD1501 and PD1502 with strain PBGD-2.

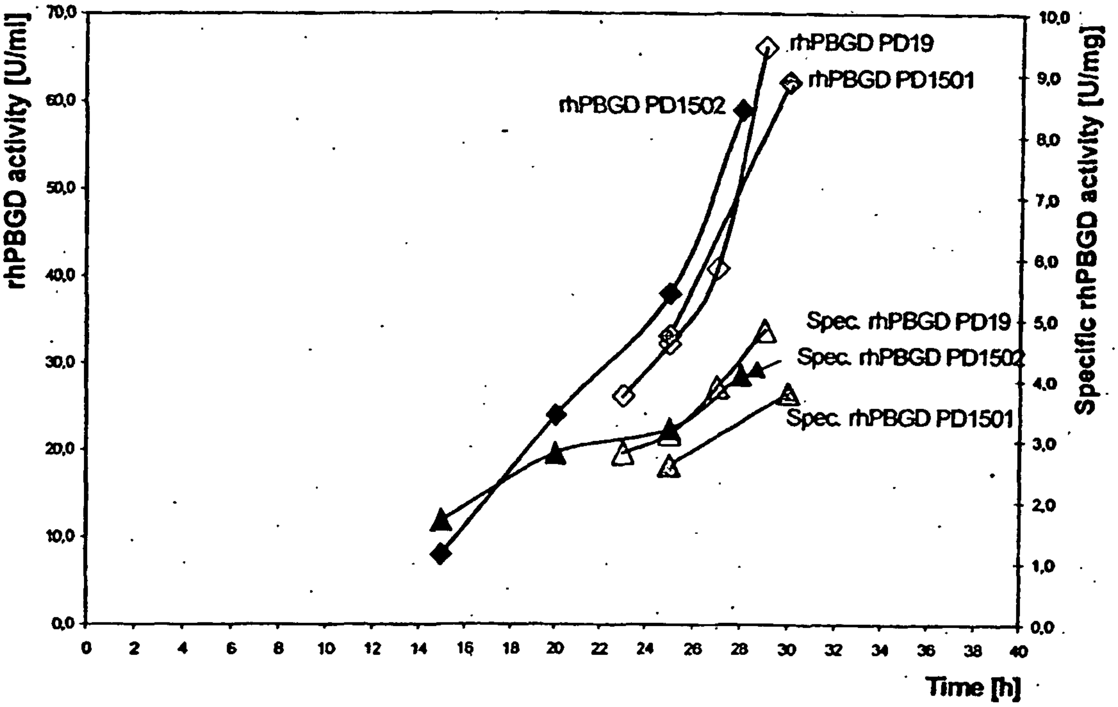


Fig. 33

Summary of Fermentation and down stream process visualized by SDS-Page. Comparison between different samples.

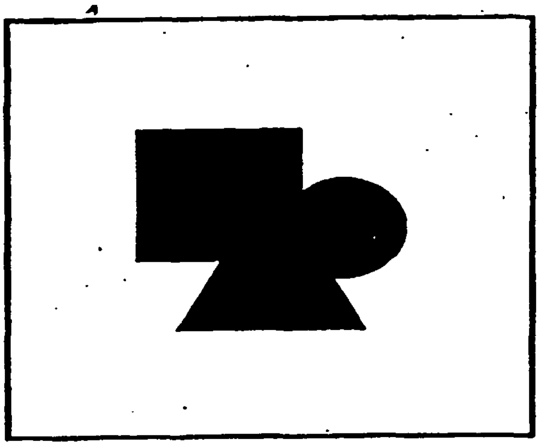

| Well | Samples from PD22 |
|---|---|
| 1 | Mark 12 MW st |
| 2 | Extract |
| 3 | Homogenate |
| 4 | Broth 30 h |
| 5 | Broth 26 h |
| 6 | Broth 22 h |
| 7 | Broth 18 h |
| 8 | Broth 14 h |
| 9 | Broth 10 h |
| 10 | rhPBGD-His |

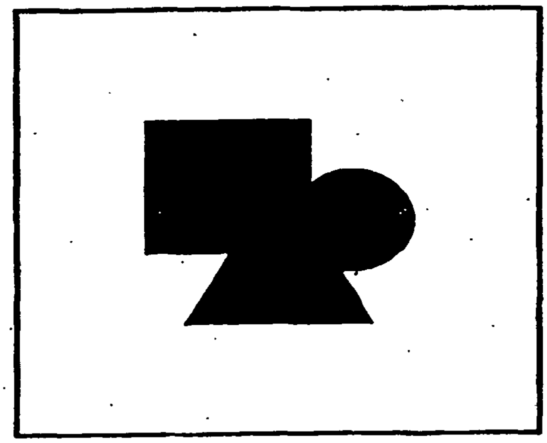

| | Samples from PD1501 |
|---|---|
| 1 | Mark 12 MW st |
| 2 | Extract |
| 3 | Extract |
| 4 | Extract |
| 5 | Broth 30 h |
| 6 | Broth 30 h |
| 7 | Broth 25 h |
| 8 | Broth 20 h |
| 9 | Broth 15 h |
| 10 | rhPBGD-His |

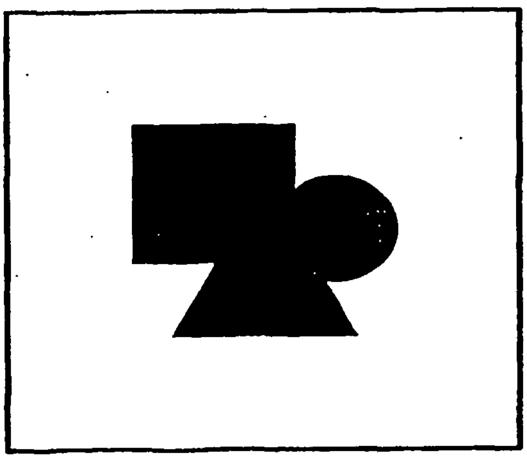

| | Samples from PD1502 |
|---|---|
| 1 | Mark 12 MW st |
| 2 | Broth 15 h |
| 3 | Broth 20 |
| 4 | Broth 25 h |
| 5 | Broth 28 h |
| 6 | Homogenate |
| 7 | Extract |
| 8 | Extract |
| 9 | Extract |
| 10 | rhPBGD-His |

Fig. 34

Fig 35. Stability studies: Single use aliquots of extract were routinely taken out of the freezer (-20°C) and the rhPBGD-activity was measured and plotted over time.

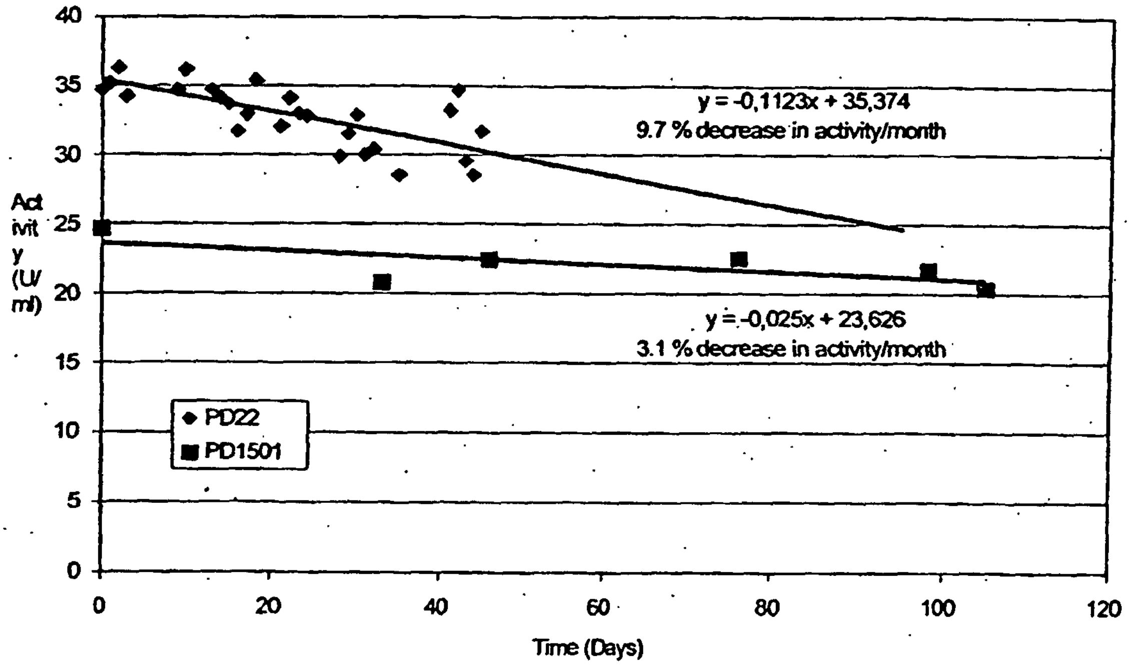


Fig. 35

**Description of the oligos used for PCR amplification**

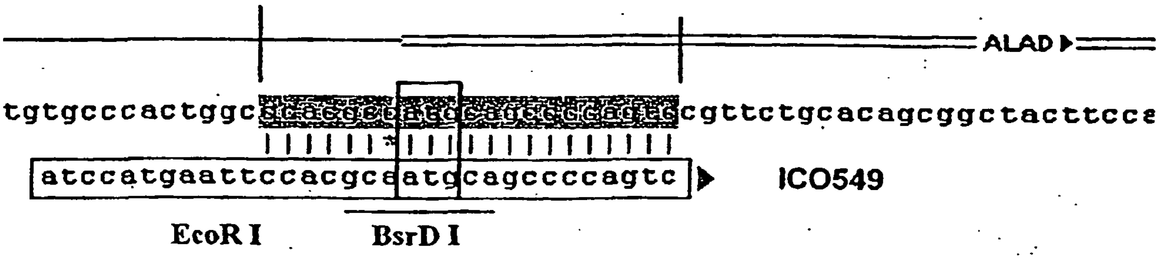


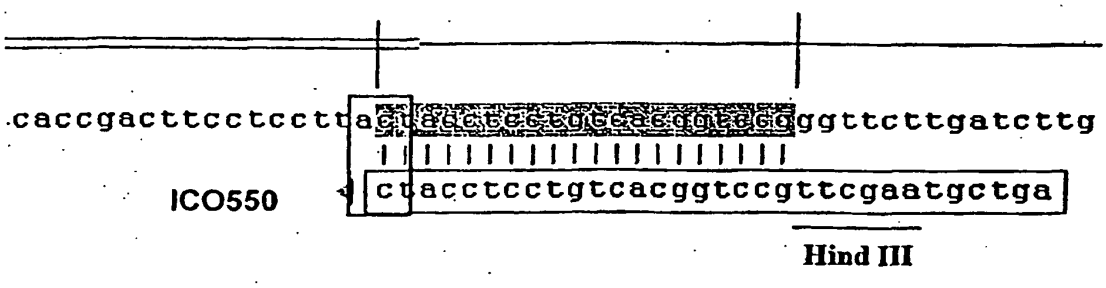


The two oligos ICO549 and ICO550 are shown below the ALAD sequence. The highlighted regions in the ALAD sequence represent the regions of homology with the oligos. The asterisk denotes the C to A change introduced to generate a *BsrD* I site for ease of manipulation of the 5' end of the cloned molecule. The initiation and termination triplets in the ALAD sequence are boxed. Both the oligos have 5' sequences extending beyond the regions of homology to incorporate restriction sites for cloning of the amplified DNA, *viz. Eco*R I for ICO549 and *Hin*d III for ICO550.

# Fig. 36

**Strategy for PCR cloning of ALAD**

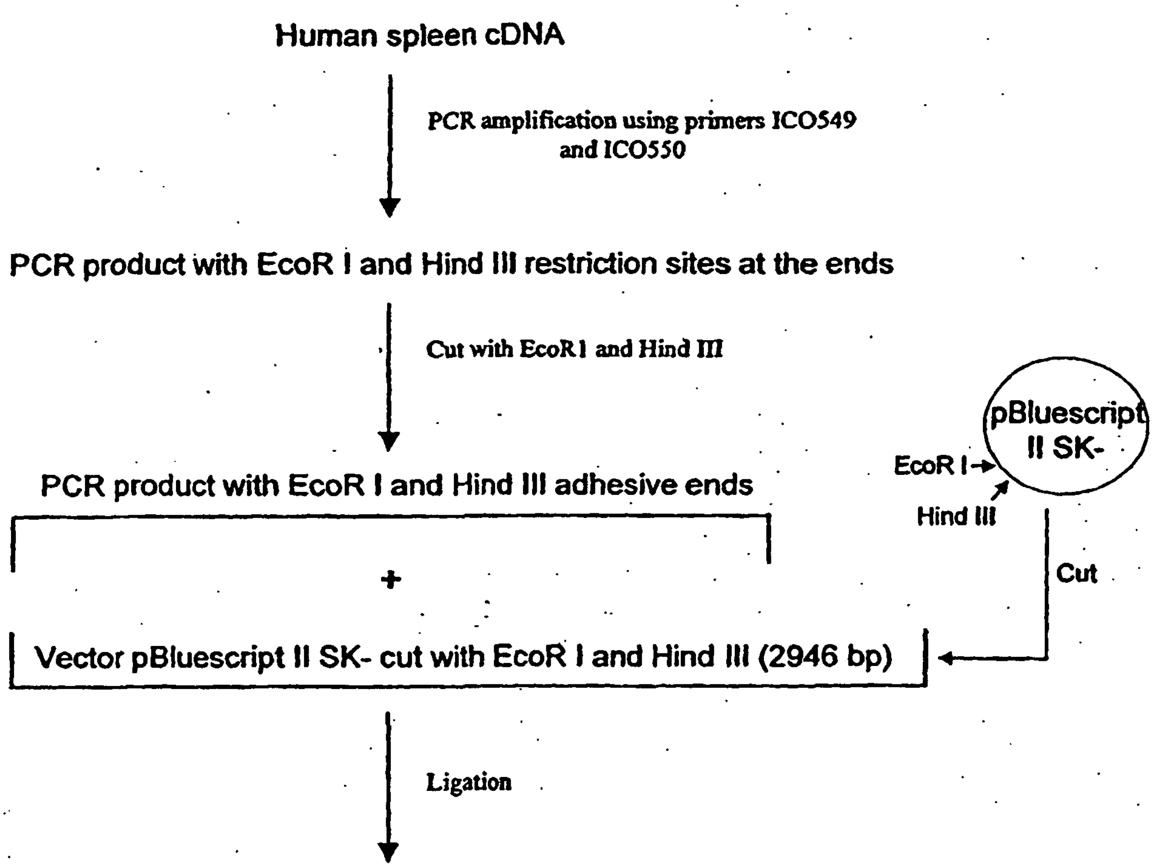


Fig. 37

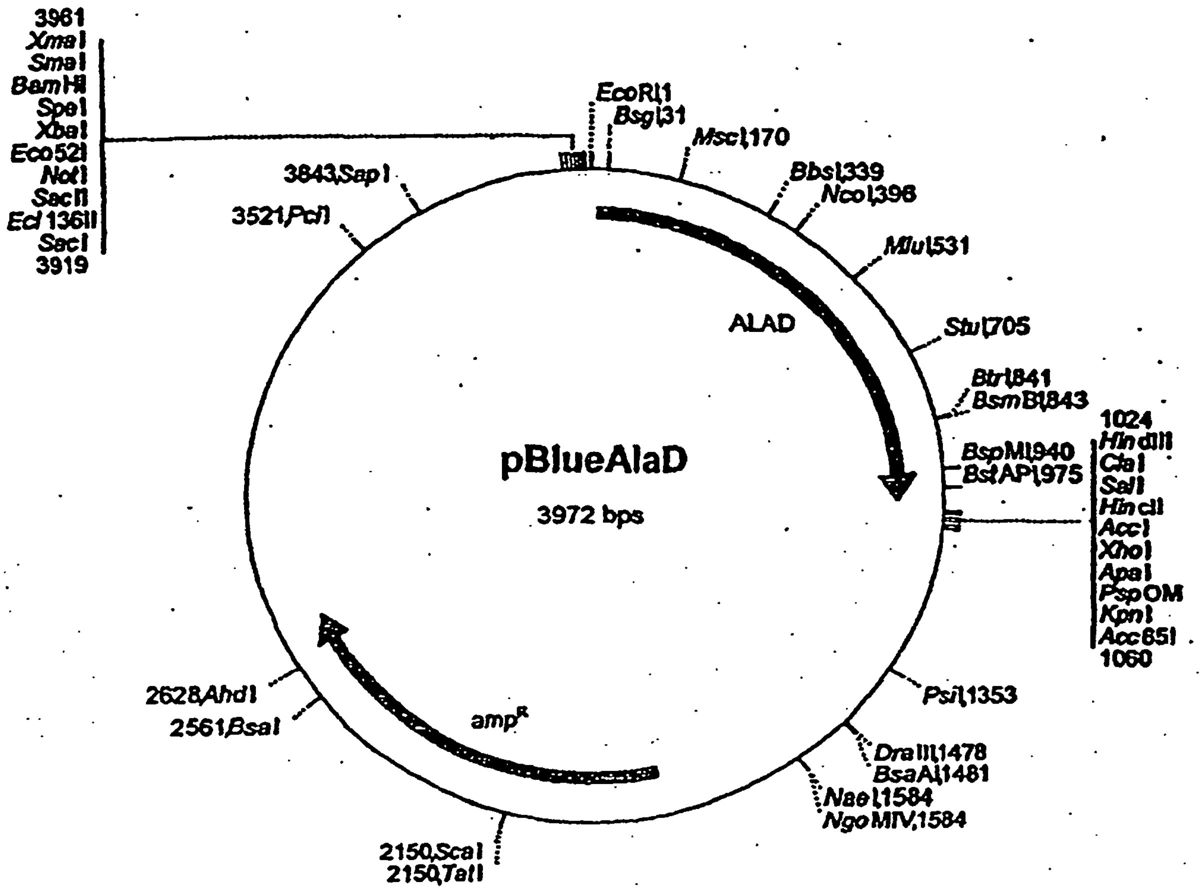
3961
XmaI
SmaI
BamHI
SpeI
XbaI
Eco52I
NotI
SacII
EclI 136II
SacI
3919
EcoRI 1
BsgI 31
MscI 170
BbsI 339
NcoI 396
MluI 531
StuI 705
BtrI 841
BsmBI 843
BspMI 940
BstAPI 975
1024
HindIII
ClaI
SalI
HincII
AccI
XhoI
ApaI
PspOMI
KpnI
Acc65I
1060
PstI 1353
DraIII 1478
BsaAI 1481
NaeI 1584
NgoMIV 1584
2150 ScaI
2150 TatI
2561 BsaI
2628 AhdI
3521 PclI
3843 SapI
ALAD
ampR
pBlueAlaD
3972 bps

Fig. 37C

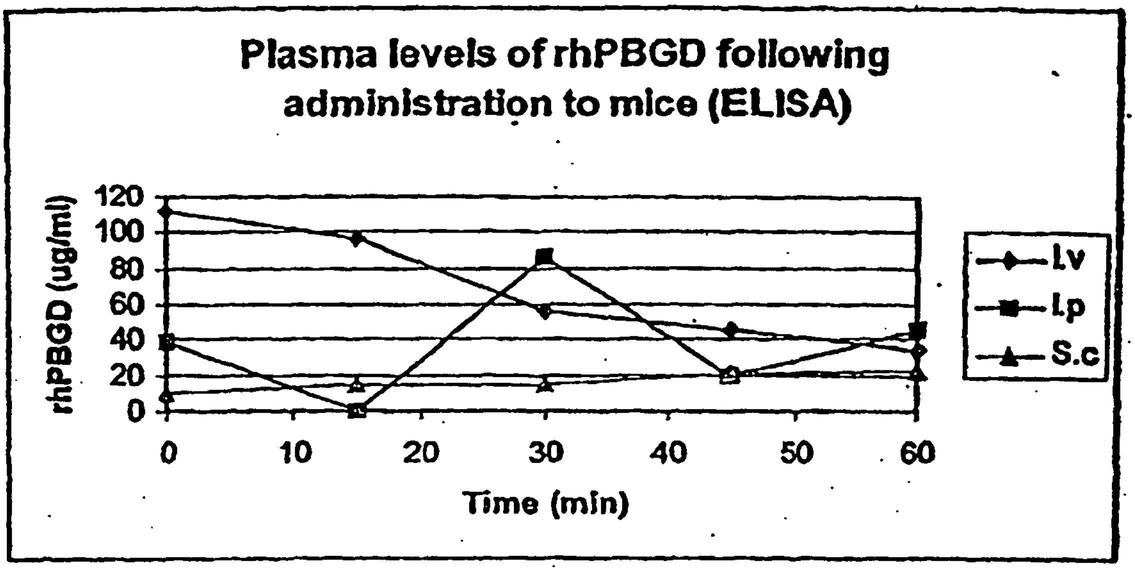


Figure 38: Plasma levels of rhPBGD following administration to mice.

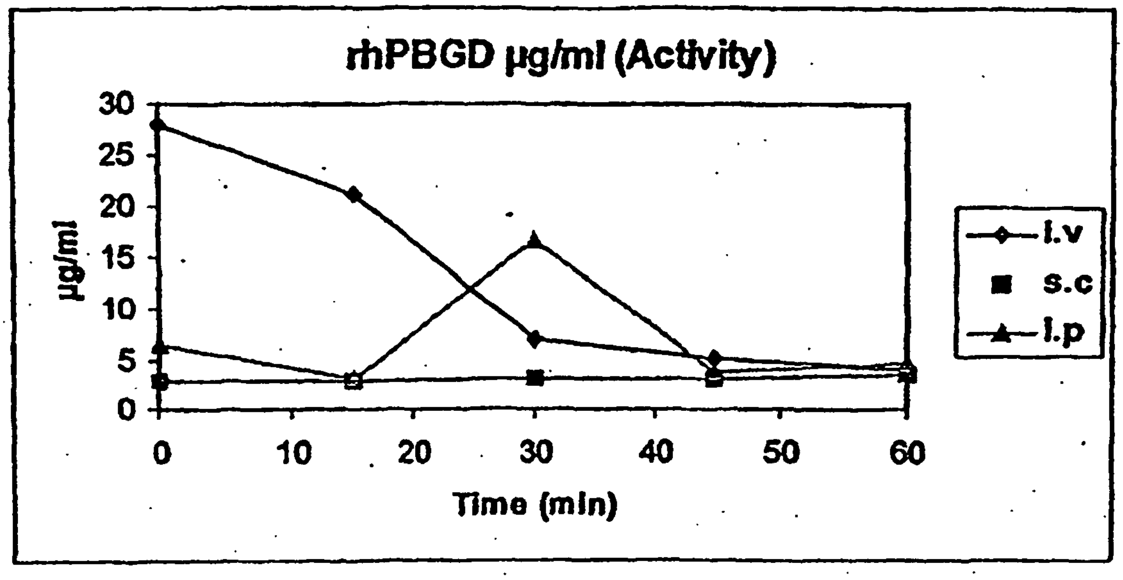


Figure 39: PBGD enzymatic activity in plasma following rhPBGD administration to mice

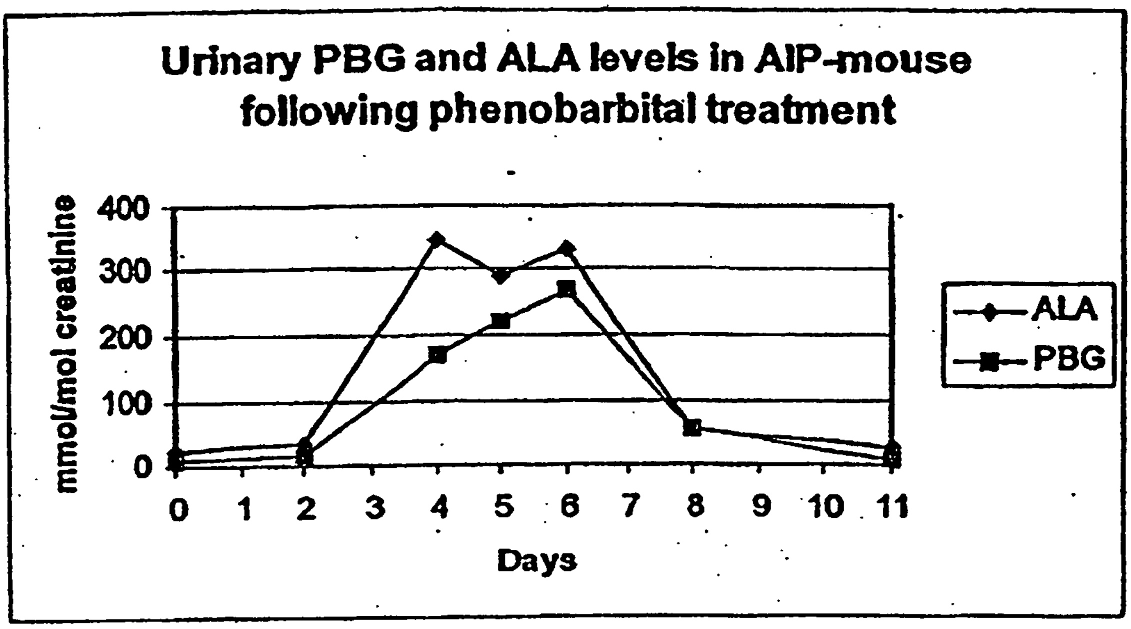


**Figure 40: Urinary content of PBG and ALA in AIP-mouse treated with phenobarbital.**

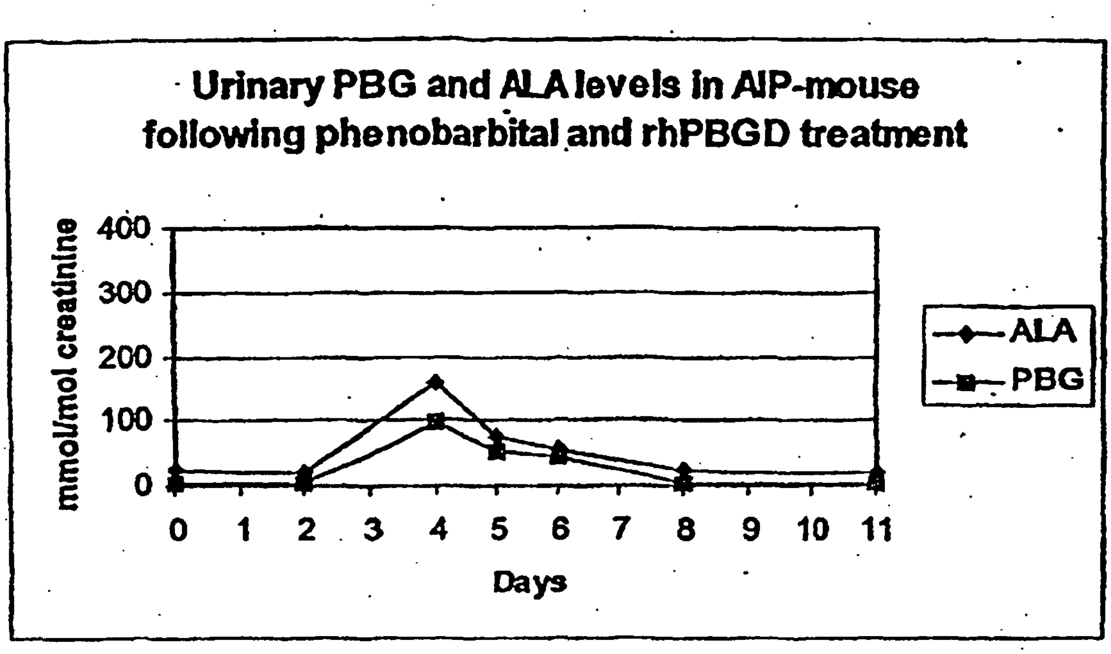


**Figure 41: Urinary content of PBG and ALA in AIP-mouse treated with phenobarbital and rhPBGD. Mice were treated with an increasing dose of phenobarbital for 4 days (day 0-4,**

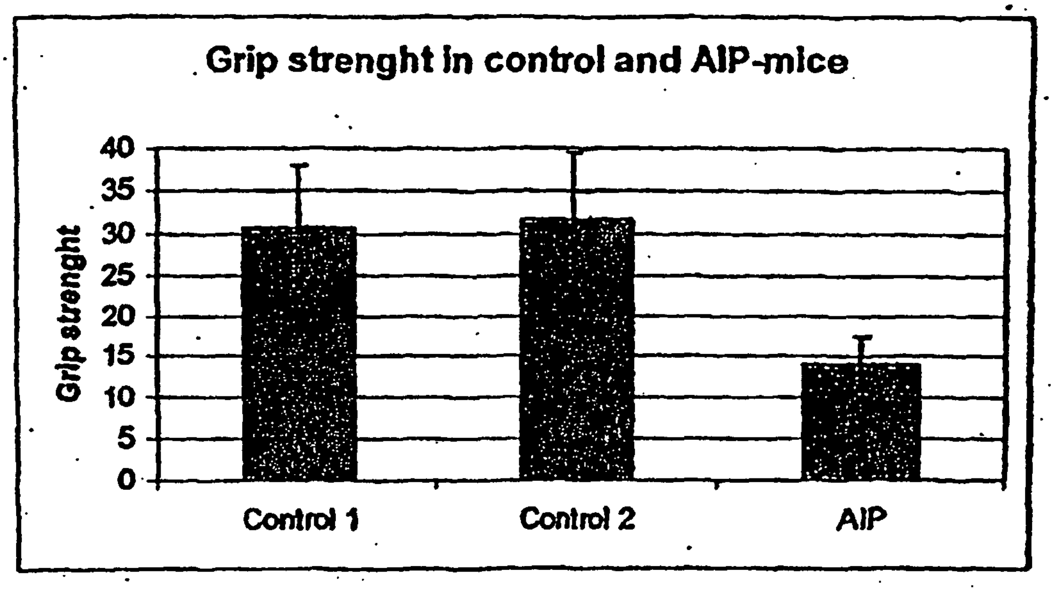


Figure 42: Grip strength analysis in control and AIP-mice. Grip strength were determined using a grip strength meter (Ugo Basile) in heterozygous control animals (control 1, n=5), in wild type controls (control 2, n=5) and in AIP-transgenic mice (AIP, n=5).

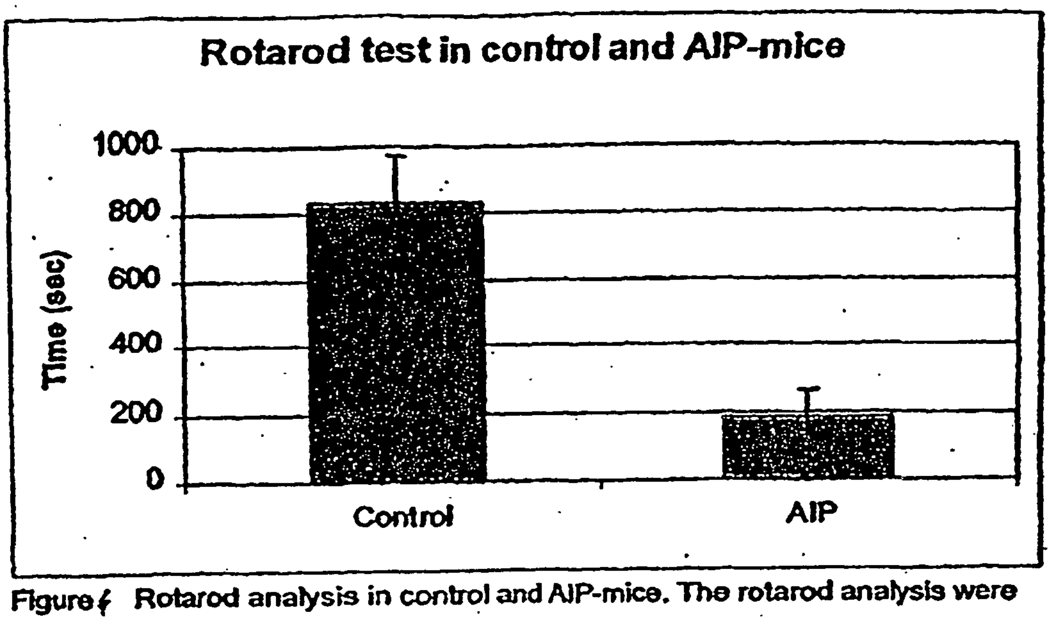


Figure 4 Rotarod analysis in control and AIP-mice. The rotarod analysis were determined using a rotarod treadmill (Ugo Basile) in wild type controls (control, n=5) and in AIP-transgenic mice (AIP, n=7).

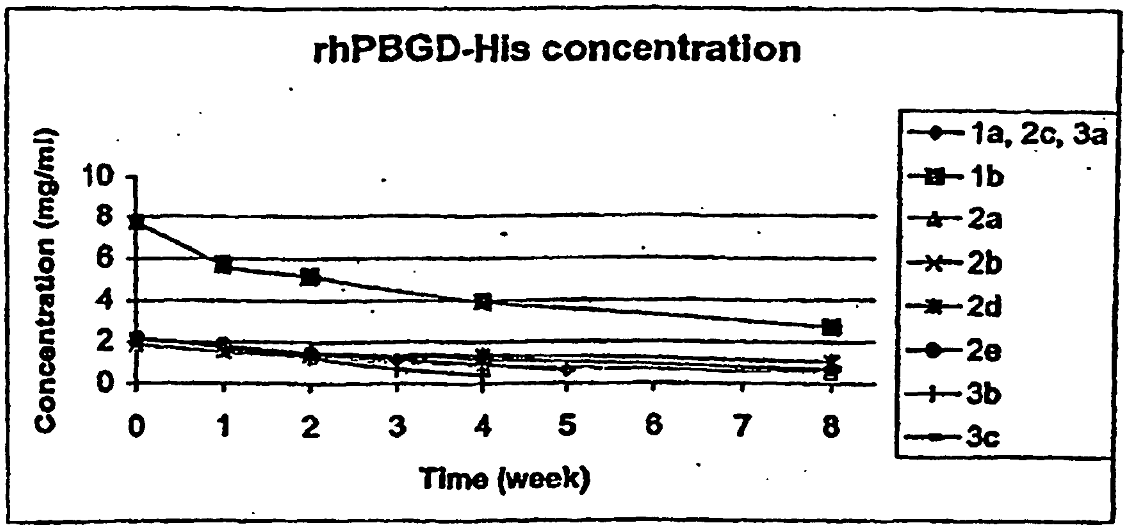


**Figure 44.** Enzyme concentration over 8 weeks at 40°C measured by HPLC. A decrease from 2 mg/ml to 0,5 mg/ml and 8 mg/ml to 2,5 was detected.

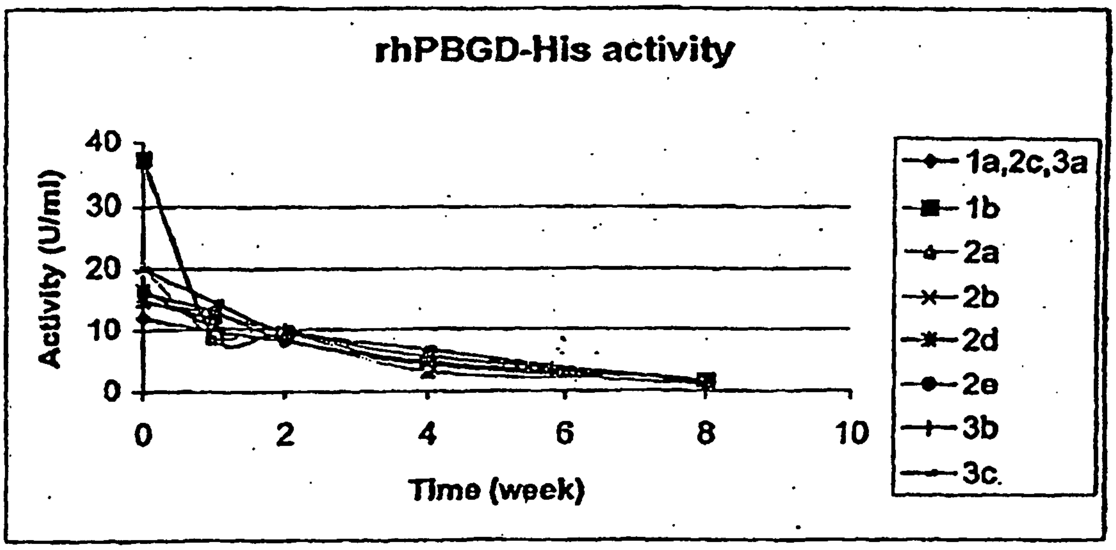


**Figure 45.** The enzyme activity measured over 8 weeks at 40°C. A significant decrease over the first week was seen for the high concentration sample, 1b. After two weeks the decrease rate was the same for all samples.

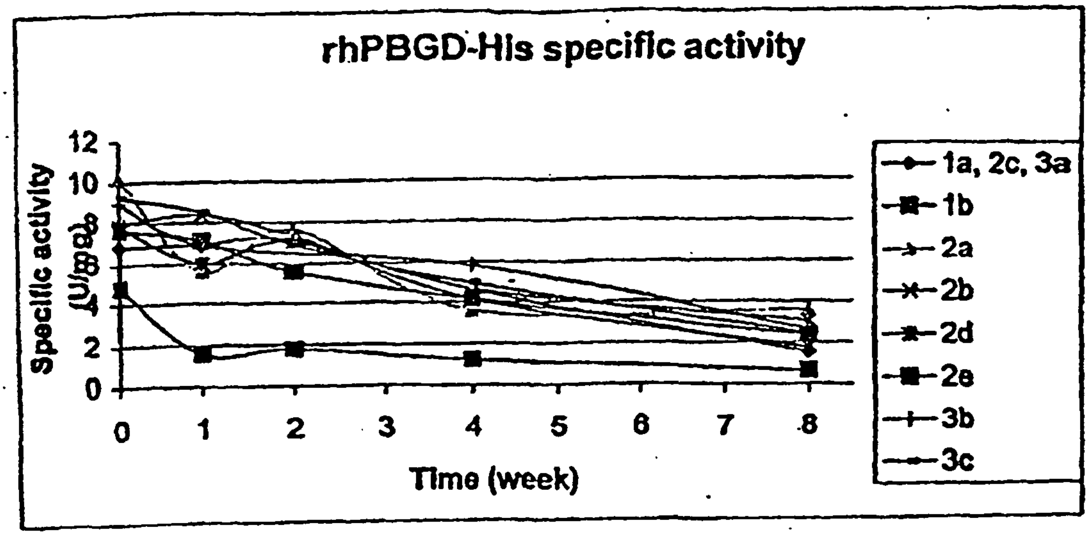


Figure 46. Enzyme specific activity measured during 8 weeks at 40°C. The activity was measured using the enzyme activity assay and the protein concentration was measured using HPLC.

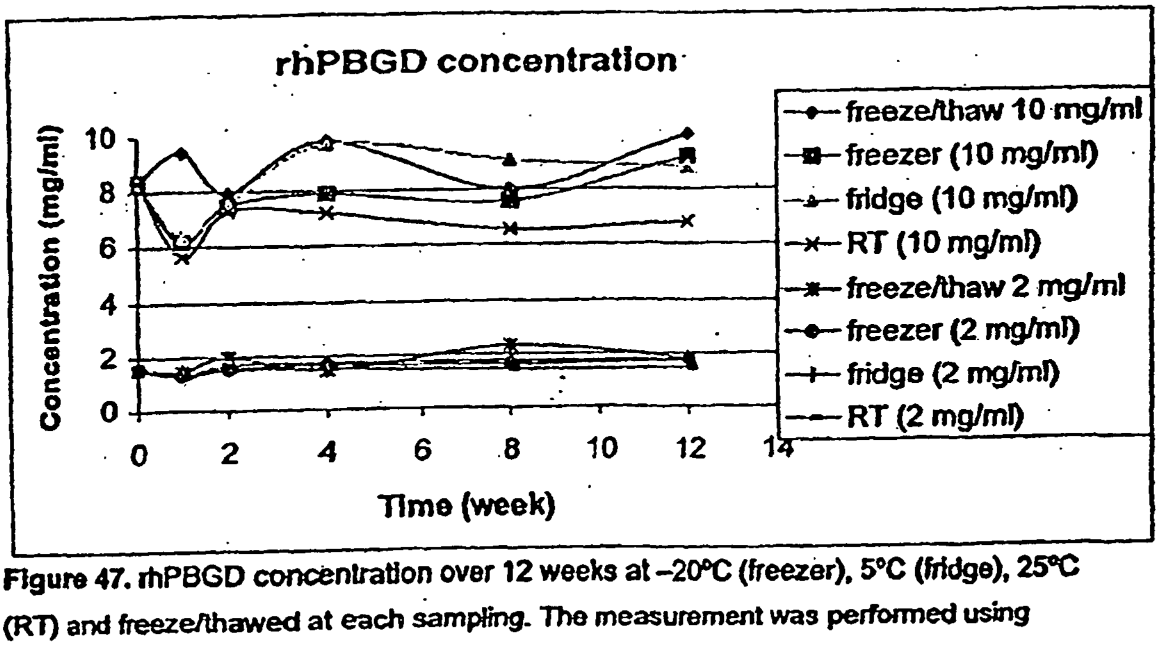


Figure 47. rhPBGD concentration over 12 weeks at -20°C (freezer), 5°C (fridge), 25°C (RT) and freeze/thawed at each sampling. The measurement was performed using HPLC.

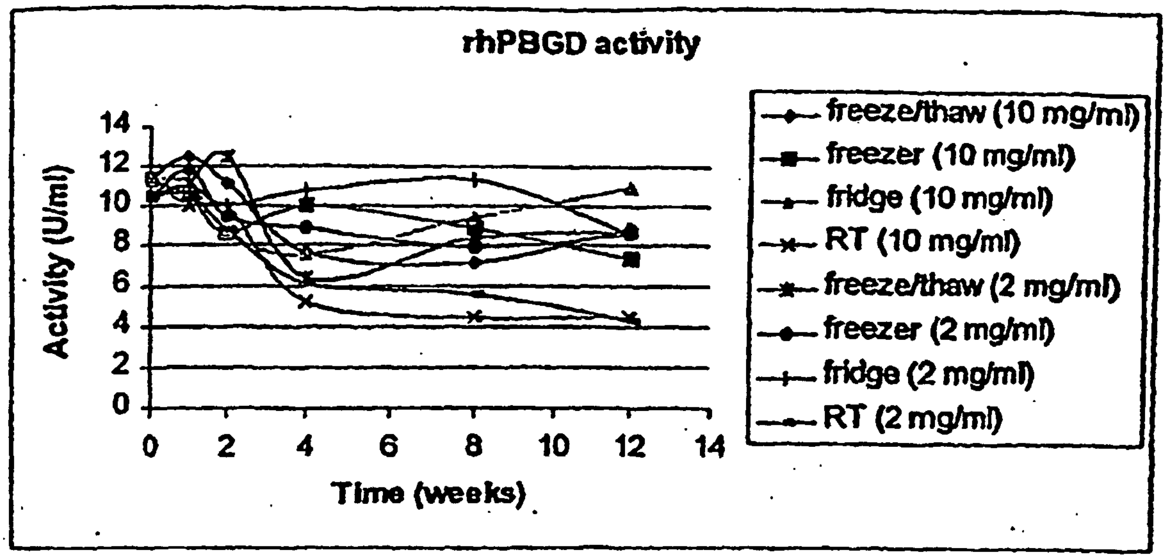


**Figure 48.** rhPBGD activity over 12 weeks at -20°C (freezer), 5°C (fridge), 25°C (RT) and freeze/thawed at each sampling.

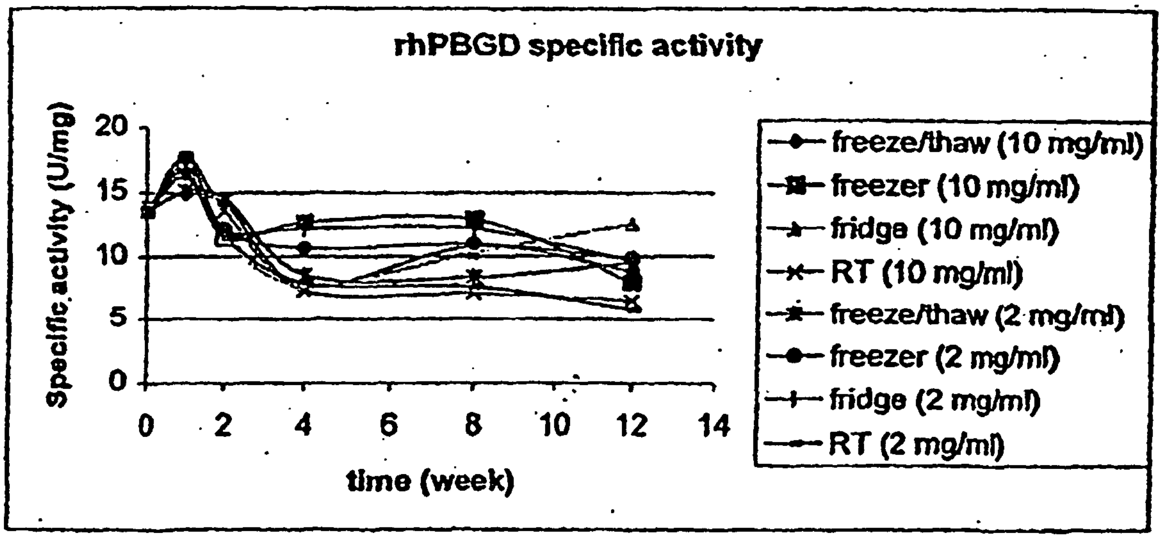


**Figure 49.** rhPBGD specific activity over 12 weeks at -20°C (freezer), 5°C (fridge), 25°C (RT) and freeze/thawed at each sampling. Measurements were performed using enzyme activity assay and HPLC.

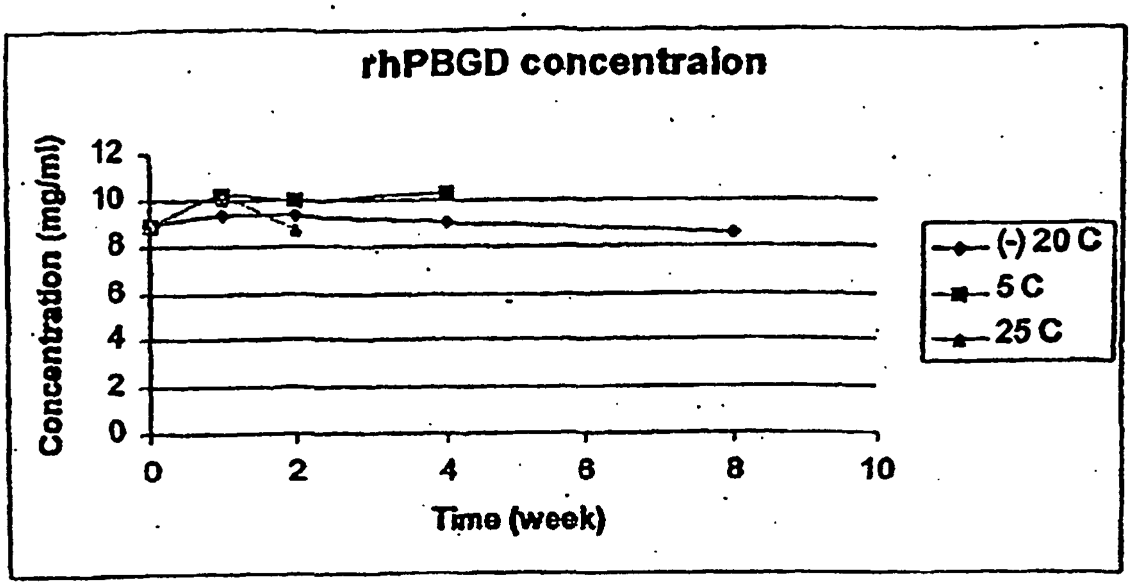


**Figure 50.** rhPBGD concentration measured over 8 weeks using BCA.

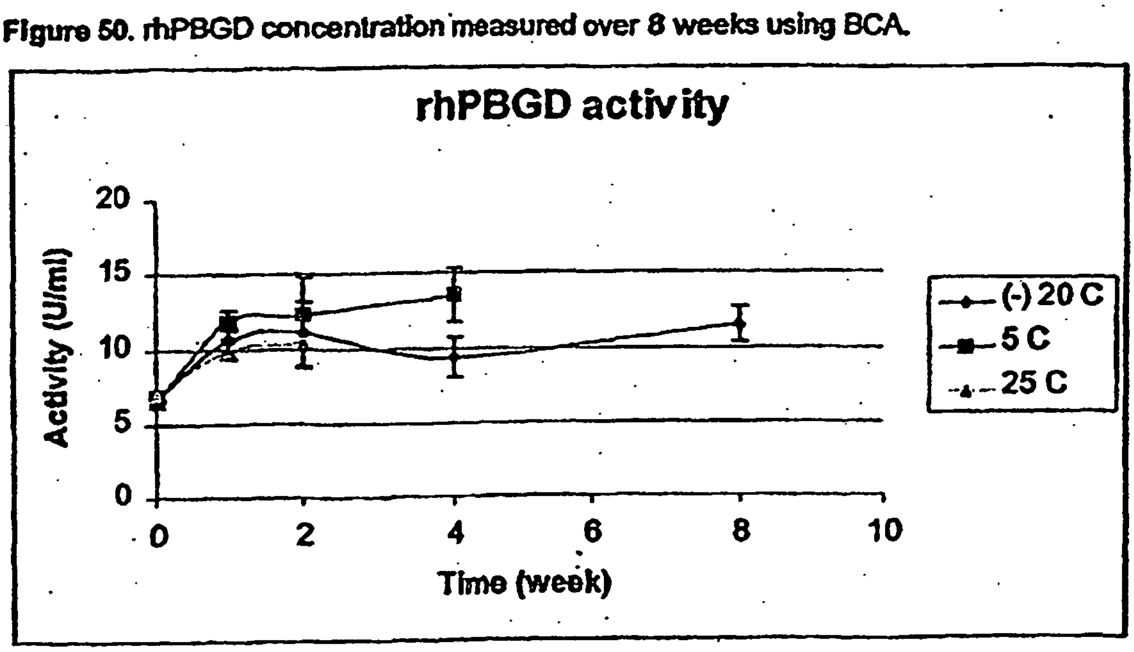


**Figure 51.** The rhPBGD activity measured over 8 weeks. The stability study has been performed under nitrogen at -20°C ± 5°C, 5°C ± 3°C and at 25°C ± 2°C.

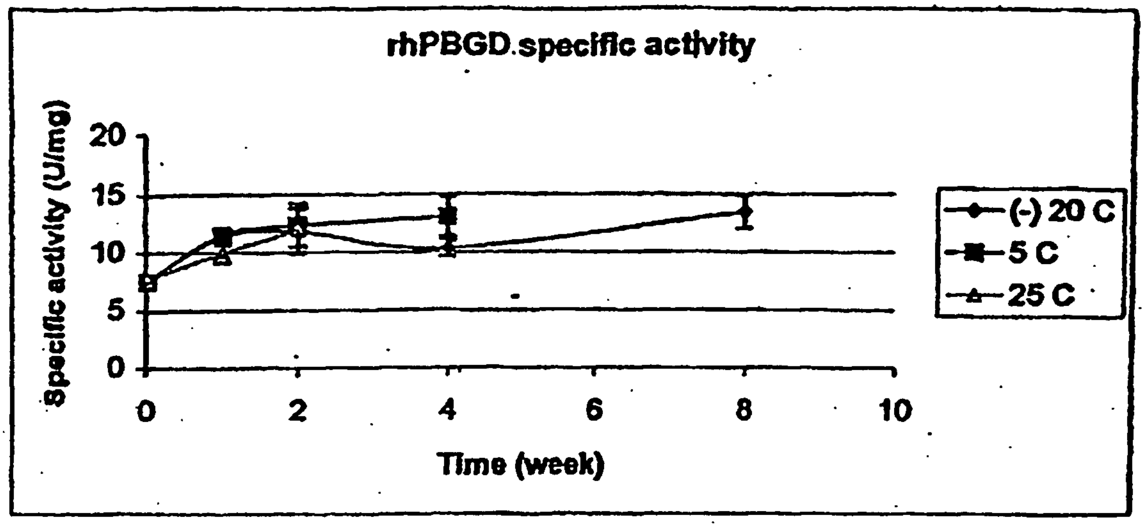


Figure 52. The specific rhPBGD activity measured using the enzyme activity assay and BCA protein concentration assay. The stability study has been performed under nitrogen at −20°C ± 5°C, 5°C ± 3°C and at 25°C ± 2°C.